(19) Europäisches Patentamt
European Patent Office
Office européen des brevets

(11) **EP 3 090 265 B1**

(12) **EUROPEAN PATENT SPECIFICATION**

(45) Date of publication and mention of the grant of the patent:
**08.04.2020 Bulletin 2020/15**

(21) Application number: **14877549.7**

(22) Date of filing: **29.12.2014**

(51) Int Cl.:
***C12Q 1/6886*** *(2018.01)*

(86) International application number:
**PCT/US2014/072610**

(87) International publication number:
**WO 2015/103166 (09.07.2015 Gazette 2015/27)**

# (54) PROSTATE CANCER GENE PROFILES AND METHODS OF USING THE SAME

PROSTATAKREBSGENPROFILE UND VERFAHREN ZUR VERWENDUNG DAVON

PROFILS DE GÈNES DU CANCER DE LA PROSTATE ET PROCÉDÉS DE LEUR UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2013 US 201361921739 P**

(43) Date of publication of application:
**09.11.2016 Bulletin 2016/45**

(73) Proprietors:
- **The Henry M. Jackson Foundation for the Advancement of Military Medicine, Inc.**
  **Bethesda, MD 20817 (US)**
- **Genomatix Software GMBH**
  **80335 München (DE)**

(72) Inventors:
- **SRIVASTAVA, Shiv, K.**
  **Potomac, MD 20854 (US)**
- **DOBI, Albert**
  **Rockville, MD 20850 (US)**
- **PETROVICS, Gyorgy**
  **Bethesda, MD 20817 (US)**
- **WERNER, Thomas**
  **80999 Munich (DE)**
- **SEIFERT, Martin**
  **82396 Fischen (Pähl) (DE)**
- **SCHERF, Matthias**
  **80335 Gröbenzell (DE)**

(74) Representative: **Dörries, Hans Ulrich**
**df-mp Dörries Frank-Molnia & Pohlman**
**Patentanwälte Rechtsanwälte PartG mbB**
**Theatinerstrasse 16**
**80333 München (DE)**

(56) References cited:

| | |
|---|---|
| **WO-A1-2010/136716** | **WO-A1-2012/024543** |
| **WO-A1-2012/152800** | **WO-A2-2006/056766** |
| **WO-A2-2008/058018** | **WO-A2-2012/031008** |
| **WO-A2-2013/033609** | **US-A1- 2009 170 075** |
| **US-A1- 2013 323 734** | |

- **KATHRYN L. PENNEY ET AL: "mRNA Expression Signature of Gleason Grade Predicts Lethal Prostate Cancer", JOURNAL OF CLINICAL ONCOLOGY, vol. 29, no. 17, 10 June 2011 (2011-06-10) , pages 2391-2396, XP055390709, US ISSN: 0732-183X, DOI: 10.1200/JCO.2010.32.6421**
- **E. K. MARKERT ET AL: "Molecular classification of prostate cancer using curated expression signatures", PROCEEDINGS NATIONAL ACADEMY OF SCIENCES PNAS, vol. 108, no. 52, 28 November 2011 (2011-11-28), pages 21276-21281, XP055390712, US ISSN: 0027-8424, DOI: 10.1073/pnas.1117029108**
- **LAIA AGELL ET AL: "A 12-Gene Expression Signature Is Associated with Aggressive Histological in Prostate Cancer", AMERICAN JOURNAL OF PATHOLOGY., vol. 181, no. 5, 1 November 2012 (2012-11-01), pages 1585-1594, XP055254671, US ISSN: 0002-9440, DOI: 10.1016/j.ajpath.2012.08.005**
- **BIBIKOVA ET AL: "Expression signatures that correlated with Gleason score and relapse in prostate cancer", GENOMICS, ACADEMIC PRESS, SAN DIEGO, US, vol. 89, no. 6, 12 May 2007 (2007-05-12), pages 666-672, XP022077660, ISSN: 0888-7543, DOI: 10.1016/J.YGENO.2007.02.005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- R RENEE REAMS ET AL: "Microarray comparison of prostate tumor gene expression in African-American and Caucasian American males: a pilot project study", INFECTIOUS AGENTS AND CANCER, vol. 4, no. Suppl 1, 1 January 2009 (2009-01-01), page S3, XP055423484, Lo ISSN: 1750-9378, DOI: 10.1186/1750-9378-4-S1-S3
- ROSE AMY E ET AL: "Copy number and gene expression differences between African American and Caucasian American prostate cancer", JOURNAL OF TRANSLATIONAL MEDICINE, BIOMED CENTRAL, vol. 8, no. 1, 22 July 2010 (2010-07-22), page 70, XP021078894, ISSN: 1479-5876, DOI: 10.1186/1479-5876-8-70
- CHAPMAN: "COL10A1 expression in elevated in diverse solid tumor types and is associated with tumor vasculature", FUTURE ONCOLOGY, FUTURE MEDICINE LTD., LONDON, GB, vol. 8, no. 8, 1 August 2012 (2012-08-01), pages 1031-1040, XP008172245, ISSN: 1479-6694, DOI: 10.2217/FON.12.79
- DELILA GASI TANDEFELT ET AL: "A 36-gene Signature Predicts Clinical Progression in a Subgroup of ERG-positive Prostate Cancers", EUROPEAN UROLOGY, vol. 64, no. 6, 1 December 2013 (2013-12-01), pages 941-950, XP055423207, AMSTERDAM, NL ISSN: 0302-2838, DOI: 10.1016/j.eururo.2013.02.039
- J. C. CHEVILLE ET AL: "Gene Panel Model Predictive of Outcome in Men at High-Risk of Systemic Progression and Death From Prostate Cancer After Radical Retropubic Prostatectomy", JOURNAL OF CLINICAL ONCOLOGY, vol. 26, no. 24, 20 August 2008 (2008-08-20), pages 3930-3936, XP055125419, ISSN: 0732-183X, DOI: 10.1200/JCO.2007.15.6752

## Description

### CROSS REFERENCE TO RELATED APPLICATIONS

This application claims the benefit of, and relies on the filing date of, U.S. provisional patent application number 61/921,739, filed 30 December 2013, the entire disclosure of which is incorporated herein by reference.

### GOVERNMENT INTEREST

This invention was made in part with Government support. The Government has certain rights in the invention.

### SEQUENCE LISTING

The instant application contains a Sequence Listing which has been submitted electronically in ASCII format. Said ASCII copy, created on December 29, 2014, is named HMJ-145-PCT_SL.txt and is 344,410 bytes in size.

### BACKGROUND

In 2013 an estimated 238,590 men will be diagnosed with carcinoma of the prostate (CaP) and an estimated 29,720 men will die from the disease [1]. This malignancy is the second leading cause of cancer-related death in men in the United States. In addition, African American (AA) men have the highest incidence and mortality from CaP compared with other races [1]. The racial disparity exists from presentation and diagnosis through treatment, survival, and quality of life [2]. Researchers have suggested that socio-economic status (SES) contributes significantly to these disparities including CaP-specific mortality [3]. As well, there is evidence that reduced access to care is associated with poor CaP outcomes, which is more prevalent among AA men than Caucasian American (CA) men [4].

However, there are populations in which AA men have similar outcomes to CA men. Sridhar and colleagues [5] published a meta-analysis in which they concluded that when SES is accounted for, there are no differences in the overall and CaP-specific survival between AA and CA men. Similarly, the military and veteran populations (systems of equal access and screening) do not observe differences in survival across race [6], and differences in pathologic stage at diagnosis narrowed by the early 2000s in a veterans' cohort [7]. Of note, both of these studies showed that AA men were more likely to have higher Gleason scores and PSA levels than CA men [6, 7].

While socio-economic factors may contribute to CaP outcomes, they do not seem to account for all variables associated with the diagnosis and disease risk. Several studies support that AA men have a higher incidence of CaP compared to CA men [1, 8, 9]. Studies also show that AA men have a significantly higher PSA at diagnosis, higher grade disease on biopsy, greater tumor volume for each stage, and a shorter PSA doubling time before radical prostatectomy [10-12]. Biological differences between prostate cancers from CA and AA men have been noted in the tumor microenvironment with regard to stress and inflammatory responses [13]. Although controversy remains over the role of biological differences, observed differences in incidence and disease aggressiveness at presentation indicate a potential role for different pathways of prostate carcinogenesis between AA and CA men.

Over the past decade, much research has focused on alterations of cancer genes and their effects in CaP [14-16]. Variations in prevalence across ethnicity and race have been noted in the TMPRSS2/ERG gene fusion that is overexpressed in CaP and is the most common known oncogene in CaP [17, 18]. Accumulating data suggest that there are differences of *ERG* oncogenic alterations across ethnicities [17, 19-21]. Significantly greater *ERG* expression in CA men compared to AA men was noted in initial papers describing *ERG* overexpression and *ERG* splice variants [17, 21]. The difference is even more pronounced between CA and AA (50% versus 16%) in patients with high Gleason grade (8-10) tumors. Thus, ERG is a major somatic gene alteration between these ethnic groups. Yet beyond TMPRSS2/ERG, little is known regarding the genetic basis for the CaP disparity between AA and CA men remains unknown [24]. A pilot project study relating to microarray comparison of prostate tumor gene expression in African-American and Caucasian American males has been done by Reams et al. [28].

Therefore, new biomarkers and therapeutic markers that are specific for distinct ethnic populations and provide more accurate diagnostic and/or prognostic potential are needed. As such, separate gene expression profiles for patients of African and Caucasian descent can be used to diagnose or prognose CaP in distinct ethnic populations and offer more informed treatment options based on these ethnic-specific gene expression signatures.

### SUMMARY

The present disclosure provides gene expression profiles that are associated with prostate cancer and methods of using the same. The gene expression profiles can be used to detect prostate cancer cells in a sample or to predict the likelihood of a patient developing prostate cancer. The gene expression profiles can also be used to evaluate the severity or stage of prostate cancer or to assess the effectiveness of a therapy or monitor the progression or regression of prostate cancer following therapy (e.g., disease-free recurrence following surgery). The gene expression profiles can be measured at either the nucleic acid or protein level. In one aspect, the gene expression profile is specific for patients of African descent. In another aspect, the gene expression profile is specific for patients of Caucasian descent.

In one aspect, the present invention provides a method of collecting data for diagnosing prostate cancer, the method comprising:

detecting expression of a plurality of genes in a biological sample obtained from a patient, wherein the patient is of African descent and wherein the plurality of genes is selected because the patient is of African descent and comprises at least three of the following genes: COL10A1, HOXC4, ESPL1, MMP9, ABCA13, PCDHGA1, and AGSK1, wherein the at least three genes comprise COL10A1.

In one embodiment, the plurality of genes comprises COL10A1, HOXC4, ESPL1, MMP9, ABCA13, PCDHGA1, and AGSK1.

In another embodiment, the method further comprises a step of diagnosing prostate cancer using the expression data obtained.

In another embodiment, overexpression of the at least three genes as compared to a control sample or a threshold value indicates the presence of prostate cancer in the biological sample or an increased risk of developing prostate cancer.

In one embodiment, the method may further comprise detecting expression of an ERG gene in the biological sample. In one embodiment, the biological sample is a tissue sample, a cell sample, a blood sample, a serum sample, or a urine sample. In one embodiment, the biological sample comprises prostate cells or nucleic acids or polypeptides isolated from prostate cells. In one embodiment, nucleic acid expression is detected. In one embodiment, polypeptide expression is detected.

In another aspect, the invention provides a kit for use in diagnosing prostate cancer, the kit comprising a plurality of probes for detecting at least three of the following genes: COL10A1, HOXC4, ESPL1, MMP9, ABCA13, PCDHGA1, and AGSK1, wherein the plurality of probes contains probes for detecting no more than 500, 250, 100, 50, 25, 15, 10, 9, 8, 7, 6, 5, 4, or 3 different genes, wherein the at least three genes comprise COL10A1.

In one embodiment, the plurality of probes is selected from a plurality of oligonucleotide probes, a plurality of antibodies, or a plurality of polypeptide probes. In one embodiment, the plurality of probes contains probes for detecting no more than 250, 100, 50, 25, 15, 10, or 5 different genes. In one embodiment, the plurality of probes are attached to the surface of an array, wherein the array optionally comprises no more than 500, 250, 100, 50, 25, 15, or 10 addressable elements. In one embodiment, the plurality of probes are labeled. In one embodiment, the plurality of probes further comprises a probe for detecting an ERG gene.

In another aspect, the invention provides a method of obtaining a gene expression profile in a biological sample, the method comprising:

incubating the aforementioned array with the biological sample, wherein the biological sample is obtained from a patient of African descent, and measuring the expression level of the at least three genes to obtain the gene expression profile.

In one embodiment, the biological sample is a tissue sample, a cell sample, a blood sample, a serum sample, or a urine sample. In one embodiment, the biological sample comprises nucleic acids or polypeptides isolated from prostate cells. In one embodiment, the measuring step comprises measuring nucleic acid expression levels. In one embodiment, the measuring step comprises measuring polypeptide expression levels. In one embodiment, the measuring step further comprises measuring the expression level of an ERG gene.

In yet another aspect, the invention provides a method of identifying a patient in need of prostate cancer treatment, wherein the patient is of African descent, the method comprising:

testing a biological sample from the patient for the overexpression of a plurality of genes, wherein the plurality of genes is selected because the patient is of African descent and comprises at least three of the following genes: COL10A1, HOXC4, ESPL1, MMP9, ABCA13, PCDHGA1, and AGSK1, wherein the at least three genes comprise COL10A1, and identifying the patient as in need of prostate cancer treatment if one or more of the at least three genes is overexpressed in the biological sample as compared to a control sample or a threshold value.

In one embodiment, the plurality of genes consists of no more than 100, 90, 80, 70, 60, 50, 40, 30, 25, 20, 15, 14, 13, 12, 11, 10, 9, 8, 7, 6, 5, 4, or 3 genes.

In one embodiment, the method of any of the above aspects is a computer-implemented method.

A further aspect described herein is directed to a gene expression profile that is associated with prostate cancer in a patient of African descent where the gene expression profile comprises a combination of the following genes: COL10A1, HOXC4, ESPL1, MMP9, ABCA13, PCDHGA1, and AGSK1.

Another aspect described herein is directed to a gene expression profile that is associated with prostate cancer in a patient of Caucasian descent where the gene expression profile comprises a combination of the following genes: PCA3, ALOX15, AMACR, CDH19, OR51E2/PSGR, F5, FZD8, and CLDN.

Yet another aspect described herein is directed to a gene expression profile that represents the top differentially expressed genes in prostate cancer in both ethnic groups and includes a combination of the following genes: DLX1, NKX2-3, CRISP3, PHGR1, THBS4, AMACR, GAP43, FFAR2, GCNT1, SIM2, STX19, KLB, APOF, LOC283177, and TRPM4. In certain embodiments, the gene expression profile comprises at least DLX1 and NKX2-3. In one embodiment, the combination includes at least DLX1 and NKX2-3.

These gene profiles can be used in a method of collecting data described or claimed herein for diagnosing prostate cancer, the method comprising measuring the expression of a representative number of genes in one of the disclosed gene profiles, where gene expression is measured in a sample obtained from a patient. The collected gene expression data can be used to predict whether a subject has prostate cancer or will develop prostate cancer or to predict the stage or severity of prostate cancer. The collected gene expression data can be also used to inform decisions about treating or monitoring a patient. Given the identification of these unique gene expression profiles, one of skill in the art can determine which of the identified genes to include in the gene profiling analysis. A representative number of genes may include all of the genes listed in a particular profile or some lesser number, for example, three or four or more of the genes. In certain embodiments, the method further comprises detecting expression of one or more other genes associated with prostate cancer, including, but not limited to ERG, PSA, and PCA3.

Another aspect is directed to kits for use in diagnosing prostate cancer. In one embodiment, the kit is designed for use in diagnosing prostate cancer in a patient of African descent and comprises a plurality of probes for detecting at least three of the following genes (or polypeptides encoded by the same): COL10A1, HOXC4, ESPL1, MMP9, ABCA13, PCDHGA1, and AGSK1. In certain embodiments, the plurality of probes is selected from a plurality of oligonucleotide probes, a plurality of antibodies, or a plurality of polypeptide probes. In other embodiments, the plurality of probes contains probes for no more than 500, 250, 100, 50, 25, 15, 10, 9, 8, 7, 6, 5, 4, 3, genes (or polypeptides). In one embodiment, the kit further comprises a probe for detecting expression of one or more other genes associated with prostate cancer, including, but not limited to ERG, PSA, and PCA3.

Another kit is designed for use in diagnosing prostate cancer in a patient of Caucasian descent and comprises a plurality of probes for detecting at least one (preferably, at least three) of the following genes (or polypeptides encoded by the same): PCA3, ALOX15, AMACR, CDH19, OR51E2/PSGR, F5, FZD8, and CLDN. The plurality of probes is selected from a plurality of oligonucleotide probes, a plurality of antibodies, or a plurality of polypeptide probes and contains probes for no more than 500, 100, 50, 25, 15, 10, 9, 8, 7, 6, 5, 4, 3, or 2 genes (or polypeptides). The kit may be used for detecting expression of one or more other genes associated with prostate cancer, including, but not limited to ERG, PSA, and PCA3.

Another kit for diagnosing prostate cancer comprises a plurality of probes for detecting at least one (preferably, at least four) of the following genes (or polypeptides encoded by the same): DLX1, NKX2-3, CRISP3, PHGR1, THBS4, AMACR, GAP43, FFAR2, GCNT1, SIM2, STX19, KLB, APOF, LOC283177, and TRPM4. The genes comprise DLX1 and/or NKX2-3. The plurality of probes is selected from a plurality of oligonucleotide probes, a plurality of antibodies, or a plurality of polypeptide probes and contains probes for no more than 500, 100, 50, 25, 15, 10, 9, 8, 7, 6, 5, 4, 3, or 2 genes (or polypeptides). The kit may be used for detecting expression of one or more other genes associated with prostate cancer, including, but not limited to ERG, PSA, and PCA3.

As described herein the disclosure provides an array for diagnosing prostate cancer. The array comprises (a) a substrate and (b) a plurality of probes immobilized on the substrate for detecting the expression of at least 3 of the following human genes: COL10A1, HOXC4, ESPL1, MMP9, ABCA13, PCDHGA1, and AGSK1, or the expression of at least 3 of the following human genes: PCA3, ALOX15, AMACR, CDH19, OR51E2/PSGR, F5, FZD8, and CLDN, or the expression of at least 4 of the following human genes: DLX1, NKX2-3, CRISP3, PHGR1, THBS4, AMACR, GAP43, FFAR2, GCNT1, SIM2, STX19, KLB, APOF, LOC283177, and TRPM4. The array may further comprise probes for detecting expression of one or more other genes associated with prostate cancer, including, but not limited to ERG, PSA, and PCA3.

The probes are preferably arranged on the substrate within addressable elements to facilitate detection. Preferably, the array comprises a limited number of addressable elements so as to distinguish the array from a more comprehensive array, such as a genomic array or the like. Thus, the array comprises 500 or fewer addressable elements, e.g. no more than 250, 100, 50, or 25 addressable elements. No more than 1000 polynucleotide probes are immobilized on the array. The disclosure provides methods of using the arrays described herein to detect gene expression in a biological sample. Using these arrays to detect gene expression can also be part of a method for detecting prostate cancer in a biological sample.

Further, the disclosure provides methods of using the gene expression profiles to identify a patient in need of prostate cancer treatment. In one embodiment, the patient is of African descent and the method comprises a) testing a biological sample from the patient for the overexpression of a plurality of genes, wherein the plurality of genes is selected because the patient is of African descent and comprises at least three of the following genes: COL10A1, HOXC4, ESPL1, MMP9, ABCA13, PCDHGA1, and AGSK1; and b) identifying the patient as in need of prostate cancer treatment if one or more of the COL10A1, HOXC4, ESPL1, MMP9, ABCA13, PCDHGA1, and AGSK1 genes is overexpressed in the biological sample as compared to a control sample or a threshold value. If the patient is of Caucasian descent and the method may comprise a) testing a biological sample from the patient for the overexpression of a plurality of genes, wherein the plurality of genes is selected because the patient is of Caucasian descent and comprises at least four of the following genes: PCA3, ALOX15, AMACR, CDH19, OR51E2/PSGR, F5, FZD8, and CLDN3; and b) identifying the patient as in need of prostate cancer treatment if one or more of the PCA3, ALOX15, AMACR, CDH19, OR51E2/PSGR, F5, FZD8, and CLDN3 genes is overexpressed in the biological sample as compared to a control sample or a threshold value. In certain embodiments, the method further comprise detecting expression of one or more other genes associated with prostate cancer, including, but not limited to ERG, PSA, and PCA3. The methods can also further comprise a step of treating the patient.

## BRIEF DESCRIPTION OF THE DRAWINGS

The accompanying drawings, which are incorporated in and constitute a part of this specification, illustrate and together with the written description, serve to explain certain principles of the antibodies and methods disclosed herein.

**Figure 1** shows hierarchical clustering analysis (HCA) of 14 tumor and 4 normal samples using average-linkage method. The HCA reveals a distinct cluster of normal patients (GP-04, GP-10, GP-09, and GP-06) and another distinct cluster of AA, ERG fusion negative patients (GP-02, GP-10, and GP-04). Clustering is based on the expression levels of the genes. All the groups are color coded.

**Figure 2A** is a heatmap with clustering of 14 tumor and 4 normal samples, with African-American patients on the left and CA on the right of the Heatmap. Genes presented in the Heatmap are the overlaps of over and under expressed genes (tumor vs. normal) for AA and CA patients.

**Figure 2B** provides expression values (log2) of top 3 over expressed genes in both tumor and normal samples from AA and CA patients.

**Figure 3A** is a heatmap showing genes that are consistently over expressed in AA patients and simultaneously under expressed or show no change in CA patients.

**Figure 3B** is a heatmap showing genes that are consistently over expressed in CA patients and simultaneously under expressed or show no change in AA patients.

**Figure 4** shows a schematic diagram of a system according to some embodiments of the invention. In particular, this figure illustrates various hardware, software, and other resources that may be used in implementations of computer system 106 according to disclosed systems and methods. In embodiments as shown, computer system 106 may include one or more processors 110 coupled to random access memory operating under control of or in conjunction with an operating system. The processor(s) 110 in embodiments may be included in one or more servers, clusters, or other computers or hardware resources, or may be implemented using cloud-based resources. The operating system may be, for example, a distribution of the Linux™ operating system, the Unix™ operating system, or other open-source or proprietary operating system or platform. Processor(s) 110 may communicate with data store 112, such as a database stored on a hard drive or drive array, to access or store program instructions other data.

Processor(s) 110 may further communicate via a network interface 108, which in turn may communicate via the one or more networks 104, such as the Internet or other public or private networks, such that a query or other request may be received from client 102, or other device or service. Additionally, processor(s) 110 may utilize network interface 108 to send information, instructions, workflows query partial workflows, or other data to a user via the one or more networks 104. Network interface 104 may include or be communicatively coupled to one or more servers. Client 102 may be, e.g., a personal computer coupled to the internet.

Processor(s) 110 may, in general, be programmed or configured to execute control logic and control operations to implement methods disclosed herein. Processors 110 may be further communicatively coupled (i.e., coupled by way of a communication channel) to co-processors 114. Co-processors 114 can be dedicated hardware and/or firmware components configured to execute the methods disclosed herein. Thus, the methods disclosed herein can be executed by processor 110 and/or co-processors 114.

Other configurations of computer system 106, associated network connections, and other hardware, software, and service resources are possible.

## DETAILED DESCRIPTION

Reference will now be made in detail to various exemplary embodiments, examples of which are illustrated in the accompanying drawings. It is to be understood that the following detailed description is provided to give the reader a fuller understanding of certain embodiments, features, and details of aspects of the invention.

## Definitions

In order that the present invention may be more readily understood, certain terms are first defined. Additional definitions are set forth throughout the detailed description.

The term **"of African descent"** refers to individuals who self-identify as being of African descent, including individuals who self-identify as being African-American, and individuals determined to have genetic markers correlated with African ancestry, also called Ancestry Informative Markers (AIM), such as the AIMs identified in Judith Kidd et al., Analyses of a set of 128 ancestry informative single-nucleotide polymorphisms in a global set of 119 population samples, Investigative Genetics, (2):1, 2011.

The term **"of Caucasian descent"** refers to individuals who self-identify as being of Caucasian descent, including individuals who self-identify as being Caucasian-American, and individuals determined to have genetic markers correlated with Caucasian (e.g., European, North African, or Asian (Western, Central or Southern) ancestry, also called Ancestry Informative Markers (AIM), such as the AIMs identified in Judith Kidd et al., Analyses of a set of 128 ancestry informative single-nucleotide polymorphisms in a global set of 119 population samples, Investigative Genetics, (2):1, 2011.

The term **"antibody"** refers to an immunoglobulin or antigen-binding fragment thereof, and encompasses any polypeptide comprising an antigen-binding fragment or an antigen-binding domain. The term includes but is not limited to polyclonal, monoclonal, monospecific, polyspecific, humanized, human, single-chain, chimeric, synthetic, recombinant, hybrid, mutated, grafted, and *in vitro* generated antibodies. Unless preceded by the word "intact", the term "antibody" includes antibody fragments such as Fab, $F(ab')_2$, Fv, scFv, Fd, dAb, and other antibody fragments that retain antigen-binding function. Unless otherwise specified, an antibody is not necessarily from any particular source, nor is it produced by any particular method.

The term **"detecting"** or **"detection"** means any of a variety of methods known in the art for determining the presence or amount of a nucleic acid or a protein. As used throughout the specification, the term "detecting" or "detection" includes either qualitative or quantitative detection.

The term **"gene expression profile"** refers to the expression levels of a plurality of genes in a sample. As is understood in the art, the expression level of a gene can be analyzed by measuring the expression of a nucleic acid (e.g., genomic DNA or mRNA) or a polypeptide that is encoded by the nucleic acid.

The term **"isolated,"** when used in the context of a polypeptide or nucleic acid refers to a polypeptide or nucleic acid that is substantially free of its natural environment and is thus distinguishable from a polypeptide or nucleic acid that might happen to occur naturally. For instance, an isolated polypeptide or nucleic acid is substantially free of cellular material or other polypeptides or nucleic acids from the cell or tissue source from which it was derived.

The terms **"polypeptide," "peptide,"** and **"protein"** are used interchangeably herein to refer to polymers of amino acids.

The term **"polypeptide probe"** as used herein refers to a labeled (e.g., isotopically labeled) polypeptide that can be used in a protein detection assay (e.g., mass spectrometry) to quantify a polypeptide of interest in a biological sample.

The term **"primer"** means a polynucleotide capable of binding to a region of a target nucleic acid, or its complement, and promoting nucleic acid amplification of the target nucleic acid. Generally, a primer will have a free 3' end that can be extended by a nucleic acid polymerase. Primers also generally include a base sequence capable of hybridizing via complementary base interactions either directly with at least one strand of the target nucleic acid or with a strand that is complementary to the target sequence. A primer may comprise target-specific sequences and optionally other sequences that are non-complementary to the target sequence. These non-complementary sequences may comprise, for example, a promoter sequence or a restriction endonuclease recognition site.

A **"variation"** or **"variant"** refers to an allele sequence that is different from the reference at as little as a single base or for a longer interval.

The term **"ERG"** or **"ERG gene"** refers to Ets-related gene (ERG), which has been assigned the unique Hugo Gene Nomenclature Committee (HGNC) identifier code: HGNC:3446, and includes ERG gene fusion products that are prevalent in prostate cancer, including TMPRSS2-ERG fusion products. Analyzing the expression of ERG or the ERG gene includes analyzing the expression of ERG gene fusion products that are associated with prostate cancer, such as TMPRSS2-ERG.

## Gene Expression Profiles in Prostate Cancer

Next generation sequencing techniques were used to identify new biomarkers and therapeutic targets for CaP. High quality genome sequence data and coverage obtained from histologically defined and precisely dissected primary CaP specimens (80-95% tumor, primary Gleason pattern 3) was compared between cohorts of 7 patients of Caucasian descent and 7 patients of African descent (28 samples total including matched controls from each patient) to evaluate the observed disparities of CaP incidence and mortality between the two ethnic groups. These data and analyses provide the first evaluation of prostate cancer genomes from CaP patients of African and Caucasian descent that have been matched for clinic-pathologic features.

The top differentially expressed genes in CaP in both ethnic groups include: DLX1, NKX2-3, CRISP3, PHGR1, THBS4, AMACR, GAP43, FFAR2, GCNT1, SIM2, STX19, KLB, APOF, LOC283177, and TRPM4. Thus, collecting expression data of at least 2, 3, 4, 5, 6, 7, 8, 9, or 10 of these genes from a biological sample provides a unique gene expression profile for use in diagnosing prostate cancer in a subject.

Also described herein is a method of collecting data for use in diagnosing or prognosing CaP, the method comprising detecting expression in a biological sample of at least 2, 3, 4, 5, 6, 7, 8, 9, 10, 11, 12, 13, 14, or 15 of the following genes: DLX1, NKX2-3, CRISP3, PHGR1, THBS4, AMACR, GAP43, FFAR2, GCNT1, SIM2, STX19, KLB, APOF, LOC283177, and TRPM4. The method may optionally include an additional step of obtaining the biological sample from a subject. The method may optionally include an additional step of diagnosing or prognosing CaP using the collected gene expression data. Overexpression of at least 2, 3, 4, 5, 6, 7, 8, 9, 10, 11, 12, 1 3, 14, or 15 of the following genes: DLX1, NKX2-3, CRISP3, PHGR1, THBS4, AMACR, GAP43, FFAR2, GCNT1, SIM2, STX19, KLB, APOF, LOC283177, and TRPM4, as compared to a control sample or threshold value indicates the presence of CaP in the biological sample or an increased likelihood of developing CaP. The methods of collecting data or diagnosing and/or prognosing CaP may further comprise detecting expression of other genes associated with prostate cancer, including, but not limited to ERG, PSA, and PCA3. The methods comprise detecting expression of no more than 100, 90, 80, 70, 60, 50, 40, 30, 25, 20, 15, 14, 13, 12, 11, 10, 9, 8, 7, 6, 5, 4, 3, or 2 genes.

As described herein the methods comprise detecting expression of DLX1, NKX2-3, DLX1 and NKX2-3, PHGR1, THBS4, GAP43, FFAR2, GCNT1, SIM2, STX19, KLB, APOF, LOC283177, or TRPM4 and one or more of the other genes listed in Table 1.

The nucleic acid and amino acid sequences for human DLX1, NKX2-3, CRISP3, PHGR1, THBS4, AMACR, GAP43, FFAR2, GCNT1, SIM2, STX19, KLB, APOF, LOC283177, and TRPM4 are known. The unique identifier code assigned by Hugo Gene Nomenclature Committee (HGNC) and Entrez Gene for these genes and the accession number of a representative sequence are provided in Table 1.

**Table 1**

| Gene | HGNC ID | Entrez Gene ID | Accession No. |
|---|---|---|---|
| DLX1 | 2914 | 1745 | NM_178120.4, GI: 84043957 |
| NKX2-3 | 7836 | 159296 | NM_145285.2 GI: 148746210 |
| CRISP3 | 16904 | 10321 | NM_006061.2 GI:300244559 |
| PHGR1 | 37226 | 644844 | NM _001145643.1 GI:224548949 |
| THBS4 | 11788 | 7060 | NM_003248.4 GI:291167798 |
| AMACR | 451 | 23600 | NM_014324.5 GI:266456114 |
| GAP43 | 4140 | 2596 | AK091466.1 GI:21749841 |
| FFAR2 | 4501 | 2867 | NM_005306.2 GI:227430361 |
| GCNTI | 4203 | 2650 | NM_001097634.1 GI:148277030 |
| SIM2 | 10833 | 6493 | NM_005069.3 GI:194239685 |
| STX19 | 19300 | 415117 | NM_001001850.2 GI:344313159 |
| KLB | 15527 | 152831 | NM_175737.3 GI:198041706 |
| APOF | 615 | 319 | BC026257.1 GI:20072209 |
| LOC283177 | N/A | 283177 | AK095081.1 GI:21754271 |
| TRPM4 | 17993 | 54795 | NM_017636.3 GI:304766649 |

The following genes were identified as being over expressed in prostate tumors of patients of Caucasian descent as compared to patients of African descent: PCA3, ALOX15, AMACR, CDH19, OR51E2/PSGR, F5, FZD8, and CLDN3. Thus, obtaining expression data of at least 1, 2, 3, 4, 5, 6, 7, or 8 of these genes provides a unique gene expression profile for use in diagnosing or prognosing prostate cancer in patients of Caucasian descent.

Also described herein is a method of collecting data for use in diagnosing or prognosing CaP in a patient of Caucasian descent, the method comprising detecting expression in a biological sample of at least 1, 2, 3, 4, 5, 6, 7, or 8 of the following genes: PCA3, ALOX15, AMACR, CDH19, OR51E2/PSGR, F5, FZD8, and CLDN3, wherein the biological sample was obtained from the patient of Caucasian descent. The method may optionally include an additional step of obtaining the biological sample from the patient of Caucasian descent. The method may optionally include an additional step of diagnosing or prognosing CaP using the collected gene expression data. In methods of diagnosing or prognosing CaP, overexpression of at least 1, 2, 3, 4, 5, 6, 7, or 8 of the following genes: PCA3, ALOX15, AMACR, CDH19, OR51E2/PSGR, F5, FZD8, and CLDN3, as compared to a control sample or threshold value indicates the presence of CaP in the biological sample or an increased risk of developing CaP. The methods of collecting data for diagnosing CaP may further comprise detecting expression of other genes associated with prostate cancer, including, but not limited to ERG, PSA, and PCA3. In certain embodiments, the methods comprise detecting expression of no more than 100, 90, 80, 70, 60, 50, 40, 30, 25, 20, 15, 14, 13, 12, 11, 10, 9, 8, 7, 6, 5, 4, or 3 genes.

As described herein, the methods may comprise detecting expression of ALOX15 and one or more of PCA3, AMACR, CDH19, OR51E2/PSGR, F5, FZD8, and CLDN3. Further, the methods may comprise detecting expression of CDH19 and one or more of PCA3, AMACR, ALOX15, OR51E2/PSGR, F5, FZD8, and CLDN3. As described herein, the methods may comprise detecting expression of F5 and one or more of PCA3, AMACR, ALOX15, OR51E2/PSGR, CDH19, FZD8, and CLDN3. As described herein, the methods may comprise detecting expression of FZD8 and one or more of PCA3, AMACR, ALOX15, OR51E2/PSGR, CDH19, F5, and CLDN3. As described herein, the methods may comprise detecting expression of CLDN3 and one or more of PCA3, AMACR, ALOX15, OR51E2/PSGR, CDH19, F5, and FZD8. As described herein, the methods may comprise detecting expression of PCA3 and AMACR and one or more of ALOX15, CDH19, F5, FZD8, and CLDN3.

The unique identifier code assigned by HGNC and Entrez Gene for these genes that are more frequently overexpressed in patients of Caucasian descent and the accession number of a representative sequence are provided in Table 2.

**Table 2**

| Gene | HGNC ID | Entrez Gene ID | NCBI Reference |
|---|---|---|---|
| PCA3 | 8637 | 50652 | AF103907.1 GI:6165973 |
| ALOX15 | 433 | 246 | NM_001140.3 GI:40316936 |
| AMACR | 451 | 23600 | NM_014324.5 GI:266456114 |
| CDH19 | 1758 | 28513 | NM_021153.3 GI:402534572 |
| OR51E2/PSGR | 15195 | 81285 | AY033942.1 GI:16943640 |
| F5 | 3542 | 2153 | NM_000130.4 GI:119395710 |
| FZD8 | 4046 | 8325 | AB043703.1 GI:13623798 |
| CLDN3 | 2045 | 1365 | NM_001306.3 GI:171541813 |

The following genes were identified as being over expressed in prostate tumors of patients of African ancestry as compared to patients of Caucasian descent: COL10A1, HOXC4, ESPL1, MMP9, ABCA13, PCDHGA1, and AGSK1. Thus, obtaining expression data of at least 1, 2, 3, 4, 5, 6, or 7 of these genes provides a unique gene expression profile for use in diagnosing or prognosing prostate cancer in patients of African descent.

Certain embodiments are directed to a method of collecting data for use in diagnosing CaP in a patient of African descent, the method comprising detecting expression in a biological sample of at least 1, 2, 3, 4, 5, 6, or 7 of the following genes: COL10A1, HOXC4, ESPL1, MMP9, ABCA13, PCDHGA1, and AGSK1, wherein the biological sample was obtained from the patient of African descent. The method may optionally include an additional step of obtaining the biological sample from the patient of African descent. The method may optionally include an additional step of diagnosing CaP using the collected gene expression data. In methods of diagnosing CaP, overexpression of at least 3, 4, 5, 6, or 7 of the following genes: COL10A1, HOXC4, ESPL1, MMP9, ABCA13, PCDHGA1, and AGSK1, as compared to a control sample or threshold value indicates the presence of CaP in the biological sample or an increased risk of developing CaP. The methods of collecting data or diagnosing CaP may further comprise detecting expression of other genes associated with prostate cancer, including, but not limited to ERG, PSA, and PCA3. In certain embodiments, the methods comprise detecting expression of no more than 100, 90, 80, 70, 60, 50, 40, 30, 25, 20, 15, 14, 13, 12, 11, 10, 9, 8, 7, 6, 5, 4, or 3, genes.

As described herein, the methods, the methods comprise detecting expression of COL10A1 and one or more of HOXC4, ESPL1, MMP9, ABCA13, PCDHGA1, and AGSK1. As described herein, the methods may comprise detecting expression of HOXC4 and one or more of COL10A1, ESPL1, MMP9, ABCA13, PCDHGA1, and AGSK1. As described herein, the methods may comprise detecting expression of ESPL1 and one or more of COL10A1, HOXC4, MMP9, ABCA13, PCDHGA1, and AGSK1. As described herein, the methods may comprise detecting expression of MMP9 and one or more of COL10A1, HOXC4, ESPL1, ABCA13, PCDHGA1, and AGSK1. As described herein, the methods may comprise detecting expression of ABCA13 and one or more of COL10A1, HOXC4, ESPL1, MMP9, PCDHGA1, and AGSK1. As described herein, the methods may comprise detecting expression of PCDHGA1 and one or more of COL10A1, HOXC4, ESPL1, MMP9, ABCA13, and AGSK1. As described herein, the methods may comprise detecting expression of AGSK1 and one or more of COL10A1, HOXC4, ESPL1, MMP9, ABCA13, and PCDHGA1.

The unique identifier codes assigned by HGNC and Entrez Gene for these genes that are more frequently overexpressed in patients of African descent and the accession number of a representative sequence are provided in Table 3.

**Table 3**

| Gene | HGNC ID | Entrez Gene ID | NCBI Reference |
|---|---|---|---|
| COL10A1 | 2185 | 1300 | NM_000493.3 GI:98985802 |
| HOXC4 | 5126 | 3221 | NM_014620.5 GI:546232084 |
| ESPL1 | 16856 | 9700 | NM_012291.4 GI:134276942 |
| MMP9 | 7176 | 4318 | NM_004994.2 GI:74272286 |
| ABCA13 | 14638 | 154664 | AY204751.1 GI:30089663 |
| PCDHGA1 | 8696 | 56114 | NM_018912.2 GI:14196453 |
| AGSK1 | N/A | 80154 | NR_026811 GI:536293433<br>NR_033936.3 GI:536293365<br>NR_103496.2 GI:536293435 |

Additionally, whole genome sequence analysis of the 28 samples identified 65 gene mutations present with higher confidence in at least one of the 14 prostate tumors analyzed. The 65 gene mutations having the highest allele frequency in the prostate tumors analyzed occurred in the following genes: GLI1, IRX4, PAPPA, SPOP, TEX15, ZNF292, ANKRD11, FAT4, HECW2, KIAA1109, SHROOM3, SPOP, TTC36, ZNRF3, C17orf65, DEGS2, NEK3, KIAA0947, LSP1, NOX3, AKR1B1, ARHGAP12, ITGA4, PVRL4, RBM26, UCN3, CATSPERB, FCRL2, CACNA1E, CORO6, DMKN, EXT1, HEATR7B2, NDUFB5, GPR180, LRRC4, TPRA1, ZIM2, C12orf50, ELMO2, RBM26, SEC14L1, TNFSF11, C9orf125, CDC73, ITSN1, KCNK16, LRRC7, METTL6, MOSC1, RP11-50B3.2, STAB2, STARD13, PTPRT, RBPJ, UBA2, DIAPH3, IL18R1, LIPF, SLITRK5, TMEM132E, POT1, RB1CC1, TAOK1, and UNC5A. Of these 65 genes, only SPOP is known to have a mutation that is associated with prostate cancer. Thus, identifying one or more of these gene mutations in a sample can provide gene signatures useful for diagnosing or prognosing prostate cancer.

Also described herein is a method of collecting data for use in diagnosing or prognosing CaP, the method comprising detecting expression in a biological sample of one or more mutations in at least 2, 3, 4, 5, 6, 7, 8, 9, 10, 15, 20, 25, 30, 35, 40, 45, or 50 of the following genes: GLI1, IRX4, PAPPA, SPOP, TEX15, ZNF292, ANKRD11, FAT4, HECW2, KIAA1109, SHROOM3, SPOP, TTC36, ZNRF3, C17orf65, DEGS2, NEK3, KIAA0947, LSP1, NOX3, AKR1B1, ARHGAP12, ITGA4, PVRL4, RBM26, UCN3, CATSPERB, FCRL2, CACNA1E, CORO6, DMKN, EXT1, HEATR7B2, NDUFB5, GPR180, LRRC4, TPRA1, ZIM2, C12orf50, ELMO2, RBM26, SEC14L1, TNFSF11, C9orf125, CDC73, ITSN1, KCNK16, LRRC7, METTL6, MOSC1, RP11-50B3.2, STAB2, STARD13, PTPRT, RBPJ, UBA2, DIAPH3, IL18R1, LIPF, SLITRK5, TMEM132E, POT1, RB1CC1, TAOK1, and UNC5A. The method may optionally include an additional step of obtaining the biological sample from a subject. The method may optionally include an additional step of diagnosing or prognosing CaP using the collected gene mutation data. In methods of diagnosing or prognosing CaP, detection of one or more mutations in at least 2, 3, 4, 5, 6, 7, 8, 9, 10, 15, 20, 25, 30, 35, 40, 45, or 50 of the following genes: GLI1, IRX4, PAPPA, SPOP, TEX15, ZNF292, ANKRD11, FAT4, HECW2, KIAA1109, SHROOM3, SPOP, TTC36, ZNRF3, C17orf65, DEGS2, NEK3, KIAA0947, LSP1, NOX3, AKR1B1, ARHGAP12, ITGA4, PVRL4, RBM26, UCN3, CATSPERB, FCRL2, CACNA1E, CORO6, DMKN, EXT1, HEATR7B2, NDUFB5, GPR180, LRRC4, TPRA1, ZIM2, C12orf50, ELMO2, RBM26, SEC14L1, TNFSF11, C9orf125, CDC73, ITSN1, KCNK16, LRRC7, METTL6, MOSC1, RP11-50B3.2, STAB2, STARD13, PTPRT, RBPJ, UBA2, DIAPH3, IL18R1, LIPF, SLITRK5, TMEM132E, POT1, RB1CC1, TAOK1, and UNC5A indicates the presence of CaP in the biological sample or an increased risk to develop CaP. The methods comprise detecting expression of no more than 100, 90, 80, 70, 60, 50, 40, 30, 25, 20, 15, 14, 13, 12, 11, 10, 9, 8, 7, 6, 5, 4, 3, or 2 mutated genes.

The unique identifier code assigned by HGNC for these genes and their Entrez Gene ID are provided in Table 4. In addition, Table 4 provides the frequency with which each mutation was identified in prostate tumors and a matched normal sample.

Table 4

| Gene | Tumor Freq. | Normal Freq. | HGNC ID | Entrez Gene ID |
|---|---|---|---|---|
| GLI1 | G:21/T:23(52%) | G:37/T:0(0%) | 20500 | 79820 |
| IRX4 | G:18/A:19(51%) | G:42/A:0(0%) | 14875 | 79368 |
| PAPPA | C:21/T:20(48%) | C:40/T:0(0%) | 1392 | 777 |
| SPOP | A:18/C:15(45%) | A:39/C:0(0%) | 21356 | 84940 |
| TEX15 | C:25/T:21(45%) | C:32/T:0(0%) | 25063 | 93099 |
| ZNF292 | T:14/A:11(44%) | T:40/A:0(0%) | 3512 | 2131 |
| ANKRD11 | C:19/A:14(42%) | C:41/A:0(0%) | 26857 | 133558 |
| FAT4 | C:26/T:19(42%) | C:30/T:0(0%) | 7700 | 4711 |
| HECW2 | C:22/T:16(42%) | C:42/T:0(0%) | 28899 | 160897 |
| KIAA1109 | A:19/G:14(42%) | A:47/G:0(0%) | 15586 | 64101 |
| SHROOM3 | G:23/A:16(41%) | G:39/A:0(0%) | 30413 | 131601 |
| SPOP | G:20/C:14(41%) | G:37/C:0(0%) | 12875 | 23619 |
| TTC36 | G:21/A:15(41%) | G:30/A:0(0%) | 26665 | 160419 |
| ZNRF3 | C:15/T:10(40%) | C:31/T:0(0%) | 17233 | 63916 |
| C17orf65 | T:19/C:12(38%) | T:41/C:0(0%) | 20327 | 64062 |
| DEGS2 | C:19/G:12(38%) | C:41/G:0(0%) | 10698 | 6397 |
| NEK3 | G:24/C:15(38%) | G:32/C:0(0%) | 11926 | 8600 |
| KIAA0947 | G:28/A:17(37%) | G:35/A:0(0%) | 28180 | 84302 |
| LSP1 | G:18/T:11(37%) | G:38/T:0(0%) | 16783 | 79577 |
| NOX3 | C:22/T:13(37%) | C:38/T:0(0%) | 6183 | 6453 |
| AKR1B1 | T:19/A:11(36%) | T:47/A:0(0%) | 14464 | 83795 |
| ARHGAP12 | G:24/A:14(36%) | G:40/A:0(0%) | 18531 | 57554 |
| ITGA4 | A:30/G:17(36%) | A:35/G:0(0%) | 28343 | 131965 |
| PVRL4 | C:26AT:15(36%) | C:21/T:0(0%) | 26189 | 64757 |
| RBM26 | C:30/G:17(36%) | C:61/G:0(0%) | 15446 | 26121 |
| UCN3 | T:24/G:14(36%) | T:41/G:0(0%) | 18629 | 55576 |
| CATSPERB | T:36/G:20(35%) | T:46/G:0(0%) | 19164 | 90627 |
| FCRL2 | G:26/A:14(35%) | G:32/A:0(0%) | 9682 | 11122 |
| CACNA1E | C:25/T:13(34%) | C:40/T:0(0%) | 5724 | 3516 |
| CORO6 | T:30/A:16(34%) | T:24/A:0(0%) | 30661 | 10054 |
| DMKN | A:23/C:12(34%) | A:37/C:0(0%) | 15480 | 81624 |
| EXT1 | G:23/A:12(34%) | G:31/A:0(0%) | 5988 | 8809 |
| HEATR7B2 | C:23/T:12(34%) | C:41/T:0(0%) | 6622 | 8513 |
| NDUFB5 | A:32/C:17(34%) | A:55/C:0(0%) | 20295 | 26050 |
| GPR180 | A:32/G:16(33%) | A:41/G:0(0%) | 26991 | 124842 |

(continued)

| Gene | Tumor Freq. | Normal Freq. | HGNC ID | Entrez Gene ID |
|---|---|---|---|---|
| LRRC4 | T:14/G:7(33%) | T:40/G:0(0%) | 17284 | 25913 |
| TPRA1 | A:18/C:9(33%) | A:33/C:0(0%) | 15574 | 9821 |
| ZIM2 | C:28/T:14(33%) | C:30/T:0(0%) | 29259 | 57551 |
| C12orf50 | C:35/A:17(32%) | C:38/A:0(0%) | 12567 | 90249 |
| ELMO2 | T:35/C:17(32%) | T:45/C:0(0%) | 20500 | 79820 |
| RBM26 | C:34/T:16(32%) | C:58/T:0(0%) | 14875 | 79368 |
| SEC14L1 | T:31/G:15(32%) | T:35/G:0(0%) | 1392 | 777 |
| TNFSF11 | A:33/C:16(32%) | A:44/C:0(0%) | 21356 | 84940 |
| C9orf125 | T:26/G:12(31%) | T:27/G:0(0%) | 25063 | 93099 |
| CDC73 | G:31/T:14(31%) | G:44/T:0(0%) | 3512 | 2131 |
| ITSN1 | T:31/C:14(31%) | T:40/C:0(0%) | 26857 | 133558 |
| KCNK16 | C:24/A:11(31%) | C:39/A:0(0%) | 7700 | 4711 |
| LRRC7 | C:39/T:18(31%) | C:32/T:0(0%) | 28899 | 160897 |
| METTL6 | A:28/G:13(31%) | A:35/G:0(0%) | 15586 | 64101 |
| MOSC1 | A:20/G:9(31%) | A:35/G:0(0%) | 30413 | 131601 |
| RP11-50B3.2 | G:26/A:12(31%) | G:44/A:0(0%) | 12875 | 23619 |
| STAB2 | G:20/A:9(31%) | G:38/A:0(0%) | 26665 | 160419 |
| STARD13 | O:24/T:11(31%) | C:35/T:0(0%) | 17233 | 63916 |
| PTPRT | C:30/T:13(30%) | C:40/T:0(0%) | 20327 | 64062 |
| RBPJ | C:23/T:9/G:1(30%) | C:30/T:0(0%) | 10698 | 6397 |
| UBA2 | T:25/A:11(30%) | T:46/A:0(0%) | 11926 | 8600 |
| DIAPH3 | C:39/A:16(29%) | C:32/A:0(0%) | 28180 | 84302 |
| 1L18R1 | G:34/T:14(29%) | G:42/T:0(0%) | 16783 | 79577 |
| LIPF | G:29/T:12(29%) | G:43/T:0(0%) | 6183 | 6453 |
| SLITRK5 | G:22/A:9(29%) | G:45/A:0(0%) | 14464 | 83795 |
| TMEM132E | C:34/T:14(29%) | 0:32/T:0(0%) | 18531 | 57554 |
| POT1 | T:30/C:12(28%) | T:46/C:0(0%) | 28343 | 131965 |
| RB1CC1 | A:27/C:11(28%) | A:42/C:0(0%) | 26189 | 64757 |
| TAOK1 | A:25/C:10(28%) | A:44/C:0(0%) | 15446 | 26121 |
| UNC5A | G:27/A:11(28%) | G:38/A:0(0%) | 18629 | 55576 |

The GLI1 mutation showed the highest allele frequency in the tumors analyzed and also shares a common pathway with SPOP, a gene with a mutation known to be associated with prostate cancer. Therefore, the methods described herein include detecting the GLI1 mutation either alone or in combination with one or more of the gene mutations listed in Table 4.

The methods of collecting data or diagnosing and/or prognosing CaP may further comprise detecting expression of other genes associated with prostate cancer, including, but not limited to ERG, PSA, and PCA3.

**Detecting Gene Expression**

As used herein, measuring or detecting the expression of any of the foregoing genes or nucleic acids comprises measuring or detecting any nucleic acid transcript (e.g., mRNA, cDNA, or genomic DNA) corresponding to the gene of interest or the protein encoded thereby. If a gene is associated with more than one mRNA transcript or isoform, the expression of the gene can be measured or detected by measuring or detecting one or more of the mRNA transcripts of the gene, or all of the mRNA transcripts associated with the gene.

Typically, gene expression can be detected or measured on the basis of mRNA or cDNA levels, although protein levels also can be used when appropriate. Any quantitative or qualitative method for measuring mRNA levels, cDNA, or protein levels can be used. Suitable methods of detecting or measuring mRNA or cDNA levels include, for example, Northern Blotting, microarray analysis, or a nucleic acid amplification procedure, such as reverse-transcription PCR (RT-PCR) or real-time RT-PCR, also known as quantitative RT-PCR (qRT-PCR). Such methods are well known in the art. *See e.g.,* Sambrook et al., Molecular Cloning: A Laboratory Manual, 4th Ed., Cold Spring Harbor Press, Cold Spring Harbor, N.Y., 2012. Other techniques include digital, multiplexed analysis of gene expression, such as the nCounter® (NanoString Technologies, Seattle, WA) gene expression assays, which are further described in [22], [23], US20100112710 and US20100047924.

Detecting a nucleic acid of interest generally involves hybridization between a target (e.g. mRNA, cDNA, or genomic DNA) and a probe. Sequences of the genes used in the prostate cancer gene expression profile are known (see above). Therefore, one of skill in the art can readily design hybridization probes for detecting those genes. *See e.g.,* Sambrook et al., Molecular Cloning: A Laboratory Manual, 4th Ed., Cold Spring Harbor Press, Cold Spring Harbor, N.Y., 2012. Each probe should be substantially specific for its target, to avoid any cross-hybridization and false positives. An alternative to using specific probes is to use specific reagents when deriving materials from transcripts (e.g., during cDNA production, or using target-specific primers during amplification). In both cases specificity can be achieved by hybridization to portions of the targets that are substantially unique within the group of genes being analyzed, e.g. hybridization to the polyA tail would not provide specificity. If a target has multiple splice variants, it is possible to design a hybridization reagent that recognizes a region common to each variant and/or to use more than one reagent, each of which may recognize one or more variants.

Preferably, microarray analysis or a PCR-based method is used. In this respect, measuring the expression of the foregoing nucleic acids in prostate cancer tissue can comprise, for instance, contacting a sample containing or suspected of containing prostate cancer cells with polynucleotide probes specific to the genes of interest, or with primers designed to amplify a portion of the genes of interest, and detecting binding of the probes to the nucleic acid targets or amplification of the nucleic acids, respectively. Detailed protocols for designing PCR primers are known in the art. *See e.g.,* Sambrook et al., Molecular Cloning: A Laboratory Manual, 4th Ed., Cold Spring Harbor Press, Cold Spring Harbor, N.Y., 2012. Similarly, detailed protocols for preparing and using microarrays to analyze gene expression are known in the art and described herein.

Alternatively or additionally, expression levels of genes can be determined at the protein level, meaning that levels of proteins encoded by the genes discussed above are measured. Several methods and devices are well known for determining levels of proteins including immunoassays such as described in e.g., U.S. Pat. Nos. 6,143,576; 6,113,855; 6,019,944; 5,985,579; 5,947,124; 5,939,272; 5,922,615; 5,885,527; 5,851,776; 5,824,799; 5,679,526; 5,525,524; 5,458,852; and 5,480,792. These assays include various sandwich, competitive, or non-competitive assay formats, to generate a signal that is related to the presence or amount of a protein of interest. Any suitable immunoassay may be utilized, for example, lateral flow, enzyme-linked immunoassays (ELISA), radioimmunoassays (RIAs), competitive binding assays, and the like. Numerous formats for antibody arrays have been described. Such arrays typically include different antibodies having specificity for different proteins intended to be detected. For example, at least 100 different antibodies are used to detect 100 different protein targets, each antibody being specific for one target. Other ligands having specificity for a particular protein target can also be used, such as the synthetic antibodies disclosed in WO/2008/048970. Other compounds with a desired binding specificity can be selected from random libraries of peptides or small molecules. U.S. Pat. No. 5,922,615, describes a device that uses multiple discrete zones of immobilized antibodies on membranes to detect multiple target antigens in an array. Microtiter plates or automation can be used to facilitate detection of large numbers of different proteins.

One type of immunoassay, called nucleic acid detection immunoassay (NADIA), combines the specificity of protein antigen detection by immunoassay with the sensitivity and precision of the polymerase chain reaction (PCR). This amplified DNA-immunoassay approach is similar to that of an enzyme immunoassay, involving antibody binding reactions and intermediate washing steps, except the enzyme label is replaced by a strand of DNA and detected by an amplification reaction using an amplification technique, such as PCR. Exemplary NADIA techniques are described in U.S. Patent No. 5,665,539 and U.S. Patent No. 8,338,579. Briefly, NADIA uses a first (reporter) antibody that is specific for the protein of interest and labelled with an assay-specific nucleic acid. The presence of the nucleic acid does not interfere with the binding of the antibody, nor does the antibody interfere with the nucleic acid amplification and detection. Typically, a second (capturing) antibody that is specific for a different epitope on the protein of interest is coated onto a solid phase (e.g., paramagnetic particles). The reporter antibody/nucleic acid conjugate is reacted with sample in a microtiter plate to form a first immune complex with the target antigen. The immune complex is then captured onto the solid phase particles coated with the capture antibody, forming an insoluble sandwich immune complex. The microparticles are washed to remove excess, unbound reporter antibody/nucleic acid conjugate. The bound nucleic acid label is then detected by subjecting the suspended particles to an amplification reaction (e.g. PCR) and monitoring the amplified nucleic acid product.

Although immunoassays have typically been used for the identification and quantification of proteins, recent advances in mass spectrometry (MS) techniques have led to the development of sensitive, high throughput MS protein analyses. The MS methods can be used to detect low abundant proteins in complex biological samples. For example, it is possible to perform targeted MS by fractionating the biological sample prior to MS analysis. Common techniques for carrying out such fractionation prior to MS analysis include two-dimensional electrophoresis, liquid chromatography, and capillary electrophoresis [25]. Selected reaction monitoring (SRM), also known as multiple reaction monitoring (MRM), has also emerged as a useful high throughput MS-based technique for quantifying targeted proteins in complex biological samples, including prostate cancer biomarkers that are encoded by gene fusions (e.g., TMPRSS2/ERG) [26, 27].

### Samples

The methods described in this application involve analysis of gene expression profiles in prostate cells. These prostate cells are found in a biological sample, such as prostate tissue, blood, serum, plasma, urine, saliva, or prostatic fluid. Nucleic acids or polypeptides may be isolated from the cells prior to detecting gene expression.

The biological sample may comprise prostate tissue and is obtained through a biopsy, such as a transrectal or transperineal biopsy. The biological sample may be urine. Urine samples may be collected following a digital rectal examination (DRE) or a prostate biopsy. The sample may be blood, serum, or plasma, and contains circulating tumor cells that have detached from a primary tumor. The sample may also contain tumor-derived exosomes. Exosomes are small (typically 30 to 100 nm) membrane-bound particles that are released from normal, diseased, and neoplastic cells and are present in blood and other bodily fluids. The methods disclosed in this application can be used with samples collected from a variety of mammals, but preferably with samples obtained from a human subject.

### Controls

The control can be any suitable reference that allows evaluation of the expression level of the genes in the prostate cancer cells as compared to the expression of the same genes in a sample comprising non-cancerous prostate cells, such as normal prostate epithelial cells from a matched subject, or a pool of such samples. Thus, for instance, the control can be a sample from the same subject that is analyzed simultaneously or sequentially with the test sample, or the control can be the average expression level of the genes of interest, as described above, in a pool of prostate samples known to be non-cancerous. Alternatively, the control can be defined by mRNA copy numbers of other genes in the sample, such as housekeeping genes (e.g., PBGD or GAPDH) that can be used to normalize gene expression levels. Thus, the control can be embodied, for example, in a pre-prepared microarray used as a standard or reference, or in data that reflects the expression profile of relevant genes in a sample or pool of non-cancerous samples, such as might be part of an electronic database or computer program.

Over expression and decreased expression of a gene can be determined by any suitable method, such as by comparing the expression of the genes in a test sample with a control (e.g., a positive or negative control), or by using a predetermined "cut-off" or threshold value of absolute expression. A control can be provided as previously discussed. Regardless of the method used, over expression and decreased expression can be defined as any level of expression greater than or less than the level of expression of the same genes, or other genes (e.g., housekeeping genes), in non-cancerous prostate cells or tissue. By way of further illustration, over expression can be defined as expression that is at least about 1.2-fold, 1.5-fold, 2-fold, 2.5-fold, 5-fold, 10-fold, 20-fold, 50-fold, 100-fold higher or even greater expression as compared to non-cancerous prostate cells or tissue, and decreased expression can similarly be defined as expression that is at least about 1.2-fold, 1.5-fold, 2-fold, 2.5-fold, 5-fold, 10-fold, 20-fold. 50-fold, 100-fold lower or even lower expression as compared to non-cancerous prostate cells or tissue. Over expression or decreased expression as used herein is defined as expression that is at least about 2-fold higher or lower, respectively, as compared to a control sample or threshold value.

### Prostate Cancer

This disclosure provides gene expression profiles that are associated with prostate cancer. The gene expression profiles can be used to detect prostate cancer cells in a sample or to measure the severity or aggressiveness of the prostate cancer, for example, distinguishing between well differentiated prostate (WD) cancer and poorly differentiated (PD) prostate cancer.

When prostate cancer is found in a biopsy, it is typically graded to estimate how quickly it is likely to grow and spread. The most commonly used prostate cancer grading system, called Gleason grading, evaluates prostate cancer cells on a scale of 1 to 5, based on their pattern when viewed under a microscope.

Cancer cells that still resemble healthy prostate cells have uniform patterns with well-defined boundaries and are considered well differentiated (Gleason grades 1 and 2). The more closely the cancer cells resemble prostate tissue, the more the cells will behave like normal prostate tissue and the less aggressive the cancer. Gleason grade 3, the most common grade, shows cells that are moderately differentiated, that is, still somewhat well-differentiated, but with boundaries that are not as well-defined. Poorly-differentiated cancer cells have random patterns with poorly defined boundaries and no longer resemble prostate tissue (Gleason grades 4 and 5), indicating a more aggressive cancer.

Prostate cancers often have areas with different grades. A combined Gleason score is determined by adding the grades from the two most common cancer cell patterns within the tumor. For example, if the most common pattern is grade 4 and the second most common pattern is grade 3, then the combined Gleason score is 4+3=7. If there is only one pattern within the tumor, the combined Gleason score can be as low as 1+1=2 or as high as 5+5=10. Combined scores of 2 to 4 are considered well-differentiated, scores of 5 to 6 are considered moderately-differentiated and scores of 7 to 10 are considered poorly-differentiated. Cancers with a high Gleason score are more likely to have already spread beyond the prostate gland at the time they were found.

In general, the lower the Gleason score, the less aggressive the cancer and the better the prognosis (outlook for cure or long-term survival). The higher the Gleason score, the more aggressive the cancer and the poorer the prognosis for long-term, metastasis-free survival.

**Array**

A convenient way of measuring RNA transcript levels for multiple genes in parallel is to use an array (also referred to as microarrays in the art). Techniques for using arrays to assess and compare gene expression levels are well known in the art and include appropriate hybridization, detection and data processing protocols. A useful array includes multiple polynucleotide probes (typically DNA) that are immobilized on a solid substrate (e.g. a glass support such as a microscope slide, or a membrane) in separate locations (e.g., addressable elements) such that detectable hybridization can occur between the probes and the transcripts to indicate the amount of each transcript that is present. The arrays disclosed in this application can be used in methods of detecting the expression of a desired combination of genes, which combinations are discussed throughout this application.

The array may comprise (a) a substrate and (b) 2, 3, 4, 5, 6, 7, 8, 9, or 10 or more different addressable elements that each comprise at least one polynucleotide probe for detecting the expression of an mRNA transcript (or cDNA synthesized from the mRNA transcript) of one of the following human genes: DLX1, NKX2-3, CRISP3, PHGR1, THBS4, AMACR, GAP43, FFAR2, GCNT1, SIM2, STX19, KLB, APOF, LOC283177, and TRPM4.

The array may comprise (a) a substrate and (b) 2, 3, 4, 5, 6, 7, or 8 or more different addressable elements that each comprise at least one polynucleotide probe for detecting the expression of an mRNA transcript (or cDNA synthesized from the mRNA transcript) of one of the following human genes; PCA3, ALOX15, AMACR, CDH19, OR51E2/PSGR, F5, FZD8, and CLDN3.

The array may comprise (a) a substrate and (b) 2, 3, 4, 5, 6, or 7 or more different addressable elements that each comprise at least one polynucleotide probe for detecting the expression of an mRNA transcript (or cDNA synthesized from the mRNA transcript) of one of the following human genes: COL10A1, HOXC4, ESPL1, MMP9, ABCA13, PCDHGA1, and AGSK1.

As used herein, the term "addressable element" means an element that is attached to the substrate at a predetermined position and specifically binds a known target molecule, such that when target-binding is detected (e.g., by fluorescent labeling), information regarding the identity of the bound molecule is provided on the basis of the location of the element on the substrate. Addressable elements are "different" for the purposes of the present disclosure if they do not bind to the same target gene. The addressable element comprises one or more polynucleotide probes specific for an mRNA transcript of a given gene, or a cDNA synthesized from the mRNA transcript. The addressable element can comprise more than one copy of a polynucleotide, can comprise more than one different polynucleotide, provided that all of the polynucleotides bind the same target molecule. Where a gene is known to express more than one mRNA transcript, the addressable element for the gene can comprise different probes for different transcripts, or probes designed to detect a nucleic acid sequence common to two or more (or all) of the transcripts. Alternatively, the array can comprise an addressable element for the different transcripts. The addressable element also can comprise a detectable label, suitable examples of which are well known in the art.

The array can comprise addressable elements that bind to mRNA or cDNA other than that of 1) DLX1, NKX2-3, CRISP3, PHGR1, THBS4, AMACR, GAP43, FFAR2, GCNT1, SIM2, STX19, KLB, APOF, LOC283177, and TRPM4; 2) PCA3, ALOX15, AMACR, CDH19, OR51E2/PSGR, F5, FZD8, and CLDN3; or 3) COL10A1, HOXC4, ESPL1, MMP9, ABCA13, PCDHGA1, and AGSK1. However, an array capable of detecting a vast number of targets (e.g., mRNA or polypeptide targets), such as arrays designed for comprehensive expression profiling of a cell line, chromosome, genome, or the like, are not economical or convenient for collecting data to use in diagnosing an/or prognosing prostate cancer. Thus, to facilitate the convenient use of the array as a diagnostic tool or screen, for example, in conjunction with the methods described herein, the array preferably comprises a limited number of addressable elements. In this regard, the array comprises no more than about 1000 different addressable elements, more preferably no more than about 500 different addressable elements, no more than about 250 different addressable elements, or even no more than about 100 different addressable elements, such as about 75 or fewer different addressable elements, or even about 50 or fewer different addressable elements. Of course, even smaller arrays can comprise about 25 or fewer different addressable elements, such as about 15 or fewer different addressable elements or about 12 or fewer different addressable elements. The array can even be limited to about 7 different addressable elements without interfering with its functionality.

It is also possible to distinguish these diagnostic arrays from the more comprehensive genomic arrays and the like by limiting the number of polynucleotide probes on the array. Thus, the array has polynucleotide probes for no more than 1000 genes immobilized on the substrate or the array has oligonucleotide probes for no more than 500, 250, 100, 50, 25, 15, 10, 9, 8, 7, 6, 5, 4, 3, or 2 genes immobilized on the substrate.

The substrate can be any rigid or semi-rigid support to which polynucleotides can be covalently or non-covalently attached. Suitable substrates include membranes, filters, chips, slides, wafers, fibers, beads, gels, capillaries, plates, polymers, microparticles, and the like. Materials that are suitable for substrates include, for example, nylon, glass, ceramic, plastic, silica, aluminosilicates, borosilicates, metal oxides such as alumina and nickel oxide, various clays, nitrocellulose, and the like.

The polynucleotides of the addressable elements (also referred to as "probes") can be attached to the substrate in a pre-determined 1- or 2-dimensional arrangement, such that the pattern of hybridization or binding to a probe is easily correlated with the expression of a particular gene. Because the probes are located at specified locations on the substrate (i.e., the elements are "addressable"), the hybridization or binding patterns and intensities create a unique expression profile, which can be interpreted in terms of expression levels of particular genes and can be correlated with prostate cancer in accordance with the methods described herein.

Polynucleotide and polypeptide probes can be generated by any suitable method known in the art (*see e.g.,* Sambrook et al., Molecular Cloning: A Laboratory Manual, 4th Ed., Cold Spring Harbor Press, Cold Spring Harbor, N.Y., 2012). For example, polynucleotide probes that specifically bind to the mRNA transcripts of the genes described herein (or cDNA synthesized therefrom) can be created using the nucleic acid sequences of the mRNA or cDNA targets themselves (e.g., nucleic acid sequences disclosed in Tables 1-4) by routine techniques (e.g., PCR or synthesis). As used herein, the term "fragment" means a contiguous part or portion of a polynucleotide sequence comprising about 10 or more nucleotides, about 15 or more nucleotides, about 20 or more nucleotides, about 30 or more, or even about 50 or more nucleotides. By way of further illustration, a polynucleotide probe that binds to an mRNA transcript of DLX1 (or cDNA corresponding thereto) can be provided by a polynucleotide comprising a nucleic acid sequence that is complementary to the mRNA transcript (e.g., SEQ ID NO: 2) or a fragment thereof, or sufficiently complementary to SEQ ID NO: 2 or fragment thereof that it selectively binds to SEQ ID NO: 2. The same is true with respect to the other genes described herein. The exact nature of the polynucleotide probe is not critical to the invention; any probe that will selectively bind the mRNA or cDNA target can be used. Typically, the polynucleotide probes will comprise 10 or more nucleic acids, 20 or more, 50 or more, or 100 or more nucleic acids. In order to confer sufficient specificity, the probe will have a sequence identity to a complement of the target sequence (e.g., nucleic acid sequences disclosed in Tables 1-4) of about 90% or more, preferably about 95% or more (e.g., about 98% or more or about 99% or more) as determined, for example, using the well-known Basic Local Alignment Search Tool (BLAST) algorithm (available through the National Center for Biotechnology Information (NCBI), Bethesda, Md.).

Stringency of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured nucleic acid sequences to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions," as defined herein, are identified by, but not limited to, those that: (1) use low ionic strength and high temperature for washing, for example 0.015 M sodium chloride/0.0015 M sodium citrate/0.1% sodium dodecyl sulfate at 50°C; (2) use during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C; or (3) use 50% formamide, 5XSSC (0.75 M NaCl, 0.075 M sodium citrate), 50 mM sodium phosphate (pH 6.8), 0.1% sodium pyrophosphate, 5X Denhardt's solution, sonicated salmon sperm DNA (50μg/ml), 0.1% SDS, and 10% dextran sulfate at 42°C, with washes at 42°C in 0.2XSSC (sodium chloride/sodium, citrate) and 50% formamide at 55°C, followed by a high-stringency wash consisting of 0.1XSSC containing EDTA at 55°C. "Moderately stringent conditions" are described by, but not limited to, those in Sambrook et al., Molecular Cloning: A Laboratory Manual, New York: Cold Spring Harbor Press, 1989, and include the use of washing solution and hybridization conditions (e.g., temperature, ionic strength and % SDS) less stringent than those described above. An example of moderately stringent conditions is overnight incubation at 37°C in a solution comprising: 20% formamide, 5XSSC (150 mM NaCl, 15 mM trisodium citrate), 50 mM sodium phosphate (pH 7.6), 5X Denhardt's solution, 10% dextran sulfate, and 20 mg/mL denatured sheared salmon sperm DNA, followed by washing the filters in 1XSSC at about 37-50°C. The skilled artisan will recognize how to adjust the temperature, ionic strength, etc. as necessary to accommodate factors such as probe length and the like.

The array can comprise other elements common to polynucleotide arrays. For instance, the array also can include one or more elements that serve as a control, standard, or reference molecule, such as a housekeeping gene or portion thereof (e.g.. PBGD or GAPDH), to assist in the normalization of expression levels or the determination of nucleic acid quality and binding characteristics, reagent quality and effectiveness, hybridization success, analysis thresholds and success, etc. These other common aspects of the arrays or the addressable elements, as well as methods for constructing and using arrays, including generating, labeling, and attaching suitable probes to the substrate, consistent with the invention are well-known in the art. Other aspects of the array are as previously described herein with respect to the methods of the invention.

The array may comprise (a) a substrate and (b) two or more different addressable elements that each comprise at least one polynucleotide probe for detecting the expression of an mRNA transcript of one of the following human genes: DLX1, NKX2-3, CRISP3, PHGR1, THBS4, AMACR, GAP43, FFAR2, GCNT1, SIM2, STX19, KLB, APOF, LOC283177, and TRPM4, wherein the array comprises no more than 500, 250, 100, 50, 25, 15, 10, 9, 8, 7, 6, 5, 4, 3, or 2 addressable elements. The array may comprise at least 3, 4, 5, 6, 7, 10, 12, or 15 different addressable elements.

The array may comprise two or more different addressable elements each of which comprises at least one polynucleotide probe for detecting the expression of an mRNA transcript of one of the following human genes: PCA3, ALOX15, AMACR, CDH19, OR51E2/PSGR, F5, FZD8, and CLDN3, wherein the array comprises no more than 500, no more than 250, no more than 100, no more than 50, no more than 25, or no more than 15 addressable elements. The array may comprise at least 3, 4, 5, 6, 7, 10, 12, or 15 different addressable elements.

The array may comprise two or more different addressable elements each of which comprises at least one polynucleotide probe for detecting the expression of an mRNA transcript of one of the following human genes: COL10A1, HOXC4, ESPL1, MMP9, ABCA13, PCDHGA1, and AGSK1, wherein the array comprises no more than 500, 250, 100, 50, 25, 15, 10, 9, 8, 7, 6, 5, 4, 3, or 2 addressable elements. The array may comprise at least 3, 4, 5, 6, 7, 10, 12, or 15 different addressable elements.

An array can also be used to measure protein levels of multiple proteins in parallel. Such an array comprises one or more supports bearing a plurality of ligands that specifically bind to a plurality of proteins, wherein the plurality of proteins comprises no more than 500, 250, 100, 50, 25, 15, 10, 9, 8, 7, 6, 5, 4, 3, or 2 different proteins. The ligands are optionally attached to a planar support or beads. The ligands may be antibodies. The proteins that are to be detected using the array correspond to the proteins encoded by the nucleic acids of interest, as described above, including the specific gene expression profiles disclosed. Thus, each ligand (e.g. antibody) is designed to bind to one of the target proteins (e.g., polypeptide sequences disclosed in Tables 1-4). As with the nucleic acid arrays, each ligand is preferably associated with a different addressable element to facilitate detection of the different proteins in a sample.

**Patient Treatment**

This application describes methods of diagnosing and prognosing prostate cancer in a sample obtained from a subject, in which gene expression in prostate cells and/or tissues are analyzed. If a sample shows over expression of certain genes or the expression of certain gene mutations, then there is an increased likelihood that the subject has prostate cancer or a less or more advanced stage (e.g., WD or PD prostate cancer) of prostate cancer. In the event of such a result, the methods of detecting or prognosing prostate cancer may include one or more of the following steps: informing the patient that they are likely to have prostate cancer, WD prostate cancer or PD prostate cancer; confirmatory histological examination of prostate tissue; and/or treating the patient by a prostate cancer therapy.

Thus, if the detection step indicates that the subject has prostate cancer, the methods further comprise a step of taking a prostate biopsy from the subject and examining the prostate tissue in the biopsy (e.g., histological examination) to confirm whether the patient has prostate cancer. Alternatively, the methods of detecting or prognosing prostate cancer may be used to assess the need for therapy or to monitor a response to a therapy (e.g., disease-free recurrence following surgery or other therapy), and, thus may include an additional step of treating a subject having prostate cancer.

Prostate cancer treatment options include surgery, radiation therapy, hormone therapy, chemotherapy, biological therapy, or high intensity focused ultrasound. Drugs approved for prostate cancer include: Enzalutamide (XTANDI), Abiraterone Acetate, Cabazitaxel, Degarelix, Jevtana (Cabazitaxel), Prednisone, Provenge (Sipuleucel-T), Sipuleucel-T, or Docetaxel. Thus a method as described in this application may, after a positive result, include a further step of surgery, radiation therapy, hormone therapy, chemotherapy, biological therapy, or high intensity focused ultrasound.

**Drug Screening**

The gene expression profiles associated with prostate cancer or lack thereof provided by the methods described in this application can also be useful in screening drugs, either in clinical trials or in animal models of prostate cancer. A clinical trial can be performed on a drug in similar fashion to the monitoring of an individual patient, except that the drug is administered in parallel to a population of prostate cancer patients, usually in comparison with a control population administered a placebo.

The changes in expression levels of genes can be analyzed in individual patients and across a treated or control population. Analysis at the level of an individual patient provides an indication of the overall status of the patient at the end of the trial (i.e., whether gene expression profile indicates the presence or severity (e.g., WD or PD) of prostate cancer) and/or an indication whether that profile has changed toward or away from such indication in the course of the trial. Results for individual patients can be aggregated for a population allowing comparison between treated and control population.

Similar trials can be performed in non-human animal models of prostate cancer. In this case, the expression levels of genes detected are the species variants or homologs of the human genes referenced above in whatever species of non-human animal on which tests are being conducted. Although the average expression levels of human genes determined in human prostate cancer patients are not necessarily directly comparable to those of homolog genes in an animal model, the human values can nevertheless be used to provide an indication whether a change in expression level of a non-human homolog is in a direction toward or away from the diagnosis of prostate cancer or prognosis of WD or PD prostate cancer. The expression profile of individual animals in a trial can provide an indication of the status of the animal at the end of the trial (i.e., whether gene expression profile indicates the presence or severity (e.g., WD or PD) of prostate cancer) and/or change in such status during the trial. Results from individual animals can be aggregated across a population and treated and control populations compared. Average changes in the expression levels of genes can then be compared between the two populations.

**Computer Implemented Models**

In accordance with all aspects and embodiments of the invention, the methods provided may be computer-implemented.

Gene expression levels can be analyzed and associated with status of a subject (e.g., presence of prostate cancer or severity of disease (e.g., WD or PD prostate cancer)) in a digital computer. Optionally, such a computer is directly linked to a scanner or the like receiving experimentally determined signals related to gene expression levels. Alternatively, expression levels can be input by other means. The computer can be programmed to convert raw signals into expression levels (absolute or relative), compare measured expression levels with one or more reference expression levels, or a scale of such values. The computer can also be programmed to assign values or other designations to expression levels based on the comparison with one or more reference expression levels, and to aggregate such values or designations for multiple genes in an expression profile. The computer can also be programmed to output a value or other designation providing an indication of the presence or severity of prostate cancer as well as any of the raw or intermediate data used in determining such a value or designation.

A typical computer (*see* U.S. Pat. No. 6,785,613; FIGS. 4 and 5) includes a bus which interconnects major subsystems such as a central processor, a system memory, an input/output controller, an external device such as a printer via a parallel port, a display screen via a display adapter, a serial port, a keyboard, a fixed disk drive and a port (e.g., USB port) operative to receive an external memory storage device. Many other devices can be connected such as a scanner via I/O controller, a mouse connected to serial port or a network interface. The computer contains computer readable media holding codes to allow the computer to perform a variety of functions. These functions include controlling automated apparatus, receiving input and delivering output as described above. The automated apparatus can include a robotic arm for delivering reagents for determining expression levels, as well as small vessels, e.g., microtiter wells for performing the expression analysis.

A typical computer system 106 may also include one or more processors 110 coupled to random access memory operating under control of or in conjunction with an operating system as set forth in Figure 4 and discussed above.

Any of the computer-implemented methods of the invention may comprise a step of obtaining by at least one processor information reflecting the expression level of at least 2, 3, 4, 5, 6, 7, 8, 9, 10, 11, 12, 13, 14, or 15 of the following human genes: DLX1, NKX2-3, CRISP3, PHGR1, THBS4, AMACR, GAP43, FFAR2, GCNT1, SIM2, STX19, KLB, APOF, LOC283177, and TRPM4 in a biological sample.

The computer-implemented methods may comprise obtaining by at least one processor information reflecting the expression level of DLX1, NKX2-3, DLX1 and NKX2-3, PHGR1, THBS4, GAP43, FFAR2, GCNT1, SIM2, STX19, KLB, APOF, LOC283177, or TRPM4 and one or more of the other genes listed in Table 1.

Any of the computer-implemented methods describe herein may comprise a step of obtaining by at least one processor information reflecting the expression level of at least 2, 3, 4, 5, 6, 7, or 8 of the following human genes: PCA3, ALOX15, AMACR, CDH19, OR51E2/PSGR, F5, FZD8, and CLDN3 in a biological sample obtained from a patient of Caucasian descent.

The computer-implemented methods may comprise obtaining by at least one processor information reflecting the expression level of ALOX15 and one or more of PCA3, AMACR, CDH19, OR51E2/PSGR, F5, FZD8, and CLDN3. The computer-implemented methods may comprise obtaining by at least one processor information reflecting the expression level of CDH19 and one or more of PCA3, AMACR, ALOX15, OR51E2/PSGR, F5, FZD8, and CLDN3. The computer-implemented methods may comprise obtaining by at least one processor information reflecting the expression level of F5 and one or more of PCA3, AMACR, ALOX15, OR51E2/PSGR, CDH19, FZD8, and CLDN3. The computer-implemented methods may comprise obtaining by at least one processor information reflecting the expression level of FZD8 and one or more of PCA3, AMACR, ALOX15, OR5IE2/PSGR, CDH19, F5, and CLDN3. The computer-implemented methods may comprise obtaining by at least one processor information reflecting the expression level of CLDN3 and one or more of PCA3, AMACR, ALOX15, OR51E2/PSGR, CDH19, F5, and FZD8. The computer-implemented methods may comprise obtaining by at least one processor information reflecting the expression level of PCA3 and AMACR and one or more of ALOX15, CDH19, F5, FZD8, and CLDN3.

Any of the computer-implemented methods of the invention may comprise a step of obtaining by at least one processor information reflecting the expression level of at least 2, 3, 4, 5, 6, or 7 of the following human genes: COL10A1, HOXC4, ESPL1, MMP9, ABCA13, PCDHGA1, and AGSK1 in a biological sample obtained from a patient of African descent.

The computer-implemented methods may comprise obtaining by at least one processor information reflecting the expression level of COL10A1 and one or more of HOXC4, ESPL1, MMP9, ABCA13, PCDHGA1, and AGSK1. The computer-implemented method may comprise obtaining by at least one processor information reflecting the expression level of HOXC4 and one or more of COL10A1, ESPL1, MMP9, ABCA13, PCDHGA1, and AGSK1. The computer-implemented methods may comprise obtaining by at least one processor information reflecting the expression level of ESPL1 and one or more of COL10A1, HOXC4, MMP9, ABCA13, PCDHGA1, and AGSK1. The computer-implemented methods may comprise obtaining by at least one processor information reflecting the expression level of MMP9 and one or more of COL10A1, HOXC4, ESPL1, ABCA13, PCDHGA1, and AGSK1. The computer-implemented methods may comprise obtaining by at least one processor information reflecting the expression level of ABCA13 and one or more of COL10A1, HOXC4, ESPL1, MMP9, PCDHGA1, and AGSK1. The computer-implemented methods may comprise obtaining by at least one processor information reflecting the expression level of PCDHGA1 and one or more of COL10A1, HOXC4, ESPL1, MMP9, ABCA13, and AGSK1. The computer-implemented methods may comprise obtaining by at least one processor information reflecting the expression level of AGSK1 and one or more of COL10A1, HOXC4, ESPL1, MMP9, ABCA13, and PCDHGA1.

The computer-implemented methods may additionally comprise the steps of i) determining by at least one processor a difference between the expression level of one or more control genes and the expression level of 1) at least 2, 3, 4, 5, 6, 7, 8, 9, 10, 11, 12, 13, 14, or 15 of the following human genes: DLX1, NKX2-3, CRISP3, PHGR1, THBS4, AMACR, GAP43, FFAR2, GCNT1, S1M2, STX19, KLB, APOF, LOC283177, and TRPM4 in a biological sample; 2) at least 2, 3, 4, 5, 6, 7, or 8 of the following human genes: PCA3, ALOX15, AMACR, CDH19, OR51E2/PSGR, F5, FZD8, and CLDN3 in a biological sample obtained from a patient of Caucasian descent; or 3) at least 2, 3, 4, 5, 6, or 7 of the following human genes: COL10A1, HOXC4, ESPL1, MMP9, ABCA13, PCDHGA1, and AGSK1 in a biological sample obtained from a patient of African descent; and (ii) outputting in user readable format the difference obtained in the determining step.

The computer-implemented methods may further comprise outputting in user readable format a determination that the subject has prostate cancer, well differentiated prostate cancer, or poorly differentiated prostate cancer based on the difference obtained in the outputting step.

Kits

The polynucleotide probes and/or primers or antibodies or polypeptide probes that are used in the methods described in this application can be arranged in a kit. Thus, also described herein is a kit for diagnosing or prognosing prostate cancer comprising a plurality of polynucleotide probes for detecting at least 2, 3, 4, 5, 6, 7, 8, 9, 10, 11, 12, 13, 14, or 15 of the following human genes: DLX1, NKX2-3, CRISP3, PHGR1, THBS4, AMACR, GAP43, FFAR2, GCNT1, SIM2, STX19, KLB, APOF, LOC283177, and TRPM4, wherein the plurality of polynucleotide probes contains polynucleotide probes for no more than 500, 250, 100, 50, 25, 15, 10, 9, 8, 7, 6, 5, 4, 3, or 2 genes. Optionally, the plurality of polynucleotide probes comprises polynucleotide probes for detecting at least 4 or 5 of the aforementioned genes, wherein the plurality of polynucleotide probes contains polynucleotide probes for no more than 10 genes. The polynucleotide probes may be optionally labeled. The kit may optionally include polynucleotide primers for amplifying a portion of the mRNA transcripts from at least 2, 3, 4, 5, 6, 7, 8, 9, 10, 11, 12, 13, 14, or 15 of the following human genes: DLX1, NKX2-3, CRISP3, PHGR1, THBS4, AMACR, GAP43, FFAR2, GCNT1, SIM2, STX19, KLB, APOF, LOC283177, and TRPM4.

Also described herein is a kit for diagnosing or prognosing prostate cancer in a patient of Caucasian descent, the kit comprising a plurality of polynucleotide probes for detecting at least 3, 4, 5, 6, 7, or 8 of the following human genes: PCA3, ALOX15, AMACR, CDH19, OR51E2/PSGR, F5, FZD8, and CLDN3, wherein the plurality of polynucleotide probes contains polynucleotide probes for no more than 500, 250, 100, 50, 25, 15, 10, 9, 8, 7, 6, 5, 4, 3, or 2 genes. Optionally, the plurality of polynucleotide probes comprises polynucleotide probes for detecting at least 4 or 5 of the aforementioned genes, wherein the plurality of polynucleotide probes contains polynucleotide probes for no more than 10 genes. The polynucleotide probes may be optionally labeled. The kit may optionally include polynucleotide primers for amplifying a portion of the mRNA transcripts from at least 3, 4, 5, 6, 7, or 8 of the following human genes: PCA3, ALOX15, AMACR, CDH19, OR51E2/PSGR, F5, FZD8, and CLDN3.

One embodiment is directed to a kit for diagnosing prostate cancer in a patient of African descent, the kit comprising a plurality of polynucleotide probes for detecting at least 3, of the following human genes: COL10A1, HOXC4, ESPL1, MMP9, ABCA13, PCDHGA1, and AGSK1, wherein the plurality of polynucleotide probes contains polynucleotide probes for no more than 500, 250, 100, 50, 25, 15, 10, 9, 8, 7. 6, 5, 4, or 3 genes. Optionally, the plurality of polynucleotide probes comprises polynucleotide probes for detecting at least 4 or 5 of the aforementioned genes, wherein the plurality of polynucleotide probes contains polynucleotide probes for no more than 10 genes. The polynucleotide probes may be optionally labeled. The kit may optionally include polynucleotide primers for amplifying a portion of the mRNA transcripts from at least 3, 4, 5, 6, or 7 of the following human genes: COL10A1, HOXC4, ESPL1, MMP9, ABCA13, PCDHGA1, and AGSK1.

Another aspect described herein is directed to a kit for diagnosing or prognosing prostate cancer comprising antibodies. Thus, the kit for diagnosing or prognosing prostate cancer may comprise a plurality of antibodies for detecting at least 3, 4, 5, 6, 7, 8, 9, 10, 11, 12, 13, 14, or 15 of the polypeptides encoded by the following human genes: DLX1, NKX2-3, CRISP3, PHGR1, THBS4, AMACR, GAP43, FFAR2, GCNT1, SIM2, STX19, KLB, APOF, LOC283177, and TRPM, wherein the plurality of antibodies contains antibodies for no more than 500, 250, 100, 50, 25, 15, 10,9, 8, 7, 6, 5, 4, 3, or 2 polypeptides. Optionally, the plurality of antibodies comprises antibodies for detecting at least 4 or 5 of the polypeptides encoded by the aforementioned genes and wherein the plurality of antibodies contains antibodies for no more than 10 polypeptides. The antibodies may be optionally labeled.

As described herein the kit for diagnosing or prognosing prostate cancer in a patient of Caucasian descent may comprise a plurality of antibodies for detecting at least 3, 4, 5, 6, 7, or 8 of the polypeptides encoded by the following human genes: PCA3, ALOX15, AMACR, CDH19, OR51E2/PSGR, F5, FZD8, and CLDN3, wherein the plurality of antibodies contains antibodies for no more than 500, 250, 100, 50, 25, 15, 10, 9, 8, 7, 6, 5, 4, 3, or 2 polypeptides. Optionally, the plurality of antibodies comprises antibodies for detecting at least 4 or 5 of the polypeptides encoded by the aforementioned genes and wherein the plurality of antibodies contains antibodies for no more than 10 polypeptides. The antibodies may be optionally labeled.

In one embodiment, the kit for diagnosing prostate cancer in a patient of African descent comprises a plurality of antibodies for detecting at least 3, 4, 5, 6, or 7 of the polypeptides encoded by following-human genes: COL10A1, HOXC4, ESPL1, MMP9, ABCA13, PCDHGA1, and AGSK1, wherein the plurality of antibodies contains antibodies for no more than 500, 250, 100, 50, 25, 15, 10, 9, 8, 7, 6, 5, 4, 3, or 2 polypeptides. Optionally, the plurality of antibodies comprises antibodies for detecting at least 4 or 5 of the polypeptides encoded by the aforementioned genes and wherein the plurality of antibodies contains antibodies for no more than 10 polypeptides. The antibodies may be optionally labeled.

In another aspect, the kit for diagnosing prostate cancer may comprise polypeptide probes that can be used, for example, in spectrometry methods, such as mass spectrometry. The kit for diagnosing or prognosing prostate cancer comprises a plurality of polypeptide probes for detecting at least 3, 4, 5, 6, 7, 8, 9, 10, 11, 12, 13, 14, or 15 of the polypeptides encoded by the following human genes: DLX1, NKX2-3, CRISP3, PHGR1, THBS4, AMACR, GAP43, FFAR2, GCNT1, SIM2, STX19, KLB, APOF, LOC283177, and TRPM, wherein the plurality of polypeptide probes contains polypeptide probes for no more than 500, 250, 100, 50, 25, 15, 10, 9, 8, 7, 6, 5, 4, 3, or 2 polypeptides. Optionally, the plurality of polypeptide probes comprises polypeptide probes for detecting at least 4 or 5 of the polypeptides encoded by the aforementioned genes and wherein the plurality of polypeptide probes contains polypeptide probes for no more than 10 polypeptides. The polypeptide probes may be optionally labeled.

As described herein, the kit for diagnosing or prognosing prostate cancer in a patient of Caucasian descent comprises a plurality of polypeptide probes for detecting at least 3, 4, 5, 6, 7, or 8 of the polypeptides encoded by the following human genes: PCA3, ALOX15, AMACR, CDH19, OR51E2/PSGR, F5, FZD8, and CLDN3, wherein the plurality of polypeptide probes contains polypeptide probes for no more than 500, 250, 100, 50, 25, 15, 10, 9, 8, 7, 6, 5, 4, 3, or 2 polypeptides. Optionally, the plurality of polypeptide probes comprises polypeptide probes for detecting at least 4 or 5 of the polypeptides encoded by the aforementioned genes and wherein the plurality of polypeptide probes contains polypeptide probes for no more than 10 polypeptides. The polypeptide probes may be optionally labeled.

In one embodiment, the kit for diagnosing prostate cancer in a patient of African descent comprises a plurality of polypeptide probes for detecting at least 3, 4, 5, 6, or 7 of the polypeptides encoded by following human genes: COL10A1, HOXC4, ESPL1, MMP9, ABCA13, PCDHGA1, and AGSK1, wherein the plurality of polypeptide probes contains polypeptide probes for no more than 500, 250, 100, 50, 25, 15, 10, 9, 8, 7, 6, 5, 4 or 3 polypeptides. Optionally, the plurality of polypeptide probes comprises polypeptide probes for detecting at least 4 or 5 of the polypeptides encoded by the aforementioned genes and wherein the plurality of polypeptide probes contains polypeptide probes for no more than 10 polypeptides. The polypeptide probes may be optionally labeled.

In one embodiment, a kit includes instructional materials disclosing methods of use of the kit contents in a disclosed method. The instructional materials may be provided in any number of forms, including, but not limited to, written form (e.g., hardcopy paper, etc.), in an electronic form (e.g., computer diskette or compact disk) or may be visual (e.g., video files). The kits may also include additional components to facilitate the particular application for which the kit is designed. Thus, for example, the kits may additionally include other reagents routinely used for the practice of a particular method, including, but not limited to buffers, enzymes, labeling compounds, and the like. Such kits and appropriate contents are well known to those of skill in the art. The kit can also include a reference or control sample. The reference or control sample can be a biological sample or a data base.

As noted above, the polynucleotide or polypeptide probes and antibodies described in this application are optionally labeled with a detectable label. Any detectable label used in conjunction with probe or antibody technology, as known by one of ordinary skill in the art, can be used. In a particular embodiment, the probe is labeled with a detectable label selected from the group consisting of: a fluorescent label, a chemiluminescent label, a quencher, a radioactive label, biotin, mass tags and/or gold.

Unless otherwise defined, all technical and scientific terms used herein have the same meaning as commonly understood by one of ordinary skill in the art. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

## EXAMPLES

### Example 1. Comparative Genomic DNA Analysis

A comparative full genome analysis was conducted using primary prostate tumors and corresponding normal tissue (blood) in a cohort of seven AA and seven CA CaP patients (28 specimens). The cohort was selected based on the following criteria: primary treatment radical prostatectomy, no neo-adjuvant treatment, Gleason grade 3 +3 and 3+4 (representing the majority of PSA-screened CaP at diagnosis/primary treatment), frozen tumor tissue with 80% or more tumor cell content, dissected tumor tissue yielding over 2 μg high molecular weight genomic DNA, availability of corresponding blood genomic DNA and patient clinico-pathological data.

28 samples were sent to Illumina Inc. (UK) for sequencing. Sequences from tumor samples were mapped to the reference genome using Illumina's ELAND alignment algorithm. Sequencing reported good coverage (average 37). Variant calling for single nucleotide polymorphisms (SNPs), small insertions and deletions (InDels), copy number variants (CNVs), and structural variants (SVs) was performed concurrently using the Strelka algorithm. All established CaP mutations (TMPRSS2/ERG, SPOP, CHD1, and PTEN) were identified at expected frequencies in this cohort.

Thirty one genes (including known mutations) with SNV, CNV or InDel somatic mutations in at least two of 14 patients were identified: AC091435.2; APC; ASMTL; ASMTL-AS1; CDC73; CHD1; CSF2RA; EYS; FRG1; FRG1B; HK2; IL3RA; KLLN; LIPF; LOC100293744; MT-ATP6; MT-BD4; MT-CO1; MT-CYB; MT-ND2; MT-ND3; MUC16; MUC6; NOX3; PDHA2; PTEN; SLC25A6; SLC9B1; SPOP; TRAV20; and USH2A.

The mutations did not appear to exhibit association with any specific group (AA-, AA+, CA-, CA+). However, the absence of PTEN deletions in AA patients was unexpected. Unequivocal PTEN deletion was detected in two CA cases with lesser apparent PTEN deletion in three additional CA cases, indicating the potential exclusivity of PTEN deletions in CA cases.

### Example 2. Comparative RNA Analysis

To complement the genomic DNA analysis, RNA-Seq analysis was performed in the same cohort of prostate tumor samples. RNA-Seq technology has the ability to interrogate multiple aspects of the transcriptome including gene fusion, gene and transcript expression. Surrounding normal tissue was collected from 4 of the 14 patients. Two of the normal tissue samples were from AA men and two were from CA men.

RNA samples were shipped to Expression Analysis (Durham, NC) for transcriptome sequencing. The details of sequencing statistics are as follows: Sequencing type: paired-end, average read length of each sample: 50nt, average read quality: 37, number of reads: approximately 31 million in each sample.

Raw reads from expression analysis were obtained in fastq format for each sample. These files contain all sequences passing Illumina's purity filter and per-base quality score as defined by Illumina phred metric. These raw reads were filtered for low quality reads (quality score <20), artifact/duplicate sequences and adapter sequences prior to actual analysis.

The human reference genome (hg19) used for mapping in this analysis was downloaded from UCSC website. Clean Paired end reads were aligned to the hg19 reference genome using TopHat software version 2.0.8 (a free open source tool available for mapping), and for each read no more than 2 mismatches were allowed in the alignment. TopHat maps reads to the reference genome using Bowtie, an ultra high-throughput short read aligner. Software outputs numerous files for further analysis: mapped reads, fusion junction, splice junction, insertions and deletions files.

Aligned reads were assembled to transcripts using Cufflinks, an open source tool that assembles transcripts, estimates their abundances, and tests for differential expression and regulation in RNA-Seq samples. Cufflinks calculates the expression level of gene depending on all the known splice variants/isoform of that gene.

Cufflinks measures transcript and Gene abundance levels in Fragments per Kilobase of transcript per Million mapped reads (FPKM). FPKM formula is defined as below:

$$FPKM = C/_{LN}$$

C is the number of mappable reads in the feature (transcript, exon), L is the length in feature (in kb) and N is the total number of mappable reads in the feature (in millions). The FPKM normalization method eliminates discrepancies of gene lengths and sequencing for comparing the differences in gene expression between samples.

The Cuffdiff program, included in the Cufflinks package, calculates differential expression between CaP tumor and normal samples and includes a p-value for each observed change in expression between samples. Cuffdiff allows inputting multiple sample files based on the experimental condition. Gene and transcript expression levels are reported in tabular format and these files contain gene information, log2 scale fold change for each gene, P values and false discovery rate (FDR). Pathway analysis and Gene Ontology Biological Processes on statistically significant genes was performed with Genomatix Pathway Analysis Software.

Hierarchical clustering (performed using R package) was used to group samples based on their expression levels. Figure 1. Clustering of all 18 samples (14 tumors and 4 normal) indicate a clear demarcation between tumor and normal samples. However, most of the tumor samples were not clustered according to AA and CA groups. Interestingly, three out of four fusion negative AA samples clustered in to one group, and based on the patient follow up studies, two of the 3 patients developed metastasis (the only two metastasis in this cohort), and the third had biochemical recurrence.

Gene expression profiles were obtained from 14 tumor (7 AA and 7 CA) and 4 normal (2 AA and 2CA) samples. A limitation for the comparison analysis between tumor and normal samples was the availability of normal samples for all tumor samples. Hence, in the current analysis two normal samples within each group were pooled together and the average value was compared to their respective groups.

In the initial analysis, gene expression profiles for each patient were generated by comparing tumor with normal sample within each group. Statistically significant genes were extracted using fold change (tumor/normal ratios), at least 2 fold over/under expressed in tumors and P-value < 0.05. 101 genes and 180 genes were statistically significant (2fold & p-value<0.05) in African- and Caucasian-American groups respectively. Few of the prostate cancer literature associated genes in African-American list included CRISP3, SIM2, THBS4 and MMP9; Caucasian-American list included AMACR, APOF, CRISP3, OR51E2 (PSGR), SIM2 and THBS4.

Comparison of AA gene list to CA gene lists showed 84 genes to be common in both the ethnic groups. The list of common genes included a few well studied genes in prostate cancer like AMACR, CRIPS3 and SIM2. DLX1, NKX3-2 and CRISP3 were the top over expressed genes in this list. Figure 2. The most consistently overexpressed genes in both ethnic groups were DLX1 and NKX2-3. The top 15 over expressed genes in both AA and CA prostate tumors are listed in Table 5 (ranked by fold change).

Table 5

| Gene Symbol | AA | CA |
|---|---|---|
| DLX1 | 168.76 | 94.24 |

(continued)

| Gene Symbol | AA | CA |
|---|---|---|
| NKX2-3 | 128.58 | 49.14 |
| CRISP3* | 128.08 | 711.14 |
| PHGR1 | 44.04 | 100.24 |
| THBS4 | 17.37 | 18.87 |
| AMACR* | 11.89 | 25 |
| GAP43 | 7.19 | 7.99 |
| FFAR2 | 7.03 | 8.54 |
| GCNT1 | 6.62 | 15.23 |
| SIM2* | 6.41 | 9.68 |
| STX19 | 5.98 | 7.67 |
| KLB | 5.07 | 5.91 |
| APOF | 5.04 | 19.42 |
| LOC283177 | 4.86 | 7 |
| TRPM4 | 4.35 | 6.48 |
| * Known gene alternation in prostate cancer | | |

Similarly, tumor/normal ratios of CA group (180 genes) were compared to the tumor/normal ratios in AA group. This gene list revealed that some of the well-studied prostate cancer genes, such as, PCA3 (10-fold), PSGR (5-fold) and AMACR (2-fold) were over expressed in the CA group as compared to the AA group (Figure 3B). Additionally, gene expression levels of TMPRSS2/ERG fusion positive samples were compared with fusion negative samples. CRISP3, GLDC, and TDRD1 were the top differentially expressed genes in TMPRSS2/ERG fusion positive samples, while COL2A1 and PLA2G7 were the top differentially expressed genes in TMPRSS2/ERG fusion negative samples. The top differential ly expressed genes in prostate tumors of the CA group as compared to the AA group are set forth in Table 6.

Table 6

| Gene Symbol | CA | AA |
|---|---|---|
| PCA3* | 94.76 | 6.09 |
| ALOX15 | 79.68 | 9.66 |
| AMACR* | 25 | 11.89 |
| CDH19 | 13.73 | 1.43 |
| OR51E2/PSGR | 10.79 | 2.8 |
| F5 | 8.89 | 4.16 |
| FZD8 | 7.72 | 3.08 |
| CLDN3 | 5.28 | 2.58 |
| * current prostate cancer diagnostic markers | | |

The tumor/normal ratios of differentially expressed gene list in AA group (101 genes) were compared to the tumor/normal ratios in CA group to evaluate AA race specific gene expression trend. The heatmap in Figure 3A shows genes that were consistently up-regulated in the AA group and simultaneously down-regulated (or no change of expression) in the CA group. In this list, MMP9 was the top gene which was found to be very strongly up-regulated in the AA group but down-regulated in the CA group. The top differentially expressed genes in prostate tumors of the AA group as compared to the CA group are set forth in Table 7.

Table 7

| Gene Symbol | AA | CA |
|---|---|---|
| COL10A1 | 539.86 | 16.81 |
| HOXC4 | 72.06 | 13.13 |
| ESPL1 | 35.49 | 1.92 |
| MMP9 | 32.23 | 0.27 |
| ABCA13 | 22.65 | 2.02 |
| PCDHGA1 | 15.15 | 1.82 |
| AGSK1 | 6.09 | 0.98 |

REFERENCES

The following references are cited in the application and provide general information on the field of the invention and provide assays and other details discussed in the application.

1. Siegel, R.; Naishadham, D.; Jemal, A. Cancer statistics. CA Cancer J. Clin. 2013, 63, 11-30.
2. Chornokur, G.; Dalton, K.; Borysova, M.E.; Kumar, N.B. Disparities at presentation, diagnosis, treatment, and survival in African American men affected by prostate cancer. Prostate 2011, 71, 985-997.
3. Schwartz, K.; Powell, I.J.; Underwood, W., 3rd; George, J.; Yee, C.; Banerjee, M. Interplay of race, socioeconomic status, and treatment on survival of patients with prostate cancer. Urology 2009, 74, 1296-1302.
4. Major, J.M.; Oliver, M.N.; Doubeni, C.A.; Hollenbeck, A.R.; Graubard, B.I.; Sinha, R. Socioeconomic status, healthcare density, and risk of prostate cancer among African American and Caucasian men in a large prospective study. Cancer Causes Control 2012, 23, 1185-1191.
5. Sridhar, G.; Masho, S.W.; Adera, T.; Ramakrishnan, V.; Roberts, J.D. Do African American men have lower survival from prostate cancer compared with White men? A meta-analysis. Am. J Mens. Health 2010, 4, 189-206.
6. Cullen, J.; Brassell, S.; Chen, Y.; Porter, C.; L'Esperance, J.; Brand, T.; McLeod, D.G. Racial/ethnic patterns in prostate cancer outcomes in an active surveillance cohort. Prostate Cancer 2011, 2011, doi:10.1155/2011/234519.
7. Berger, A.D.; Satagopan, J.; Lee, P.; Taneja, S.S.; Osman, I. Differences in clinicopathologic features of prostate cancer between black and white patients treated in the 1990s and 2000s. Urology 2006, 67, 120-124.
8. Kheirandish, P.; Chinegwundoh, F. Ethnic differences in prostate cancer. Br. J. Cancer 2011, 105, 481-485.
9. Odedina, F.T.; Akinremi, T.O.; Chinegwundoh, F.; Roberts, R.; Yu, D.; Reams, R.R.; Freedman, M.L.; Rivers, B.; Green, B.L.; Kumar, N. Prostate cancer disparities in black men of African descent: A comparative literature review of prostate cancer burden among black men in the United States, Caribbean, United Kingdom, and West Africa. Infect, Agents Cancer 2009, 4, doi:10.1186/1750-9378-4S1-S2.
10. Heath, E.I.; Kattan, M.W.; Powell, I.J.; Sakr, W.; Brand, T.C.; Rybicki, B.A.; Thompson, I.M.; Aronson, W.J.; Terris, M.K.; Kane, C.J.; et al. The effect of race/ethnicity on the accuracy of the 2001 Partin Tables for predicting pathologic stage of localized prostate cancer. Urology 2008, 71, 151-155.
11. Moul, J.W.; Sesterhenn, I.A.; Connelly, R.R.; Douglas, T.; Srivastava, S.; Mostofi, F.K.; McLeod, D.G. Prostate-specific antigen values at the time of prostate cancer diagnosis in African-American men. JAMA 1995, 274, 1277-1281.
12. Tewari, A.; Horninger, W.; Badani, K.K.; Hasan, M.; Coon, S.; Crawford, E.D.; Gamito, E.J.; Wei, J.; Taub, D.; Montie, J.; et al. Racial differences in serum prostate-specific (PSA) doubling time, histopathological variables and long-term PSA recurrence between African-American and white American men undergoing radical prostatectomy for clinically localized prostate cancer. BJU Int. 2005, 96, 29-33.
13. Wallace, T.A.; Prueitt, R.L.; Yi, M.; Howe, T.M.; Gillespie, J.W.; Yfantis, H.G.; Stephens, R.M.; Caporaso, N.E.; Loffredo, C.A.; Ambs, S. Tumor immunobiological differences in prostate cancer between African-American and Caucasian-American men. Cancer Res. 2008, 68, 927-936.
14. Prensner, J.R.; Rubin, M.A.; Wei, J.T.; Chinnaiyan, A.M. Beyond PSA: The next generation of prostate cancer biomarkers. Sci. Transl. Med. 2012, 4, doi: 10.1126/scitranslmed.3003180.
15. Rubin, M.A.; Maher, C.A.; Chinnaiyan, A.M. Common gene rearrangements in prostate cancer. J. Clin. Oncol. 2011, 29, 3659-3668.
16. Sreenath, T.L.; Dobi, A.; Petrovics, G.; Srivastava, S. Oncogenic activation of ERG: A predominant mechanism in prostate cancer. J. Carcinog. 2011, 11. 10-21.

17. Petrovics, G.; Liu, A.; Shaheduzzaman, S.; Furasato, B.; Sun, C.; Chen, Y.; Nau, M. Ravindranath, L.; Chen, Y.; Dobi, A.; et al. Frequent overexpression of ETS-related gene-1 (ERG1) in prostate cancer transcriptome. Oncogene 2005, 24, 3847-3852.
18. Tomlins, S.A.; Rhodes, D.R.; Perner, S.; Dhanasekaran, S.M.; Mehra, R.; Sun, X.W.; Varambally, S.; Cao, X.; Tchinda, J.; Kuefer, R.; et al. Recurrent fusion of TMPRSS2 and ETS transcription factor genes in prostate cancer. Science 2005, 310, 644-648.
19. Magi-Galluzzi, C.; Tsusuki, T.; Elson, P.; Simmerman, K.; LaFarque, C.; Esqueva, R.; Klein, E.; Rubin, M.A.; Zhou, M. TMPRSS2-ERG gene fusion prevalence and class are significantly different in prostate cancer of Caucasian, African-American and Japanese patients. Prostate 2011, 71, 489-497.
20. Rosen, P.; Pfister, D.; Young, D.; Petrovics, G.; Chen, Y.; Cullen, J.; Bohm, D.; Perner, S.; Dobi, A.; McLeod, D.G.; et al. Differences in frequency of ERG oncoprotein expression between index tumors of Caucasian and African American patients with prostate cancer. Urology 2012, 80, 749-753.
21. Hu, Y.; Dobi, A.; Sreenath, T.; Cook, C.; Tadase, A.Y.; Ravindranath, L.; Cullen,J.; Furusato, B.; Chen, Y.; Thanqapazham, R.L.; et al. Delineation of TMPRSS2-ERG splice variants in prostate cancer. Clin. Cancer Res. 2008, 14, 4719-4725.
22. Gary K Geiss, et al. (2008) Direct multiplexed measurement of gene expression with color-coded probe pairs, Nature Biotechnology 26:317-25.
23.Paolo Fortina and Saul Surrey, (2008) Digital mRNA Profiling, Nature Biotechnology 26:317-25.
24. Farrell J, Petrovics G, McLeod DG, Srivastava S.: Genetic and molecular differences in prostate carcinogenesis between African American and Caucasian American men. International Journal of Molecular Sciences. 2013; 14(8):15510-31.
25. Rodriquez-Suarez et al., Urine as a source for clinical proteome analysis: From discovery to clinical application, Biochimica et Biophysica Acta (2013).
26. Shi et al., Antibody-free, targeted mass-spectrometric approach for quantification of proteins at low picogram per milliliter levels in human plasma/serum, PNAS, 109(38): 15395-15400 (2012).
27. Elentiboba-Johnson and Lim, Fusion peptides from oncogenic chimeric proteins as specific biomarkers of cancer, Mol Cell Proteomics, 12:2714 (2013).
28. Reams et al., Microarray comparison of prostate tumor gene expression in African-American and Caucasian American males: a pilot project, Infectious Agents and Cancer, 4(Suppl I):S3 (2009).

## Claims

1. A method of collecting data for diagnosing prostate cancer, the method comprising:

   detecting expression of a plurality of genes in a biological sample obtained from a patient, wherein the patient is of African descent and wherein the plurality of genes is selected because the patient is of African descent and comprises at least three of the following genes: COL10A1, HOXC4, ESPL1, MMP9, ABCA13, PCDHGA1, and AGSK1,
   wherein the at least three genes comprise COL10A1.

2. The method of claim 1, wherein the plurality of genes comprises COL10A1, HOXC4, ESPL1, MMP9, ABCA13, PCDHGA1, and AGSK1.

3. The method of claim 1, further comprising a step of diagnosing prostate cancer using the expression data obtained.

4. The method of claim 3, wherein overexpression of the at least three genes as compared to a control sample or a threshold value indicates the presence of prostate cancer in the biological sample or an increased risk of developing prostate cancer.

5. The method of any one of claims 1-4, wherein

   i) the method further comprises detecting expression of an ERG gene in the biological sample,
   ii) the biological sample is a tissue sample, a cell sample, a blood sample, a serum sample, or a urine sample,
   iii) the biological sample comprises prostate cells or nucleic acids or polypeptides isolated from prostate cells,
   iv) nucleic acid expression is detected, and/or
   v) polypeptide expression is detected.

6. A kit for use in diagnosing prostate cancer, the kit comprising a plurality of probes for detecting at least three of the following genes: COL10A1, HOXC4, ESPL1, MMP9, ABCA13, PCDHGA1, and AGSK1, wherein the plurality of probes contains probes for detecting no more than 500, 250, 100, 50, 25, 15, 10, 9, 8, 7, 6, 5, 4, or 3 different genes, wherein the at least three genes comprise COL10A1.

7. The kit of claim 6, wherein

   a) the plurality of probes is selected from a plurality of oligonucleotide probes, a plurality of antibodies, or a plurality of polypeptide probes,
   b) the plurality of probes contains probes for detecting no more than 250, 100, 50, 25, 15, 10, or 5 different genes,
   c) the plurality of probes are attached to the surface of an array,
   wherein the array optionally comprises no more than 500, 250, 100, 50, 25, 15, or 10 addressable elements,
   d) the plurality of probes are labeled, and/or
   e) the plurality of probes further comprises a probe for detecting an ERG gene.

8. A method of obtaining a gene expression profile in a biological sample, the method comprising:

   a) incubating the array of claim 7 with the biological sample, wherein the biological sample is obtained from a patient of African descent; and
   b) measuring the expression level of the at least three genes to obtain the gene expression profile.

9. The method of claim 8, wherein

   a) the biological sample is a tissue sample, a cell sample, a blood sample, a serum sample, or a urine sample,
   b) the biological sample comprises nucleic acids or polypeptides isolated from prostate cells,
   c) the measuring step comprises measuring nucleic acid expression levels,
   d) the measuring step comprises measuring polypeptide expression levels, and/or
   e) the measuring step further comprises measuring the expression level of an ERG gene.

10. A method of identifying a patient in need of prostate cancer treatment, wherein the patient is of African descent, the method comprising:

a) testing a biological sample from the patient for the overexpression of a plurality of genes, wherein the plurality of genes is selected because the patient is of African descent and comprises at least three of the following genes: COL10A1, HOXC4, ESPL1, MMP9, ABCA13, PCDHGA1, and AGSK1, wherein the at least three genes comprise COL10A1; and
b) identifying the patient as in need of prostate cancer treatment if one or more of the at least three genes is overexpressed in the biological sample as compared to a control sample or a threshold value.

**11.** The method of any one of claims 1-5, wherein the plurality of genes consists of no more than 100, 90, 80, 70, 60, 50, 40, 30, 25, 20, 15, 14, 13, 12, 11, 10, 9, 8, 7, 6, 5, 4, or 3 genes.

**12.** The method of any one of claims 1-5 or 8-11, wherein the method is a computer-implemented method.

**Patentansprüche**

**1.** Verfahren zum Sammeln von Daten zum Diagnostizieren von Prostatakrebs, wobei das Verfahren umfasst:

Detektieren der Expression mehrerer Gene in einer biologischen Probe, die von einem Patienten erhalten wird, wobei der Patient afrikanischer Abstammung ist und wobei die mehreren Gene ausgewählt werden, weil der Patient afrikanischer Abstammung ist, und wenigstens drei der folgenden Gene umfassen: COL10A1, HOXC4, ESPL1, MMP9, ABCA13, PCDHGA1 und AGSK1,
wobei die wenigstens drei Gene COL10A1 umfassen.

**2.** Verfahren nach Anspruch 1, wobei die mehreren Gene COL10A1, HOXC4, ESPL1, MMP9, ABCA13, PCDHGA1 und AGSK1 umfassen.

**3.** Verfahren nach Anspruch 1, ferner umfassend einen Schritt des Diagnostizierens von Prostatakrebs unter Verwendung der erhaltenen Expressionsdaten.

**4.** Verfahren nach Anspruch 3, wobei eine Überexpression der wenigstens drei Gene im Vergleich zu einer Kontrollprobe oder einem Schwellenwert das Vorhandensein von Prostatakrebs in der biologischen Probe oder ein erhöhtes Risiko der Entwicklung von Prostatakrebs anzeigt.

**5.** Verfahren nach einem der Ansprüche 1 bis 4, wobei

i) das Verfahren ferner die Detektion der Expression eines ERG-Gens in der biologischen Probe umfasst,
ii) die biologische Probe eine Gewebsprobe, eine Zellprobe, eine Blutprobe, eine Serumprobe oder eine Harnprobe ist,
iii) die biologische Probe Prostatazellen oder aus Prostatazellen isolierte Nukleinsäuren oder Polypeptide umfasst,
iv) die Nukleinsäureexpression detektiert wird, und/oder
v) die Polypeptidexpression detektiert wird.

**6.** Kit zur Verwendung bei der Diagnostizierung von Prostatakrebs, wobei das Kit mehrere Sonden zum Detektieren von wenigstens drei der folgenden Gene umfasst: COL10A1, HOXC4, ESPL1, MMP9, ABCA13, PCDHGA1 und AGSK1, wobei die mehreren Sonden Sonden zum Detektieren von nicht mehr als 500, 250, 100, 50, 25, 15, 10, 9, 8, 7, 6, 5, 4 oder 3 verschiedenen Genen umfassen, wobei die wenigstens drei Gene COL10A1 umfassen.

**7.** Kit nach Anspruch 6, wobei

a) die mehreren Sonden aus mehreren Oligonukleotid-Sonden, mehreren Antikörpern oder mehreren Polypeptid-Sonden ausgewählt werden,
b) die mehreren Sonden Sonden zum Detektieren von nicht mehr als 250, 100, 50, 25, 15, 10 oder 5 verschiedenen Genen umfassen,
c) die mehreren Sonden an der Oberfläche eines Arrays anhaften,
wobei der Array gegebenenfalls nicht mehr als 500, 250, 100, 50, 25, 15 oder 10 adressierbare Elemente umfasst,
d) die mehreren Sonden markiert sind, und/oder
e) die mehreren Sonden ferner eine Sonde zum Detektieren eines ERG-Gens umfassen.

**8.** Verfahren zum Erhalten eines Genexpressionsprofils in einer biologischen Probe, wobei das Verfahren umfasst:

a) Inkubieren des Arrays nach Anspruch 7 mit der biologischen Probe, wobei die biologische Probe von einem Patienten afrikanischer Herkunft erhalten wird; und
b) Messen des Expressionsniveaus der wenigstens drei Gene, um das Genexpressionsprofil zu erhalten.

**9.** Verfahren nach Anspruch 8, wobei

a) die biologische Probe eine Gewebsprobe, eine Zellprobe, eine Blutprobe, eine Serumprobe oder eine Harnprobe ist,
b) die biologische Probe aus Prostatazellen isolierte Nukleinsäuren oder Polypeptide umfasst,
c) der Messschritt das Messen der Nukleinsäureexpressionsniveaus umfasst,
d) der Messschritt das Messen der Polypeptidexpressionsniveaus umfasst, und/oder
e) der Messschritt ferner das Messen des Expressionsniveaus eines ERG-Gens umfasst.

**10.** Verfahren zum Identifizieren eines Patienten, der eine Prostatakrebsbehandlung benötigt, wobei der Patient afrikanischer Abstammung ist, wobei das Verfahren umfasst:

a) Testen einer biologischen Probe von dem Patienten auf die Überexpression mehrerer Gene, wobei die mehreren Gene ausgewählt werden, weil der Patient afrikanischer Abstammung ist, und wenigstens drei der folgenden Gene umfassen: COL10A1, HOXC4, ESPL1, MMP9, ABCA13, PCDHGA1 und AGSK1, wobei die wenigstens drei Gene COL10A1 umfassen; und
b) Identifizieren des Patienten als eine Prostatakrebsbehandlung benötigend, wenn eines oder mehrere der wenigstens drei Gene in der biologischen Probe im Vergleich zu einer Kontrollprobe oder einem Schwellenwert überexprimiert sind.

**11.** Verfahren nach einem der Ansprüche 1 bis 5, wobei die mehreren Gene aus nicht mehr als 100, 90, 80, 70, 60, 50, 40, 30, 25, 20, 15, 14, 13, 12, 11, 10, 9, 8, 7, 6, 5, 4 oder 3 Genen bestehen.

**12.** Verfahren nach einem der Ansprüche 1 bis 5 oder 8 bis 11, wobei das Verfahren ein computerimplementiertes Verfahren ist.

## Revendications

**1.** Procédé de collecte de données pour le diagnostic du cancer de la prostate, le procédé comprenant :

la détection de l'expression d'une pluralité de gènes dans un échantillon biologique obtenu d'un patient, dans lequel le patient est d'ascendance africaine et dans lequel la pluralité de gènes est sélectionnée parce que le patient et d'ascendance africaine et comprend au moins trois des gènes suivants : COL10A1, HOXC4, ESPL1, MMP9, ABCA13, PCDHGA1, et AGSK1 ,
dans lequel les aux moins trois gènes comprennent COL10A1.

**2.** Procédé selon la revendication 1, dans lequel la pluralité de gènes comprend COL10A1, HOXC4, ESPL1, MMP9, ABCA13, PCDHGA1, et AGSK1.

**3.** Procédé selon la revendication 1, comprenant en outre une étape de diagnostic du cancer de la prostate à l'aide des données d'expression obtenues.

**4.** Procédé selon la revendication 3, dans lequel la surexpression des aux moins trois gènes comparé à un échantillon témoin ou une valeur seuil indique la présence de cancer de la prostate dans l'échantillon biologique ou un risque accru de développement de cancer de la prostate.

**5.** Procédé selon l'une quelconque des revendications 1 à 4, dans lequel

i) le procédé comprend en outre la détection de l'expression d'un gène ERG dans l'échantillon biologique,
ii) l'échantillon biologique est un échantillon de tissu, un échantillon de cellules, un échantillon de sang, un échantillon de sérum, ou un échantillon d'urine,

iii) l'échantillon biologique comprend des cellules de prostate ou des acides nucléiques ou des polypeptides isolés de cellules de prostate,
iv) l'expression des acides nucléiques est détectée, et/ou
v) l'expression des polypeptides est détectée.

**6.** Kit pour son utilisation dans le diagnostic du cancer de la prostate, le kit comprenant une pluralité de sondes pour la détection d'au moins trois des gènes suivants: COL10A1, HOXC4, ESPL1, MMP9, ABCA13, PCDHGA1, et AGSK1, dans lequel la pluralité de sondes contient des sondes pour la détection de pas plus de 500, 250, 100, 50, 25, 15, 10, 9, 8, 7, 6, 5, 4 ou 3 gènes différents, dans lequel les au moins trois gènes comprennent COL10A1.

**7.** Kit selon la revendication 6, dans lequel

a) la pluralité de sondes est sélectionnée à partir d'une pluralité de sondes oligonucléotidiques, d'une pluralité d'anticorps, ou d'une pluralité de sondes polypeptidiques,
b) la pluralité de sondes contient des sondes pour la détection de pas plus de 250, 100, 50, 25, 15, 10, ou 5 gènes différents,
c) la pluralité de sondes sont fixées à la surface d'un réseau,
dans lequel facultativement le réseau ne comprend pas plus de 500, 250, 100, 50, 25, 15, ou 10, éléments adressables,
d) la pluralité de sondes sont marquées, et/ou
e) la pluralité de sondes comprend en outre une sonde de détection d'un gène ERG.

**8.** Procédé d'obtention d'un profil d'expression génique dans un échantillon biologique, le procédé comprenant :

a) l'incubation du réseau selon la revendication 7 avec l'échantillon biologique, dans lequel l'échantillon biologique est obtenu d'un patient d'ascendance africaine ; et
b) la mesure du niveau d'expression des au moins trois gènes pour obtenir le profil d'expression génique.

**9.** Procédé selon la revendication 8, dans lequel

a) l'échantillon biologique est un échantillon de tissu, un échantillon de cellules, un échantillon de sang, un échantillon de sérum, ou un échantillon d'urine,
b) l'échantillon biologique comprend des acides nucléiques ou des polypeptides isolés de cellules de la prostate,
c) l'étape de mesure comprend la mesure de niveaux d'expression des acides nucléiques,
d) l'étape de mesure comprend la mesure de niveaux d'expression des polypeptides, et/ou
e) l'étape de mesure comprend en outre la mesure du niveau d'expression d'un gène ERG.

**10.** Procédé d'identification d'un patient nécessitant un traitement contre le cancer de la prostate, dans lequel le patient est d'ascendance africaine, le procédé comprenant :

a) le test d'un échantillon biologique du patient à la recherche de la surexpression d'une pluralité de gènes, dans lequel la pluralité de gènes est sélectionnée parce que le patient est d'ascendance africaine et comprend au moins trois des gènes suivants: COL10A1, HOXC4, ESPL1, MMP9, ABCA13, PCDHGA1, et AGSK1, dans lequel les au moins trois gènes comprennent COL10A1 ; et
b) l'identification du patient comme nécessitant un traitement du cancer de la prostate si un ou plus des au moins trois gènes est surexprimé dans l'échantillon biologique comparé à un échantillon témoin ou une valeur seuil.

**11.** Procédé selon l'une quelconque des revendications 1 à 5, dans lequel la pluralité de gènes ne consiste pas en plus de 100, 90, 80, 70, 60, 50, 40, 30, 25, 20, 15, 14, 13, 12, 11, 10, 9, 8, 7, 6, 5, 4 ou 3 gènes.

**12.** Procédé selon l'une quelconque des revendications 1 à 5 ou 8 à 11, dans lequel le procédé est un procédé informatique.

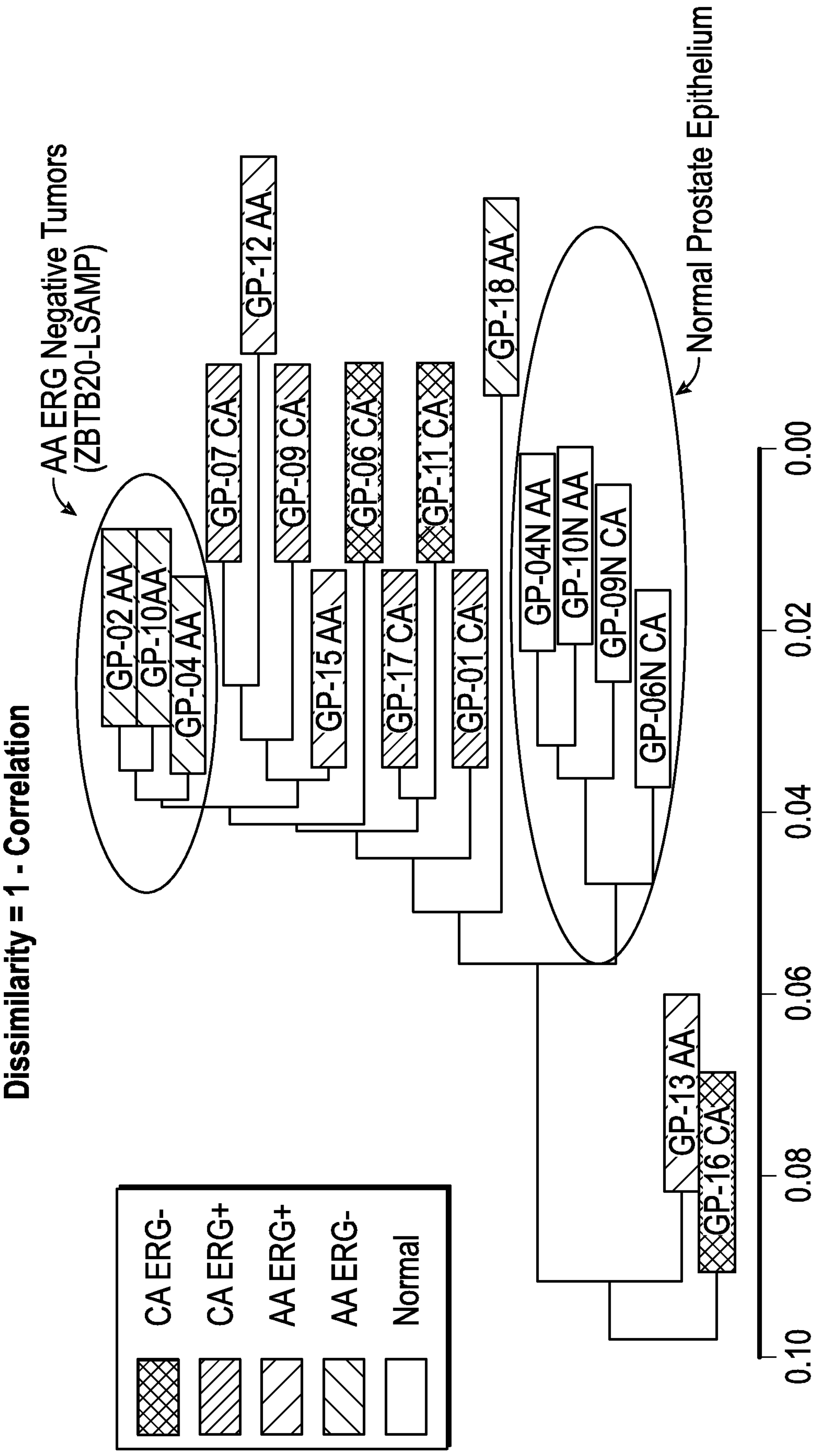
Dissimilarity = 1 - Correlation
AA ERG Negative Tumors
(ZBTB20-LSAMP)
Normal Prostate Epithelium
GP-02 AA
GP-10AA
GP-04 AA
GP-07 CA
GP-12 AA
GP-09 CA
GP-15 AA
GP-06 CA
GP-17 CA
GP-11 CA
GP-01 CA
GP-18 AA
GP-04N AA
GP-10N AA
GP-09N CA
GP-06N CA
GP-13 AA
GP-16 CA
0.10
0.08
0.06
0.04
0.02
0.00
CA ERG-
CA ERG+
AA ERG+
AA ERG-
Normal

FIG. 1

FIG. 2

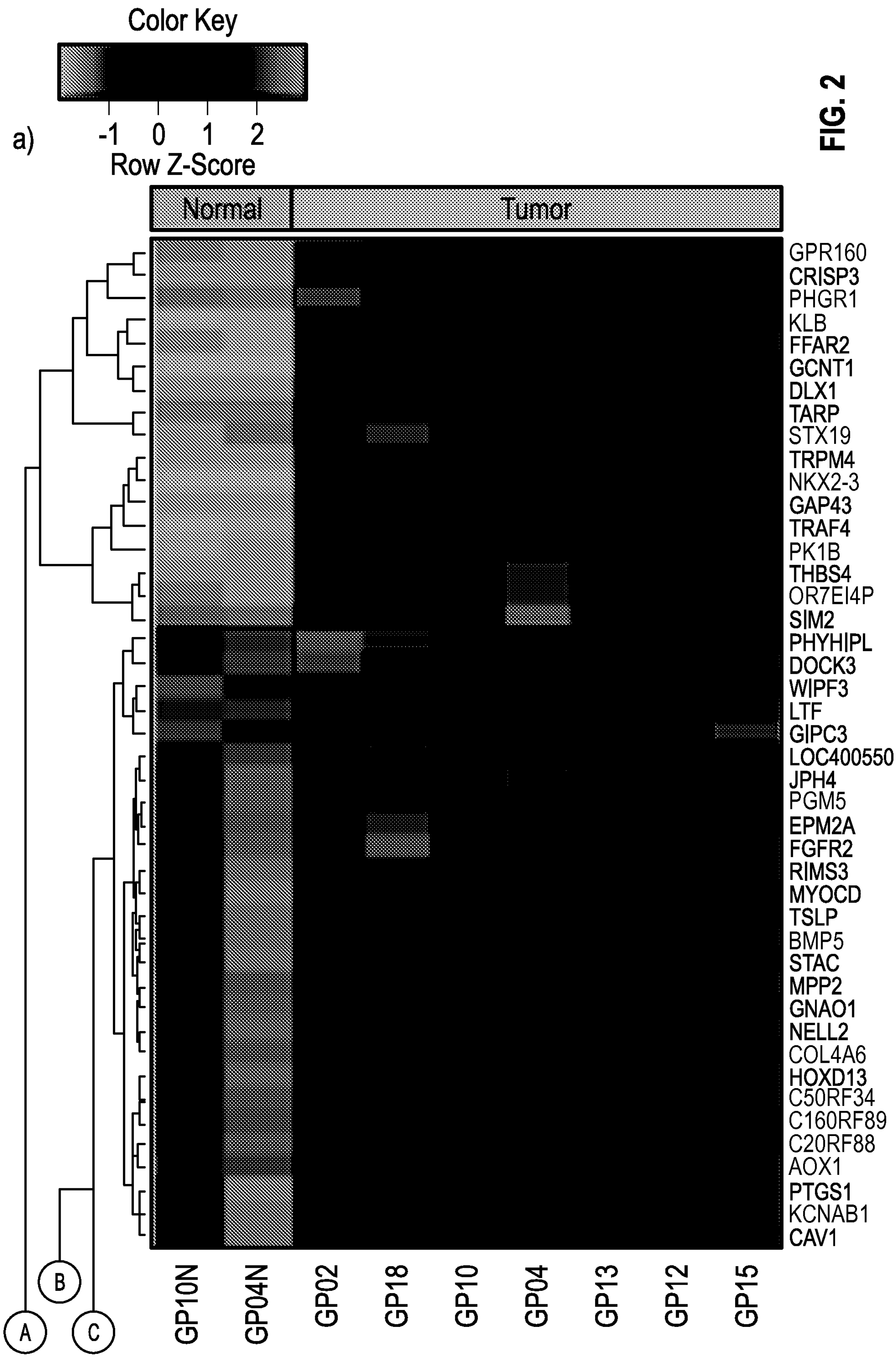
a)
Color Key
-1 0 1 2
Row Z-Score
Normal
Tumor
GPR160
CRISP3
PHGR1
KLB
FFAR2
GCNT1
DLX1
TARP
STX19
TRPM4
NKX2-3
GAP43
TRAF4
PK1B
THBS4
OR7EI4P
SIM2
PHYHIPL
DOCK3
WIPF3
LTF
GIPC3
LOC400550
JPH4
PGM5
EPM2A
FGFR2
RIMS3
MYOCD
TSLP
BMP5
STAC
MPP2
GNAO1
NELL2
COL4A6
HOXD13
C50RF34
C160RF89
C20RF88
AOX1
PTGS1
KCNAB1
CAV1
GP10N
GP04N
GP02
GP18
GP10
GP04
GP13
GP12
GP15
A
B
C

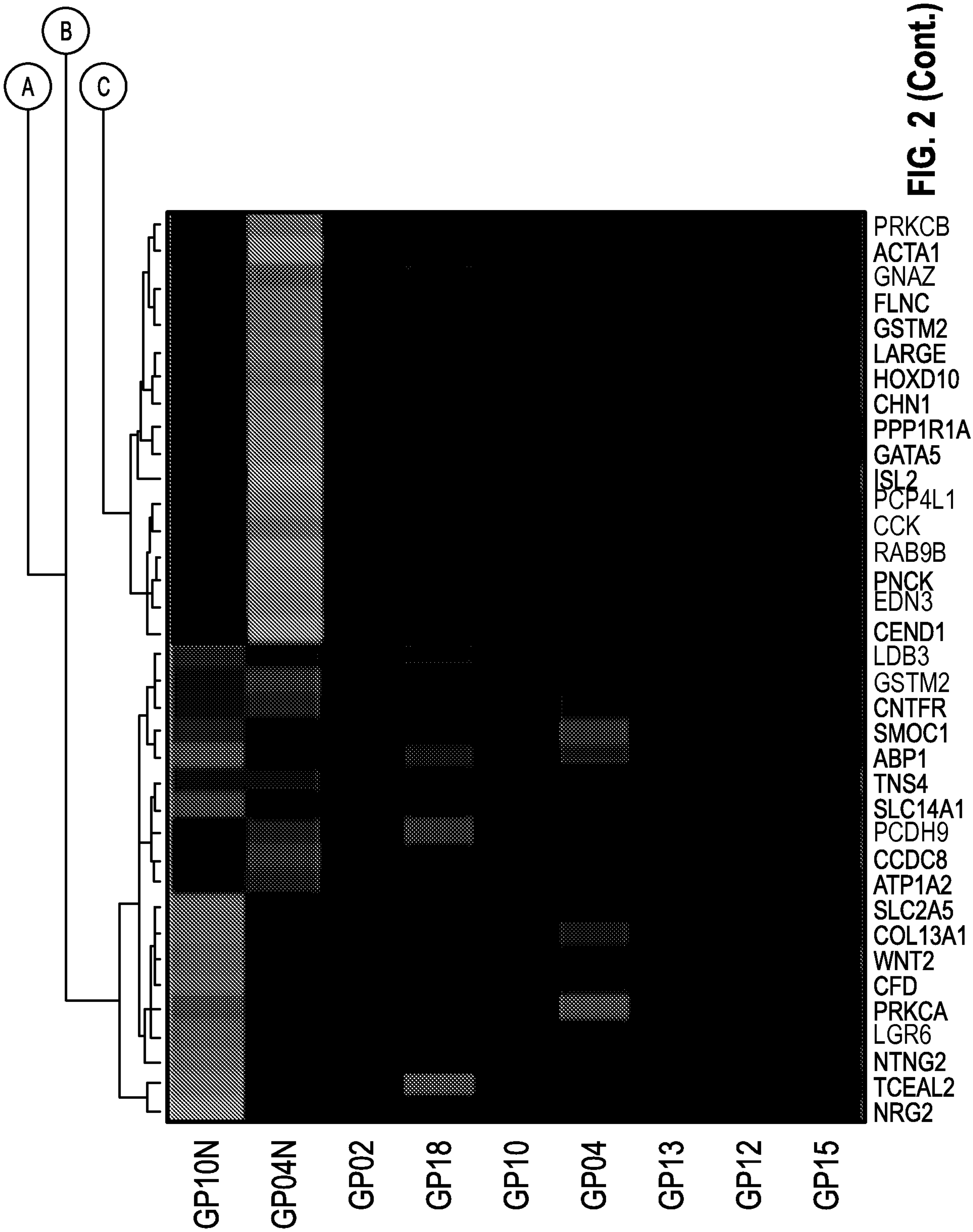
A
B
C
PRKCB
ACTA1
GNAZ
FLNC
GSTM2
LARGE
HOXD10
CHN1
PPP1R1A
GATA5
ISL2
PCP4L1
CCK
RAB9B
PNCK
EDN3
CEND1
LDB3
GSTM2
CNTFR
SMOC1
ABP1
TNS4
SLC14A1
PCDH9
CCDC8
ATP1A2
SLC2A5
COL13A1
WNT2
CFD
PRKCA
LGR6
NTNG2
TCEAL2
NRG2
GP10N
GP04N
GP02
GP18
GP10
GP04
GP13
GP12
GP15

FIG. 2 (Cont.)

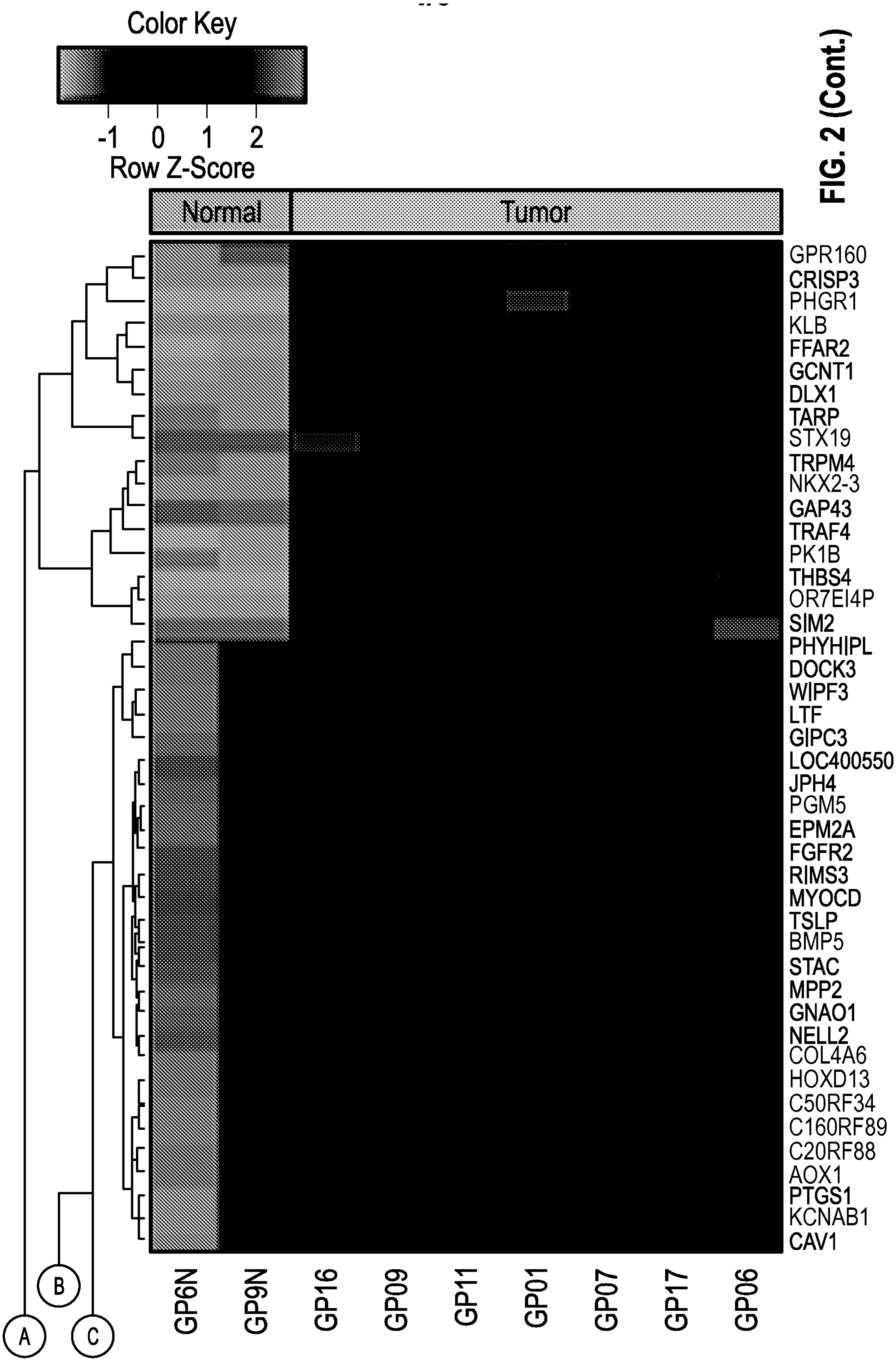
Color Key
-1 0 1 2
Row Z-Score
Normal
Tumor
GPR160
CRISP3
PHGR1
KLB
FFAR2
GCNT1
DLX1
TARP
STX19
TRPM4
NKX2-3
GAP43
TRAF4
PK1B
THBS4
OR7EI4P
SIM2
PHYHIPL
DOCK3
WIPF3
LTF
GIPC3
LOC400550
JPH4
PGM5
EPM2A
FGFR2
RIMS3
MYOCD
TSLP
BMP5
STAC
MPP2
GNAO1
NELL2
COL4A6
HOXD13
C50RF34
C160RF89
C20RF88
AOX1
PTGS1
KCNAB1
CAV1
GP6N
GP9N
GP16
GP09
GP11
GP01
GP07
GP17
GP06
A
B
C

FIG. 2 (Cont.)

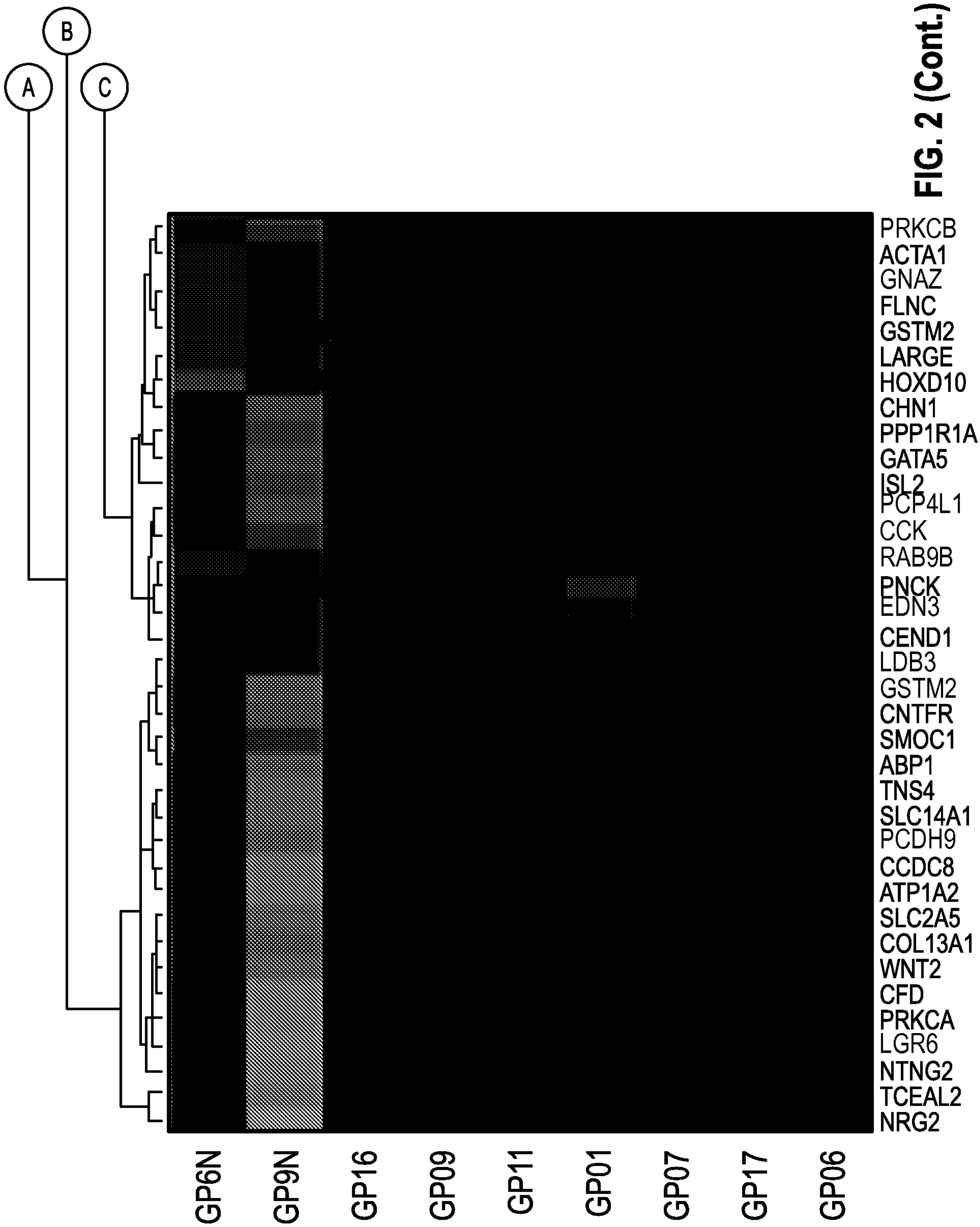
A
B
C
PRKCB
ACTA1
GNAZ
FLNC
GSTM2
LARGE
HOXD10
CHN1
PPP1R1A
GATA5
ISL2
PCP4L1
CCK
RAB9B
PNCK
EDN3
CEND1
LDB3
GSTM2
CNTFR
SMOC1
ABP1
TNS4
SLC14A1
PCDH9
CCDC8
ATP1A2
SLC2A5
COL13A1
WNT2
CFD
PRKCA
LGR6
NTNG2
TCEAL2
NRG2
GP6N
GP9N
GP16
GP09
GP11
GP01
GP07
GP17
GP06

FIG. 2 (Cont.)

b)

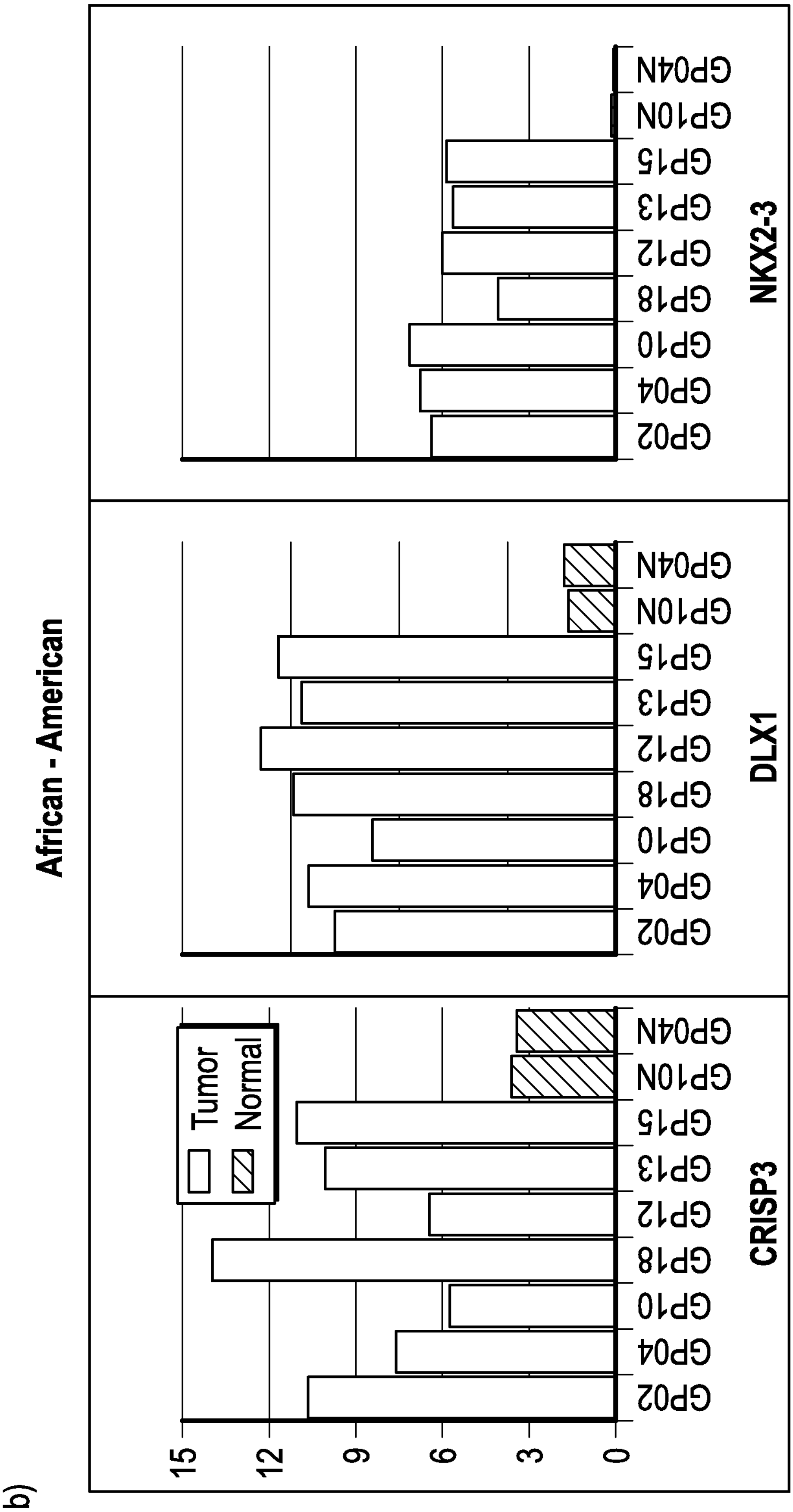
African - American
Tumor
Normal
15
12
9
6
3
0
GP02
GP04
GP10
GP18
GP12
GP13
GP15
GP10N
GP04N
CRISP3
DLX1
NKX2-3

FIG. 2

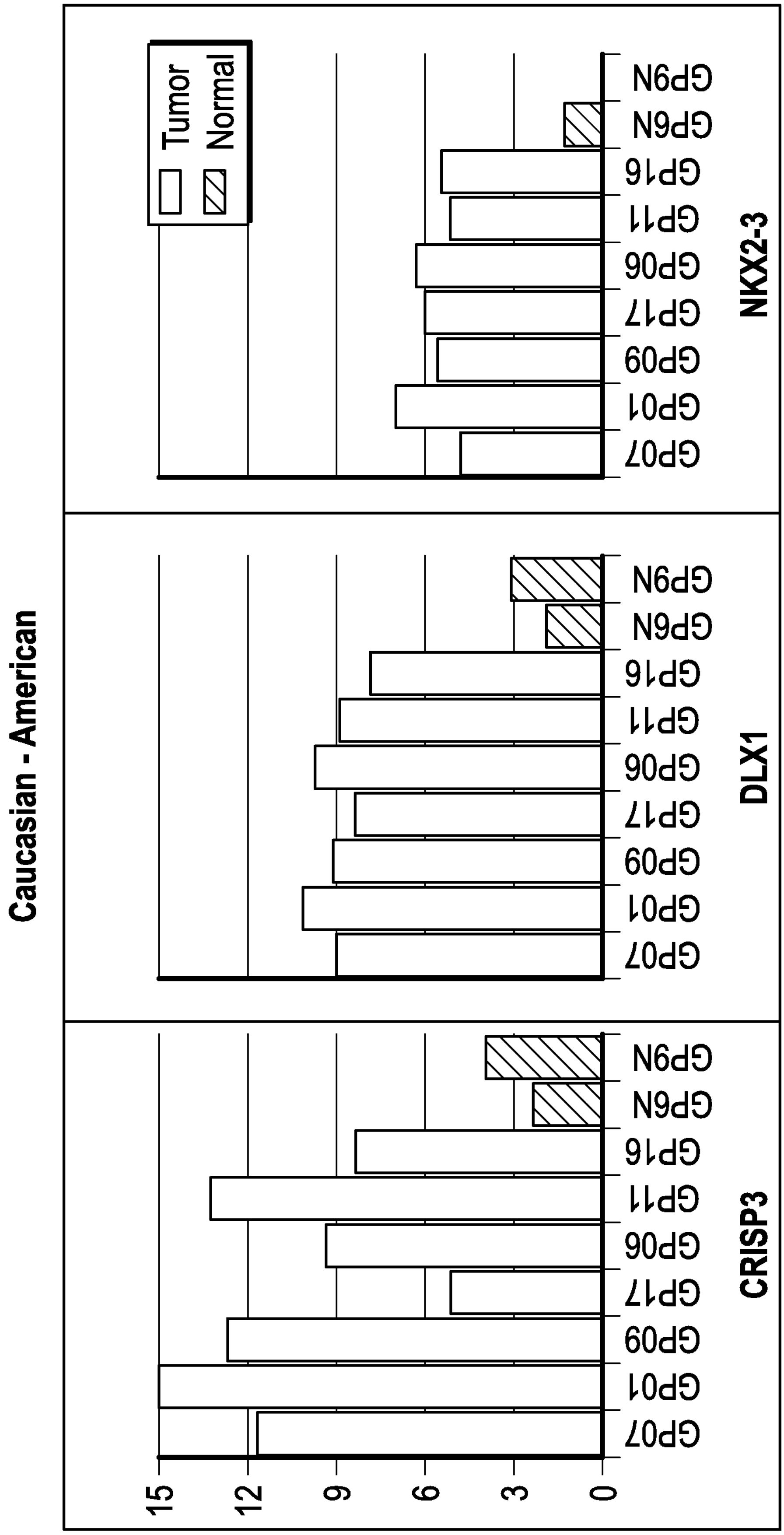
Caucasian - American
Tumor
Normal
15
12
9
6
3
0
GP07
GP01
GP09
GP17
GP06
GP11
GP16
GP6N
GP9N
CRISP3
DLX1
NKX2-3

FIG. 2 (Cont.)

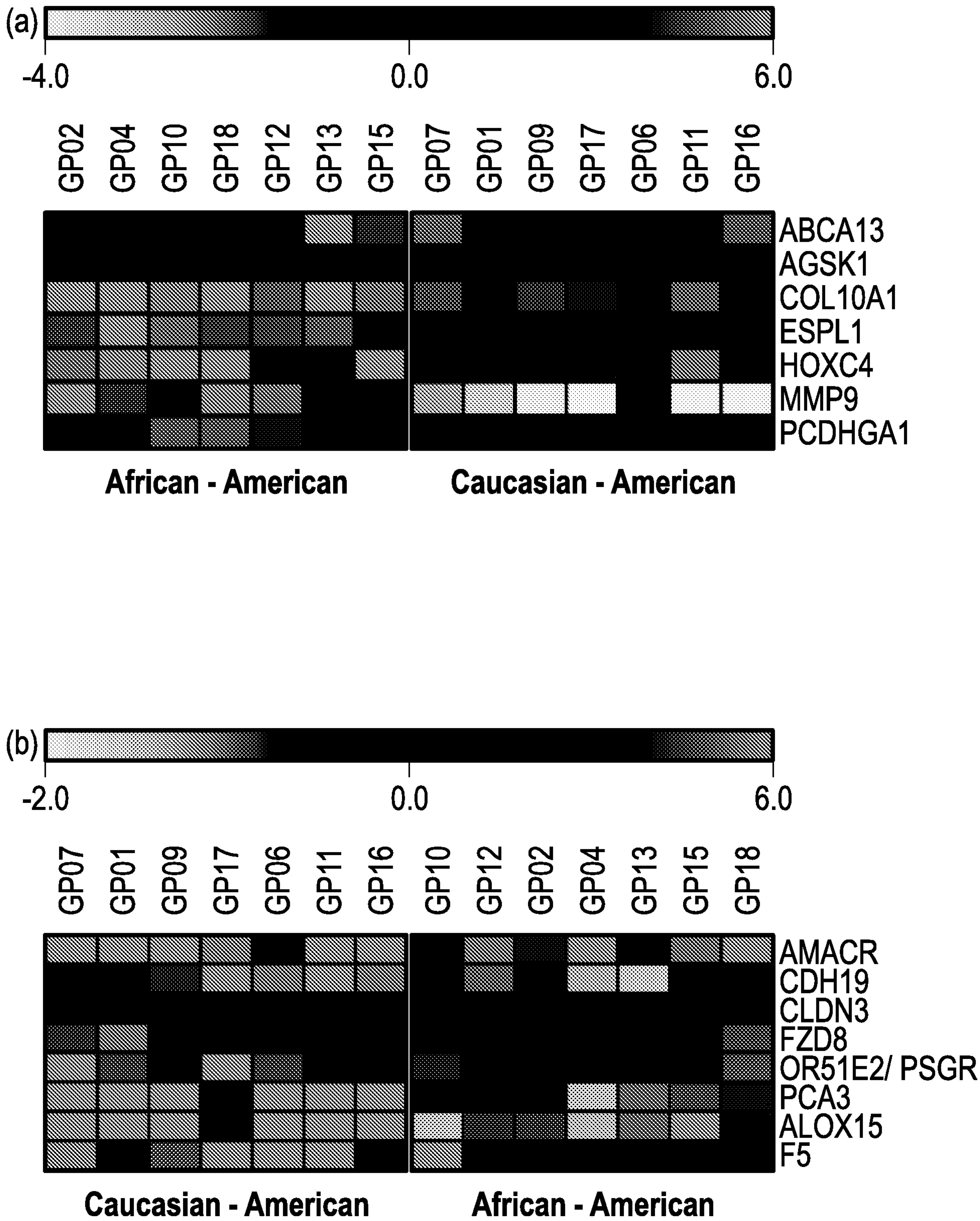
(a)
-4.0
0.0
6.0
GP02 GP04 GP10 GP18 GP12 GP13 GP15 GP07 GP01 GP09 GP17 GP06 GP11 GP16
ABCA13
AGSK1
COL10A1
ESPL1
HOXC4
MMP9
PCDHGA1
African - American
Caucasian - American
(b)
-2.0
0.0
6.0
GP07 GP01 GP09 GP17 GP06 GP11 GP16 GP10 GP12 GP02 GP04 GP13 GP15 GP18
AMACR
CDH19
CLDN3
FZD8
OR51E2/ PSGR
PCA3
ALOX15
F5
Caucasian - American
African - American

FIG. 3

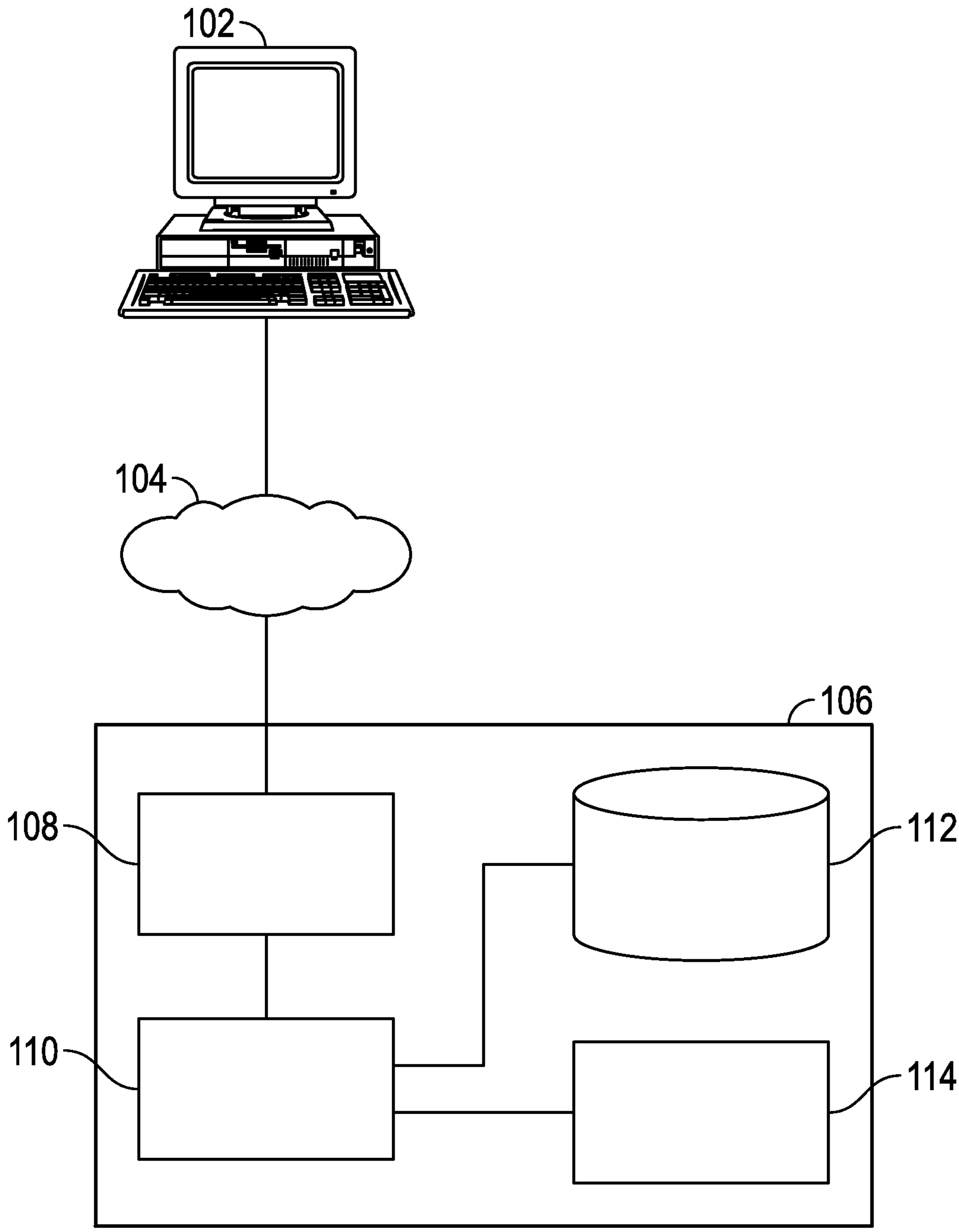


FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61921739
- US 20100112710 A
- US 20100047924 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5458852 A
- US 5480792 A
- WO 2008048970 A
- US 5665539 A
- US 8338579 B
- US 6785613 B
- WO 61921739 A


### Non-patent literature cited in the description


- **JUDITH KIDD et al.** Analyses of a set of 128 ancestry informative single-nucleotide polymorphisms in a global set of 119 population samples. *Investigative Genetics,* 2011, vol. 2, 1
- **SAMBROOK et al.** Molecular Cloning: A Laboratory Manual. Cold Spring Harbor Press, 2012
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **SIEGEL, R. ; NAISHADHAM, D. ; JEMAL, A.** Cancer statistics. *CA Cancer J. Clin.,* 2013, vol. 63, 11-30
- **CHORNOKUR, G. ; DALTON, K. ; BORYSOVA, M.E. ; KUMAR, N.B.** Disparities at presentation, diagnosis, treatment, and survival in African American men affected by prostate cancer. *Prostate,* 2011, vol. 71, 985-997
- **SCHWARTZ, K. ; POWELL, I.J. ; UNDERWOOD, W., 3RD ; GEORGE, J. ; YEE, C. ; BANERJEE, M.** Interplay of race, socioeconomic status, and treatment on survival of patients with prostate cancer. *Urology,* 2009, vol. 74, 1296-1302
- **MAJOR, J.M. ; OLIVER, M.N. ; DOUBENI, C.A. ; HOLLENBECK, A.R. ; GRAUBARD, B.I. ; SINHA, R.** Socioeconomic status, healthcare density, and risk of prostate cancer among African American and Caucasian men in a large prospective study. *Cancer Causes Control,* 2012, vol. 23, 1185-1191
- **SRIDHAR, G. ; MASHO, S.W. ; ADERA, T. ; RAMAKRISHNAN, V. ; ROBERTS, J.D.** Do African American men have lower survival from prostate cancer compared with White men? A meta-analysis. *Am. J Mens. Health,* 2010, vol. 4, 189-206
- **CULLEN, J. ; BRASSELL, S. ; CHEN, Y. ; PORTER, C. ; L'ESPERANCE, J. ; BRAND, T. ; MCLEOD, D.G.** Racial/ethnic patterns in prostate cancer outcomes in an active surveillance cohort. *Prostate Cancer,* 2011, 2011
- **BERGER, A.D. ; SATAGOPAN, J. ; LEE, P. ; TANEJA, S.S. ; OSMAN, I.** Differences in clinicopathologic features of prostate cancer between black and white patients treated in the 1990s and 2000s. *Urology,* 2006, vol. 67, 120-124
- **KHEIRANDISH, P. ; CHINEGWUNDOH, F.** Ethnic differences in prostate cancer. *Br. J. Cancer,* 2011, vol. 105, 481-485
- **ODEDINA, F.T. ; AKINREMI, T.O. ; CHINEGWUNDOH, F. ; ROBERTS, R. ; YU, D. ; REAMS, R.R. ; FREEDMAN, M.L. ; RIVERS, B. ; GREEN, B.L. ; KUMAR, N.** Prostate cancer disparities in black men of African descent: A comparative literature review of prostate cancer burden among black men in the United States, Caribbean, United Kingdom, and West Africa. *Infect, Agents Cancer,* 2009, 4
- **HEATH, E.I. ; KATTAN, M.W. ; POWELL, I.J. ; SAKR, W. ; BRAND, T.C. ; RYBICKI, B.A. ; THOMPSON, I.M. ; ARONSON, W.J. ; TERRIS, M.K. ; KANE, C.J. et al.** The effect of race/ethnicity on the accuracy of the 2001 Partin Tables for predicting pathologic stage of localized prostate cancer. *Urology,* 2008, vol. 71, 151-155

- **MOUL, J.W. ; SESTERHENN, I.A. ; CONNELLY, R.R. ; DOUGLAS, T. ; SRIVASTAVA, S. ; MOSTOFI, F.K. ; MCLEOD, D.G.** Prostate-specific antigen values at the time of prostate cancer diagnosis in African-American men. *JAMA,* 1995, vol. 274, 1277-1281
- **TEWARI, A. ; HORNINGER, W. ; BADANI, K.K. ; HASAN, M. ; COON, S. ; CRAWFORD, E.D. ; GAMITO, E.J. ; WEI, J. ; TAUB, D. ; MONTIE, J. et al.** Racial differences in serum prostate-specific (PSA) doubling time, histopathological variables and long-term PSA recurrence between African-American and white American men undergoing radical prostatectomy for clinically localized prostate cancer. *BJU Int,* 2005, vol. 96, 29-33
- **WALLACE, T.A. ; PRUEITT, R.L. ; YI, M. ; HOWE, T.M. ; GILLESPIE, J.W. ; YFANTIS, H.G. ; STEPHENS, R.M. ; CAPORASO, N.E. ; LOFFREDO, C.A. ; AMBS, S.** Tumor immunobiological differences in prostate cancer between African-American and Caucasian-American men. *Cancer Res.,* 2008, vol. 68, 927-936
- **PRENSNER, J.R. ; RUBIN, M.A. ; WEI, J.T. ; CHINNAIYAN, A.M.** Beyond PSA: The next generation of prostate cancer biomarkers. *Sci. Transl. Med.,* 2012, 4
- **RUBIN, M.A. ; MAHER, C.A. ; CHINNAIYAN, A.M.** Common gene rearrangements in prostate cancer. *J. Clin. Oncol.,* 2011, vol. 29, 3659-3668
- **SREENATH, T.L. ; DOBI, A. ; PETROVICS, G. ; SRIVASTAVA, S.** Oncogenic activation of ERG: A predominant mechanism in prostate cancer. *J. Carcinog.,* 2011, vol. 11, 10-21
- **PETROVICS, G. ; LIU, A. ; SHAHEDUZZAMAN, S. ; FURASATO, B. ; SUN, C. ; CHEN, Y. ; NAU, M. RAVINDRANATH, L. ; CHEN, Y. ; DOBI, A. et al.** Frequent overexpression of ETS-related gene-1 (ERG1) in prostate cancer transcriptome. *Oncogene,* 2005, vol. 24, 3847-3852
- **TOMLINS, S.A. ; RHODES, D.R. ; PERNER, S. ; DHANASEKARAN, S.M. ; MEHRA, R. ; SUN, X.W. ; VARAMBALLY, S. ; CAO, X. ; TCHINDA, J. ; KUEFER, R. et al.** Recurrent fusion of TMPRSS2 and ETS transcription factor genes in prostate cancer. *Science,* 2005, vol. 310, 644-648
- **MAGI-GALLUZZI, C. ; TSUSUKI, T. ; ELSON, P. ; SIMMERMAN, K. ; LAFARQUE, C. ; ESQUEVA, R. ; KLEIN, E. ; RUBIN, M.A. ; ZHOU, M.** TMPRSS2-ERG gene fusion prevalence and class are significantly different in prostate cancer of Caucasian, African-American and Japanese patients. *Prostate,* 2011, vol. 71, 489-497
- **ROSEN, P. ; PFISTER, D. ; YOUNG, D. ; PETROVICS, G. ; CHEN, Y. ; CULLEN, J. ; BOHM, D. ; PERNER, S. ; DOBI, A. ; MCLEOD, D.G. et al.** Differences in frequency of ERG oncoprotein expression between index tumors of Caucasian and African American patients with prostate cancer. *Urology,* 2012, vol. 80, 749-753
- **HU, Y. ; DOBI, A. ; SREENATH, T. ; COOK, C. ; TADASE, A.Y. ; RAVINDRANATH, L. ; CULLEN,J. ; FURUSATO, B. ; CHEN, Y. ; THANQAPAZHAM, R.L. et al.** Delineation of TMPRSS2-ERG splice variants in prostate cancer. *Clin. Cancer Res.,* 2008, vol. 14, 4719-4725
- **GARY K GEISS et al.** Direct multiplexed measurement of gene expression with color-coded probe pairs. *Nature Biotechnology,* 2008, vol. 26, 317-25
- **PAOLO FORTINA ; SAUL SURREY.** Digital mRNA Profiling. *Nature Biotechnology,* 2008, vol. 26, 317-25
- **FARRELL J ; PETROVICS G ; MCLEOD DG ; SRIVASTAVA S.** Genetic and molecular differences in prostate carcinogenesis between African American and Caucasian American men. *International Journal of Molecular Sciences,* 2013, vol. 14 (8), 15510-31
- **RODRIQUEZ-SUAREZ et al.** Urine as a source for clinical proteome analysis: From discovery to clinical application. *Biochimica et Biophysica Acta,* 2013
- **SHI et al.** Antibody-free, targeted mass-spectrometric approach for quantification of proteins at low picogram per milliliter levels in human plasma/serum. *PNAS,* 2012, vol. 109 (38), 15395-15400
- **ELENTIBOBA-JOHNSON ; LIM.** Fusion peptides from oncogenic chimeric proteins as specific biomarkers of cancer. *Mol Cell Proteomics,* 2013, vol. 12, 2714
- **REAMS et al.** Microarray comparison of prostate tumor gene expression in African-American and Caucasian American males: a pilot project. *Infectious Agents and Cancer,* 2009, vol. 4 (I), 3

SEQUENCE LISTING

<110> THE HENRY M. JACKSON FOUNDATION FOR THE ADVANCEMENT OF MILITARY MEDICINE, INC. GENOMATIX

<120> PROSTATE CANCER GENE PROFILES AND METHODS OF USING THE SAME

<130> HMJ-145-PCT

<140>
<141>

<150> 61/921,739
<151> 2013-12-30

<160> 57

<170> PatentIn version 3.5

<210> 1
<211> 255
<212> PRT
<213> Homo sapiens

<400> 1

```
Met Thr Met Thr Thr Met Pro Glu Ser Leu Asn Ser Pro Val Ser Gly
1               5                   10                  15

Lys Ala Val Phe Met Glu Phe Gly Pro Pro Asn Gln Gln Met Ser Pro
            20                  25                  30

Ser Pro Met Ser His Gly His Tyr Ser Met His Cys Leu His Ser Ala
        35                  40                  45

Gly His Ser Gln Pro Asp Gly Ala Tyr Ser Ser Ala Ser Ser Phe Ser
    50                  55                  60

Arg Pro Leu Gly Tyr Pro Tyr Val Asn Ser Val Ser Ser His Ala Ser
65                  70                  75                  80

Ser Pro Tyr Ile Ser Ser Val Gln Ser Tyr Pro Gly Ser Ala Ser Leu
                85                  90                  95

Ala Gln Ser Arg Leu Glu Asp Pro Gly Ala Asp Ser Glu Lys Ser Thr
            100                 105                 110

Val Val Glu Gly Gly Glu Val Arg Phe Asn Gly Lys Gly Lys Lys Ile
        115                 120                 125

Arg Lys Pro Arg Thr Ile Tyr Ser Ser Leu Gln Leu Gln Ala Leu Asn
    130                 135                 140

Arg Arg Phe Gln Gln Thr Gln Tyr Leu Ala Leu Pro Glu Arg Ala Glu
```

```
145                 150                 155                 160

Leu Ala Ala Ser Leu Gly Leu Thr Gln Thr Gln Val Lys Ile Trp Phe
                165                 170                 175

Gln Asn Lys Arg Ser Lys Phe Lys Lys Leu Met Lys Gln Gly Gly Ala
            180                 185                 190

Ala Leu Glu Gly Ser Ala Leu Ala Asn Gly Arg Ala Leu Ser Ala Gly
        195                 200                 205

Ser Pro Pro Val Pro Pro Gly Trp Asn Pro Asn Ser Ser Ser Gly Lys
    210                 215                 220

Gly Ser Gly Gly Asn Ala Gly Ser Tyr Ile Pro Ser Tyr Thr Ser Trp
225                 230                 235                 240

Tyr Pro Ser Ala His Gln Glu Ala Met Gln Gln Pro Gln Leu Met
                245                 250                 255
```

<210> 2
<211> 2403
<212> DNA
<213> Homo sapiens

<400> 2

```
aagctttgaa ccgagtttgg ggagctcagc agcatcatgc ttagactttt caaagagaca      60
aactccattt tcttatgaat ggaaagtgaa aacccctgtt ccgcttaaat tgggttcctt     120
cctgtcctga gaaacataga gacccccaaa agggaagcag aggagagaaa gtcccacacc     180
cagaccccgc gagaagagat gaccatgacc accatgccag aaagtctcaa cagccccgtg     240
tcgggcaagg cggtgtttat ggagtttggg ccgcccaacc agcaaatgtc tccttctccc     300
atgtcccacg ggcactactc catgcactgt ttacactcgg cgggccattc gcagcccgac     360
ggcgcctaca gctcagcctc gtccttctcc cgaccgctgg gctaccccta cgtcaactcg     420
gtcagcagcc acgcatccag cccctacatc agttcggtgc agtcctaccc gggcagcgcc     480
agcctcgccc agagccgcct ggaggaccca ggggcggact cggagaagag cacggtggtg     540
gaaggcggtg aagtgcgctt caatggcaag ggaaaaaaga tccgtaaacc caggacgatt     600
tattccagtt tgcagttgca ggctttgaac cggaggttcc agcaaactca gtacctagct     660
ctgccggaga gggcggagct cgcggcctct ttgggactca cacagactca ggtcaagatc     720
tggttccaaa acaagcgatc caagttcaag aagctgatga agcagggtgg ggcggctctg     780
gagggtagtg cgttggccaa cggtcgggcc ctgtctgctg gctccccacc cgtgccgccc     840
ggctggaacc ctaactcttc atccgggaag ggctcaggag gaaacgcggg ctcctatatc     900
```

```
cccagctaca catcgtggta cccttcagcg caccaagaag ctatgcagca accccaactt      960
atgtgaggtt gcccgcccgt ctccttcttg tctccccggc ccaggtccct cccgcctcca     1020
ggtccatcca tcccgtccgg aaaagaagga cccagaggga agaaggaaca gtggaggcgg     1080
gacgccctcc atctcctcgg agccccgcga ggtccggccc agcaacttcc cggcatccgc     1140
gctctagcct gaaccctggc ctgggccgag cagtggcagc agagagtggc ctcggaggga     1200
agccactgcc acctgagaca gcccaagcag caagataaac ccgctccacc cgacccgccg     1260
accttcagct ttgtgggact atcaggaaaa aacaaaacaa aaacaaaatg tagaaaaagc     1320
aaaagctctt ttctgtcctg tcagtctcct gtctcctttt gctctgtctg tgcgctggta     1380
aagtccaggt cctcatccgt ccgctgtcct cattctgcgg cctcagcaaa aagccacaag     1440
gtctgagcgg cccgggtcct gccgggctga ccatctccgg atcctgggac actctgcctg     1500
accatctgtg tagctggtgt gggaatctgg gggcattgga gggagggggt tttatttatt     1560
gagaaatgga cttcgcctga ggctgtttgc caattcaggg ttctgctggg cgcaaggaac     1620
gcactgttca aacgcactgt ttactttaag cgcacgggga gaaacgaata aggaggacgt     1680
ggtgattttt aatttataca gtaacttttg tacttctctg gtatggagag tttggagccg     1740
aatgatttgc atttttttaca tgtccgacat tatttaataa ataattttta aaagaaaaga     1800
acgataaatg aagccaacat gattttctca tttcgggagg aactctgttg cttcgcctgg     1860
acaagaagga aaatgctgat ttcctccttg ggtagaaaga gggagcgagg gcaaatgggg     1920
agtagagaga aaacaggcga gaacaagcac tctaattcca gtgggcttta aaataagaca     1980
aaatcagctt tacaacaatc cctagaggct cgaccacaga ataatgccag tcaccaccct     2040
gaacgcacaa tctccagtgc aggatctaat gactgtacat attattgtta ttattattat     2100
tgttattatt gttgttctgt aaacatgttg cacaagctta gcctttttgc gttctgttgt     2160
gtgtggctgt aaaaccccat gctttgtgaa atgagaatct tgacattttt cttgtgaaat     2220
ttggaaaatg tgatcaattg aaatcaactg tgttttgtgt tctctatgtc aaagtttagt     2280
tttatattga gaatgttaac ttattgcttt gtatcttggg aaaaaaactt tgtaaataag     2340
ttataaagtt tctttgagac agtaaaatta tgatttcttg aaaaaaaaaa aaaaaaaaaa     2400
aaa                                                                   2403
```

<210> 3
<211> 364
<212> PRT
<213> Homo sapiens

<400> 3

```
Met Met Leu Pro Ser Pro Val Thr Ser Thr Pro Phe Ser Val Lys Asp
1               5                   10                  15
```

Ile Leu Asn Leu Glu Gln Gln His Gln His Phe His Gly Ala His Leu
20 25 30

Gln Ala Asp Leu Glu His His Phe His Ser Ala Pro Cys Met Leu Ala
35 40 45

Ala Ala Glu Gly Thr Gln Phe Ser Asp Gly Gly Glu Glu Asp Glu Glu
50 55 60

Asp Glu Gly Glu Lys Leu Ser Tyr Leu Asn Ser Leu Ala Ala Ala Asp
65 70 75 80

Gly His Gly Asp Ser Gly Leu Cys Pro Gln Gly Tyr Val His Thr Val
85 90 95

Leu Arg Asp Ser Cys Ser Glu Pro Lys Glu His Glu Glu Glu Pro Glu
100 105 110

Val Val Arg Asp Arg Ser Gln Lys Ser Cys Gln Leu Lys Lys Ser Leu
115 120 125

Glu Thr Ala Gly Asp Cys Lys Ala Ala Glu Glu Ser Glu Arg Pro Lys
130 135 140

Pro Arg Ser Arg Arg Lys Pro Arg Val Leu Phe Ser Gln Ala Gln Val
145 150 155 160

Phe Glu Leu Glu Arg Arg Phe Lys Gln Gln Arg Tyr Leu Ser Ala Pro
165 170 175

Glu Arg Glu His Leu Ala Ser Ser Leu Lys Leu Thr Ser Thr Gln Val
180 185 190

Lys Ile Trp Phe Gln Asn Arg Arg Tyr Lys Cys Lys Arg Gln Arg Gln
195 200 205

Asp Lys Ser Leu Glu Leu Gly Ala His Ala Pro Pro Pro Pro Pro Arg
210 215 220

Arg Val Ala Val Pro Val Leu Val Arg Asp Gly Lys Pro Cys Val Thr
225 230 235 240

Pro Ser Ala Gln Ala Tyr Gly Ala Pro Tyr Ser Val Gly Ala Ser Ala
245 250 255

Tyr Ser Tyr Asn Ser Phe Pro Ala Tyr Gly Tyr Gly Asn Ser Ala Ala
260 265 270

```
Ala Ala Ala Ala Ala Ala Ala Ala Ala Ala Ala Ala Ala Ala Tyr Ser
        275                 280                 285

Ser Ser Tyr Gly Cys Ala Tyr Pro Ala Gly Gly Gly Gly Gly Gly Gly
    290                 295                 300

Gly Thr Ser Ala Ala Thr Thr Ala Met Gln Pro Ala Cys Ser Ala Ala
305                 310                 315                 320

Gly Gly Gly Pro Phe Val Asn Val Ser Asn Leu Gly Gly Phe Gly Ser
                325                 330                 335

Gly Gly Ser Ala Gln Pro Leu His Gln Gly Thr Ala Ala Gly Ala Ala
            340                 345                 350

Cys Ala Gln Gly Thr Leu Gln Gly Ile Arg Ala Trp
        355                 360
```

<210> 4
<211> 2117
<212> DNA
<213> Homo sapiens

<400> 4

```
agtccctgca gtggctgtaa caaaacccag acccccaggt cccggccaat ggaggcgatt        60
tagactggag tgggaccgcg tctgtcaaaa gcccgactcg gcagcagcgg cggagtccag       120
gaggagagct ggagccgccg cgctgcctcc ccgcccccgc cgggatttat tatttggact       180
ggacaattaa gtggccctga tgatgttacc aagcccggtc acctccaccc ctttctcagt       240
caaagacatt ttgaatctgg agcagcagca ccagcacttc catggtgcgc acttgcaggc       300
ggacttggag caccacttcc actctgcgcc ctgcatgctg gccgccgctg aggggacgca       360
attttctgac ggaggggagg aggacgagga agacgagggc gagaaattgt cctatttgaa       420
ctcactagcc gcagcagacg gccacgggga ttcagggctg tgtccccagg gctatgtcca       480
cacggtcctg cgagactcgt gcagcgagcc caaggaacat gaagaggagc ccgaggtcgt       540
gagggaccgg agccaaaaaa gctgccagct gaagaagtct ctagagacgg ccggagactg       600
caaggcggcg gaggagagcg agaggccgaa gccacgcagc cgccggaagc cccgggtcct       660
cttctcgcaa gcccaggtct tcgagctgga acgcaggttc aagcagcagc ggtacctgtc       720
ggcacccgag cgcgagcacc tcgccagcag cctgaagctc acatccactc aggtgaaaat       780
ctggttccag aatcgcaggt acaagtgcaa gagacagcgg caggacaagt ctctggagct       840
tggcgcacac gcgcccccgc cgccgccgcg ccgcgtggct gtcccggtgc tggtgcggga       900
cggcaagccg tgcgtcacgc ccagcgcgca ggcctacggc gcgccctaca gcgtgggcgc       960
```

```
cagcgcctac tcctacaaca gcttccccgc ctacggctat gggaactcgg ccgcggccgc      1020
cgccgccgcc gccgccgccg ccgcagcagc ggcggcctac agcagcagct atggctgtgc      1080
gtacccggcg ggcggcggcg gcggcggcgg cgggacctcc gcggcgacca ctgccatgca      1140
gcccgcctgc agcgcggccg gaggcggccc ctttgtgaac gtgagcaacc taggaggctt      1200
cggcagcggc ggcagcgcac agccgttgca ccagggtact gcagccgggg ccgcgtgcgc      1260
tcagggcacc ttgcagggca tccgggcctg gtagggacgg ggcgggtcac gcggcgggca      1320
ccccagcgca gcctggcgcc gcgggactga agctcgagaa gggcctgacc taaaggtcag      1380
gtcccctcgt taaaaaaata tgtacgtcta gctcctcagg gcttcggatc gcagctcact      1440
cgaggcctgg ggaaggggac tcaggggcga ggaggatgac tgggtccggt cgccaggact      1500
gtctctgagg cagaaacgcc ggctgggcgc cggggaggac gatggccccg accctggcag      1560
cgagaggaga ccaggaggct aggaccctgg ccgcgcttgg ttcttccaaa gcgagaaggg      1620
cttctctccc tctgcctttc cgcggcctcc gcgaagcgtt ggcggggagc ccaaggacat      1680
aacaaattaa aagcatgaag gagagaaaaa tgggggtcgt ggcttgagaa attccaggcc      1740
ctaccgatcc tctgccccct ttgcgggcct ggagcgccat agcacagtcg atttcgtttc      1800
gcagctgtct cccctccgca gcagatacct cggtccagat ctccggattg tcgggggacg      1860
caggactctt cgaggaaaac cagccgaatg agatcaaaag ttgggggtgg ggggaggctg      1920
aacaaactca ggacctggtg gcccaccgga ggtgttaccg ggtttccttt ctgtttcgta      1980
ttctgtattc agcacatgtt atctatctat ctatctatat aactataacc acacgccgtg      2040
tagacacccg ctgccacaca ctacaggagt caataaacaa ggtgcaatat tttcaaaaaa      2100
aaaaaaaaaa aaaaaaa                                                     2117
```

<210> 5
<211> 258
<212> PRT
<213> Homo sapiens

<400> 5

```
Met Lys Gln Ile Leu His Pro Ala Leu Glu Thr Thr Ala Met Thr Leu
1               5                   10                  15

Phe Pro Val Leu Leu Phe Leu Val Ala Gly Leu Leu Pro Ser Phe Pro
            20                  25                  30

Ala Asn Glu Asp Lys Asp Pro Ala Phe Thr Ala Leu Leu Thr Thr Gln
        35                  40                  45

Thr Gln Val Gln Arg Glu Ile Val Asn Lys His Asn Glu Leu Arg Arg
    50                  55                  60
```

```
Ala Val Ser Pro Pro Ala Arg Asn Met Leu Lys Met Glu Trp Asn Lys
65                  70                  75                  80

Glu Ala Ala Ala Asn Ala Gln Lys Trp Ala Asn Gln Cys Asn Tyr Arg
                85                  90                  95

His Ser Asn Pro Lys Asp Arg Met Thr Ser Leu Lys Cys Gly Glu Asn
            100                 105                 110

Leu Tyr Met Ser Ser Ala Ser Ser Ser Trp Ser Gln Ala Ile Gln Ser
        115                 120                 125

Trp Phe Asp Glu Tyr Asn Asp Phe Asp Phe Gly Val Gly Pro Lys Thr
    130                 135                 140

Pro Asn Ala Val Val Gly His Tyr Thr Gln Val Val Trp Tyr Ser Ser
145                 150                 155                 160

Tyr Leu Val Gly Cys Gly Asn Ala Tyr Cys Pro Asn Gln Lys Val Leu
                165                 170                 175

Lys Tyr Tyr Tyr Val Cys Gln Tyr Cys Pro Ala Gly Asn Trp Ala Asn
            180                 185                 190

Arg Leu Tyr Val Pro Tyr Glu Gln Gly Ala Pro Cys Ala Ser Cys Pro
        195                 200                 205

Asp Asn Cys Asp Asp Gly Leu Cys Thr Asn Gly Cys Lys Tyr Glu Asp
    210                 215                 220

Leu Tyr Ser Asn Cys Lys Ser Leu Lys Leu Thr Leu Thr Cys Lys His
225                 230                 235                 240

Gln Leu Val Arg Asp Ser Cys Lys Ala Ser Cys Asn Cys Ser Asn Ser
                245                 250                 255

Ile Tyr
```

<210> 6
<211> 2219
<212> DNA
<213> Homo sapiens

<400> 6

```
gcacaaccag aatttgccaa aacaggaaat aggtgtttca tatatacggc tctaaccttc        60
tctctctgca ccttccttct gtcaatagat gaaacaaata cttcatcctg ctctggaaac       120
cactgcaatg acattattcc cagtgctgtt gttcctggtt gctgggctgc ttccatcttt       180
```

tccagcaaat gaagataagg atcccgcttt tactgctttg ttaaccaccc aaacacaagt 240
gcaaagggag attgtgaata agcacaatga actgaggaga gcagtatctc cccctgccag 300
aaacatgctg aagatggaat ggaacaaaga ggctgcagca aatgcccaaa agtgggcaaa 360
ccagtgcaat tacagacaca gtaacccaaa ggatcgaatg acaagtctaa aatgtggtga 420
gaatctctac atgtcaagtg cctccagctc atggtcacaa gcaatccaaa gctggtttga 480
tgagtacaat gattttgact ttggtgtagg gccaaagact cccaacgcag tggttggaca 540
ttatacacag gttgtttggt actcttcata cctcgttgga tgtggaaatg cctactgtcc 600
caatcaaaaa gttctaaaat actactatgt ttgccaatat tgtcctgctg gtaattgggc 660
taatagacta tatgtccctt atgaacaagg agcaccttgt gccagttgcc cagataactg 720
tgacgatgga ctatgcacca atggttgcaa gtacgaagat ctctatagta actgtaaaag 780
tttgaagctc acattaacct gtaaacatca gttggtcagg gacagttgca aggcctcctg 840
caattgttca aacagcattt attaaatacg cattacacac cgagtagggc tatgtagaga 900
ggagtcagat tatctactta gatttggcat ctacttagat ttaacatata ctagctgaga 960
aattgtaggc atgtttgata cacatttgat ttcaaatgtt tttcttctgg atctgctttt 1020
tattttacaa aaatattttt catacaaatg gttaaaaaga aacaaaatct ataacaacaa 1080
ctttggattt ttatatataa actttgtgat ttaaatttac tgaatttaat tagggtgaaa 1140
attttgaaag ttgtattctc atatgactaa gttcactaaa accctggatt gaaagtgaaa 1200
attatgttcc tagaacaaaa tgtacaaaaa gaacaatata attttcacat gaacccttgg 1260
ctgtagttgc ctttcctagc tccactctaa ggctaagcat cttcaaagac gttttcccat 1320
atgctgtctt aattcttttc actcattcac ccttcttccc aatcatctgg ctggcatcct 1380
cacaattgag ttgaagctgt tcctcctaaa acaatcctga cttttatttt gccaaaatca 1440
atacaatcct ttgaattttt tatctgcata aattttacag tagaatatga tcaaaccttc 1500
atttttaaac ctctcttctc tttgacaaaa cttccttaaa aaagaataca agataatata 1560
ggtaaatacc ctccactcaa ggaggtagaa ctcagtcctc tcccttgtga gtcttcacta 1620
aaatcagtga ctcacttcca aagagtggag tatggaaagg gaaacatagt aactttacag 1680
gggagaaaaa tgacaaatga cgtcttcacc aagtgatcaa aattaacgtc accagtgata 1740
agtcattcag atttgttcta gataatcttt ctaaaaattc ataatcccaa tctaattatg 1800
agctaaaaca tccagcaaac tcaagttgaa ggacattcta caaaatatcc ctggggtatt 1860
ttagagtatt cctcaaaact gtaaaaatca tggaaaataa gggaatcctg agaaacaatc 1920
acagaccaca tgagactaag gagacatgtg agccaaatgc aatgtgcttc ttggatcaga 1980
tcctggaaca gaaaaagatc agtaatgaaa aaactgatga agtctgaata gaatctggag 2040

tatttttaac agtagtgttg atttcttaat cttgataaat atagcagggt aatgtaagat 2100

gataacgtta gagaaactga aactgggtga gggctatcta ggaattctct gtactatctt 2160

accaaatttt cggtaagtct aagaaagcaa tgcaaaataa aaagtgtctt gaaaaaaa 2219

<210> 7
<211> 82
<212> PRT
<213> Homo sapiens

<400> 7

Met Asp Pro Gly Pro Lys Gly His Cys His Cys Gly Gly His Gly His
1 5 10 15

Pro Pro Gly His Cys Gly Pro Pro Pro Gly His Gly Pro Gly Pro Cys
20 25 30

Gly Pro Pro Pro His His Gly Pro Gly Pro Cys Gly Pro Pro Pro Gly
35 40 45

His Gly Pro Gly Pro Cys Gly Pro Pro Pro His His Gly Pro Gly Pro
50 55 60

Cys Gly Pro Pro Pro Gly His Gly Pro Gly His Pro Pro Pro Gly Pro
65 70 75 80

His His

<210> 8
<211> 464
<212> DNA
<213> Homo sapiens

<400> 8

agacttcctg cccctgctct gcactctcag gtattccctg ctcttactcc aaaaagatgg 60

acccaggtcc gaaggggcac tgccactgtg gggggcatgg ccatcctcca ggtcactgcg 120

ggccaccccc tggccatggc ccagggccct gcgggccacc cccccaccat ggtccagggc 180

cctgcgggcc acccccctggc catggcccag ggccctgcgg gccacccccc caccatggtc 240

cagggccctg cgggcctccc cctggccatg gcccaggtca cccacccccct ggtccacatc 300

actgaggaag tagaagaaaa caggacacaa gatggcaagc ctgagagaat tgcccagctg 360

acctggaatg aggcctaaac cacaatcttc tcttcctaat aaacagcctc ctagaggcca 420

cattctattc tttaaaaaaa aaaaaaaaaa aaaaaaaaaa aaaa 464

<210> 9

<211> 961
<212> PRT
<213> Homo sapiens

<400> 9

```
Met Leu Ala Pro Arg Gly Ala Ala Val Leu Leu Leu His Leu Val Leu
1               5                   10                  15

Gln Arg Trp Leu Ala Ala Gly Ala Gln Ala Thr Pro Gln Val Phe Asp
            20                  25                  30

Leu Leu Pro Ser Ser Ser Gln Arg Leu Asn Pro Gly Ala Leu Leu Pro
        35                  40                  45

Val Leu Thr Asp Pro Ala Leu Asn Asp Leu Tyr Val Ile Ser Thr Phe
    50                  55                  60

Lys Leu Gln Thr Lys Ser Ser Ala Thr Ile Phe Gly Leu Tyr Ser Ser
65                  70                  75                  80

Thr Asp Asn Ser Lys Tyr Phe Glu Phe Thr Val Met Gly Arg Leu Asn
                85                  90                  95

Lys Ala Ile Leu Arg Tyr Leu Lys Asn Asp Gly Lys Val His Leu Val
            100                 105                 110

Val Phe Asn Asn Leu Gln Leu Ala Asp Gly Arg Arg His Arg Ile Leu
        115                 120                 125

Leu Arg Leu Ser Asn Leu Gln Arg Gly Ala Gly Ser Leu Glu Leu Tyr
    130                 135                 140

Leu Asp Cys Ile Gln Val Asp Ser Val His Asn Leu Pro Arg Ala Phe
145                 150                 155                 160

Ala Gly Pro Ser Gln Lys Pro Glu Thr Ile Glu Leu Arg Thr Phe Gln
                165                 170                 175

Arg Lys Pro Gln Asp Phe Leu Glu Glu Leu Lys Leu Val Val Arg Gly
            180                 185                 190

Ser Leu Phe Gln Val Ala Ser Leu Gln Asp Cys Phe Leu Gln Gln Ser
        195                 200                 205

Glu Pro Leu Ala Ala Thr Gly Thr Gly Asp Phe Asn Arg Gln Phe Leu
    210                 215                 220

Gly Gln Met Thr Gln Leu Asn Gln Leu Leu Gly Glu Val Lys Asp Leu
225                 230                 235                 240
```

```
Leu Arg Gln Gln Val Lys Glu Thr Ser Phe Leu Arg Asn Thr Ile Ala
                245                 250                 255

Glu Cys Gln Ala Cys Gly Pro Leu Lys Phe Gln Ser Pro Thr Pro Ser
            260                 265                 270

Thr Val Val Pro Pro Ala Pro Pro Ala Pro Pro Thr Arg Pro Pro Arg
        275                 280                 285

Arg Cys Asp Ser Asn Pro Cys Phe Arg Gly Val Gln Cys Thr Asp Ser
    290                 295                 300

Arg Asp Gly Phe Gln Cys Gly Pro Cys Pro Glu Gly Tyr Thr Gly Asn
305                 310                 315                 320

Gly Ile Thr Cys Ile Asp Val Asp Glu Cys Lys Tyr His Pro Cys Tyr
                325                 330                 335

Pro Gly Val His Cys Ile Asn Leu Ser Pro Gly Phe Arg Cys Asp Ala
            340                 345                 350

Cys Pro Val Gly Phe Thr Gly Pro Met Val Gln Gly Val Gly Ile Ser
        355                 360                 365

Phe Ala Lys Ser Asn Lys Gln Val Cys Thr Asp Ile Asp Glu Cys Arg
    370                 375                 380

Asn Gly Ala Cys Val Pro Asn Ser Ile Cys Val Asn Thr Leu Gly Ser
385                 390                 395                 400

Tyr Arg Cys Gly Pro Cys Lys Pro Gly Tyr Thr Gly Asp Gln Ile Arg
                405                 410                 415

Gly Cys Lys Ala Glu Arg Asn Cys Arg Asn Pro Glu Leu Asn Pro Cys
            420                 425                 430

Ser Val Asn Ala Gln Cys Ile Glu Glu Arg Gln Gly Asp Val Thr Cys
        435                 440                 445

Val Cys Gly Val Gly Trp Ala Gly Asp Gly Tyr Ile Cys Gly Lys Asp
    450                 455                 460

Val Asp Ile Asp Ser Tyr Pro Asp Glu Glu Leu Pro Cys Ser Ala Arg
465                 470                 475                 480

Asn Cys Lys Lys Asp Asn Cys Lys Tyr Val Pro Asn Ser Gly Gln Glu
                485                 490                 495
```

```
Asp Ala Asp Arg Asp Gly Ile Gly Asp Ala Cys Asp Glu Asp Ala Asp
            500                 505                 510

Gly Asp Gly Ile Leu Asn Glu Gln Asp Asn Cys Val Leu Ile His Asn
        515                 520                 525

Val Asp Gln Arg Asn Ser Asp Lys Asp Ile Phe Gly Asp Ala Cys Asp
    530                 535                 540

Asn Cys Leu Ser Val Leu Asn Asn Asp Gln Lys Asp Thr Asp Gly Asp
545                 550                 555                 560

Gly Arg Gly Asp Ala Cys Asp Asp Asp Met Asp Gly Asp Gly Ile Lys
                565                 570                 575

Asn Ile Leu Asp Asn Cys Pro Lys Phe Pro Asn Arg Asp Gln Arg Asp
            580                 585                 590

Lys Asp Gly Asp Gly Val Gly Asp Ala Cys Asp Ser Cys Pro Asp Val
        595                 600                 605

Ser Asn Pro Asn Gln Ser Asp Val Asp Asn Asp Leu Val Gly Asp Ser
    610                 615                 620

Cys Asp Thr Asn Gln Asp Ser Asp Gly Asp Gly His Gln Asp Ser Thr
625                 630                 635                 640

Asp Asn Cys Pro Thr Val Ile Asn Ser Ala Gln Leu Asp Thr Asp Lys
                645                 650                 655

Asp Gly Ile Gly Asp Glu Cys Asp Asp Asp Asp Asp Asn Asp Gly Ile
            660                 665                 670

Pro Asp Leu Val Pro Pro Gly Pro Asp Asn Cys Arg Leu Val Pro Asn
        675                 680                 685

Pro Ala Gln Glu Asp Ser Asn Ser Asp Gly Val Gly Asp Ile Cys Glu
    690                 695                 700

Ser Asp Phe Asp Gln Asp Gln Val Ile Asp Arg Ile Asp Val Cys Pro
705                 710                 715                 720

Glu Asn Ala Glu Val Thr Leu Thr Asp Phe Arg Ala Tyr Gln Thr Val
                725                 730                 735

Val Leu Asp Pro Glu Gly Asp Ala Gln Ile Asp Pro Asn Trp Val Val
            740                 745                 750
```

Leu Asn Gln Gly Met Glu Ile Val Gln Thr Met Asn Ser Asp Pro Gly
755 760 765

Leu Ala Val Gly Tyr Thr Ala Phe Asn Gly Val Asp Phe Glu Gly Thr
770 775 780

Phe His Val Asn Thr Gln Thr Asp Asp Asp Tyr Ala Gly Phe Ile Phe
785 790 795 800

Gly Tyr Gln Asp Ser Ser Ser Phe Tyr Val Val Met Trp Lys Gln Thr
805 810 815

Glu Gln Thr Tyr Trp Gln Ala Thr Pro Phe Arg Ala Val Ala Glu Pro
820 825 830

Gly Ile Gln Leu Lys Ala Val Lys Ser Lys Thr Gly Pro Gly Glu His
835 840 845

Leu Arg Asn Ser Leu Trp His Thr Gly Asp Thr Ser Asp Gln Val Arg
850 855 860

Leu Leu Trp Lys Asp Ser Arg Asn Val Gly Trp Lys Asp Lys Val Ser
865 870 875 880

Tyr Arg Trp Phe Leu Gln His Arg Pro Gln Val Gly Tyr Ile Arg Val
885 890 895

Arg Phe Tyr Glu Gly Ser Glu Leu Val Ala Asp Ser Gly Val Thr Ile
900 905 910

Asp Thr Thr Met Arg Gly Gly Arg Leu Gly Val Phe Cys Phe Ser Gln
915 920 925

Glu Asn Ile Ile Trp Ser Asn Leu Lys Tyr Arg Cys Asn Asp Thr Ile
930 935 940

Pro Glu Asp Phe Gln Glu Phe Gln Thr Gln Asn Phe Asp Arg Phe Asp
945 950 955 960

Asn

<210> 10
<211> 3233
<212> DNA
<213> Homo sapiens

<400> 10

cagcagccag ctccccagca ccgcacggcg gggacgcgag cgcgccccg acggcagccc 60
ggacgccgag cacgggtcac ctgcggcgcc ggcccgggcg ccgaccgagg ttcaacgcac 120
ggcccgggga cccccaggcg gggccaacgc cgccgtcgcc cccggcctcg cggggagcag 180
gaagagccaa catgctggcc ccgcgcggag ccgccgtcct cctgctgcac ctggtcctgc 240
agcggtggct agcggcaggc gcccaggcca ccccccaggt ctttgacctt ctcccatctt 300
ccagtcagag gctaaaccca ggcgctctgc tgccagtcct gacagacccc gccctgaatg 360
atctctatgt gatttccacc ttcaagctgc agactaaaag ttcagccacc atcttcggtc 420
tttactcttc aactgacaac agtaaatatt ttgaatttac tgtgatggga cgcttaaaca 480
aagccatcct ccgttacctg aagaacgatg ggaaggtgca tttggtggtt ttcaacaacc 540
tgcagctggc agacggaagg cggcacagga tcctcctgag gctgagcaat ttgcagcgag 600
gggccggctc cctagagctc tacctggact gcatccaggt ggattccgtt cacaatctcc 660
ccagggcctt tgctggcccc tcccagaaac ctgagaccat tgaattgagg actttccaga 720
ggaagccaca ggacttcttg gaagagctga agctggtggt gagaggctca ctgttccagg 780
tggccagcct gcaagactgc ttcctgcagc agagtgagcc actggctgcc acaggcacag 840
gggactttaa ccggcagttc ttgggtcaaa tgacacaatt aaaccaactc ctgggagagg 900
tgaaggacct tctgagacag caggttaagg aaacatcatt tttgcgaaac accatagctg 960
aatgccaggc ttgcggtcct ctcaagtttc agtctccgac cccaagcacg gtggtgcccc 1020
cggctccccc tgcaccgcca acacgcccac ctcgtcggtg tgactccaac ccatgtttcc 1080
gaggtgtcca atgtaccgac agtagagatg gcttccagtg tgggccctgc cccgagggct 1140
acacaggaaa cgggatcacc tgtattgatg ttgatgagtg caaataccat ccctgctacc 1200
cgggcgtgca ctgcataaat ttgtctcctg gcttcagatg tgacgcctgc ccagtgggct 1260
tcacagggcc catggtgcag ggtgttggga tcagttttgc caagtcaaac aagcaggtct 1320
gcactgacat tgatgagtgt cgaaatggag cgtgcgttcc caactcgatc tgcgttaata 1380
ctttgggatc ttaccgctgt gggccttgta agccggggta tactggtgat cagataaggg 1440
gatgcaaagc ggaaagaaac tgcagaaacc cagagctgaa cccttgcagt gtgaatgccc 1500
agtgcattga agagaggcag gggatgtga catgtgtgtg tggagtcggt tgggctggag 1560
atggctatat ctgtggaaag gatgtggaca tcgacagtta ccccgacgaa gaactgccat 1620
gctctgccag gaactgtaaa aaggacaact gcaaatatgt gccaaattct ggccaagaag 1680
atgcagacag agatggcatt ggcgacgctt gtgacgagga tgctgacgga gatgggatcc 1740
tgaatgagca ggataactgt gtcctgattc ataatgtgga ccaaaggaac agcgataaag 1800
atatctttgg ggatgcctgt gataactgcc tgagtgtctt aaataacgac cagaaagaca 1860

ccgatgggga tggaagagga gatgcctgtg atgatgacat ggatggagat ggaataaaaa 1920
acattctgga caactgccca aaatttccca atcgtgacca acgggacaag gatggtgatg 1980
gtgtggggga tgcctgtgac agttgtcctg atgtcagcaa ccctaaccag tctgatgtgg 2040
ataatgatct ggttggggac tcctgtgaca ccaatcagga cagtgatgga gatgggcacc 2100
aggacagcac agacaactgc cccaccgtca ttaacagtgc ccagctggac accgataagg 2160
atggaattgg tgacgagtgt gatgatgatg atgacaatga tggtatccca gacctggtgc 2220
cccctggacc agacaactgc cggctggtcc ccaacccagc ccaggaggat agcaacagcg 2280
acggagtggg agacatctgt gagtctgact ttgaccagga ccaggtcatc gatcggatcg 2340
acgtctgccc agagaacgca gaggtcaccc tgaccgactt cagggcttac cagaccgtgg 2400
tcctggatcc tgaaggggat gcccagatcg atcccaactg ggtggtcctg aaccagggca 2460
tggagattgt acagaccatg aacagtgatc ctggcctggc agtggggtac acagctttta 2520
atggagttga cttcgaaggg accttccatg tgaataccca gacagatgat gactatgcag 2580
gctttatctt tggctaccaa gatagctcca gcttctacgt ggtcatgtgg aagcagacgg 2640
agcagacata ttggcaagcc accccattcc gagcagttgc agaacctggc attcagctca 2700
aggctgtgaa gtctaagaca ggtccagggg agcatctccg gaactccctg tggcacacgg 2760
gggacaccag tgaccaggtc aggctgctgt ggaaggactc caggaatgtg ggctggaagg 2820
acaaggtgtc ctaccgctgg ttcctacagc acaggcccca ggtgggctac atcagggtac 2880
gattttatga aggctctgag ttggtggctg actctggcgt caccatagac accacaatgc 2940
gtggaggccg acttggcgtt ttctgcttct ctcaagaaaa catcatctgg tccaacctca 3000
agtatcgctg caatgacacc atccctgagg acttccaaga gtttcaaacc cagaatttcg 3060
accgcttcga taattaaacc aaggaagcaa tctgtaactg cttttcggaa cactaaaacc 3120
atatatattt taacttcaat tttctttagc ttttaccaac ccaaatatat caaaacgttt 3180
tatgtgaatg tggcaataaa ggagaagaga tcatttttaa aaaaaaaaaa aaa 3233

<210> 11
<211> 382
<212> PRT
<213> Homo sapiens

<400> 11

```
Met Ala Leu Gln Gly Ile Ser Val Val Glu Leu Ser Gly Leu Ala Pro
1               5                   10                  15

Gly Pro Phe Cys Ala Met Val Leu Ala Asp Phe Gly Ala Arg Val Val
            20                  25                  30

Arg Val Asp Arg Pro Gly Ser Arg Tyr Asp Val Ser Arg Leu Gly Arg
        35                  40                  45
```

```
Gly Lys Arg Ser Leu Val Leu Asp Leu Lys Gln Pro Arg Gly Ala Ala
    50                  55                  60

Val Leu Arg Arg Leu Cys Lys Arg Ser Asp Val Leu Leu Glu Pro Phe
65                  70                  75                  80

Arg Arg Gly Val Met Glu Lys Leu Gln Leu Gly Pro Glu Ile Leu Gln
                85                  90                  95

Arg Glu Asn Pro Arg Leu Ile Tyr Ala Arg Leu Ser Gly Phe Gly Gln
            100                 105                 110

Ser Gly Ser Phe Cys Arg Leu Ala Gly His Asp Ile Asn Tyr Leu Ala
        115                 120                 125

Leu Ser Gly Val Leu Ser Lys Ile Gly Arg Ser Gly Glu Asn Pro Tyr
    130                 135                 140

Ala Pro Leu Asn Leu Leu Ala Asp Phe Ala Gly Gly Gly Leu Met Cys
145                 150                 155                 160

Ala Leu Gly Ile Ile Met Ala Leu Phe Asp Arg Thr Arg Thr Gly Lys
                165                 170                 175

Gly Gln Val Ile Asp Ala Asn Met Val Glu Gly Thr Ala Tyr Leu Ser
            180                 185                 190

Ser Phe Leu Trp Lys Thr Gln Lys Leu Ser Leu Trp Glu Ala Pro Arg
        195                 200                 205

Gly Gln Asn Met Leu Asp Gly Gly Ala Pro Phe Tyr Thr Thr Tyr Arg
    210                 215                 220

Thr Ala Asp Gly Glu Phe Met Ala Val Gly Ala Ile Glu Pro Gln Phe
225                 230                 235                 240

Tyr Glu Leu Leu Ile Lys Gly Leu Gly Leu Lys Ser Asp Glu Leu Pro
                245                 250                 255

Asn Gln Met Ser Met Asp Asp Trp Pro Glu Met Lys Lys Lys Phe Ala
            260                 265                 270

Asp Val Phe Ala Glu Lys Thr Lys Ala Glu Trp Cys Gln Ile Phe Asp
        275                 280                 285

Gly Thr Asp Ala Cys Val Thr Pro Val Leu Thr Phe Glu Glu Val Val
```

```
    290                 295                 300

His His Asp His Asn Lys Glu Arg Gly Ser Phe Ile Thr Ser Glu Glu
305                 310                 315                 320

Gln Asp Val Ser Pro Arg Pro Ala Pro Leu Leu Leu Asn Thr Pro Ala
                325                 330                 335

Ile Pro Ser Phe Lys Arg Asp Pro Phe Ile Gly Glu His Thr Glu Glu
            340                 345                 350

Ile Leu Glu Glu Phe Gly Phe Ser Arg Glu Glu Ile Tyr Gln Leu Asn
        355                 360                 365

Ser Asp Lys Ile Ile Glu Ser Asn Lys Val Lys Ala Ser Leu
    370                 375                 380
```

<210> 12
<211> 3352
<212> DNA
<213> Homo sapiens

<400> 12

```
ggggcgtggc gccggggatt gggagggctt cttgcaggct gctgggctgg ggctaagggc      60
tgctcagttt ccttcagcgg ggcactggga agcgccatgg cactgcaggg catctcggtc     120
gtggagctgt ccggcctggc cccgggcccg ttctgtgcta tggtcctggc tgacttcggg     180
gcgcgtgtgg tacgcgtgga ccggcccggc tcccgctacg acgtgagccg cttgggccgg     240
ggcaagcgct cgctagtgct ggacctgaag cagccgcggg gagccgccgt gctgcggcgt     300
ctgtgcaagc ggtcggatgt gctgctggag cccttccgcc gcggtgtcat ggagaaactc     360
cagctgggcc cagagattct gcagcgggaa aatccaaggc ttatttatgc caggctgagt     420
ggatttggcc agtcaggaag cttctgccgg ttagctggcc acgatatcaa ctatttggct     480
ttgtcaggtg ttctctcaaa aattggcaga agtggtgaga atccgtatgc cccgctgaat     540
ctcctggctg actttgctgg tggtggcctt atgtgtgcac tgggcattat aatggctctt     600
tttgaccgca cacgcactgg caagggtcag gtcattgatg caaatatggt ggaaggaaca     660
gcatatttaa gttcttttct gtggaaaact cagaaattga gtctgtggga agcacctcga     720
ggacagaaca tgttggatgg tggagcacct ttctatacga cttacaggac agcagatggg     780
gaattcatgg ctgttggagc aatagaaccc cagttctacg agctgctgat caaaggactt     840
ggactaaagt ctgatgaact tcccaatcag atgagcatgg atgattggcc agaaatgaag     900
aagaagtttg cagatgtatt tgcagagaag acgaaggcag agtggtgtca aatctttgac     960
ggcacagatg cctgtgtgac tccggttctg acttttgagg aggttgttca tcatgatcac    1020
```

aacaaggaac ggggctcgtt tatcaccagt gaggagcagg acgtgagccc ccgccctgca 1080
cctctgctgt taaacacccc agccatccct tctttcaaaa gggatccttt cataggagaa 1140
cacactgagg agatacttga agaatttgga ttcagccgcg aagagattta tcagcttaac 1200
tcagataaaa tcattgaaag taataaggta aaagctagtc tctaacttcc aggcccacgg 1260
ctcaagtgaa tttgaatact gcatttacag tgtagagtaa cacataacat tgtatgcatg 1320
gaaacatgga ggaacagtat tacagtgtcc taccactcta atcaagaaaa gaattacaga 1380
ctctgattct acagtgatga ttgaattcta aaaatggtta tcattagggc ttttgattta 1440
taaaactttg ggtacttata ctaaattatg gtagttattc tgccttccag tttgcttgat 1500
atatttgttg atattaagat tcttgactta tattttgaat gggttctagt gaaaaaggaa 1560
tgatatattc ttgaagacat cgatatacat ttatttacac tcttgattct acaatgtaga 1620
aaatgaggaa atgccacaaa ttgtatggtg ataaaagtca cgtgaaacag agtgattggt 1680
tgcatccagg ccttttgtct tggtgttcat gatctccctc taagcacatt ccaaacttta 1740
gcaacagtta tcacactttg taatttgcaa agaaaagttt cacctgtatt gaatcagaat 1800
gccttcaact gaaaaaaaca tatccaaaat aatgaggaaa tgtgttggct cactacgtag 1860
agtccagagg gacagtcagt tttagggttg cctgtatcca gtaactcggg gcctgtttcc 1920
ccgtgggtct ctgggctgtc agctttcctt tctccatgtg tttgatttct cctcaggctg 1980
gtagcaagtt ctggatctta tacccaacac acagcaacat ccagaaataa agatctcagg 2040
accccccagc aagtcgtttt gtgtctcctt ggactgagtt aagttacaag cctttcttat 2100
acctgtcttt gacaaagaag acgggattgt ctttacataa aaccagcctg ctcctggagc 2160
ttccctggac tcaacttcct aaaggcatgt gaggaagggg tagattccac aatctaatcc 2220
gggtgccatc agagtagagg gagtagagaa tggatgttgg gtaggccatc aataaggtcc 2280
attctgcgca gtatctcaac tgccgttcaa caatcgcaag aggaaggtgg agcaggtttc 2340
ttcatcttac agttgagaaa acagagactc agaagggctt cttagttcat gtttccctta 2400
gcgcctcagt gatttttca tggtggctta ggccaaaaga aatatctaac cattcaattt 2460
ataaataatt aggtccccaa cgaattaaat attatgtcct accaacttat tagctgcttg 2520
aaaaatataa tacacataaa taaaaaaata tatttttcat ttctatttca ttgttaatca 2580
caactactta ctaaggagat gtatgcacct attggacact gtgcaacttc tcacctggaa 2640
tgagattgga cactgctgcc ctcattttct gctccatgtt ggtgtccata tagtacttga 2700
ttttttatca gatggcctgg aaaacccagt ctcacaaaaa tatgaaatta tcagaaggat 2760
tatagtgcaa tcttatgttg aaagaatgaa ctacctcact agtagttcac gtgatgtctg 2820
acagatgttg agtttcattg tgtttgtgtg ttcaaatttt taaatattct gagatactct 2880
tgtgaggtca ctctaatgcc ctgggtgcct tggcacagtt ttagaaatac cagttgaaaa 2940

```
tatttgctca ggaatatgca actaggaagg ggcagaatca gaatttaagc tttcatattc     3000
tagccttcag tcttgttctt caaccatttt taggaacttt cccataaggt tatgttttcc     3060
agcccaggca tggaggatca cttgaggcca agagttcgag accagcctgg ggaacttggc     3120
tggacctccg tttctacgaa ataaaaataa aaaaattatc caggtatggt ggtgtgtgcc     3180
tgtagtccta tctactcaag ggtggggcag gaggatcact tgagcccagg aatttgaggc     3240
cacagtgaat taggattgca ccactgcact ctagcccagg caacagaaca agaacctgtc     3300
tctaaataaa taaataaaaa taataataat aaaaaagatg ttttccctac aa             3352
```

<210> 13
<211> 238
<212> PRT
<213> Homo sapiens

<400> 13

```
Met Leu Cys Cys Met Arg Arg Thr Lys Gln Val Glu Lys Asn Asp Asp
1               5                   10                  15

Asp Gln Lys Ile Glu Gln Asp Gly Ile Lys Pro Glu Asp Lys Ala His
            20                  25                  30

Lys Ala Ala Thr Lys Ile Gln Ala Ser Phe Arg Gly His Ile Thr Arg
        35                  40                  45

Lys Lys Leu Lys Gly Glu Lys Lys Asp Asp Val Gln Ala Ala Glu Ala
    50                  55                  60

Glu Ala Asn Lys Lys Asp Glu Ala Pro Val Ala Asp Gly Val Glu Lys
65                  70                  75                  80

Lys Gly Glu Gly Thr Thr Thr Ala Glu Ala Ala Pro Ala Thr Gly Ser
                85                  90                  95

Lys Pro Asp Glu Pro Gly Lys Ala Gly Glu Thr Pro Ser Glu Glu Lys
            100                 105                 110

Lys Gly Glu Gly Asp Ala Ala Thr Glu Gln Ala Ala Pro Gln Ala Pro
        115                 120                 125

Ala Ser Ser Glu Glu Lys Ala Gly Ser Ala Glu Thr Glu Ser Ala Thr
    130                 135                 140

Lys Ala Ser Thr Asp Asn Ser Pro Ser Ser Lys Ala Glu Asp Ala Pro
145                 150                 155                 160

Ala Lys Glu Glu Pro Lys Gln Ala Asp Val Pro Ala Ala Val Thr Ala


                165                 170                 175

Ala Ala Ala Thr Thr Pro Ala Ala Glu Asp Ala Ala Ala Lys Ala Thr
            180                 185                 190

Ala Gln Pro Pro Thr Glu Thr Gly Glu Ser Ser Gln Ala Glu Glu Asn
        195                 200                 205

Ile Glu Ala Val Asp Glu Thr Lys Pro Lys Glu Ser Ala Arg Gln Asp
    210                 215                 220

Glu Gly Lys Glu Glu Glu Pro Glu Ala Asp Gln Glu His Ala
225                 230                 235
```

<210> 14
<211> 3710
<212> DNA
<213> Homo sapiens

<400> 14

```
tctctgggtt gttttcaaca tctcaagtgt gaattttccc tgtcaaaatc ttcacaagga        60
aaatgagtca cagcatcacc tgggtgacga ggtcataaca cctcagccct tgcttaaaaa       120
attttatttc tacttttcta ttgtaaagag atctcaaaac aggaagataa aattggactg       180
acagctctac agcctagtct tttagacagt gaactaggcc agcattggca gacactggcg       240
atgacaaagt cctgctctga attatgccac cccgcactcc actttttacc ttgcctggga       300
ggcttgagga aaaatcttca gagagcagtt cgacctagtc cttattcact tggcttcttg       360
actttctgga tttcaagggt aattttacct cacatgtaac ctatacaagt attttaggtt       420
aaaaccctga ctctgccact tactagctgt atgactttga gcaaattact taattactta       480
actaatgtcc ttctacttag tcttttcatc tgtaagataa tagtacccat tgcatagttt       540
tgttcttaga attgaattag ttaatataca aactatatgc aataaatatg tttggggtgt       600
gtataatcat ataaaagtgc ttagaagagt gcttgacata tagttttaga atttgctatt       660
attatgttct ccaagcatat gagggtattt tatgtcatta cttttaaagg tcgctttttt       720
atatgtgtta gtgggaggca tttattattt ttttaagtgt gcatatccct gaaacatccc       780
tctgtggagc tataaaagaa aactataaaa tatttaccat ataattcact caatccccag       840
tctccagaaa gtaacaattt gtacttcatt tgtattgtct tcttccaggc attttacaaa       900
tactattttc tttcatcctc aaaaaaactc tgcacaatag gtaataggat tctaatatgg       960
gtgataagaa aactgaaatt tagagaaatt aagtaattta tcccaggtca tacatttaag      1020
cagtgaatga gccaggattc aaactcagat cttttgaccc caaaaccatg ccaggtagca      1080
agtaatcaat gaatgtcaat gacagatcat cgagagagaa tccagtgcca aaaatgaaga      1140
```

gggtgtcaac aaaataagaa caaggacaat ggcagcatga gcaacaagaa caaacttaga 1200
tccaaaatta acagttaaag atttttctag agtcctgcat atttttcctt aaactgtcca 1260
gcagcttggg gacagaaatc taattaaaat ggaagtctat gcatgctcaa accacatgcc 1320
ttgtacctga tgcaatccgc ttccacccca gtcctgaaaa aaggcattca gttatttttt 1380
tctacatgtg gaagaataca acaacatacc ttctagaatc agggagaaat gtacttgctc 1440
tttttatttt tttttaattt ctcaaatact gttctataag gataatcact cacctggtga 1500
ttaatgtaga atgttaaggt gtgtacaaat tactgctgat aggtaacact gtccttgcaa 1560
aatcatccca tgtgcccccc tcaaataaat aagtatactt caaaaagcag tgtgcttgct 1620
atacaagtgc agttttaaga aaaatcactc agctctctct tgcaaacatg ttctttcccc 1680
taatgcctcc tacacagaga ttctggagcc accccccttca gaatatctga aatatagacc 1740
aattttgctc gttaaaacta cttcaagtag tcctagagct gatattagga ttgctactta 1800
ttcagtcata cttttcgagt gttcattgtt gaattcttag attgaatttt ctattagttt 1860
ccttaaaata taagaggttg gagggctaag tttaagaccc attctactaa ttgtgaggct 1920
ttccacaatc tgtgtttcct tattttttta aaggggatgg cacacttgct tagccttctt 1980
cattatcatt ctcagaggaa tcattttaag ttgaaagtac ttcataacct ataatgcata 2040
aatttaaacc actgtcattt ccccccacca cagcccttgg caaatggggt cttaaattcc 2100
ttctcccacc aactagactt ttagcatgca ttctgacacg tattgtgtat ccttactaga 2160
tatgaggtat ataatatttg ggctttacta actatccagt gggggtgcat tggatatact 2220
tggcttggca atttcaaatt ataatgatct tcagtaccag atgctcttaa gctagtcccc 2280
tacaaatgtc tggcagcttc atttgtaact tttatcaggc ttgagtatta ccacacacaa 2340
gcacatagag tagctctcag aacctgcaga ggcaattgtt ttactagatg tgtgagggat 2400
atagttggat caatgggata gatactgtat atgaaatagt ggctaaatga gtttgtgtgt 2460
gtgtgtgtgt gtgtgtgtgt gtgtggttgg gggatgtcag gtgaaggtca catattttgg 2520
gaatgaatag atttctagtt tcagtgaatt tctatcacta agctcctaca ctaggtgaag 2580
ttttgggggg tgggtaagtt agagaagact gagaaggaat cagttggtgt gtgtcaggtg 2640
atagtagagt gggactgtgg tttaactttg acaatgtaaa cagatcttcc cccttcccaa 2700
gataagaaag aacagagttg agtactatgg cacattttac atggtttcct gcttatctct 2760
ttctttgaga atagatatag ctcagtagta ctgtcgagtt aagaaatagc ttttccaccc 2820
tctgagtgat gatacctaca atcatcagga tgaatacagt ctcatgacca aatctcctct 2880
ggtatttgtc ttatcaacat gccttaactt aatgtcacga tgctctagaa attactctga 2940
gagaataatg gtgatggcaa taattatgat gagaataatg gtagcagcca cccctttctg 3000
agtatgtaca aagtgtcagg gactgtgctg ggtgctttat gtgcattaac tcatttgatt 3060

ctcatatcag cccaatggga tagttttttt attccctcat cctgcagatg aggagcctaa 3120

agctgacttg cccaaggtca ggatctacca agtaactggc agagctggaa ttcaaactta 3180

cacatgtcta actctgcagc ccatgctctt aagcaccagg gaatattgac ttttacagga 3240

ggaggactaa gcaagggctt tgtcattcag aagctggatg acgctgggct ccttcctggt 3300

tcctcatctg tgttgattat aaaacaggca cagtgatgtg ttcctcacat gatcatgatg 3360

aggattgaat tgaaatacta aatatatata ctgtgcactt ataaatgtaa gaagagagag 3420

gaaaatgact gctggagagt ggcgtgatga aggctccagc attagtgacc tggagcgaga 3480

acacccagcc agtgagctga gtgttcacac acagagagtt tacacactct ctgagcacca 3540

acacagtatg gcatgtggtc atgcaacaac taaaaataac attttttttt agtatcaact 3600

atgtgccagc actattttag ccactgaagc ttagaagatt gtaaaaaaac agataaagtt 3660

ctgttctcag attgtttata gcccaagcga agttcagtta gtaataaaat 3710

<210> 15
<211> 330
<212> PRT
<213> Homo sapiens

<400> 15

```
Met Leu Pro Asp Trp Lys Ser Ser Leu Ile Leu Met Ala Tyr Ile Ile
1               5                   10                  15

Ile Phe Leu Thr Gly Leu Pro Ala Asn Leu Leu Ala Leu Arg Ala Phe
            20                  25                  30

Val Gly Arg Ile Arg Gln Pro Gln Pro Ala Pro Val His Ile Leu Leu
        35                  40                  45

Leu Ser Leu Thr Leu Ala Asp Leu Leu Leu Leu Leu Leu Leu Pro Phe
    50                  55                  60

Lys Ile Ile Glu Ala Ala Ser Asn Phe Arg Trp Tyr Leu Pro Lys Val
65                  70                  75                  80

Val Cys Ala Leu Thr Ser Phe Gly Phe Tyr Ser Ser Ile Tyr Cys Ser
                85                  90                  95

Thr Trp Leu Leu Ala Gly Ile Ser Ile Glu Arg Tyr Leu Gly Val Ala
            100                 105                 110

Phe Pro Val Gln Tyr Lys Leu Ser Arg Arg Pro Leu Tyr Gly Val Ile
        115                 120                 125

Ala Ala Leu Val Ala Trp Val Met Ser Phe Gly His Cys Thr Ile Val
```

```
        130                 135                 140

    Ile Ile Val Gln Tyr Leu Asn Thr Thr Glu Gln Val Arg Ser Gly Asn
    145                 150                 155                 160

    Glu Ile Thr Cys Tyr Glu Asn Phe Thr Asp Asn Gln Leu Asp Val Val
                    165                 170                 175

    Leu Pro Val Arg Leu Glu Leu Cys Leu Val Leu Phe Phe Ile Pro Met
                180                 185                 190

    Ala Val Thr Ile Phe Cys Tyr Trp Arg Phe Val Trp Ile Met Leu Ser
            195                 200                 205

    Gln Pro Leu Val Gly Ala Gln Arg Arg Arg Arg Ala Val Gly Leu Ala
        210                 215                 220

    Val Val Thr Leu Leu Asn Phe Leu Val Cys Phe Gly Pro Tyr Asn Val
    225                 230                 235                 240

    Ser His Leu Val Gly Tyr His Gln Arg Lys Ser Pro Trp Trp Arg Ser
                    245                 250                 255

    Ile Ala Val Val Phe Ser Ser Leu Asn Ala Ser Leu Asp Pro Leu Leu
                260                 265                 270

    Phe Tyr Phe Ser Ser Ser Val Val Arg Arg Ala Phe Gly Arg Gly Leu
            275                 280                 285

    Gln Val Leu Arg Asn Gln Gly Ser Ser Leu Leu Gly Arg Arg Gly Lys
        290                 295                 300

    Asp Thr Ala Glu Gly Thr Asn Glu Asp Arg Gly Val Gly Gln Gly Glu
    305                 310                 315                 320

    Gly Met Pro Ser Ser Asp Phe Thr Thr Glu
                    325                 330
```

<210> 16
<211> 2069
<212> DNA
<213> Homo sapiens

<400> 16

atgctgccgg actggaagag ctccttgatc ctcatggctt acatcatcat cttcctcact 60

ggcctccctg ccaacctcct ggccctgcgg gcctttgtgg ggcggatccg ccagccccag 120

cctgcacctg tgcacatcct cctgctgagc ctgacgctgg ccgacctcct cctgctgctg 180

```
ctgctgccct tcaagatcat cgaggctgcg tcgaacttcc gctggtacct gcccaaggtc      240
gtctgcgccc tcacgagttt tggcttctac agcagcatct actgcagcac gtggctcctg      300
gcgggcatca gcatcgagcg ctacctggga gtggctttcc ccgtgcagta caagctctcc      360
cgccggcctc tgtatggagt gattgcagct ctggtggcct gggttatgtc ctttggtcac      420
tgcaccatcg tgatcatcgt tcaatacttg aacacgactg agcaggtcag aagtggcaat      480
gaaattacct gctacgagaa cttcaccgat aaccagttgg acgtggtgct gcccgtgcgg      540
ctggagctgt gcctggtgct cttcttcatc cccatggcag tcaccatctt ctgctactgg      600
cgttttgtgt ggatcatgct ctcccagccc cttgtggggg cccagaggcg gcgccgagcc      660
gtggggctgg ctgtggtgac gctgctcaat ttcctggtgt gcttcggacc ttacaacgtg      720
tcccacctgg tggggtatca ccagagaaaa agcccctggt ggcggtcaat agccgtggtg      780
ttcagttcac tcaacgccag tctggacccc ctgctcttct atttctcttc ttcagtggtg      840
cgcagggcat ttgggagagg gctgcaggtg ctgcggaatc agggctcctc cctgttggga      900
cgcagaggca aagacacagc agaggggaca aatgaggaca ggggtgtggg tcaaggagaa      960
gggatgccaa gttcggactt cactacagag tagcagtttc cctggacctt cagaggtcgc     1020
ctgggttaca caggagctgg gaagcctggg agaggcggag caggaaggct cccatccaga     1080
ttcagaaatc cttagaccca gcccaggact gcgactttga aaaaaatgcc tttcaccagc     1140
ttggtatccc ttcctgactg aattgtccta ctcaaaggag cataagtcag agatgcacga     1200
agaagtagtt aggtatagaa gcacctgccg ggtgtggtgg ctcatgccta taatcccaga     1260
actttgggag gctgaggcag gtggatcact tgaggtcggg agattgagaa catcctggtc     1320
aacatgggaa aaccccgtct ctactaaaaa tacaaaaaaa ttagctgggc atggtggcac     1380
atgcctataa tcccagctac tctggaggct gaggcaggag aatccttgaa cccgggagtt     1440
ggaggttgca gtgagctgag atcacgccac tgcactccag cctagcgaca gagcaagact     1500
ccatttaaaa aaaaaaaaaa aaaaaaaaag aagcaccttc aggctggaga agcagcgtag     1560
ctaacacaag tccagtcctt gtgatgtggc tggtagttgg ggatggccag gctgaagcag     1620
agagtcctag agaaatctcg atacaagctt caaagcaaca cctagacact gctctagcgg     1680
ttgatcctgg agataaacca acaagagaga gatggaagag aaatactaaa tgaggtcaaa     1740
gaagactcag aaaggttctg agcctggaga tgagcaggga ggcctcaggg cttagacctt     1800
taatgatagg ggtttccctg cattggtttg acctgttgcc tttttgatgt gctctgtttg     1860
ttttcatgtg ttgtcttgtc tcccctgcta aactgggagc tgccaggggt ctgggtctta     1920
tctccttcct ccatggtacc ccacacaggc caggatgtgg tttggtaccc agcaatcaga     1980
gattggcact ccctcataca ggggaaagca acctggtcta gcaaattgaa aataaagatg     2040
ataaaactct gaaaaaaaaa aaaaaaaaa                                       2069
```

<210> 17
<211> 428
<212> PRT
<213> Homo sapiens

<400> 17

```
Met Leu Arg Thr Leu Leu Arg Arg Arg Leu Phe Ser Tyr Pro Thr Lys
1               5                   10                  15

Tyr Tyr Phe Met Val Leu Val Leu Ser Leu Ile Thr Phe Ser Val Leu
            20                  25                  30

Arg Ile His Gln Lys Pro Glu Phe Val Ser Val Arg His Leu Glu Leu
        35                  40                  45

Ala Gly Glu Asn Pro Ser Ser Asp Ile Asn Cys Thr Lys Val Leu Gln
    50                  55                  60

Gly Asp Val Asn Glu Ile Gln Lys Val Lys Leu Glu Ile Leu Thr Val
65                  70                  75                  80

Lys Phe Lys Lys Arg Pro Arg Trp Thr Pro Asp Asp Tyr Ile Asn Met
                85                  90                  95

Thr Ser Asp Cys Ser Ser Phe Ile Lys Arg Arg Lys Tyr Ile Val Glu
            100                 105                 110

Pro Leu Ser Lys Glu Glu Ala Glu Phe Pro Ile Ala Tyr Ser Ile Val
        115                 120                 125

Val His His Lys Ile Glu Met Leu Asp Arg Leu Leu Arg Ala Ile Tyr
    130                 135                 140

Met Pro Gln Asn Phe Tyr Cys Ile His Val Asp Thr Lys Ser Glu Asp
145                 150                 155                 160

Ser Tyr Leu Ala Ala Val Met Gly Ile Ala Ser Cys Phe Ser Asn Val
                165                 170                 175

Phe Val Ala Ser Arg Leu Glu Ser Val Val Tyr Ala Ser Trp Ser Arg
            180                 185                 190

Val Gln Ala Asp Leu Asn Cys Met Lys Asp Leu Tyr Ala Met Ser Ala
        195                 200                 205

Asn Trp Lys Tyr Leu Ile Asn Leu Cys Gly Met Asp Phe Pro Ile Lys
    210                 215                 220
```

```
Thr Asn Leu Glu Ile Val Arg Lys Leu Lys Leu Leu Met Gly Glu Asn
225                 230                 235                 240

Asn Leu Glu Thr Glu Arg Met Pro Ser His Lys Glu Glu Arg Trp Lys
                245                 250                 255

Lys Arg Tyr Glu Val Val Asn Gly Lys Leu Thr Asn Thr Gly Thr Val
            260                 265                 270

Lys Met Leu Pro Pro Leu Glu Thr Pro Leu Phe Ser Gly Ser Ala Tyr
        275                 280                 285

Phe Val Val Ser Arg Glu Tyr Val Gly Tyr Val Leu Gln Asn Glu Lys
    290                 295                 300

Ile Gln Lys Leu Met Glu Trp Ala Gln Asp Thr Tyr Ser Pro Asp Glu
305                 310                 315                 320

Tyr Leu Trp Ala Thr Ile Gln Arg Ile Pro Glu Val Pro Gly Ser Leu
                325                 330                 335

Pro Ala Ser His Lys Tyr Asp Leu Ser Asp Met Gln Ala Val Ala Arg
            340                 345                 350

Phe Val Lys Trp Gln Tyr Phe Glu Gly Asp Val Ser Lys Gly Ala Pro
        355                 360                 365

Tyr Pro Pro Cys Asp Gly Val His Val Arg Ser Val Cys Ile Phe Gly
    370                 375                 380

Ala Gly Asp Leu Asn Trp Met Leu Arg Lys His His Leu Phe Ala Asn
385                 390                 395                 400

Lys Phe Asp Val Asp Val Asp Leu Phe Ala Ile Gln Cys Leu Asp Glu
                405                 410                 415

His Leu Arg His Lys Ala Leu Glu Thr Leu Lys His
            420                 425
```

<210> 18
<211> 5984
<212> DNA
<213> Homo sapiens

<400> 18

acagaccata ggtgaactgt taaggaggga gaagccgcag tgtcaccttc cacagtcctg 60

ctcccattaa gacctatgaa gtggaaaatg cagattattc tttgaggttt cctgggacca 120

gaaaaggtca gagactctta atgggtaatg cagacaactg gaatgcaatc atcaggtatt 180
catttctgtt ttaaggtttt atgtctgaga aacctgaaac ctgaagaagt aaagcaatca 240
tgattttttta aaaacttaat aatatggttt tggatttaaa aagaaagact gactcagagc 300
tggggaaaac actcacttgt gatacggatc aatgtgaaaa cagcttaact ctgttccatg 360
gcaattcgca cagtgtgact cagagacacc tctcttcacc agctctggct caggggatgt 420
gaagccagaa gcagaggtca cagtgacttc atctgagaag gacttaactg accattgctg 480
gttttgaagc cggaggaagg cccacagtca atgaatgaga gaggcttcga gaagctagaa 540
atggcaagga aatagatttt cctctaaagc tcccagaaag aaacacggtt ctgctgacac 600
ctcgatttta ggccagtgag acctgcttca gacttctgac ctacagaacg atttctttaa 660
agactcgtgc agcacatcat tatcgctgga tgcccggaca tgtaatacac ctgacagcat 720
gtgaagtgct cagaatgggg caggatgtca cctggaatca gcactaagtg attcagactt 780
tccttacttt taaatgtgct gctcttcatt tcaagatgcc gttgcagctc tgataaatgc 840
aaactgacaa ccttcaaggc cacgacggag ggaaaatcat tggtgcttgg agcatagaag 900
actgcccttc acaaaggaaa tccctgatta ttgtttgaaa tgctgaggac gttgctgcga 960
aggagacttt tttcttatcc caccaaatac tactttatgg ttcttgtttt atccctaatc 1020
accttctccg ttttaaggat tcatcaaaag cctgaatttg taagtgtcag acacttggag 1080
cttgctgggg agaatcctag tagtgatatt aattgcacca aagttttaca gggtgatgta 1140
aatgaaatcc aaaaggtaaa gcttgagatc ctaacagtga aatttaaaaa gcgccctcgg 1200
tggacacctg acgactatat aaacatgacc agtgactgtt cttctttcat caagagacgc 1260
aaatatattg tagaacccct tagtaaagaa gaggcggagt ttccaatagc atattctata 1320
gtggttcatc acaagattga aatgcttgac aggctgctga gggccatcta tatgcctcag 1380
aatttctatt gcattcatgt ggacacaaaa tccgaggatt cctatttagc tgcagtgatg 1440
ggcatcgctt cctgttttag taatgtcttt gtggccagcc gattggagag tgtggtttat 1500
gcatcgtgga gccgggttca ggctgacctc aactgcatga aggatctcta tgcaatgagt 1560
gcaaactgga agtacttgat aaatctttgt ggtatggatt ttcccattaa aaccaaccta 1620
gaaattgtca ggaagctcaa gttgttaatg ggagaaaaca acctggaaac ggagaggatg 1680
ccatcccata aagaagaaag gtggaagaag cggtatgagg tcgttaatgg aaagctgaca 1740
aacacaggga ctgtcaaaat gcttcctcca ctcgaaacac ctctcttttc tggcagtgcc 1800
tacttcgtgg tcagtaggga gtatgtgggg tatgtactac agaatgaaaa aatccaaaag 1860
ttgatggagt gggcacaaga cacatacagc cctgatgagt atctctgggc caccatccaa 1920
aggattcctg aagtcccggg ctcactccct gccagccata agtatgatct gtctgacatg 1980
caagcagttg ccaggtttgt caagtggcag tactttgagg gtgatgtttc caagggtgct 2040

ccctacccgc cctgcgatgg agtccatgtg cgctcagtgt gcattttcgg agctggtgac 2100
ttgaactgga tgctgcgcaa acaccacttg tttgccaata agtttgacgt ggatgttgac 2160
ctctttgcca tccagtgttt ggatgagcat ttgagacaca aagctttgga gacattaaaa 2220
cactgaccat tacgggcaat tttatgaaca agaagaagga tacacaaaac gtacccttat 2280
ctgtttcccc ttccttgtca gcatcgggaa gatggtatga agtcctcttt ggggcaggga 2340
ctctagtaga tcttcttgtc agagaagctg catggtttct gcagagcaca gttagctaga 2400
aaggtgatag cattaaatgt tcatctagag ttaatagtgg gaggagtaaa ggtagccttg 2460
aggccagagc aggtagcaag gcattgtgga aagaggggac cagggtggct ggggaagagg 2520
ccgatgcata aagtcagcct gttcaaagtg ctcagggact tagcaaaatg agaagatgtg 2580
acctgtgcca aaactatttt gagaatttta aatgtgacca tttttctggt atgaataaac 2640
ttacagcaac aaataatcaa agatacaatt aatctgatat tatatttgtt gaaatagaaa 2700
tttgattgta ctataaatga tttttgtaaa taatttatat tctgctctaa tactgtactg 2760
tgtagtgtgt ctccgtatgt catctcaggg agcttaaaat gggcttgatt taacattgtt 2820
tttgtgttat ttttgcttga aacaacgcac acattttcaa caaccaaaaa atgacaattt 2880
ctagtttagt taatttctac aaatcatctt atgttattag caaggttaag acatcttttt 2940
taaaaaaatt atagcttcta ccaagagaaa cactcaattt ttctagagat ttgcctctat 3000
cttcctttcc tcagtcttcc cagactgcta tcaagctgtg taaaaattta ctttcactgg 3060
accctaaatt attgtctctg ctatctgact gccagtaatt agtgcagaaa actaagacag 3120
gatgatacag gtttgagggg ctggggagtg ggaggggggga gaaaaggaat gtatttaaac 3180
aatttccgat gcccatgatg agtttaaaaa ccagcattga caccatcccc aaaattaagg 3240
ctgtcgctta ttgaatccac ttgtgtccaa cctcccagga ttgttttatc ctaatgtcac 3300
ctgtatattc atttgaaagg acttggccct gttcttgggt cttcccgtta cctgcccccct 3360
gggtggtaag tttcctcctt tctcaacctt ccacgaggag gaaagaagtg tgcagtcatt 3420
ccacatggcc tgttggaagg cctggggagg gaactttggg tttgggacag attttttttt 3480
ttgtttttgg tatcattcac agcatacgat ttttactctc tccatcttca ccataagaca 3540
gataatttgg ggttgctata atgctgtcac acatctcaaa gtacattcaa atcttaaaaa 3600
gaaattctcg tacttttgcc atgttgatac tgttcagcaa acaagctacc aggaactgtg 3660
aggctttgtc atttagcatt agactttaaa caagaattaa aatcatgtgc tgtattttta 3720
aaatctagcc aaattaaata gtacatgaga aattcagagt attagacagt tttaaggcat 3780
tcaactgaga aaactttatt tgtcaaagtc agaaaacatt ttcatcttat tgagagatat 3840
gtttttaaac ttttatcatc atttgtaaat gtggaagttg gtggattgct gtgtttttgc 3900

atgattagca tgggagtctg ttggagcaag aggaacatgc ttgttttgaa aactcgagat 3960
gatgagggtg gtacatgcag tgtgttctct ctttattggc ttctaaacca gttttgtcct 4020
ttaatgcatg tcaaatattt ctcccatgct tctcttagca gaaaagtttt tacctataag 4080
acagggcacc ttttaactct aaaactagtg atactcagtg acatagactt tgtcttataa 4140
acattttttc atttttttatt ttgaaaaatt gcaaatctac agcaaaagta aaacagtaga 4200
gtgaacacca tgtaaccctc acctggtgtt aacattgtac cctatttgct ttaagttgta 4260
tgtatttctg aacttggcaa aattggaaat taaaattttt aaaaattaca aatatacaaa 4320
gttatttatc ttagcacatt tattatgtgt gactgcatct gatttatatt taaattggca 4380
ggttttgagg gattttttttc ttcatcataa atgtaaacat aggattttag agtctatttc 4440
cccaagcgcc acattataac tgtaaactta ccatcttcta tgtagctgtg atatctcatc 4500
tttctaaaat ggaacttgtt aaaaagtgtt caaacactat ccctaatgcc tgcggcagaa 4560
tttatatacg atccattcat tggggctcaa agtatctttt agacttttaa ggacaattta 4620
cagcaaatga aatttatgat gctgtgacaa gaaatttaaa gaatcaaaac gatggtttga 4680
aaaggaaacc tatgatacat gcaggagaag caaaacccaa gtgattggtg agaaatatag 4740
aaattattta aattacttta tagtaattat taaacactaa tttttgtact gtcattgaag 4800
gtgttttata gagaaatctg agaaatcaca ttcacaaatt agaagtcaaa catggccagg 4860
cacagtggct cacacctgta atcttcagga tttcaagacc agcctagcca acatgacgaa 4920
accccatctc tactgaaaat agaaaaaaaa aattagccag gcttgcacct gtaatcccag 4980
ttacctggga ggctgaggca ggagaatctc ttgagcccag gaggcggagg ttgcagtgag 5040
ctgagatgcc accaccacac cagcctgggt gacagagtga aactcgtatc tccaaacaaa 5100
caaacaaaaa gtccttaaac atatgtgaac aaaaattttg tgatggaagg attctagtta 5160
atgagtattg catcaagatt tacatctttc ttactaagga aaagagttaa taaaaattgt 5220
tctttatttt acaggcagtt actgaggctc ttcccagatc tcagtaaaca gccactcagc 5280
cttgaaaatg gagtgttgtt gtttctaaac atatatttat gtcatttatt aagtacagtt 5340
cacttaaata acataagtag attttctctt gtagtgattt gggtaggaag aggccatgtt 5400
tcagttcgtt ttctctgtag ggtcgattga attggacctt ttcagttgtt cagaaaaata 5460
aaaataattt ctcatattaa atacagacgc tcctcaactt atgatgtggg taggtcccag 5520
taaacccatt ataatttgaa aatatcacat tgaaaatgca tttaatatct cttacctgaa 5580
atcataactt agccaagcct accttaaatg ttctcagaac atttagcctg cagttgggca 5640
aaaccattta acacaaagcc tattttataa tgaagtgttg aatagctcat gttatttact 5700
gaatactgtt gtgaaagtga aaaacaatga ttgtatgggt actcaaagta taatttctac 5760
tgaatgcata tcacttgtgc actgttgtaa agctgaaaaa ccgttaagcc tctacgattt 5820

ttaagtaagt tggggaccat cagtttaaaa taaatgcaat actatttcat gataaacatg 5880

gtcactgtaa gttttactct tttgaatgag ggtgcgacag aatgcagtta gaatcagttc 5940

atatcaccat taaaatcatc cattcagaaa ccaaaaaaaa aaaa 5984

<210> 19
<211> 667
<212> PRT
<213> Homo sapiens

<400> 19

```
Met Lys Glu Lys Ser Lys Asn Ala Ala Lys Thr Arg Arg Glu Lys Glu
1               5                   10                  15

Asn Gly Glu Phe Tyr Glu Leu Ala Lys Leu Leu Pro Leu Pro Ser Ala
            20                  25                  30

Ile Thr Ser Gln Leu Asp Lys Ala Ser Ile Ile Arg Leu Thr Thr Ser
        35                  40                  45

Tyr Leu Lys Met Arg Ala Val Phe Pro Glu Gly Leu Gly Asp Ala Trp
    50                  55                  60

Gly Gln Pro Ser Arg Ala Gly Pro Leu Asp Gly Val Ala Lys Glu Leu
65                  70                  75                  80

Gly Ser His Leu Leu Gln Thr Leu Asp Gly Phe Val Phe Val Val Ala
                85                  90                  95

Ser Asp Gly Lys Ile Met Tyr Ile Ser Glu Thr Ala Ser Val His Leu
            100                 105                 110

Gly Leu Ser Gln Val Glu Leu Thr Gly Asn Ser Ile Tyr Glu Tyr Ile
        115                 120                 125

His Pro Ser Asp His Asp Glu Met Thr Ala Val Leu Thr Ala His Gln
    130                 135                 140

Pro Leu His His His Leu Leu Gln Glu Tyr Glu Ile Glu Arg Ser Phe
145                 150                 155                 160

Phe Leu Arg Met Lys Cys Val Leu Ala Lys Arg Asn Ala Gly Leu Thr
                165                 170                 175

Cys Ser Gly Tyr Lys Val Ile His Cys Ser Gly Tyr Leu Lys Ile Arg
            180                 185                 190

Gln Tyr Met Leu Asp Met Ser Leu Tyr Asp Ser Cys Tyr Gln Ile Val
```

```
        195                 200                 205

Gly Leu Val Ala Val Gly Gln Ser Leu Pro Pro Ser Ala Ile Thr Glu
    210                 215                 220

Ile Lys Leu Tyr Ser Asn Met Phe Met Phe Arg Ala Ser Leu Asp Leu
225                 230                 235                 240

Lys Leu Ile Phe Leu Asp Ser Arg Val Thr Glu Val Thr Gly Tyr Glu
                245                 250                 255

Pro Gln Asp Leu Ile Glu Lys Thr Leu Tyr His His Val His Gly Cys
            260                 265                 270

Asp Val Phe His Leu Arg Tyr Ala His His Leu Leu Leu Val Lys Gly
        275                 280                 285

Gln Val Thr Thr Lys Tyr Tyr Arg Leu Leu Ser Lys Arg Gly Gly Trp
    290                 295                 300

Val Trp Val Gln Ser Tyr Ala Thr Val Val His Asn Ser Arg Ser Ser
305                 310                 315                 320

Arg Pro His Cys Ile Val Ser Val Asn Tyr Val Leu Thr Glu Ile Glu
                325                 330                 335

Tyr Lys Glu Leu Gln Leu Ser Leu Glu Gln Val Ser Thr Ala Lys Ser
            340                 345                 350

Gln Asp Ser Trp Arg Thr Ala Leu Ser Thr Ser Gln Glu Thr Arg Lys
        355                 360                 365

Leu Val Lys Pro Lys Asn Thr Lys Met Lys Thr Lys Leu Arg Thr Asn
    370                 375                 380

Pro Tyr Pro Pro Gln Gln Tyr Ser Ser Phe Gln Met Asp Lys Leu Glu
385                 390                 395                 400

Cys Gly Gln Leu Gly Asn Trp Arg Ala Ser Pro Pro Ala Ser Ala Ala
                405                 410                 415

Ala Pro Pro Glu Leu Gln Pro His Ser Glu Ser Ser Asp Leu Leu Tyr
            420                 425                 430

Thr Pro Ser Tyr Ser Leu Pro Phe Ser Tyr His Tyr Gly His Phe Pro
        435                 440                 445
```

```
Leu Asp Ser His Val Phe Ser Ser Lys Lys Pro Met Leu Pro Ala Lys
    450                 455                 460

Phe Gly Gln Pro Gln Gly Ser Pro Cys Glu Val Ala Arg Phe Phe Leu
465                 470                 475                 480

Ser Thr Leu Pro Ala Ser Gly Glu Cys Gln Trp His Tyr Ala Asn Pro
                485                 490                 495

Leu Val Pro Ser Ser Ser Ser Pro Ala Lys Asn Pro Pro Glu Pro Pro
            500                 505                 510

Ala Asn Thr Ala Arg His Ser Leu Val Pro Ser Tyr Glu Ala Pro Ala
        515                 520                 525

Ala Ala Val Arg Arg Phe Gly Glu Asp Thr Ala Pro Pro Ser Phe Pro
    530                 535                 540

Ser Cys Gly His Tyr Arg Glu Glu Pro Ala Leu Gly Pro Ala Lys Ala
545                 550                 555                 560

Ala Arg Gln Ala Ala Arg Asp Gly Ala Arg Leu Ala Leu Ala Arg Ala
                565                 570                 575

Ala Pro Glu Cys Cys Ala Pro Pro Thr Pro Glu Ala Pro Gly Ala Pro
            580                 585                 590

Ala Gln Leu Pro Phe Val Leu Leu Asn Tyr His Arg Val Leu Ala Arg
        595                 600                 605

Arg Gly Pro Leu Gly Gly Ala Ala Pro Ala Ala Ser Gly Leu Ala Cys
    610                 615                 620

Ala Pro Gly Gly Pro Glu Ala Ala Thr Gly Ala Leu Arg Leu Arg His
625                 630                 635                 640

Pro Ser Pro Ala Ala Thr Ser Pro Pro Gly Ala Pro Leu Pro His Tyr
                645                 650                 655

Leu Gly Ala Ser Val Ile Ile Thr Asn Gly Arg
            660                 665
```

<210> 20
<211> 4177
<212> DNA
<213> Homo sapiens

<400> 20

gggggctccgc gggcctggag cacggccggg tctaatatgc ccggagccga ggcgcgatga 60

```
aggagaagtc caagaatgcg gccaagacca ggagggagaa ggaaaatggc gagttttacg     120
agcttgccaa gctgctcccg ctgccgtcgg ccatcacttc gcagctggac aaagcgtcca     180
tcatccgcct caccacgagc tacctgaaga tgcgcgccgt cttccccgaa ggtttaggag     240
acgcgtgggg acagccgagc cgcgccgggc ccctggacgg cgtcgccaag gagctgggat     300
cgcacttgct gcagactttg gatggatttg tttttgtggt agcatctgat ggcaaaatca     360
tgtatatatc cgagaccgct tctgtccatt taggcttatc ccaggtggag ctcacgggca     420
acagtattta tgaatacatc catccttctg accacgatga gatgaccgct gtcctcacgg     480
cccaccagcc gctgcaccac cacctgctcc aagagtatga gatagagagg tcgttctttc     540
ttcgaatgaa atgtgtcttg gcgaaaagga acgcgggcct gacctgcagc ggatacaagg     600
tcatccactg cagtggctac ttgaagatca ggcagtatat gctggacatg tccctgtacg     660
actcctgcta ccagattgtg gggctggtgg ccgtgggcca gtcgctgcca cccagtgcca     720
tcaccgagat caagctgtac agtaacatgt tcatgttcag ggccagcctt gacctgaagc     780
tgatattcct ggattccagg gtgaccgagg tgacggggta cgagccgcag gacctgatcg     840
agaagaccct ataccatcac gtgcacggct gcgacgtgtt ccacctccgc tacgcacacc     900
acctcctgtt ggtgaagggc caggtcacca ccaagtacta ccggctgctg tccaagcggg     960
gcggctgggt gtgggtgcag agctacgcca ccgtggtgca caacagccgc tcgtcccggc    1020
cccactgcat cgtgagtgtc aattatgtac tcacggagat tgaatacaag gaacttcagc    1080
tgtccctgga gcaggtgtcc actgccaagt cccaggactc ctggaggacc gccttgtcta    1140
cctcacaaga aactaggaaa ttagtgaaac ccaaaaatac caagatgaag acaaagctga    1200
gaacaaaccc ttacccccca cagcaataca gctcgttcca aatggacaaa ctggaatgcg    1260
gccagctcgg aaactggaga gccagtcccc ctgcaagcgc tgctgctcct ccagaactgc    1320
agccccactc agaaagcagt gaccttctgt acacgccatc ctacagcctg cccttctcct    1380
accattacgg acacttccct ctggactctc acgtcttcag cagcaaaaag ccaatgttgc    1440
cggccaagtt cgggcagccc caaggatccc cttgtgaggt ggcacgcttt ttcctgagca    1500
cactgccagc cagcggtgaa tgccagtggc attatgccaa ccccctagtg cctagcagct    1560
cgtctccagc taaaaatcct ccagagccac cggcgaacac tgctaggcac agcctggtgc    1620
caagctacga agcgcccgcc gccgccgtgc gcaggttcgg cgaggacacc gcgcccccga    1680
gcttcccgag ctgcggccac taccgcgagg agcccgcgct gggcccggcc aaagccgccc    1740
gccaggccgc ccgggacggg gcgcggctgg cgctggcccg cgcggcaccc gagtgctgcg    1800
cgcccccgac ccccgaggcc ccgggcgcgc cggcgcagct gcccttcgtg ctgctcaact    1860
accaccgcgt gctggcccgg cgcggaccgc tgggggcgc cgcacccgcc gcctccggcc    1920
```

tggcctgcgc tcccggcggc cccgaggcgg cgaccggcgc gctgcggctc cggcacccga 1980
gccccgccgc cacctccccg cccggcgcgc ccctgccgca ctacctgggc gcctcggtca 2040
tcatcaccaa cgggaggtga cccgctggcc gcccgcgcca ggagcctgga cccggcctcc 2100
cggggctgcg gcgccaccga gcccggcaaa tgcgcacgac ctacattaat ttatgcagag 2160
acagctgttt gaattggacc ccgccgccga cttgcggatt tccaccgcgg aggccccgcg 2220
cgccggtgcc gagggccgag gagcgcccgg gtccgggcag gtgaccgccc gcctctgtcc 2280
tgcgagggcc ggtgcgaccc agttgctggg ggcttggttt cctcaccttg aaatcgggct 2340
tcacgcgtct tgccttgtcc ccaacgttcc acaacagtcc cgctgggga ttgaagcggt 2400
ttcactccgc aaatatcctc cactttcagg agggaaaacc caccctacca cagtccgctc 2460
ttccaagtgg acggcagacc tgggaggga cgcctgtgtc acgagccctt ttagatgctt 2520
aggtgaaggc agaagtgatg attgtaagtc ccatgaatac acaactccac tgtctttaaa 2580
agtcattcaa gagtctcatt atttttgttt ttatttaacc ctttcttcaa tacaaaaagc 2640
caacaaacca agactaaggg ggtgaccatg caattccatt ttgtgtctgt gaacataggt 2700
gtgcttccca aatacattaa caagctctta cttcccccta acccctatga actcttgata 2760
acaccaagag tagcaccttc agaatatatt gaataggcat taaatgcaaa aatatatatg 2820
tagccagaca gtttatgaga atgaccctgt caagcttcat tattacgtgg caaaatccct 2880
ctggcccaca cagatctgta attcactagg ctcgtgtttg ctacaaatag tgctaataaa 2940
gttaaattgc acgtgcaata cggaacactg tcaatggact gcaccttgtg aaggaaaaac 3000
atgcttaagg gggtgtaatg aaaatgatgt agacatttta agcattttct acacagcgag 3060
aaaacttcgt aagaacatgt tacgtgtgca acaggtaaac agaaatcctt tcataaagca 3120
ccagcagtgt ttaaaaaatg agcttccatt aatttttact ttttatgggt tttgcttaaa 3180
gatctcaaca tggaaaaatc ctgtcatggc tctgaactgc acaatgcatt gaaccgccgt 3240
ccttcaattt tcttcacact atcaacactg cagcattttg ctgctttatc aaaatggttt 3300
attttaggaa actttttcca cctttctgaa tggaaagagg ttttcacaaa tgttttaaac 3360
tcatcgttct aaaatcaagt gcacctacac caactgctct caaaatgtga actgactttt 3420
tttttttttt ttttgccaac cctgtgtcac ttagtgagga cctgacacaa tccctacagg 3480
gtgtctgtca gtgggcctca tggtaagagt cacaatttgc aaatttagga ccgtgggtca 3540
tgcagcgaag gggctggatg gtaggaaggg atgtgcccgc ctctccacgc actcagctat 3600
acctcattca cagctccttg tgagtgtgtg cacaggaaat aagccgaggg tattattttt 3660
ttatgttcat gagtcttgta attaaaccgt gattcttgaa aggtgtaggt ttgattacta 3720
ggagatacca ccgacatttt tcaataaagt actgcaaaat gcttttgtgt ctaccttgtt 3780
attaactttt ggggctgtat ttagtaaaaa taaatcaagg ctatcggagc agttcaataa 3840

```
caaaggttac tgttgagaaa aaagacccta tcatagattt acaagtgaca acgtggacag     3900
gcgtaacaga gtgtgtatgt acttgaaaat gtgtactagt gaaaacgtcc ttgtctaaac     3960
aaacttcgtt ttcataacct agaacatgaa ggaagattct tacacgagct ggcttcagga     4020
cggccagggc tgagtgtgaa gtcagcagag ctgttactgt ggcaacctaa tattgtgacc     4080
tccatcttga agagtctgca catttaagct ggggatagcg tttcttgact tctgtgaggc     4140
tccggcaggg gaagaggaag cattttaata catatgc                              4177
```

<210> 21
<211> 294
<212> PRT
<213> Homo sapiens

<400> 21

```
Met Lys Asp Arg Leu Gln Glu Leu Lys Gln Arg Thr Lys Glu Ile Glu
1               5                   10                  15

Leu Ser Arg Asp Ser His Val Ser Thr Thr Glu Thr Glu Glu Gln Gly
            20                  25                  30

Val Phe Leu Gln Gln Ala Val Ile Tyr Glu Arg Glu Pro Val Ala Glu
        35                  40                  45

Arg His Leu His Glu Ile Gln Lys Leu Gln Glu Ser Ile Asn Asn Leu
    50                  55                  60

Ala Asp Asn Val Gln Lys Phe Gly Gln Gln Gln Lys Ser Leu Val Ala
65                  70                  75                  80

Ser Met Arg Arg Phe Ser Leu Leu Lys Arg Glu Ser Thr Ile Thr Lys
                85                  90                  95

Glu Ile Lys Ile Gln Ala Glu Tyr Ile Asn Arg Ser Leu Asn Asp Leu
            100                 105                 110

Val Lys Glu Val Lys Lys Ser Glu Val Glu Asn Gly Pro Ser Ser Val
        115                 120                 125

Val Thr Arg Ile Leu Lys Ser Gln His Ala Ala Met Phe Arg His Phe
    130                 135                 140

Gln Gln Ile Met Phe Ile Tyr Asn Asp Thr Ile Ala Ala Lys Gln Glu
145                 150                 155                 160

Lys Cys Lys Thr Phe Ile Leu Arg Gln Leu Glu Val Ala Gly Lys Glu
                165                 170                 175
```

```
Met Ser Glu Glu Asp Val Asn Asp Met Leu His Gln Gly Lys Trp Glu
            180                 185                 190

Val Phe Asn Glu Ser Leu Leu Thr Glu Ile Asn Ile Thr Lys Ala Gln
        195                 200                 205

Leu Ser Glu Ile Glu Gln Arg His Lys Glu Leu Val Asn Leu Glu Asn
    210                 215                 220

Gln Ile Lys Asp Leu Arg Asp Leu Phe Ile Gln Ile Ser Leu Leu Val
225                 230                 235                 240

Glu Glu Gln Gly Glu Ser Ile Asn Asn Ile Glu Met Thr Val Asn Ser
                245                 250                 255

Thr Lys Glu Tyr Val Asn Asn Thr Lys Glu Lys Phe Gly Leu Ala Val
            260                 265                 270

Lys Tyr Lys Lys Arg Asn Pro Cys Arg Val Leu Cys Cys Trp Cys Cys
        275                 280                 285

Pro Cys Cys Ser Ser Lys
    290
```

<210> 22
<211> 1198
<212> DNA
<213> Homo sapiens

<400> 22

```
gtgtgaagta agcaatctat tttcacttgc tattaacagc aaacgtacaa ctttatctct      60
tatttgactt cattatttgg ctttcaaatt aacaacatga aaactataca gaaattagtc     120
tgtgccctat aagacagaga tttagataat ttgttcaaaa catgagatga tattcagata     180
ttctctcaaa ggcaggactc caacaccgca ggtggaaaac caacaaaagg aagaaaacag     240
aaaggaggaa agggaagatg aaagaccgac ttcaagaact aaagcagaga acaaaggaaa     300
ttgaactctc tagagacagt catgtatcaa ctacagaaac agaggaacaa ggggtgtttc     360
tacagcaagc tgttatttat gaaagagagc ctgtagctga gagacaccta catgaaatcc     420
aaaaactaca ggaaagtatt aacaatttgg cagataatgt tcaaaaattt gggcagcaac     480
agaaaagtct ggtggcttca atgagaaggt ttagtctact taagagagag tctaccatta     540
caaaggagat aaaaattcag gcagaataca tcaacagaag tttgaatgat ttagttaaag     600
aagttaaaaa gtcagaggtt gaaaatggtc catcttcagt ggtcacaagg atacttaaat     660
ctcagcatgc tgcaatgttc cgccattttc agcaaatcat gtttatatac aatgacacaa     720
tagcagcaaa gcaagagaag tgcaagacat ttattttacg tcagcttgaa gttgctggaa     780
aagagatgtc tgaagaagat gtaaatgata tgcttcatca aggaaaatgg gaagttttta     840
atgaaagctt acttacagaa atcaatatca ctaaagcaca actttcagag attgaacaga     900
gacacaagga acttgttaat ttggagaacc aaataaagga tttaagggat cttttcattc     960
agatatctct tttagtagag gaacaaggag agagcatcaa caatattgaa atgacagtga    1020
atagtacaaa agagtatgtt aacaatacta aagagaaatt tggactagct gtaaaataca    1080
aaaaaagaaa tccttgcaga gtactgtgtt gttggtgctg tccatgctgt agctcaaaat    1140
aaagaagcta ttttaaaaaa aaaaaaaaaa aaaaaaaaaa aaaaaaaaaa aaaaaaaa      1198
```

<210> 23
<211> 1044
<212> PRT
<213> Homo sapiens

<400> 23

```
Met Lys Pro Gly Cys Ala Ala Gly Ser Pro Gly Asn Glu Trp Ile Phe
1               5                   10                  15

Phe Ser Thr Asp Glu Ile Thr Thr Arg Tyr Arg Asn Thr Met Ser Asn
            20                  25                  30

Gly Gly Leu Gln Arg Ser Val Ile Leu Ser Ala Leu Ile Leu Leu Arg
        35                  40                  45

Ala Val Thr Gly Phe Ser Gly Asp Gly Arg Ala Ile Trp Ser Lys Asn
    50                  55                  60

Pro Asn Phe Thr Pro Val Asn Glu Ser Gln Leu Phe Leu Tyr Asp Thr
65                  70                  75                  80

Phe Pro Lys Asn Phe Phe Trp Gly Ile Gly Thr Gly Ala Leu Gln Val
                85                  90                  95

Glu Gly Ser Trp Lys Lys Asp Gly Lys Gly Pro Ser Ile Trp Asp His
            100                 105                 110

Phe Ile His Thr His Leu Lys Asn Val Ser Ser Thr Asn Gly Ser Ser
        115                 120                 125

Asp Ser Tyr Ile Phe Leu Glu Lys Asp Leu Ser Ala Leu Asp Phe Ile
    130                 135                 140

Gly Val Ser Phe Tyr Gln Phe Ser Ile Ser Trp Pro Arg Leu Phe Pro
145                 150                 155                 160
```

Asp Gly Ile Val Thr Val Ala Asn Ala Lys Gly Leu Gln Tyr Tyr Ser
165 170 175

Thr Leu Leu Asp Ala Leu Val Leu Arg Asn Ile Glu Pro Ile Val Thr
180 185 190

Leu Tyr His Trp Asp Leu Pro Leu Ala Leu Gln Glu Lys Tyr Gly Gly
195 200 205

Trp Lys Asn Asp Thr Ile Ile Asp Ile Phe Asn Asp Tyr Ala Thr Tyr
210 215 220

Cys Phe Gln Met Phe Gly Asp Arg Val Lys Tyr Trp Ile Thr Ile His
225 230 235 240

Asn Pro Tyr Leu Val Ala Trp His Gly Tyr Gly Thr Gly Met His Ala
245 250 255

Pro Gly Glu Lys Gly Asn Leu Ala Ala Val Tyr Thr Val Gly His Asn
260 265 270

Leu Ile Lys Ala His Ser Lys Val Trp His Asn Tyr Asn Thr His Phe
275 280 285

Arg Pro His Gln Lys Gly Trp Leu Ser Ile Thr Leu Gly Ser His Trp
290 295 300

Ile Glu Pro Asn Arg Ser Glu Asn Thr Met Asp Ile Phe Lys Cys Gln
305 310 315 320

Gln Ser Met Val Ser Val Leu Gly Trp Phe Ala Asn Pro Ile His Gly
325 330 335

Asp Gly Asp Tyr Pro Glu Gly Met Arg Lys Lys Leu Phe Ser Val Leu
340 345 350

Pro Ile Phe Ser Glu Ala Glu Lys His Glu Met Arg Gly Thr Ala Asp
355 360 365

Phe Phe Ala Phe Ser Phe Gly Pro Asn Asn Phe Lys Pro Leu Asn Thr
370 375 380

Met Ala Lys Met Gly Gln Asn Val Ser Leu Asn Leu Arg Glu Ala Leu
385 390 395 400

Asn Trp Ile Lys Leu Glu Tyr Asn Asn Pro Arg Ile Leu Ile Ala Glu
405 410 415

```
Asn Gly Trp Phe Thr Asp Ser Arg Val Lys Thr Glu Asp Thr Thr Ala
            420                 425                 430

Ile Tyr Met Met Lys Asn Phe Leu Ser Gln Val Leu Gln Ala Ile Arg
        435                 440                 445

Leu Asp Glu Ile Arg Val Phe Gly Tyr Thr Ala Trp Ser Leu Leu Asp
    450                 455                 460

Gly Phe Glu Trp Gln Asp Ala Tyr Thr Ile Arg Arg Gly Leu Phe Tyr
465                 470                 475                 480

Val Asp Phe Asn Ser Lys Gln Lys Glu Arg Lys Pro Lys Ser Ser Ala
                485                 490                 495

His Tyr Tyr Lys Gln Ile Ile Arg Glu Asn Gly Phe Ser Leu Lys Glu
            500                 505                 510

Ser Thr Pro Asp Val Gln Gly Gln Phe Pro Cys Asp Phe Ser Trp Gly
        515                 520                 525

Val Thr Glu Ser Val Leu Lys Pro Glu Ser Val Ala Ser Ser Pro Gln
    530                 535                 540

Phe Ser Asp Pro His Leu Tyr Val Trp Asn Ala Thr Gly Asn Arg Leu
545                 550                 555                 560

Leu His Arg Val Glu Gly Val Arg Leu Lys Thr Arg Pro Ala Gln Cys
                565                 570                 575

Thr Asp Phe Val Asn Ile Lys Lys Gln Leu Glu Met Leu Ala Arg Met
            580                 585                 590

Lys Val Thr His Tyr Arg Phe Ala Leu Asp Trp Ala Ser Val Leu Pro
        595                 600                 605

Thr Gly Asn Leu Ser Ala Val Asn Arg Gln Ala Leu Arg Tyr Tyr Arg
    610                 615                 620

Cys Val Val Ser Glu Gly Leu Lys Leu Gly Ile Ser Ala Met Val Thr
625                 630                 635                 640

Leu Tyr Tyr Pro Thr His Ala His Leu Gly Leu Pro Glu Pro Leu Leu
                645                 650                 655

His Ala Asp Gly Trp Leu Asn Pro Ser Thr Ala Glu Ala Phe Gln Ala
            660                 665                 670
```

```
Tyr Ala Gly Leu Cys Phe Gln Glu Leu Gly Asp Leu Val Lys Leu Trp
        675                 680                 685

Ile Thr Ile Asn Glu Pro Asn Arg Leu Ser Asp Ile Tyr Asn Arg Ser
    690                 695                 700

Gly Asn Asp Thr Tyr Gly Ala Ala His Asn Leu Leu Val Ala His Ala
705                 710                 715                 720

Leu Ala Trp Arg Leu Tyr Asp Arg Gln Phe Arg Pro Ser Gln Arg Gly
                725                 730                 735

Ala Val Ser Leu Ser Leu His Ala Asp Trp Ala Glu Pro Ala Asn Pro
            740                 745                 750

Tyr Ala Asp Ser His Trp Arg Ala Ala Glu Arg Phe Leu Gln Phe Glu
        755                 760                 765

Ile Ala Trp Phe Ala Glu Pro Leu Phe Lys Thr Gly Asp Tyr Pro Ala
    770                 775                 780

Ala Met Arg Glu Tyr Ile Ala Ser Lys His Arg Arg Gly Leu Ser Ser
785                 790                 795                 800

Ser Ala Leu Pro Arg Leu Thr Glu Ala Glu Arg Arg Leu Leu Lys Gly
                805                 810                 815

Thr Val Asp Phe Cys Ala Leu Asn His Phe Thr Thr Arg Phe Val Met
            820                 825                 830

His Glu Gln Leu Ala Gly Ser Arg Tyr Asp Ser Asp Arg Asp Ile Gln
        835                 840                 845

Phe Leu Gln Asp Ile Thr Arg Leu Ser Ser Pro Thr Arg Leu Ala Val
    850                 855                 860

Ile Pro Trp Gly Val Arg Lys Leu Leu Arg Trp Val Arg Arg Asn Tyr
865                 870                 875                 880

Gly Asp Met Asp Ile Tyr Ile Thr Ala Ser Gly Ile Asp Asp Gln Ala
                885                 890                 895

Leu Glu Asp Asp Arg Leu Arg Lys Tyr Tyr Leu Gly Lys Tyr Leu Gln
            900                 905                 910

Glu Val Leu Lys Ala Tyr Leu Ile Asp Lys Val Arg Ile Lys Gly Tyr
```

915 920 925

Tyr Ala Phe Lys Leu Ala Glu Glu Lys Ser Lys Pro Arg Phe Gly Phe
930 935 940

Phe Thr Ser Asp Phe Lys Ala Lys Ser Ser Ile Gln Phe Tyr Asn Lys
945 950 955 960

Val Ile Ser Ser Arg Gly Phe Pro Phe Glu Asn Ser Ser Ser Arg Cys
965 970 975

Ser Gln Thr Gln Glu Asn Thr Glu Cys Thr Val Cys Leu Phe Leu Val
980 985 990

Gln Lys Lys Pro Leu Ile Phe Leu Gly Cys Cys Phe Phe Ser Thr Leu
995 1000 1005

Val Leu Leu Leu Ser Ile Ala Ile Phe Gln Arg Gln Lys Arg Arg
1010 1015 1020

Lys Phe Trp Lys Ala Lys Asn Leu Gln His Ile Pro Leu Lys Lys
1025 1030 1035

Gly Lys Arg Val Val Ser
1040

<210> 24
<211> 6079
<212> DNA
<213> Homo sapiens

<400> 24

```
atcctcagtc tcccagttca agctaatcat tgacagagct ttacaatcac aagcttttac      60
tgaagctttg ataagacagt ccagcagttg gtggcaaatg aagccaggct gtgcggcagg     120
atctccaggg aatgaatgga ttttcttcag cactgatgaa ataaccacac gctataggaa     180
tacaatgtcc aacgggggat tgcaaagatc tgtcatcctg tcagcactta ttctgctacg     240
agctgttact ggattctctg gagatggaag agctatatgg tctaaaaatc ctaattttac     300
tccggtaaat gaaagtcagc tgtttctcta tgacactttc cctaaaaact tttctgggg      360
tattgggact ggagcattgc aagtggaagg gagttggaag aaggatggaa aaggaccttc     420
tatatgggat catttcatcc acacacacct taaaaatgtc agcagcacga atggttccag     480
tgacagttat atttttctgg aaaaagactt atcagccctg gattttatag gagtttcttt     540
ttatcaattt tcaatttcct ggccaaggct tttccccgat ggaatagtaa cagttgccaa     600
cgcaaaaggt ctgcagtact acagtactct tctggacgct ctagtgctta gaaacattga     660
```

acctatagtt actttatacc actgggattt gcctttggca ctacaagaaa aatatggggg 720
gtggaaaaat gataccataa tagatatctt caatgactat gccacatact gtttccagat 780
gtttggggac cgtgtcaaat attggattac aattcacaac ccatatctag tggcttggca 840
tgggtatggg acaggtatgc atgcccctgg agagaaggga aatttagcag ctgtctacac 900
tgtgggacac aacttgatca aggctcactc gaaagtttgg cataactaca acacacattt 960
ccgcccacat cagaagggtt ggttatcgat cacgttggga tctcattgga tcgagccaaa 1020
ccggtcggaa aacacgatgg atatattcaa atgtcaacaa tccatggttt ctgtgcttgg 1080
atggtttgcc aaccctatcc atggggatgg cgactatcca gaggggatga gaaagaagtt 1140
gttctccgtt ctacccattt tctctgaagc agagaagcat gagatgagag gcacagctga 1200
tttctttgcc ttttcttttg gacccaacaa cttcaagccc ctaaacacca tggctaaaat 1260
gggacaaaat gtttcactta atttaagaga agcgctgaac tggattaaac tggaatacaa 1320
caaccctcga atcttgattg ctgagaatgg ctggttcaca gacagtcgtg tgaaaacaga 1380
agacaccacg gccatctaca tgatgaagaa tttcctcagc caggtgcttc aagcaataag 1440
gttagatgaa atacgagtgt ttggttatac tgcctggtct ctcctggatg gctttgaatg 1500
gcaggatgct tacaccatcc gccgaggatt attttatgtg gattttaaca gtaaacagaa 1560
agagcggaaa cctaagtctt cagcacacta ctacaaacag atcatacgag aaaatggttt 1620
ttctttaaaa gagtccacgc cagatgtgca gggccagttt ccctgtgact tctcctgggg 1680
tgtcactgaa tctgttctta agcccgagtc tgtggcttcg tccccacagt tcagcgatcc 1740
tcatctgtac gtgtggaacg ccactggcaa cagactgttg caccgagtgg aaggggtgag 1800
gctgaaaaca cgacccgctc aatgcacaga ttttgtaaac atcaaaaaac aacttgagat 1860
gttggcaaga atgaaagtca cccactaccg gtttgctctg gattgggcct cggtccttcc 1920
cactggcaac ctgtccgcgg tgaaccgaca ggccctgagg tactacaggt gcgtggtcag 1980
tgaggggctg aagcttggca tctccgcgat ggtcaccctg tattatccga cccacgccca 2040
cctaggcctc cccgagcctc tgttgcatgc cgacgggtgg ctgaacccat cgacggccga 2100
ggccttccag gcctacgctg ggctgtgctt ccaggagctg gggggacctgg tgaagctctg 2160
gatcaccatc aacgagccta accggctaag tgacatctac aaccgctctg gcaacgacac 2220
ctacggggcg gcgcacaacc tgctggtggc ccacgccctg gcctggcgcc tctacgaccg 2280
gcagttcagg ccctcacagc gcggggccgt gtcgctgtcg ctgcacgcgg actgggcgga 2340
acccgccaac ccctatgctg actcgcactg gagggcggcc gagcgcttcc tgcagttcga 2400
gatcgcctgg ttcgccgagc cgctcttcaa gaccggggac taccccgcgg ccatgaggga 2460
atacattgcc tccaagcacc gacgggggct ttccagctcg gccctgccgc gcctcaccga 2520
ggccgaaagg aggctgctca agggcacggt cgacttctgc gcgctcaacc acttcaccac 2580

taggttcgtg atgcacgagc agctggccgg cagccgctac gactcggaca gggacatcca 2640
gtttctgcag gacatcaccc gcctgagctc ccccacgcgc ctggctgtga ttccctgggg 2700
ggtgcgcaag ctgctgcggt gggtccggag gaactacggc gacatggaca tttacatcac 2760
cgccagtggc atcgacgacc aggctctgga ggatgaccgg ctccggaagt actacctagg 2820
gaagtacctt caggaggtgc tgaaagcata cctgattgat aaagtcagaa tcaaaggcta 2880
ttatgcattc aaactggctg aagagaaatc taaacccaga tttggattct tcacatctga 2940
ttttaaagct aaatcctcaa tacaatttta caacaaagtg atcagcagca ggggcttccc 3000
ttttgagaac agtagttcta gatgcagtca gacccaagaa aatacagagt gcactgtctg 3060
cttattcctt gtgcagaaga aaccactgat attcctgggt tgttgcttct tctccaccct 3120
ggttctactc ttatcaattg ccatttttca aaggcagaag agaagaaagt tttggaaagc 3180
aaaaaactta caacacatac cattaaagaa aggcaagaga gttgttagct aaactgatct 3240
gtctgcatga tagacagttt aaaaattcat cccagttcca tatgctggta acttacagga 3300
gatatacctg tattatagaa agacaatctg agatacagct gtaaccaagg tgatgacaat 3360
tgtctctgct gtgtggttca aagaacattc ccttaggtgt tgacatcagt gaactcagtt 3420
cttggatgta aacataaagg cttcatcctg acagtaagct atgaggatta catgctacat 3480
tgcttcttaa agtttcatca actgtattcc atcattctgc tttagctttc atctctacca 3540
atagctactt gtggtacaat aaattatttt taagaagtaa aactctgggg ctggacgctg 3600
tggctcacac ctgtaatctc agcactttgg gaggccgagg cggggagatc acctgaggtg 3660
aggagttcga gaccagcctg gccaacatgg tgaaaccatg tctctactaa aaatacaaaa 3720
aattagccag gcgtggtgac agtggcacct gtaatcccag ctacttggga ggctgaggca 3780
gaagtttgaa cccaggaaac aggttacagt aggccaaaat tgcgccactg cactccagcc 3840
taggcgacaa cagcaagact gtgtccaaaa aaaaaaaaaa aagcaaaagc aaaactttgt 3900
tttgttagac tctacagcag agatttaaca cccttcttta aactgggtag tcagtgatag 3960
ataatatata ttctgtcact tctaataagg tgccttctcc tttaggtcag ggtggttcta 4020
aaatggaaag aaaacacaat agggtaagta gtgcttgtct aagccagtta caacacagac 4080
tcttaaagag gatcaagccc ttcatttttc taacaacaaa aaatcaccta tagaatatct 4140
aatttgtgat cttttactag atctgatttt ttaaaataat gtaatttccg gccaggcacg 4200
gtggcaccgc ctgtaatccc agcactttgg gaggccaagg caggtggatc acctgaggtt 4260
aggagttcga gactagcctg gccaacatgg caaaacccca tctctactaa aaatacaaaa 4320
gttagccggg catggtggtg ggcacctgta atcccagcta ctcaggaggc cgaggcagga 4380
gaatcgcttg aacccgagag gcagaggttg caatgagcca agatcgtgcc attgcactcc 4440

```
agcctggggg acagggcaag actgtctctc aaaataaaaa aaaataataa aaataaaaat      4500
aaaagtaatt tccaaaacct catctcatgg aaagatcaca ggatgaagga aagctagact      4560
caactctgtg aatagaagtt gctatactgt aagtaaagca acaattcaga atactgaatg      4620
agtttaaatt gttttatata gcaccctttt gggctagggt taattactag atctgacttg      4680
gataatttga cactttggga aatgaactct gttcttgaga cttgttcagt gtattttaaa      4740
catctgagga agaaaactta aatatgcacc tatttatacc tattctttct ttaggtcaac      4800
atttaacacc cactgcatac attaatttgt ccttgtctgc tcactccagc aatttagacc      4860
ttaacagtca caagagacgt tcttctgtta caaagcctta gtaaattaag gcagttttga      4920
ttatattcta ggtccaccta tgtctgaagc taaattcagt atctaactgc taatgaacaa      4980
gtttccaaaa tactgtaaaa atacaattag tcaatttgag taaatgcaaa tatgatgaga      5040
aatcaatttg ctatttggcc tggcaaatgg gaacagtaaa attctgcttt actcttctct      5100
agtctccttg ccccagctgc acccactacc ccaaagttgg cagttttgag gtatgatttt      5160
caaggaattt ttttagtatt aacatctccc tctgagaact atgtacctaa ggtcacgcat      5220
acaactagtc aattctgttt ttattactct aactatgtag aaacagtaag tcacttaaaa      5280
caatcacttg gctgggtttt ttcccctttg tgccacattg attcaccctg acccaagaac      5340
tccagggaaa attctttaat gtcaactggg caactcatta acctctcttt aacatcaagg      5400
gcttgggaaa aaaaaaaaaa aggttagcca caggaataac aaaaacctgg aatttatctt      5460
tcaggttttg ctttctcttt ctcactttgt ttaaagtatc tcgtactcac agttcacaaa      5520
ttaaccttca ctgtctcttt cacattaaga gcttatgctt aaagcatgcc ccccttttct      5580
aacttgctgg tttaccataa actcccctaa gtaataaaat tcctaaccca gtactgagag      5640
tcctccttct ctgccacttg ggcattattt tactagtttt taagccatca tcgcacaaga      5700
atccaaaaac ccttaaattt tttaaccact ggcaaatatg tacagcaaat taggttaagc      5760
atttaatctg gctcatgctc tatcatacta aatattcagg tttatcataa actccttaaa      5820
aaccatcaaa ggtcaaccag aaactgataa ctcttgaaag gagcaaacag gtaagatctt      5880
tggagtttaa gcttttctga gatgtgttgt gaaaaatcta acgtgtttat cgtatattca      5940
atgtaacaac ctggagaatc acaactatat ttaaagagcc tctggaaaat gaggccagta      6000
cagtgtgact acatgtttaa ttttcaatgt aatttattcc aaataaactg gttcatgctg      6060
accacttgta ttcaactaa                                                  6079
```

<210> 25
<211> 326
<212> PRT
<213> Homo sapiens

<400> 25

```
Met Thr Gly Leu Cys Gly Tyr Ser Ala Pro Asp Met Arg Gly Leu Arg
1               5                   10                  15

Leu Ile Met Ile Pro Val Glu Leu Leu Leu Cys Tyr Leu Leu Leu His
            20                  25                  30

Pro Val Asp Ala Thr Ser Tyr Gly Lys Gln Thr Asn Val Leu Met His
        35                  40                  45

Phe Pro Leu Ser Leu Glu Ser Gln Thr Pro Ser Ser Asp Pro Leu Ser
    50                  55                  60

Cys Gln Phe Leu His Pro Lys Ser Leu Pro Gly Phe Ser His Met Ala
65                  70                  75                  80

Pro Leu Pro Lys Phe Leu Val Ser Leu Ala Leu Arg Asn Ala Leu Glu
                85                  90                  95

Glu Ala Gly Cys Gln Ala Asp Val Trp Ala Leu Gln Leu Gln Leu Tyr
            100                 105                 110

Arg Gln Gly Gly Val Asn Ala Thr Gln Val Leu Ile Gln His Leu Arg
        115                 120                 125

Gly Leu Gln Lys Gly Arg Ser Thr Glu Arg Asn Val Ser Val Glu Ala
    130                 135                 140

Leu Ala Ser Ala Leu Gln Leu Leu Ala Arg Glu Gln Gln Ser Thr Gly
145                 150                 155                 160

Arg Val Gly Arg Ser Leu Pro Thr Glu Asp Cys Glu Asn Glu Lys Glu
                165                 170                 175

Gln Ala Val His Asn Val Val Gln Leu Leu Pro Gly Val Gly Thr Phe
            180                 185                 190

Tyr Asn Leu Gly Thr Ala Leu Tyr Tyr Ala Thr Gln Asn Cys Leu Gly
        195                 200                 205

Lys Ala Arg Glu Arg Gly Arg Asp Gly Ala Ile Asp Leu Gly Tyr Asp
    210                 215                 220

Leu Leu Met Thr Met Ala Gly Met Ser Gly Gly Pro Met Gly Leu Ala
225                 230                 235                 240

Ile Ser Ala Ala Leu Lys Pro Ala Leu Arg Ser Gly Val Gln Gln Leu
                245                 250                 255
```

```
Ile Gln Tyr Tyr Gln Asp Gln Lys Asp Ala Asn Ile Ser Gln Pro Glu
            260                 265                 270

Thr Thr Lys Glu Gly Leu Arg Ala Ile Ser Asp Val Ser Asp Leu Glu
        275                 280                 285

Glu Thr Thr Thr Leu Ala Ser Phe Ile Ser Glu Val Val Ser Ser Ala
    290                 295                 300

Pro Tyr Trp Gly Trp Ala Ile Ile Lys Ser Tyr Asp Leu Asp Pro Gly
305                 310                 315                 320

Ala Gly Ser Leu Glu Ile
                325
```

<210> 26
<211> 1722
<212> DNA
<213> Homo sapiens

<400> 26

```
aggcagaaca ggatcaggaa gcgatcaaac ctaccaaggc agtctcactt ctcaatgact      60
ggactgtgtg ggtactctgc tccagacatg cgtggcctca gactcatcat gataccagtt     120
gagctgctac tttgctacct cctgctgcac cctgtggatg ccacttcata tggaaagcag     180
acaaatgtct tgatgcactt tcccttgtcc ttggaatccc agacaccctc ctcagacccc     240
ttgtcctgcc aatttctgca cccaaagtca ctgcctggtt tcagccacat ggcccctcta     300
cccaagttct tggtaagcct ggctctaagg aatgccctgg aggaagctgg ttgtcaggct     360
gatgtttggg ctctacagct acagctctac cgccagggtg gtgtgaatgc tacacaggtc     420
ctcatccagc atcttcgagg gctccagaaa ggcagaagca cagagaggaa cgtgtcagtg     480
gaagccctgg cctctgctct gcagctgtta gccagggagc agcaaagcac aggaagggtc     540
gggcgctccc tcccgacaga ggactgtgag aatgagaagg agcaagctgt gcacaatgta     600
gtccagctgc tgccaggagt gggaaccttc tacaacctgg gcacagcttt gtattatgct     660
actcaaaact gcctgggcaa ggccagggaa cgaggccgag atggggccat agatctggga     720
tatgaccttc tgatgaccat ggctgggatg tcaggggggc ctatgggtct agcgatcagt     780
gctgcactta aacctgcatt aaggtctggg gttcagcagt tgatccagta ttaccaagat     840
cagaaagacg caaacatctc tcagccggag accaccaagg agggtttgag ggccatctca     900
gatgtgagtg acttggaaga aacaactact ctggcttctt tcatatcaga agtagtaagt     960
tcagctccct actgggggtg ggccataatc aagagctatg acttagatcc tggggctggg    1020
agtcttgaga tataaaagaa tgtggtaacc acagaattaa taactgtact accctgacaa    1080
```

```
gctatataca tgtcttcaaa attttaatct gatttatcca ggaggaaggc tgtacagtaa     1140
aacgtaagaa cgtaaatgtt tgggtgttga agtcacaggg tttggtttcg aatctaggct     1200
ccacttgtta gagcctcggt gatcactgaa tagtaacttc tttcttgaac taagatcagt     1260
tttgaagttt ctaaaggaga tagaatgatt ttaacctcaa tgagttgccc tgtaaattta     1320
aaatgataca atgaatctaa aatgcttatc acagtacttt caataaatag ctattagcca     1380
ggtgcggtgg ctcacgcctg taatcccagc actgtgagag gctgaggcgg gatgatcacc     1440
tgaggtcagg agttcaagat cagcctgggc aacatggcga aaccccgtct ctacaataaa     1500
tacaaaaaat tatcctggcg gagttatgca cgcttgtagt cccaactacc tgggaggctg     1560
aggcgggaga atcacctgag cctgggaggt cgaggctgca gcgagccgag atcgcgccgc     1620
tgcattccag cctgggtgac agagcgagac catgtctcaa aaaaaataaa aataaaaaaa     1680
aattgttttc acaaaaaaaa aaaaaaaaaa aaaaaaaaaa aa                        1722
```

<210> 27
<211> 3653
<212> DNA
<213> Homo sapiens

<400> 27

```
gcgccagcgc cggcgggacc cgagctgcta atgaggcagc catgactggc cacttcatgt      60
gctcctggag aagggcttgc accagccgtt ttcaggaaag tcaagcagct gttgactcct     120
gagtctgggt gaatttgtgt gaagagcata aggcgctgtt tcttaaccaa aacgcttcct     180
cttgcagtgc agatgggatg tgcttctcca caggaggccc cacggcttcc ccacccctca     240
gaggagcgcc gtgcgtgcgt ctgtgtggag gattggcagc tcctgcagtc ggcccttggt     300
cctatttggc gacgcctctg ccttcccctt aattatacag tcatgagccg ccctggaatc     360
acggcagctc cggatggatc ctggatgcca gaatgcagcc tcagcacggg gctgcaggat     420
aggagtgagc gaggggctgc agagccggcg gccgcggtgg gcaccatgga gggggctgcc     480
ctgggcagca cgggcatgag tctcaaggcc caggtttgag taacaggtgt tgagagctta     540
cttacttttc ctgagacaca gtttcctcat ctcgagagca cggaaaatca ttctaacttc     600
agaggattgt tgtgaaagtt aaatgagatt aaagaggtaa agcccatgac gtgcttagct     660
cgtgcttggc tcttggtcaa tgccagttag cgctgcattt tctcccctct ccctccctcc     720
ttctctcttt cttttcttct attctccatt cctgttttct cccccacccc actccccaaa     780
gctctgcgtt gagaaccaga tgctgtctgg tgggttaggg ccagaggagg aaaagctgcc     840
tgccgtgggc tgcacccata ccctcttcat tccaatgaca tgaggggagg ggaaaggaca     900
gaggtagact gtcctcccct acctcctcct aatacaaatg gaattcctgg aactggaaaa     960
caaagaatac ccccataaaa ataagacagt acttctggtg cggtgtaata aaggggaaag    1020
```

taaccctcaa tgtcaggaaa ctccgcacct cccagctcat atttgtgtgg aggaaaagtt 1080
aaatattaat ttggactcaa ctgaatgtgg acacaaacaa tggtcaccaa gtcccggaac 1140
aggttgtgtg agcctcttca ggggttcatc cagcgctgtt ttggagaaat ctctatttca 1200
atttattcct atacgttagt tactgaaaaa caacagacaa tcgcaaaagc aagttgcccg 1260
ttttgtgttc cttgagccca atcatgaagt gccgtcgtga ctgggcctca tgacaaacaa 1320
cttgtaataa gtaacaacag agctcaggtc ccagaccgca ctgaagctct gtgagacctc 1380
tcctcatctg tgcatgaacg agtgtctgac tctggagccc agcctgctgc ttcccagtct 1440
ggtggtgaat cctccgtagt ctgatggagg tttgctcttg ttgcccaggc tggagtgcaa 1500
tggcacaatc tcggctcact gcagcccctg cctcccaggc tcaagcaatt cttatgcctc 1560
agcctcctga gtagatggaa ctacagggca tggactggaa ctcccaatac ccctgacatg 1620
ggctgagtca acgtggtcat gaacatgtga caggaggcag cagaagttgc agaaaagagt 1680
gaggcacgtt tgaaaaaggc tgaaaaatgt ttctgtccag gcaagggtgt gtgctgaatg 1740
actcaaggat tttttggaga gaattggagt gtctcaccag aggagaccac gtctgaaggg 1800
ctttgcatcc ctccttggac atgtctaata cctagcactc agaaagcatc cagtaaatat 1860
tcgtggaaag aaaggagtgg agaaggggag aaaggggaaa gggagtaggc gagagagaag 1920
aaagactctg cttcttgccc agggcctggc atggggcgga ggcaaagcag tggggtcctc 1980
agctatgtcc cactgtgagt gcacagcgag tcctgacctt cagagggtgc agcccgaggg 2040
gccctggcct gtctgaaggg tgcgccagcc gagtggcctg ctctgaccac caggctcacc 2100
catgactacc tgggtggcta cagccagttc ctgacaatga gtacagcact cagttatcgg 2160
ggcccttcca cccacacgct gtccacttcc tggggtactg ctgtgggcat gcgagtgctt 2220
gctccccggg gcactgctgt gggcatgcga gtgcttgctc cccggggcac tgctgtgggc 2280
atgtgagtgc ttgctcccca gctccactag tgacactggt ggccccttgc tggggccttc 2340
cccgcctgct ccgctccatt accgctgccg ggctcctcac gtctctcctt gctgcttcct 2400
gcactggggt gaggagagtg gggctggtcc ccttgagacc ggagaagctc caggctttta 2460
aggaaaactg ccagggacga agagaagata tcacttcccc acgtggttgg cttccagatt 2520
cagaaggaat gtctgtcctt gtggattccg taccagatga ccccagatgc tgcctcagta 2580
ctaggtccct gtggctctgg agcctttgct gggtctgggc agtgtctctt cctctccagt 2640
tcatccttgg gtctcttcac ccttgccagg ggcaggcttc ctggtgagag gtcgacctcc 2700
tgcatgaagg ctctcaagag gccagttcaa agccaagctc cgggtctgtg cctgtggggc 2760
tgctcctcga tcaggagatg gtcactcccc tcctggtctg tatctgtggg attctcctcc 2820
atcaggagat ggtctctccc ctcctggtct atacccgtgg gattctcctc catcaggaga 2880
tggtcactcc ccatcctggt ctatacccgt gggattctcc tccattagat ggtcactccc 2940

ctcctggtct attcccgtgg ggctgctcct ccatcaggag gtggtcactc cccctcctgg 3000

tctatacccg tggggctgct cctccatcag gagatagtca ctccccctcc tggtctatac 3060

ccgtgggatt ctcctccatc tggagatggt cactcccctc ctggtctata cccatgggat 3120

tctcctccat ctggagatgg tcactcccct cctggtctat acccatggga ttctcctcca 3180

tctggagata gtcactcccc tcctggtcta tacccgtggg gttctcctca atcaggaggt 3240

ggtcactccc ctcctggtct atacccgtgg gattctcctc catcaggaga tggtcactct 3300

ccctcctggt ctatacccgt ggggctgctc ctccatcagg agatggtcac tcccctcctg 3360

gtctatacct gtgggattct cctccctcag aagatggtca ctccccctcc tggtctatac 3420

ccatgggatt ctcctccctc agaagatggt cactccccct cctggtctat acccgtgggg 3480

ctgctcctcc atcaggagat ggtcactctc cctctcggtt gctcagtcca aaaacaacct 3540

ctctggaaaa ctgcgtggaa ttttttttta aagaattgaa actagaacta gcatttgatc 3600

cagccatctg cctactggga atacacccaa agaaaaataa atcattatat cag 3653

<210> 28
<211> 1214
<212> PRT
<213> Homo sapiens

<400> 28

```
Met Val Val Pro Glu Lys Glu Gln Ser Trp Ile Pro Lys Ile Phe Lys
1               5                   10                  15

Lys Lys Thr Cys Thr Thr Phe Ile Val Asp Ser Thr Asp Pro Gly Gly
            20                  25                  30

Thr Leu Cys Gln Cys Gly Arg Pro Arg Thr Ala His Pro Ala Val Ala
        35                  40                  45

Met Glu Asp Ala Phe Gly Ala Ala Val Val Thr Val Trp Asp Ser Asp
    50                  55                  60

Ala His Thr Thr Glu Lys Pro Thr Asp Ala Tyr Gly Glu Leu Asp Phe
65                  70                  75                  80

Thr Gly Ala Gly Arg Lys His Ser Asn Phe Leu Arg Leu Ser Asp Arg
                85                  90                  95

Thr Asp Pro Ala Ala Val Tyr Ser Leu Val Thr Arg Thr Trp Gly Phe
            100                 105                 110

Arg Ala Pro Asn Leu Val Val Ser Val Leu Gly Gly Ser Gly Gly Pro
        115                 120                 125
```

```
Val Leu Gln Thr Trp Leu Gln Asp Leu Leu Arg Arg Gly Leu Val Arg
    130                 135                 140

Ala Ala Gln Ser Thr Gly Ala Trp Ile Val Thr Gly Gly Leu His Thr
145                 150                 155                 160

Gly Ile Gly Arg His Val Gly Val Ala Val Arg Asp His Gln Met Ala
                165                 170                 175

Ser Thr Gly Gly Thr Lys Val Val Ala Met Gly Val Ala Pro Trp Gly
            180                 185                 190

Val Val Arg Asn Arg Asp Thr Leu Ile Asn Pro Lys Gly Ser Phe Pro
        195                 200                 205

Ala Arg Tyr Arg Trp Arg Gly Asp Pro Glu Asp Gly Val Gln Phe Pro
    210                 215                 220

Leu Asp Tyr Asn Tyr Ser Ala Phe Phe Leu Val Asp Asp Gly Thr His
225                 230                 235                 240

Gly Cys Leu Gly Gly Glu Asn Arg Phe Arg Leu Arg Leu Glu Ser Tyr
                245                 250                 255

Ile Ser Gln Gln Lys Thr Gly Val Gly Gly Thr Gly Ile Asp Ile Pro
            260                 265                 270

Val Leu Leu Leu Leu Ile Asp Gly Asp Glu Lys Met Leu Thr Arg Ile
        275                 280                 285

Glu Asn Ala Thr Gln Ala Gln Leu Pro Cys Leu Leu Val Ala Gly Ser
    290                 295                 300

Gly Gly Ala Ala Asp Cys Leu Ala Glu Thr Leu Glu Asp Thr Leu Ala
305                 310                 315                 320

Pro Gly Ser Gly Gly Ala Arg Gln Gly Glu Ala Arg Asp Arg Ile Arg
                325                 330                 335

Arg Phe Phe Pro Lys Gly Asp Leu Glu Val Leu Gln Ala Gln Val Glu
            340                 345                 350

Arg Ile Met Thr Arg Lys Glu Leu Leu Thr Val Tyr Ser Ser Glu Asp
        355                 360                 365

Gly Ser Glu Glu Phe Glu Thr Ile Val Leu Lys Ala Leu Val Lys Ala
    370                 375                 380
```

Cys Gly Ser Ser Glu Ala Ser Ala Tyr Leu Asp Glu Leu Arg Leu Ala
385 390 395 400

Val Ala Trp Asn Arg Val Asp Ile Ala Gln Ser Glu Leu Phe Arg Gly
405 410 415

Asp Ile Gln Trp Arg Ser Phe His Leu Glu Ala Ser Leu Met Asp Ala
420 425 430

Leu Leu Asn Asp Arg Pro Glu Phe Val Arg Leu Leu Ile Ser His Gly
435 440 445

Leu Ser Leu Gly His Phe Leu Thr Pro Met Arg Leu Ala Gln Leu Tyr
450 455 460

Ser Ala Ala Pro Ser Asn Ser Leu Ile Arg Asn Leu Leu Asp Gln Ala
465 470 475 480

Ser His Ser Ala Gly Thr Lys Ala Pro Ala Leu Lys Gly Gly Ala Ala
485 490 495

Glu Leu Arg Pro Pro Asp Val Gly His Val Leu Arg Met Leu Leu Gly
500 505 510

Lys Met Cys Ala Pro Arg Tyr Pro Ser Gly Gly Ala Trp Asp Pro His
515 520 525

Pro Gly Gln Gly Phe Gly Glu Ser Met Tyr Leu Leu Ser Asp Lys Ala
530 535 540

Thr Ser Pro Leu Ser Leu Asp Ala Gly Leu Gly Gln Ala Pro Trp Ser
545 550 555 560

Asp Leu Leu Leu Trp Ala Leu Leu Leu Asn Arg Ala Gln Met Ala Met
565 570 575

Tyr Phe Trp Glu Met Gly Ser Asn Ala Val Ser Ser Ala Leu Gly Ala
580 585 590

Cys Leu Leu Leu Arg Val Met Ala Arg Leu Glu Pro Asp Ala Glu Glu
595 600 605

Ala Ala Arg Arg Lys Asp Leu Ala Phe Lys Phe Glu Gly Met Gly Val
610 615 620

Asp Leu Phe Gly Glu Cys Tyr Arg Ser Ser Glu Val Arg Ala Ala Arg

625 630 635 640

Leu Leu Leu Arg Arg Cys Pro Leu Trp Gly Asp Ala Thr Cys Leu Gln
645 650 655

Leu Ala Met Gln Ala Asp Ala Arg Ala Phe Phe Ala Gln Asp Gly Val
660 665 670

Gln Ser Leu Leu Thr Gln Lys Trp Trp Gly Asp Met Ala Ser Thr Thr
675 680 685

Pro Ile Trp Ala Leu Val Leu Ala Phe Phe Cys Pro Pro Leu Ile Tyr
690 695 700

Thr Arg Leu Ile Thr Phe Arg Lys Ser Glu Glu Glu Pro Thr Arg Glu
705 710 715 720

Glu Leu Glu Phe Asp Met Asp Ser Val Ile Asn Gly Glu Gly Pro Val
725 730 735

Gly Thr Ala Asp Pro Ala Glu Lys Thr Pro Leu Gly Val Pro Arg Gln
740 745 750

Ser Gly Arg Pro Gly Cys Cys Gly Gly Arg Cys Gly Gly Arg Arg Cys
755 760 765

Leu Arg Arg Trp Phe His Phe Trp Gly Ala Pro Val Thr Ile Phe Met
770 775 780

Gly Asn Val Val Ser Tyr Leu Leu Phe Leu Leu Leu Phe Ser Arg Val
785 790 795 800

Leu Leu Val Asp Phe Gln Pro Ala Pro Pro Gly Ser Leu Glu Leu Leu
805 810 815

Leu Tyr Phe Trp Ala Phe Thr Leu Leu Cys Glu Glu Leu Arg Gln Gly
820 825 830

Leu Ser Gly Gly Gly Gly Ser Leu Ala Ser Gly Gly Pro Gly Pro Gly
835 840 845

His Ala Ser Leu Ser Gln Arg Leu Arg Leu Tyr Leu Ala Asp Ser Trp
850 855 860

Asn Gln Cys Asp Leu Val Ala Leu Thr Cys Phe Leu Leu Gly Val Gly
865 870 875 880

```
Cys Arg Leu Thr Pro Gly Leu Tyr His Leu Gly Arg Thr Val Leu Cys
                885                 890                 895

Ile Asp Phe Met Val Phe Thr Val Arg Leu Leu His Ile Phe Thr Val
            900                 905                 910

Asn Lys Gln Leu Gly Pro Lys Ile Val Ile Val Ser Lys Met Met Lys
        915                 920                 925

Asp Val Phe Phe Phe Leu Phe Phe Leu Gly Val Trp Leu Val Ala Tyr
    930                 935                 940

Gly Val Ala Thr Glu Gly Leu Leu Arg Pro Arg Asp Ser Asp Phe Pro
945                 950                 955                 960

Ser Ile Leu Arg Arg Val Phe Tyr Arg Pro Tyr Leu Gln Ile Phe Gly
                965                 970                 975

Gln Ile Pro Gln Glu Asp Met Asp Val Ala Leu Met Glu His Ser Asn
            980                 985                 990

Cys Ser Ser Glu Pro Gly Phe Trp  Ala His Pro Pro Gly  Ala Gln Ala
        995                 1000                 1005

Gly Thr  Cys Val Ser Gln Tyr  Ala Asn Trp Leu Val  Val Leu Leu
    1010                 1015                 1020

Leu Val  Ile Phe Leu Leu Val  Ala Asn Ile Leu Leu  Val Asn Leu
    1025                 1030                 1035

Leu Ile  Ala Met Phe Ser Tyr  Thr Phe Gly Lys Val  Gln Gly Asn
    1040                 1045                 1050

Ser Asp  Leu Tyr Trp Lys Ala  Gln Arg Tyr Arg Leu  Ile Arg Glu
    1055                 1060                 1065

Phe His  Ser Arg Pro Ala Leu  Ala Pro Pro Phe Ile  Val Ile Ser
    1070                 1075                 1080

His Leu  Arg Leu Leu Leu Arg  Gln Leu Cys Arg Arg  Pro Arg Ser
    1085                 1090                 1095

Pro Gln  Pro Ser Ser Pro Ala  Leu Glu His Phe Arg  Val Tyr Leu
    1100                 1105                 1110

Ser Lys  Glu Ala Glu Arg Lys  Leu Leu Thr Trp Glu  Ser Val His
    1115                 1120                 1125
```

```
Lys Glu  Asn Phe Leu Leu Ala  Arg Ala Arg Asp Lys  Arg Glu Ser
    1130                 1135                 1140

Asp Ser  Glu Arg Leu Lys Arg  Thr Ser Gln Lys Val  Asp Leu Ala
    1145                 1150                 1155

Leu Lys  Gln Leu Gly His Ile  Arg Glu Tyr Glu Gln  Arg Leu Lys
    1160                 1165                 1170

Val Leu  Glu Arg Glu Val Gln  Gln Cys Ser Arg Val  Leu Gly Trp
    1175                 1180                 1185

Val Ala  Glu Ala Leu Ser Arg  Ser Ala Leu Leu Pro  Pro Gly Gly
    1190                 1195                 1200

Pro Pro  Pro Pro Asp Leu Pro  Gly Ser Lys Asp
    1205                 1210
```

<210> 29
<211> 4103
<212> DNA
<213> Homo sapiens

<400> 29

```
aggctggaaa gtggaggatc cggtttgctc tgggcgggtc tggaagcaga gccggcggag       60
ggagcgccgg ggccctgggc tgcaggaggt tgcggcggcc gcggcagcat ggtggtgccg      120
gagaaggagc agagctggat ccccaagatc ttcaagaaga agacctgcac gacgttcata      180
gttgactcca cagatccggg agggaccttg tgccagtgtg ggcgcccccg gaccgcccac      240
cccgcagtgg ccatggagga tgccttcggg gcagccgtgg tgaccgtgtg ggacagcgat      300
gcacacacca cggagaagcc caccgatgcc tacggagagc tggacttcac gggggccggc      360
cgcaagcaca gcaatttcct ccggctctct gaccgaacgg atccagctgc agtttatagt      420
ctggtcacac gcacatgggg cttccgtgcc ccgaacctgg tggtgtcagt gctgggggga      480
tcgggggggcc ccgtcctcca gacctggctg caggacctgc tgcgtcgtgg gctggtgcgg      540
gctgcccaga gcacaggagc ctggattgtc actgggggtc tgcacacggg catcggccgg      600
catgttggtg tggctgtacg ggaccatcag atggccagca ctgggggcac caaggtggtg      660
gccatgggtg tggccccctg gggtgtggtc cggaatagag acaccctcat caaccccaag      720
ggctcgttcc ctgcgaggta ccggtggcgc ggtgacccgg aggacggggt ccagtttccc      780
ctggactaca actactcggc cttcttcctg gtggacgacg gcacacacgg ctgcctgggg      840
ggcgagaacc gcttccgctt gcgcctggag tcctacatct cacagcagaa gacgggcgtg      900
ggagggactg gaattgacat ccctgtcctg ctcctcctga ttgatggtga tgagaagatg      960
```

ttgacgcgaa tagagaacgc cacccaggct cagctcccat gtctcctcgt ggctggctca 1020

gggggagctg cggactgcct ggcggagacc ctggaagaca ctctggcccc agggagtggg 1080

ggagccaggc aaggcgaagc ccgagatcga atcaggcgtt tctttcccaa aggggacctt 1140

gaggtcctgc aggcccaggt ggagaggatt atgacccgga aggagctcct gacagtctat 1200

tcttctgagg atgggtctga ggaattcgag accatagttt tgaaggccct tgtgaaggcc 1260

tgtgggagct cggaggcctc agcctacctg gatgagctgc gtttggctgt ggcttggaac 1320

cgcgtggaca ttgcccagag tgaactcttt cggggggaca tccaatggcg gtccttccat 1380

ctcgaagctt ccctcatgga cgccctgctg aatgaccggc ctgagttcgt gcgcttgctc 1440

atttcccacg gcctcagcct gggccacttc ctgaccccga tgcgcctggc ccaactctac 1500

agcgcggcgc cctccaactc gctcatccgc aacctttgg accaggcgtc ccacagcgca 1560

ggcaccaaag ccccagccct aaaaggggga gctgcggagc tccggccccc tgacgtgggg 1620

catgtgctga ggatgctgct ggggaagatg tgcgcgccga ggtacccctc cgggggcgcc 1680

tgggaccctc acccaggcca gggcttcggg gagagcatgt atctgctctc ggacaaggcc 1740

acctcgccgc tctcgctgga tgctggcctc gggcaggccc cctggagcga cctgcttctt 1800

tgggcactgt tgctgaacag ggcacagatg gccatgtact tctgggagat gggttccaat 1860

gcagtttcct cagctcttgg ggcctgtttg ctgctccggg tgatggcacg cctggagcct 1920

gacgctgagg aggcagcacg gaggaaagac ctggcgttca agtttgaggg gatgggcgtt 1980

gacctctttg gcgagtgcta tcgcagcagt gaggtgaggg ctgcccgcct cctcctccgt 2040

cgctgcccgc tctgggggga tgccacttgc ctccagctgg ccatgcaagc tgacgcccgt 2100

gccttctttg cccaggatgg ggtacagtct ctgctgacac agaagtggtg gggagatatg 2160

gccagcacta cacccatctg ggccctggtt ctcgccttct tttgccctcc actcatctac 2220

acccgcctca tcaccttcag gaaatcagaa gaggagccca cacgggagga gctagagttt 2280

gacatggata gtgtcattaa tggggaaggg cctgtcggga cggcggaccc agccgagaag 2340

acgccgctgg gggtcccgcg ccagtcgggc cgtccgggtt gctgcggggg ccgctgcggg 2400

gggcgccggt gcctacgccg ctggttccac ttctggggcg cgccggtgac catcttcatg 2460

ggcaacgtgg tcagctacct gctgttcctg ctgcttttct cgcgggtgct gctcgtggat 2520

ttccagccgg cgccgcccgg ctccctggag ctgctgctct atttctgggc tttcacgctg 2580

ctgtgcgagg aactgcgcca gggcctgagc ggaggcgggg gcagcctcgc cagcgggggc 2640

cccgggcctg gccatgcctc actgagccag cgcctgcgcc tctacctcgc cgacagctgg 2700

aaccagtgcg acctagtggc tctcacctgc ttcctcctgg gcgtgggctg ccggctgacc 2760

ccgggtttgt accacctggg ccgcactgtc ctctgcatcg acttcatggt tttcacggtg 2820

cggctgcttc acatcttcac ggtcaacaaa cagctggggc ccaagatcgt catcgtgagc 2880

```
aagatgatga aggacgtgtt cttcttcctc ttcttcctcg gcgtgtggct ggtagcctat     2940
ggcgtggcca cggaggggct cctgaggcca cgggacagtg acttcccaag tatcctgcgc     3000
cgcgtcttct accgtcccta cctgcagatc ttcgggcaga ttccccagga ggacatggac     3060
gtggccctca tggagcacag caactgctcg tcggagcccg gcttctgggc acaccctcct     3120
ggggcccagg cgggcacctg cgtctcccag tatgccaact ggctggtggt gctgctcctc     3180
gtcatcttcc tgctcgtggc caacatcctg ctggtcaact tgctcattgc catgttcagt     3240
tacacattcg gcaaagtaca gggcaacagc gatctctact ggaaggcgca gcgttaccgc     3300
ctcatccggg aattccactc tcggcccgcg ctggccccgc cctttatcgt catctcccac     3360
ttgcgcctcc tgctcaggca attgtgcagg cgaccccgga gcccccagcc gtcctccccg     3420
gccctcgagc atttccgggt ttacctttct aaggaagccg agcggaagct gctaacgtgg     3480
gaatcggtgc ataaggagaa ctttctgctg gcacgcgcta gggacaagcg ggagagcgac     3540
tccgagcgtc tgaagcgcac gtcccagaag gtggacttgg cactgaaaca gctgggacac     3600
atccgcgagt acgaacagcg cctgaaagtg ctggagcggg aggtccagca gtgtagccgc     3660
gtcctggggt gggtggccga ggccctgagc cgctctgcct tgctgccccc aggtgggccg     3720
ccacccccctg acctgcctgg gtccaaagac tgagccctgc tggcggactt caaggagaag     3780
cccccacagg ggattttgct cctagagtaa ggctcatctg ggcctcggcc cccgcacctg     3840
gtggccttgt ccttgaggtg agccccatgt ccatctgggc cactgtcagg accacctttg     3900
ggagtgtcat ccttacaaac cacagcatgc ccggctcctc ccagaaccag tcccagcctg     3960
ggaggatcaa ggcctggatc ccgggccgtt atccatctgg aggctgcagg gtccttgggg     4020
taacagggac cacagacccc tcaccactca cagattcctc acactgggga aataaagcca     4080
tttcagagga atcgtgaaaa aaa                                             4103
```

<210> 30
<211> 3923
<212> DNA
<213> Homo sapiens

<400> 30

```
acagaagaaa tagcaagtgc cgagaagctg gcatcagaaa aacagagggg agatttgtgt       60
ggctgcagcc gagggagacc aggaagatct gcatggtggg aaggacctga tgatacagag      120
gaattacaac acatatactt agtgtttcaa tgaacaccaa gataaataag tgaagagcta      180
gtccgctgtg agtctcctca gtgacacagg gctggatcac catcgacggc actttctgag      240
tactcagtgc agcaaagaaa gactacagac atctcaatgg caggggtgag aaataagaaa      300
ggctgctgac tttaccatct gaggccacac atctgctgaa atggagataa ttaacatcac      360
tagaaacagc aagatgacaa tataatgtct aagtagtgac atgtttttgc acatttccag      420
```

ccccctttaaa tatccacaca cacaggaagc acaaaaggaa gcacagagat ccctgggaga 480
aatgcccggc cgccatcttg ggtcatcgat gagcctcgcc ctgtgcctgg tcccgcttgt 540
gagggaagga cattagaaaa tgaattgatg tgttccttaa aggatgggca ggaaaacaga 600
tcctgttgtg gatatttatt tgaacgggat tacagatttg aaatgaagtc acaaagtgag 660
cattaccaat gagaggaaaa cagacgagaa aatcttgatg gcttcacaag acatgcaaca 720
aacaaaatgg aatactgtga tgacatgagg cagccaagct ggggaggaga taaccacggg 780
gcagagggtc aggattctgg ccctgctgcc taaactgtgc gttcataacc aaatcatttc 840
atatttctaa ccctcaaaac aaagctgttg taatatctga tctctacggt tccttctggg 900
cccaacattc tccatatatc cagccacact catttttaat atttagttcc cagatctgta 960
ctgtgacctt tctacactgt agaataacat tactcatttt gttcaaagac ccttcgtgtt 1020
gctgcctaat atgtagctga ctgtttttcc taaggagtgt tctggcccag gggatctgtg 1080
aacaggctgg gaagcatctc aagatctttc cagggttata cttactagca cacagcatga 1140
tcattacgga gtgaattatc taatcaacat catcctcagt gtctttgccc atactgaaat 1200
tcatttccca cttttgtgcc cattctcaag acctcaaaat gtcattccat taatatcaca 1260
ggattaactt tttttttaa cctggaagaa ttcaatgtta catgcagcta tgggaattta 1320
attacatatt ttgttttcca gtgcaaagat gactaagtcc tttatccctc ccctttgttt 1380
gatttttttt ccagtataaa gttaaaatgc ttagccttgt actgaggctg tatacagcac 1440
agcctctccc catccctcca gccttatctg tcatcaccat caacccctcc cataccacct 1500
aaacaaaatc taacttgtaa ttccttgaac atgtcaggac atacattatt ccttctgcct 1560
gagaagctct tccttgtctc ttaaatctag aatgatgtaa agttttgaat aagttgacta 1620
tcttacttca tgcaaagaag ggacacatat gagattcatc atcacatgag acagcaaata 1680
ctaaaagtgt aatttgatta taagagttta gataaatata tgaaatgcaa gagccacaga 1740
gggaatgttt atggggcacg tttgtaagcc tgggatgtga agcaaaggca gggaacctca 1800
tagtatctta tataatatac ttcatttctc tatctctatc acaatatcca acaagctttt 1860
cacagaattc atgcagtgca aatccccaaa ggtaaccttt atccatttca tggtgagtgc 1920
gctttagaat tttggcaaat catactggtc acttatctca actttgagat gtgtttgtcc 1980
ttgtagttaa ttgaaagaaa tagggcactc ttgtgagcca ctttagggtt cactcctggc 2040
aataaagaat ttacaaagag ctactcagga ccagttgtta agagctctgt gtgtgtgtgt 2100
gtgtgtgtgt gagtgtacat gccaaagtgt gcctctctct cttgacccat tatttcagac 2160
ttaaaacaag catgttttca aatggcacta tgagctgcca atgatgtatc accaccatat 2220
ctcattattc tccagtaaat gtgataataa tgtcatctgt taacataaaa aaagtttgac 2280

```
ttcacaaaag cagctggaaa tggacaacca caatatgcat aaatctaact cctaccatca   2340
gctacacact gcttgacata tattgttaga agcacctcgc atttgtgggt tctcttaagc   2400
aaaatacttg cattaggtct cagctggggc tgtgcatcag gcggtttgag aaatattcaa   2460
ttctcagcag aagccagaat ttgaattccc tcatctttta ggaatcattt accaggtttg   2520
gagaggattc agacagctca ggtgctttca ctaatgtctc tgaacttctg tccctctttg   2580
tgttcatgga tagtccaata aataatgtta tctttgaact gatgctcata ggagagaata   2640
taagaactct gagtgatatc aacattaggg attcaaagaa atattagatt taagctcaca   2700
ctggtcaaaa ggaaccaaga tacaaagaac tctgagctgt catcgtcccc atctctgtga   2760
gccacaacca acagcaggac ccaacgcatg tctgagatcc ttaaatcaag gaaaccagtg   2820
tcatgagttg aattctccta ttatggatgc tagcttctgg ccatctctgg ctctcctctt   2880
gacacatatt agcttctagc ctttgcttcc acgactttta tcttttctcc aacacatcgc   2940
ttaccaatcc tctctctgct ctgttgcttt ggacttcccc acaagaattt caacgactct   3000
caagtctttt cttccatccc caccactaac ctgaatgcct agacccttat ttttattaat   3060
ttccaataga tgctgcctat gggctatatt gctttagatg aacattagat atttaaagct   3120
caagaggttc aaaatccaac tcattatctt ctctttcttt cacctccctg ctcctctccc   3180
tatattactg attgcactga acagcatggt ccccaatgta gccatgcaaa tgagaaaccc   3240
agtggctcct tgtggtacat gcatgcaaga ctgctgaagc cagaaggatg actgattacg   3300
cctcatgggt ggaggggacc actcctgggc cttcgtgatt gtcaggagca agacctgaga   3360
tgctccctgc cttcagtgtc ctctgcatct cccctttcta atgaagatcc atagaatttg   3420
ctacatttga gaattccaat taggaactca catgttttat ctgccctatc aatttttttaa   3480
acttgctgaa aattaagttt tttcaaaatc tgtccttgta aattactttt tcttacagtg   3540
tcttggcata ctatatcaac tttgattctt tgttacaact tttcttactc ttttatcacc   3600
aaagtggctt ttattctctt tattattatt attttctttt actactatat tacgttgtta   3660
ttattttgtt ctctatagta tcaatttatt tgatttagtt tcaatttatt tttattgctg   3720
acttttaaaa taagtgattc gggggtggg agaacagggg agggagagca ttaggacaaa   3780
tacctaatgc atgtgggact taaaacctag atgatgggtt gataggtgca gcaaaccact   3840
atggcacacg tatacctgtg taacaaacct acacattctg cacatgtatc ccagaacgta   3900
aagtaaaatt taaaaaaaag tga                                           3923
```

<210> 31
<211> 662
<212> PRT
<213> Homo sapiens

<400> 31

```
Met Gly Leu Tyr Arg Ile Arg Val Ser Thr Gly Ala Ser Leu Tyr Ala
1               5                   10                  15

Gly Ser Asn Asn Gln Val Gln Leu Trp Leu Val Gly Gln His Gly Glu
            20                  25                  30

Ala Ala Leu Gly Lys Arg Leu Trp Pro Ala Arg Gly Lys Glu Thr Glu
        35                  40                  45

Leu Lys Val Glu Val Pro Glu Tyr Leu Gly Pro Leu Leu Phe Val Lys
    50                  55                  60

Leu Arg Lys Arg His Leu Leu Lys Asp Asp Ala Trp Phe Cys Asn Trp
65                  70                  75                  80

Ile Ser Val Gln Gly Pro Gly Ala Gly Asp Glu Val Arg Phe Pro Cys
                85                  90                  95

Tyr Arg Trp Val Glu Gly Asn Gly Val Leu Ser Leu Pro Glu Gly Thr
            100                 105                 110

Gly Arg Thr Val Gly Glu Asp Pro Gln Gly Leu Phe Gln Lys His Arg
        115                 120                 125

Glu Glu Glu Leu Glu Glu Arg Arg Lys Leu Tyr Arg Trp Gly Asn Trp
    130                 135                 140

Lys Asp Gly Leu Ile Leu Asn Met Ala Gly Ala Lys Leu Tyr Asp Leu
145                 150                 155                 160

Pro Val Asp Glu Arg Phe Leu Glu Asp Lys Arg Val Asp Phe Glu Val
                165                 170                 175

Ser Leu Ala Lys Gly Leu Ala Asp Leu Ala Ile Lys Asp Ser Leu Asn
            180                 185                 190

Val Leu Thr Cys Trp Lys Asp Leu Asp Asp Phe Asn Arg Ile Phe Trp
        195                 200                 205

Cys Gly Gln Ser Lys Leu Ala Glu Arg Val Arg Asp Ser Trp Lys Glu
    210                 215                 220

Asp Ala Leu Phe Gly Tyr Gln Phe Leu Asn Gly Ala Asn Pro Val Val
225                 230                 235                 240

Leu Arg Arg Ser Ala His Leu Pro Ala Arg Leu Val Phe Pro Pro Gly
                245                 250                 255
```

```
Met Glu Glu Leu Gln Ala Gln Leu Glu Lys Glu Leu Glu Gly Gly Thr
            260                 265                 270

Leu Phe Glu Ala Asp Phe Ser Leu Leu Asp Gly Ile Lys Ala Asn Val
        275                 280                 285

Ile Leu Cys Ser Gln Gln His Leu Ala Ala Pro Leu Val Met Leu Lys
    290                 295                 300

Leu Gln Pro Asp Gly Lys Leu Leu Pro Met Val Ile Gln Leu Gln Leu
305                 310                 315                 320

Pro Arg Thr Gly Ser Pro Pro Pro Pro Leu Phe Leu Pro Thr Asp Pro
                325                 330                 335

Pro Met Ala Trp Leu Leu Ala Lys Cys Trp Val Arg Ser Ser Asp Phe
            340                 345                 350

Gln Leu His Glu Leu Gln Ser His Leu Leu Arg Gly His Leu Met Ala
        355                 360                 365

Glu Val Ile Val Val Ala Thr Met Arg Cys Leu Pro Ser Ile His Pro
    370                 375                 380

Ile Phe Lys Leu Ile Ile Pro His Leu Arg Tyr Thr Leu Glu Ile Asn
385                 390                 395                 400

Val Arg Ala Arg Thr Gly Leu Val Ser Asp Met Gly Ile Phe Asp Gln
                405                 410                 415

Ile Met Ser Thr Gly Gly Gly Gly His Val Gln Leu Leu Lys Gln Ala
            420                 425                 430

Gly Ala Phe Leu Thr Tyr Ser Ser Phe Cys Pro Pro Asp Asp Leu Ala
        435                 440                 445

Asp Arg Gly Leu Leu Gly Val Lys Ser Ser Phe Tyr Ala Gln Asp Ala
    450                 455                 460

Leu Arg Leu Trp Glu Ile Ile Tyr Arg Tyr Val Glu Gly Ile Val Ser
465                 470                 475                 480

Leu His Tyr Lys Thr Asp Val Ala Val Lys Asp Asp Pro Glu Leu Gln
                485                 490                 495

Thr Trp Cys Arg Glu Ile Thr Glu Ile Gly Leu Gln Gly Ala Gln Asp
            500                 505                 510
```

```
Arg Gly Phe Pro Val Ser Leu Gln Ala Arg Asp Gln Val Cys His Phe
            515                 520                 525

Val Thr Met Cys Ile Phe Thr Cys Thr Gly Gln His Ala Ser Val His
    530                 535                 540

Leu Gly Gln Leu Asp Trp Tyr Ser Trp Val Pro Asn Ala Pro Cys Thr
545                 550                 555                 560

Met Arg Leu Pro Pro Pro Thr Thr Lys Asp Ala Thr Leu Glu Thr Val
                565                 570                 575

Met Ala Thr Leu Pro Asn Phe His Gln Ala Ser Leu Gln Met Ser Ile
            580                 585                 590

Thr Trp Gln Leu Gly Arg Arg Gln Pro Val Met Val Ala Val Gly Gln
        595                 600                 605

His Glu Glu Glu Tyr Phe Ser Gly Pro Glu Pro Lys Ala Val Leu Lys
    610                 615                 620

Lys Phe Arg Glu Glu Leu Ala Ala Leu Asp Lys Glu Ile Glu Ile Arg
625                 630                 635                 640

Asn Ala Lys Leu Asp Met Pro Tyr Glu Tyr Leu Arg Pro Ser Val Val
                645                 650                 655

Glu Asn Ser Val Ala Ile
            660
```

<210> 32
<211> 2707
<212> DNA
<213> Homo sapiens

<400> 32

```
catctttgag caagatgggt ctctaccgca tccgcgtgtc cactggggcc tcgctctatg      60
ccggttccaa caaccaggtg cagctgtggc tggtcggcca gcacggggag gcggcgctcg     120
ggaagcgact gtggcccgca cggggcaagg agacagaact caaggtggaa gtaccggagt     180
atctggggcc gctgctgttt gtgaaactgc gcaaacggca cctccttaag gacgacgcct     240
ggttctgcaa ctggatctct gtgcagggcc ccggagccgg ggacgaggtc aggttccctt     300
gttaccgctg ggtggagggc aacggcgtcc tgagcctgcc tgaaggcacc ggccgcactg     360
tgggcgagga ccctcagggc ctgttccaga aacaccggga agaagagctg gaagagagaa     420
ggaagttgta ccggtgggga aactggaagg acgggttaat tctgaatatg gctggggcca     480
```

aactatatga cctccctgtg gatgagcgat ttctggaaga caagagagtt gactttgagg 540
tttcgctggc caaggggctg gccgacctcg ctatcaaaga ctctctaaat gttctgactt 600
gctggaagga tctagatgac ttcaaccgga ttttctggtg tggtcagagc aagctggctg 660
agcgcgtgcg ggactcctgg aaggaagatg ccttatttgg gtaccagttt cttaatggcg 720
ccaaccccgt ggtgctgagg cgctctgctc accttcctgc tcgcctagtg ttccctccag 780
gcatggagga actgcaggcc cagctggaga aggagctgga gggaggcaca ctgttcgaag 840
ctgacttctc cctgctggat gggatcaagg ccaacgtcat tctctgtagc cagcagcacc 900
tggctgcccc tctagtcatg ctgaaattgc agcctgatgg gaaactcttg cccatggtca 960
tccagctcca gctgccccgc acaggatccc caccacctcc ccttttcttg cctacggatc 1020
ccccaatggc ctggcttctg gccaaatgct gggtgcgcag ctctgacttc cagctccatg 1080
agctgcagtc tcatcttctg aggggacact tgatggctga ggtcattgtt gtggccacca 1140
tgaggtgcct gccgtcgata catcctatct tcaagcttat aattccccac ctgcgataca 1200
ccctggaaat taacgtccgg gccaggactg ggctggtctc tgacatggga attttcgacc 1260
agataatgag cactggtggg ggaggccacg tgcagctgct caagcaagct ggagccttcc 1320
taacctacag ctccttctgt ccccctgatg acttggccga ccgggggctc ctgggagtga 1380
agtcttcctt ctatgcccaa gatgcgctgc ggctctggga aatcatctat cggtatgtgg 1440
aaggaatcgt gagtctccac tataagacag acgtggctgt gaaagacgac ccagagctgc 1500
agacctggtg tcgagagatc actgaaatcg ggctgcaagg ggcccaggac cgagggtttc 1560
ctgtctcttt acaggctcgg gaccaggttt gccactttgt caccatgtgt atcttcacct 1620
gcaccggcca acacgcctct gtgcacctgg gccagctgga ctggtactct tgggtgccta 1680
atgcaccctg cacgatgcgg ctgcccccgc caaccaccaa ggatgcaacg ctggagacag 1740
tgatggcgac actgcccaac ttccaccagg cttctctcca gatgtccatc acttggcagc 1800
tgggcagacg ccagcccgtt atggtggctg tgggccagca tgaggaggag tatttttcgg 1860
gccctgagcc taaggctgtg ctgaagaagt tcagggagga gctggctgcc ctggataagg 1920
aaattgagat ccggaatgca aagctggaca tgccctacga gtacctgcgg cccagcgtgg 1980
tggaaaacag tgtggccatc taagcgtcgc caccctttgg ttatttcagc ccccatcacc 2040
caagccacaa gctgacccct tcgtggttat agccctgccc tcccaagtcc caccctcttc 2100
ccatgtccca ccctccctag aggggcacct tttcatggtc tctgcaccca gtgaacacat 2160
tttactctag aggcatcacc tgggacctta ctcctctttc cttccttcct cctttcctat 2220
cttccttcct ctctctcttc ctctttcttc attcagatct atatggcaaa tagccacaat 2280
tatataaatc atttcaagac tagaataggg ggatataata catattactc cacacctttt 2340

```
atgaatcaaa tatgattttt ttgttgttgt taagacagag tctcactttg acacccaggc     2400
tggagtgcag tggtgccatc accacggctc actgcagcct cagcgtcctg ggctcaaatg     2460
atcctcccac ctcagcctcc tgagtagctg ggactacagg ctcatgccat catgcccagc     2520
taatattttt ttattttcgt ggagacgggg cctcactatg ttgcctaggc tggaaatagg     2580
attttgaacc caaattgagt ttaacaataa taaaaagttg ttttacgcta aagatggaaa     2640
agaactagga ctgaactatt ttaaataaaa tattggcaaa agaaaaaaaa aaaaaaaaaa     2700
aaaaaaa                                                               2707
```

<210> 33
<211> 772
<212> PRT
<213> Homo sapiens

<400> 33

```
Met Asn Cys Tyr Leu Leu Leu Arg Phe Met Leu Gly Ile Pro Leu Leu
1               5                   10                  15

Trp Pro Cys Leu Gly Ala Thr Glu Asn Ser Gln Thr Lys Lys Val Lys
            20                  25                  30

Gln Pro Val Arg Ser His Leu Arg Val Lys Arg Gly Trp Val Trp Asn
        35                  40                  45

Gln Phe Phe Val Pro Glu Glu Met Asn Thr Thr Ser His His Ile Gly
    50                  55                  60

Gln Leu Arg Ser Asp Leu Asp Asn Gly Asn Asn Ser Phe Gln Tyr Lys
65                  70                  75                  80

Leu Leu Gly Ala Gly Ala Gly Ser Thr Phe Ile Ile Asp Glu Arg Thr
                85                  90                  95

Gly Asp Ile Tyr Ala Ile Gln Lys Leu Asp Arg Glu Glu Arg Ser Leu
            100                 105                 110

Tyr Ile Leu Arg Ala Gln Val Ile Asp Ile Ala Thr Gly Arg Ala Val
        115                 120                 125

Glu Pro Glu Ser Glu Phe Val Ile Lys Val Ser Asp Ile Asn Asp Asn
    130                 135                 140

Glu Pro Lys Phe Leu Asp Glu Pro Tyr Glu Ala Ile Val Pro Glu Met
145                 150                 155                 160

Ser Pro Glu Gly Thr Leu Val Ile Gln Val Thr Ala Ser Asp Ala Asp
                165                 170                 175
```

```
Asp Pro Ser Ser Gly Asn Asn Ala Arg Leu Leu Tyr Ser Leu Leu Gln
            180                 185                 190

Gly Gln Pro Tyr Phe Ser Val Glu Pro Thr Thr Gly Val Ile Arg Ile
        195                 200                 205

Ser Ser Lys Met Asp Arg Glu Leu Gln Asp Glu Tyr Trp Val Ile Ile
    210                 215                 220

Gln Ala Lys Asp Met Ile Gly Gln Pro Gly Ala Leu Ser Gly Thr Thr
225                 230                 235                 240

Ser Val Leu Ile Lys Leu Ser Asp Val Asn Asp Asn Lys Pro Ile Phe
                245                 250                 255

Lys Glu Ser Leu Tyr Arg Leu Thr Val Ser Glu Ser Ala Pro Thr Gly
            260                 265                 270

Thr Ser Ile Gly Thr Ile Met Ala Tyr Asp Asn Asp Ile Gly Glu Asn
        275                 280                 285

Ala Glu Met Asp Tyr Ser Ile Glu Glu Asp Asp Ser Gln Thr Phe Asp
    290                 295                 300

Ile Ile Thr Asn His Glu Thr Gln Glu Gly Ile Val Ile Leu Lys Lys
305                 310                 315                 320

Lys Val Asp Phe Glu His Gln Asn His Tyr Gly Ile Arg Ala Lys Val
                325                 330                 335

Lys Asn His His Val Pro Glu Gln Leu Met Lys Tyr His Thr Glu Ala
            340                 345                 350

Ser Thr Thr Phe Ile Lys Ile Gln Val Glu Asp Val Asp Glu Pro Pro
        355                 360                 365

Leu Phe Leu Leu Pro Tyr Tyr Val Phe Glu Val Phe Glu Glu Thr Pro
    370                 375                 380

Gln Gly Ser Phe Val Gly Val Val Ser Ala Thr Asp Pro Asp Asn Arg
385                 390                 395                 400

Lys Ser Pro Ile Arg Tyr Ser Ile Thr Arg Ser Lys Val Phe Asn Ile
                405                 410                 415

Asn Asp Asn Gly Thr Ile Thr Thr Ser Asn Ser Leu Asp Arg Glu Ile
```

```
            420                 425                 430

Ser Ala Trp Tyr Asn Leu Ser Ile Thr Ala Thr Glu Lys Tyr Asn Ile
        435                 440                 445

Glu Gln Ile Ser Ser Ile Pro Leu Tyr Val Gln Val Leu Asn Ile Asn
    450                 455                 460

Asp His Ala Pro Glu Phe Ser Gln Tyr Tyr Glu Thr Tyr Val Cys Glu
465                 470                 475                 480

Asn Ala Gly Ser Gly Gln Val Ile Gln Thr Ile Ser Ala Val Asp Arg
                485                 490                 495

Asp Glu Ser Ile Glu Glu His His Phe Tyr Phe Asn Leu Ser Val Glu
            500                 505                 510

Asp Thr Asn Asn Ser Ser Phe Thr Ile Ile Asp Asn Gln Asp Asn Thr
        515                 520                 525

Ala Val Ile Leu Thr Asn Arg Thr Gly Phe Asn Leu Gln Glu Glu Pro
    530                 535                 540

Val Phe Tyr Ile Ser Ile Leu Ile Ala Asp Asn Gly Ile Pro Ser Leu
545                 550                 555                 560

Thr Ser Thr Asn Thr Leu Thr Ile His Val Cys Asp Cys Gly Asp Ser
                565                 570                 575

Gly Ser Thr Gln Thr Cys Gln Tyr Gln Glu Leu Val Leu Ser Met Gly
            580                 585                 590

Phe Lys Thr Glu Val Ile Ile Ala Ile Leu Ile Cys Ile Met Ile Ile
        595                 600                 605

Phe Gly Phe Ile Phe Leu Thr Leu Gly Leu Lys Gln Arg Arg Lys Gln
    610                 615                 620

Ile Leu Phe Pro Glu Lys Ser Glu Asp Phe Arg Glu Asn Ile Phe Gln
625                 630                 635                 640

Tyr Asp Asp Glu Gly Gly Gly Glu Glu Asp Thr Glu Ala Phe Asp Ile
                645                 650                 655

Ala Glu Leu Arg Ser Ser Thr Ile Met Arg Glu Arg Lys Thr Arg Lys
            660                 665                 670
```

```
Thr Thr Ser Ala Glu Ile Arg Ser Leu Tyr Arg Gln Ser Leu Gln Val
        675                 680                 685

Gly Pro Asp Ser Ala Ile Phe Arg Lys Phe Ile Leu Glu Lys Leu Glu
    690                 695                 700

Glu Ala Asn Thr Asp Pro Cys Ala Pro Pro Phe Asp Ser Leu Gln Thr
705                 710                 715                 720

Tyr Ala Phe Glu Gly Thr Gly Ser Leu Ala Gly Ser Leu Ser Ser Leu
                725                 730                 735

Glu Ser Ala Val Ser Asp Gln Asp Glu Ser Tyr Asp Tyr Leu Asn Glu
            740                 745                 750

Leu Gly Pro Arg Phe Lys Arg Leu Ala Cys Met Phe Gly Ser Ala Val
        755                 760                 765

Gln Ser Asn Asn
    770
```

<210> 34
<211> 6241
<212> DNA
<213> Homo sapiens

<400> 34

```
atactgacag tgttgtggta ctaaaagcac aagcgtctgt aactctgggc aatggggcac      60
atcgagagtt tgctgagaag actgtgaagc aaaaagaaga aagtttttcc tactcttcct     120
tatgtgtcca acacgaagtt tgctgttcag ttttcacaga acttctagaa gttgaagtta     180
caaaggtata tagaaggtac acagaatcag aaaagattat aaaagaaagc aagatttttg     240
ttagtgacgt cctgtttcct ctgaagagta atagttggaa tcaaaagagt caacgcaatg     300
aactgttatt tactgctgcg ttttatgttg ggaattcctc tcctatggcc ttgtcttgga     360
gcaacagaaa actctcaaac aaagaaagtc aagcagccag tgcgatctca tttgagagtg     420
aagcgtggct gggtgtggaa ccaatttttt gtaccagagg aaatgaatac gactagtcat     480
cacatcggcc agctaagatc tgatttagac aatggaaaca attctttcca gtacaagctt     540
ttgggagctg gagctggaag tacttttatc attgatgaaa gaacaggtga catatatgcc     600
atacagaagc ttgatagaga ggagcgatcc ctctacatct taagagccca ggtaatagac     660
atcgctactg gaagggctgt ggaacctgag tctgagtttg tcatcaaagt ttcggatatc     720
aatgacaatg aaccaaaatt cctagatgaa ccttatgagg ccattgtacc agagatgtct     780
ccagaaggaa cattagttat ccaggtgaca gcaagtgatg ctgacgatcc ctcaagtggt     840
aataatgctc gtctcctcta cagcttactt caaggccagc catatttttc tgttgaacca     900
```

acaacaggag tcataagaat atcttctaaa atggatagag aactgcaaga tgagtattgg 960
gtaatcattc aagccaagga catgattggt cagccaggag cgttgtctgg aacaacaagt 1020
gtattaatta aactttcaga tgttaatgac aataagccta tatttaaaga aagtttatac 1080
cgcttgactg tctctgaatc tgcacccact gggacttcta taggaacaat catggcatat 1140
gataatgaca taggagagaa tgcagaaatg gattacagca ttgaagagga tgattcgcaa 1200
acatttgaca ttattactaa tcatgaaact caagaaggaa tagttatatt aaaaaagaaa 1260
gtggattttg agcaccagaa ccactacggt attagagcaa aagttaaaaa ccatcatgtt 1320
cctgagcagc tcatgaagta ccacactgag gcttccacca ctttcattaa gatccaggtg 1380
gaagatgttg atgagcctcc tcttttcctc cttccatatt atgtatttga agtttttgaa 1440
gaaaccccac agggatcatt tgtaggcgtg gtgtctgcca cagacccaga caataggaaa 1500
tctcctatca ggtattctat tactaggagc aaagtgttca atatcaatga taatggtaca 1560
atcactacaa gtaactcact ggatcgtgaa atcagtgctt ggtacaacct aagtattaca 1620
gccacagaaa aatacaatat agaacagatc tcttcgatcc cactgtatgt gcaagttctt 1680
aacatcaatg atcatgctcc tgagttctct caatactatg agacttatgt ttgtgaaaat 1740
gcaggctctg gtcaggtaat tcagactatc agtgcagtgg atagagatga atccatagaa 1800
gagcaccatt tttactttaa tctatctgta gaagacacta acaattcaag ttttacaatc 1860
atagataatc aagataacac agctgtcatt ttgactaata gaactggttt taaccttcaa 1920
gaagaacctg tcttctacat ctccatctta attgccgaca atggaatccc gtcacttaca 1980
agtacaaaca cccttaccat ccatgtctgt gactgtggtg acagtgggag cacacagacc 2040
tgccagtacc aggagcttgt gctttccatg ggattcaaga cagaagtcat cattgctatt 2100
ctcatttgca ttatgatcat atttgggttt atttttttga ctttgggttt aaaacaacgg 2160
agaaaacaga ttctatttcc tgagaaaagt gaagatttca gagagaatat attccaatat 2220
gatgatgaag gggtggaga agaagataca gaggcctttg atatagcaga gctgaggagt 2280
agtaccataa tgcgggaacg caagactcgg aaaaccacaa gcgctgagat caggagccta 2340
tacaggcagt ctttgcaagt tggccccgac agtgccatat tcaggaaatt cattctggaa 2400
aagctcgaag aagctaatac tgatccgtgt gcccctcctt ttgattccct ccagacctac 2460
gcttttgagg gaacagggtc attagctgga tccctgagct ccttagaatc agcagtctct 2520
gatcaggatg aaagctatga ttaccttaat gagttgggac ctcgctttaa aagattagca 2580
tgcatgtttg gttctgcagt gcagtcaaat aattagggct ttttaccatc aaaattttta 2640
aaagtgctaa tgtgtattcg aacccaatgg tagtcttaaa gagttttgtg ccctggctct 2700
atggcgggga aagccctagt ctatggagtt ttctgatttc cctggagtaa atactccatg 2760

gttattttaa gctacctaca tgctgtcatt gaacagagat gtggggagaa atgtaaacaa 2820
tcagctcaca ggcatcaata caaccagatt tgaagtaaaa taatttagga agatattaaa 2880
agtagatgag aggacacaag atgtagtcga tccttatgcg attatatcat tatttactta 2940
ggaaagagta aaaataccaa acgagaaaat ttaaaggagc aaaaatttgc aagtcaaata 3000
gaaatgtaca aatcgagata acatttacat ttctatcata ttgacatgaa aattgaaaat 3060
gtatagtcag agaaattttc atgaattatt ccatgaagta ttgtttcctt tatttaaaaa 3120
aaaaaaaaaa aaaaaagaat gctaggtaat cttcgtagaa aactagaaag tatgataaac 3180
aacagttgga ggaatccatg gaaaatagac gagaaaatgt aaataaggct ttctggggat 3240
cacagaactt ttgtatgaat aaaagcctta taaaaccagc tctggcatat gactaaaata 3300
ttcctctact atttttcaat gtcatctaat gcaaacgctc aacctttta aatggttaat 3360
gtgagaaagg cactgaatta agatatctta gtgttatttt atttactcaa ttttcatctt 3420
tcacactgta agaataaaat gtttttgtgg ttaagttcct gatactatat agagataaac 3480
tttaaaactt taacacctag gataatgtgg gtgattacat aaatgtttaa aaacatttac 3540
cttatatgga aagcagattt ctgactaaga tgccatgatt cattctagtt tgatatttta 3600
aaactttgcc aagagcactc tttaaaactg atttcaaatt tgaacagata cttggtgtat 3660
acaaattaat cagagttata tagtataaat ttgtattatt tttgatatta caactttgta 3720
ttaaaaagga aaatatattt aggtgtttgt catagatttt ttttcatatg caactcaaaa 3780
cacattctta tgtcaaaata caatagcaaa atacaagtca atatttactc ataattaaat 3840
gcaaacttta tgaaactggt ggaaagtgtg gaaaaattta agtaaatttt tacctctact 3900
aaaaattatt tagacaactt aaaggaatac tggattcaaa cttcagttac tattcactgt 3960
aaatattatg atcaactgaa ctgacttacc attcatggga taaaacatga tacaattctc 4020
agtatttgct tttctgttaa gagcttggaa tatgactgat gcttcagtgt cattttttta 4080
ttattgaata taaaattagg tgcaatagat acatttattg tagccttaag aaggaaatca 4140
atgctaagct gttgggcata aaactttaca gtaacattag ataacaagat aataaaacac 4200
tttaatttta atcgaccttt taagaatgat ctttattatt ctatccaaat caaggtagac 4260
ttcaaaattt atggtttgaa caggaagaaa gtagtgaata caaaattctt aaaattggtt 4320
catatgatca tttttttatt aatagcatgt gagactaagc tgaaccaaac tggccatata 4380
tgttaaatca aataatttga gtaattttga aatattgatg aagtagttaa atggttataa 4440
tatattttat tggtactttg aaatttaaac atctatatca cataataaag aaagtttttc 4500
atcaatctga aacaggcacc attaaattgg tgacacaaat attatcattt cacaaaagct 4560
tgagaaaacc agatttgaag tgaaataatt taggaagata ttaaaagtgg atgagaggac 4620
ataagatgta gtcaatcctt aacgcaatga taccattatt aggaaagagg aaaaatacca 4680

```
aacaagagaa aatttaaagg agcaaaaatt tgcaagtcaa atagaaatgc acaaatcgag     4740
agaacattta catttctatc acattgacat gaaaattgaa aatgtatagt cagagaaatt     4800
ttcatgaatt attccatgaa gtgttgtttc ctttatttaa aaagaatgct aggtaatctt     4860
agtagaaaac taaaaagtat gataaacaac agatggaaga attcatggaa aatagacgag     4920
aaaaagtaaa taaggctttc tggagatgac ggaaattttg tatgaataaa agccttataa     4980
acaatgaata catttggata aacaactcat taaaatgcac attaatggaa tacctgtaca     5040
aatagtttta atacttgtgt ccagtggcac taaagagata tattaaaata gattctggtt     5100
gagttgatta taagattgtc aatcatagga tacacaggga aagcacagac acgattgctc     5160
atctctttgg tagtctaaaa tatgttcctt ttaaccacat caaaagatac tatctgattc     5220
taatacagaa ggtgcaactt cctaggaatt atactttgta ttatattcaa taattatttt     5280
atgttttgca tcacatgggt gttgcaagaa cacagctctt aaactaaaaa ggcattacat     5340
taagaattta tttttgtaaa ttttaataaa ctctaaatta atgcaaaata tttatgaaaa     5400
tagcacagct tcacattaat acccacagta cttaaatatc tcccttagtt gactcacagc     5460
tttgtatgta agcttcaaat ttttaatggt aaaaataaga aaattaggac tagcgaactg     5520
ctagttgttc cctcagtgca tgtgcttgag cctaacagat ctttgtttga attagttact     5580
ttcaattaag cgaacatata atatttgtta aactgcttaa tctttccaga tctcactttg     5640
taaaatgacg tccaatcaac ctatttcaca tggttgttat gaagaattaa taataagata     5700
tgtaaaacca gcagcaccaa tagtgcctga caaaatataa ctgctcaata aataacagtg     5760
aattattata acaacagcta atggtattct attggaacac atggtctgtt tcttttccaa     5820
ttctgagtga tctcaacagc ctcacggcac attgaaaata aaagtcaact tcctcacctt     5880
ccccataggc catggacttt cccatctcta cccacttgtg cagtttctcc tctttcctct     5940
ctcctcctca gttactaaag caatcttcct tcaaacactt ctattttcca ttacatcact     6000
tcattcaggt tggtgatcag ctgcaacttc ctcagaaaga ccactgctgc ttccagtgac     6060
cctggctttg tcttgctttg tattagatta ctcagctccc tggtattatt gttgtcttta     6120
tcgcttatta gaatgtaacc cttaatgggc aagaaattgt cagtcttgtt catttatcat     6180
cgtataaaaa atgtttctag cacacagaaa gtgaccaata aatatttctg gaattaatgt     6240
a                                                                     6241
```

<210> 35
<211> 320
<212> PRT
<213> Homo sapiens

<400> 35

Met Ser Ser Cys Asn Phe Thr His Ala Thr Phe Val Leu Ile Gly Ile

1 5 10 15

Pro Gly Leu Glu Lys Ala His Phe Trp Val Gly Phe Pro Leu Leu Ser
20 25 30

Met Tyr Val Val Ala Met Phe Gly Asn Cys Ile Val Val Phe Ile Val
35 40 45

Arg Thr Glu Arg Ser Leu His Ala Pro Met Tyr Leu Phe Leu Cys Met
50 55 60

Leu Ala Ala Ile Asp Leu Ala Leu Ser Thr Ser Thr Met Pro Lys Ile
65 70 75 80

Leu Ala Leu Phe Trp Phe Asp Ser Arg Glu Ile Ser Phe Glu Ala Cys
85 90 95

Leu Thr Gln Met Phe Phe Ile His Ala Leu Ser Ala Ile Glu Ser Thr
100 105 110

Ile Leu Leu Ala Met Ala Phe Asp Arg Tyr Val Ala Ile Cys His Pro
115 120 125

Leu Arg His Ala Ala Val Leu Asn Asn Thr Val Thr Ala Gln Ile Gly
130 135 140

Ile Val Ala Val Val Arg Gly Ser Leu Phe Phe Phe Pro Leu Pro Leu
145 150 155 160

Leu Ile Lys Arg Leu Ala Phe Cys His Ser Asn Val Leu Ser His Ser
165 170 175

Tyr Cys Val His Gln Asp Val Met Lys Leu Ala Tyr Ala Asp Thr Leu
180 185 190

Pro Asn Val Val Tyr Gly Leu Thr Ala Ile Leu Leu Val Met Gly Val
195 200 205

Asp Val Met Phe Ile Ser Leu Ser Tyr Phe Leu Ile Ile Arg Thr Val
210 215 220

Leu Gln Leu Pro Ser Lys Ser Glu Arg Ala Lys Ala Phe Gly Thr Cys
225 230 235 240

Val Ser His Ile Gly Val Val Leu Ala Phe Tyr Val Pro Leu Ile Gly
245 250 255

```
Leu Ser Val Val His Arg Phe Gly Asn Ser Leu His Pro Ile Val Arg
            260                 265                 270

Val Val Met Gly Asp Ile Tyr Leu Leu Leu Pro Pro Val Ile Asn Pro
        275                 280                 285

Ile Ile Tyr Gly Ala Lys Thr Lys Gln Ile Arg Thr Arg Val Leu Ala
    290                 295                 300

Met Phe Lys Ile Ser Cys Asp Lys Asp Leu Gln Ala Val Gly Gly Lys
305                 310                 315                 320
```

<210> 36
<211> 2799
<212> DNA
<213> Homo sapiens

<400> 36

```
ctcacacacc ctgaagacac agtgagttag caccaccacc aggaattggc cttttagctc      60
tgtgcctgtc tccagtcagg ctggaataag tctcctcata tttgcaagct cggccctccc     120
ctggaatcta aagcctcctc agccttctga gtcagcctga aaggaacagg ccgaactgct     180
gtatgggctc tactgccagt gtgacctcac cctctccagt cacccctcct cagttccagc     240
tatgagttcc tgcaacttca cacatgccac ctttgtgctt attggtatcc caggattaga     300
gaaagcccat ttctgggttg gcttccccct cctttccatg tatgtagtgg caatgtttgg     360
aaactgcatc gtggtcttca tcgtaaggac ggaacgcagc ctgcacgctc cgatgtacct     420
ctttctctgc atgcttgcag ccattgacct ggccttatcc acatccacca tgcctaagat     480
ccttgccctt ttctggtttg attcccgaga gattagcttt gaggcctgtc ttacccagat     540
gttctttatt catgccctct cagccattga atccaccatc ctgctggcca tggcctttga     600
ccgttatgtg gccatctgcc acccactgcg ccatgctgca gtgctcaaca atacagtaac     660
agcccagatt ggcatcgtgg ctgtggtccg cggatccctc tttttttcc cactgcctct      720
gctgatcaag cggctggcct tctgccactc caatgtcctc tcgcactcct attgtgtcca     780
ccaggatgta atgaagttgg cctatgcaga cactttgccc aatgtggtat atggtcttac     840
tgccattctg ctggtcatgg gcgtggacgt aatgttcatc tccttgtcct attttctgat     900
aatacgaacg gttctgcaac tgccttccaa gtcagagcgg gccaaggcct ttggaacctg     960
tgtgtcacac attggtgtgg tactcgcctt ctatgtgcca cttattggcc tctcagttgt    1020
acaccgcttt ggaaacagcc ttcatcccat tgtgcgtgtt gtcatgggtg acatctacct    1080
gctgctgcct cctgtcatca atcccatcat ctatggtgcc aaaaccaaac agatcagaac    1140
acgggtgctg gctatgttca agatcagctg tgacaaggac ttgcaggctg tgggaggcaa    1200
gtgaccctta acactacact tctccttatc tttattggct tgataaacat aattatttct    1260
```

aacactagct tatttccagt tgcccataag cacatcagta cttttctctg gctggaatag 1320

taaactaaag tatggtacat ctacctaaag gactattatg tggaataata catactaatg 1380

aagtattaca tgatttaaag actacaataa aaccaaacat gcttataaca ttaagaaaaa 1440

caataaagat acatgattga aaccaagttg aaaaatagca tatgccttgg aggaaatgtg 1500

ctcaaattac taatgattta gtgttgtccc tactttctct ctctttttc tttcttttt 1560

ttttattatg gttagctgtc acatacaact ttttttttt tgagatgggg tctcgctctg 1620

tcaccaggct ggagtgcagt ggcgcgatct cggctcactg caacctccac atcccatgtt 1680

gaagtaattc ttctgcctca gcctcccgag tagctgggac tagaggaacg tgccaccatg 1740

actggctaat tttctgtatt ttttagtaga gacagagttt caccatgttg gccaggatgg 1800

tctcgatctc ctgaccttgt gatccacccg cctcagcctc ccaaagtgtt gggattacag 1860

gtgtgaacca ctgtgcccgg cctgtgtaca actttttaaa tagggaatat gatagcttcg 1920

catggtggtg tgcacctata gcccccactg cctggaaagc tgaggtggga gaatcgcttg 1980

agtccaggag tttgaggtta cagtgatcca cgatcgtacc actacactcc agcctgggca 2040

acggagcaag accctgtctc aaagcataaa atggaataac atatcaaatg aaacagggaa 2100

aatgaagctg acaatttatg gaagccaggg cttgtcacag tctctactgt tattatgcat 2160

tacctgggaa tttatataag cccttaataa taatgccaat gaacatctca tgtgtgctca 2220

caatgttctg gcactattat aagtgcttca caggttttat gtgttcttcg taactttatg 2280

gagtaggtac catttgtgtc tctttattat aagtgagaga aatgaagttt atattatcaa 2340

ggggactaaa gtcacacggc ttgtgggcac tgtgccaaga tttaaaatta aatttgatgg 2400

ttgaatacag ttacttaatg accatgttat attgcttcct gtgtaacatc tgccatttat 2460

ttcctcagct gtacaaatcc tctgttttct ctctgttaca cactaacatc aatggctttg 2520

tacttgtgat gagagataac cttgccctag ttgtgggcaa cacatgcaga ataatcctgt 2580

tttacagctg cctttcgtga tcttattgct tgctttttc cagattcagg gagaatgttg 2640

ttgtctattt gtctcttaca tctccttgat catgtcttca ttttttaatg tgctctgtac 2700

ctgtcaaaaa ttttgaatgt acaccacatg ctattgtctg aacctgagta taagataaaa 2760

taaaatttta ttttaaattt taaaaaaaaa aaaaaaaaa 2799

<210> 37
<211> 2224
<212> PRT
<213> Homo sapiens

<400> 37

Met Phe Pro Gly Cys Pro Arg Leu Trp Val Leu Val Val Leu Gly Thr
1 5 10 15

```
Ser Trp Val Gly Trp Gly Ser Gln Gly Thr Glu Ala Ala Gln Leu Arg
            20                  25                  30

Gln Phe Tyr Val Ala Ala Gln Gly Ile Ser Trp Ser Tyr Arg Pro Glu
        35                  40                  45

Pro Thr Asn Ser Ser Leu Asn Leu Ser Val Thr Ser Phe Lys Lys Ile
    50                  55                  60

Val Tyr Arg Glu Tyr Glu Pro Tyr Phe Lys Lys Glu Lys Pro Gln Ser
65                  70                  75                  80

Thr Ile Ser Gly Leu Leu Gly Pro Thr Leu Tyr Ala Glu Val Gly Asp
                85                  90                  95

Ile Ile Lys Val His Phe Lys Asn Lys Ala Asp Lys Pro Leu Ser Ile
            100                 105                 110

His Pro Gln Gly Ile Arg Tyr Ser Lys Leu Ser Glu Gly Ala Ser Tyr
        115                 120                 125

Leu Asp His Thr Phe Pro Ala Glu Lys Met Asp Asp Ala Val Ala Pro
    130                 135                 140

Gly Arg Glu Tyr Thr Tyr Glu Trp Ser Ile Ser Glu Asp Ser Gly Pro
145                 150                 155                 160

Thr His Asp Asp Pro Pro Cys Leu Thr His Ile Tyr Tyr Ser His Glu
                165                 170                 175

Asn Leu Ile Glu Asp Phe Asn Ser Gly Leu Ile Gly Pro Leu Leu Ile
            180                 185                 190

Cys Lys Lys Gly Thr Leu Thr Glu Gly Gly Thr Gln Lys Thr Phe Asp
        195                 200                 205

Lys Gln Ile Val Leu Leu Phe Ala Val Phe Asp Glu Ser Lys Ser Trp
    210                 215                 220

Ser Gln Ser Ser Ser Leu Met Tyr Thr Val Asn Gly Tyr Val Asn Gly
225                 230                 235                 240

Thr Met Pro Asp Ile Thr Val Cys Ala His Asp His Ile Ser Trp His
                245                 250                 255

Leu Leu Gly Met Ser Ser Gly Pro Glu Leu Phe Ser Ile His Phe Asn
            260                 265                 270
```

Gly Gln Val Leu Glu Gln Asn His His Lys Val Ser Ala Ile Thr Leu
275 280 285

Val Ser Ala Thr Ser Thr Thr Ala Asn Met Thr Val Gly Pro Glu Gly
290 295 300

Lys Trp Ile Ile Ser Ser Leu Thr Pro Lys His Leu Gln Ala Gly Met
305 310 315 320

Gln Ala Tyr Ile Asp Ile Lys Asn Cys Pro Lys Lys Thr Arg Asn Leu
325 330 335

Lys Lys Ile Thr Arg Glu Gln Arg Arg His Met Lys Arg Trp Glu Tyr
340 345 350

Phe Ile Ala Ala Glu Glu Val Ile Trp Asp Tyr Ala Pro Val Ile Pro
355 360 365

Ala Asn Met Asp Lys Lys Tyr Arg Ser Gln His Leu Asp Asn Phe Ser
370 375 380

Asn Gln Ile Gly Lys His Tyr Lys Lys Val Met Tyr Thr Gln Tyr Glu
385 390 395 400

Asp Glu Ser Phe Thr Lys His Thr Val Asn Pro Asn Met Lys Glu Asp
405 410 415

Gly Ile Leu Gly Pro Ile Ile Arg Ala Gln Val Arg Asp Thr Leu Lys
420 425 430

Ile Val Phe Lys Asn Met Ala Ser Arg Pro Tyr Ser Ile Tyr Pro His
435 440 445

Gly Val Thr Phe Ser Pro Tyr Glu Asp Glu Val Asn Ser Ser Phe Thr
450 455 460

Ser Gly Arg Asn Asn Thr Met Ile Arg Ala Val Gln Pro Gly Glu Thr
465 470 475 480

Tyr Thr Tyr Lys Trp Asn Ile Leu Glu Phe Asp Glu Pro Thr Glu Asn
485 490 495

Asp Ala Gln Cys Leu Thr Arg Pro Tyr Tyr Ser Asp Val Asp Ile Met
500 505 510

Arg Asp Ile Ala Ser Gly Leu Ile Gly Leu Leu Leu Ile Cys Lys Ser

515 520 525

Arg Ser Leu Asp Arg Arg Gly Ile Gln Arg Ala Ala Asp Ile Glu Gln
530 535 540

Gln Ala Val Phe Ala Val Phe Asp Glu Asn Lys Ser Trp Tyr Leu Glu
545 550 555 560

Asp Asn Ile Asn Lys Phe Cys Glu Asn Pro Asp Glu Val Lys Arg Asp
565 570 575

Asp Pro Lys Phe Tyr Glu Ser Asn Ile Met Ser Thr Ile Asn Gly Tyr
580 585 590

Val Pro Glu Ser Ile Thr Thr Leu Gly Phe Cys Phe Asp Asp Thr Val
595 600 605

Gln Trp His Phe Cys Ser Val Gly Thr Gln Asn Glu Ile Leu Thr Ile
610 615 620

His Phe Thr Gly His Ser Phe Ile Tyr Gly Lys Arg His Glu Asp Thr
625 630 635 640

Leu Thr Leu Phe Pro Met Arg Gly Glu Ser Val Thr Val Thr Met Asp
645 650 655

Asn Val Gly Thr Trp Met Leu Thr Ser Met Asn Ser Ser Pro Arg Ser
660 665 670

Lys Lys Leu Arg Leu Lys Phe Arg Asp Val Lys Cys Ile Pro Asp Asp
675 680 685

Asp Glu Asp Ser Tyr Glu Ile Phe Glu Pro Pro Glu Ser Thr Val Met
690 695 700

Ala Thr Arg Lys Met His Asp Arg Leu Glu Pro Glu Asp Glu Glu Ser
705 710 715 720

Asp Ala Asp Tyr Asp Tyr Gln Asn Arg Leu Ala Ala Ala Leu Gly Ile
725 730 735

Arg Ser Phe Arg Asn Ser Ser Leu Asn Gln Glu Glu Glu Glu Phe Asn
740 745 750

Leu Thr Ala Leu Ala Leu Glu Asn Gly Thr Glu Phe Val Ser Ser Asn
755 760 765

Thr Asp Ile Ile Val Gly Ser Asn Tyr Ser Ser Pro Ser Asn Ile Ser
770 775 780

Lys Phe Thr Val Asn Asn Leu Ala Glu Pro Gln Lys Ala Pro Ser His
785 790 795 800

Gln Gln Ala Thr Thr Ala Gly Ser Pro Leu Arg His Leu Ile Gly Lys
805 810 815

Asn Ser Val Leu Asn Ser Ser Thr Ala Glu His Ser Ser Pro Tyr Ser
820 825 830

Glu Asp Pro Ile Glu Asp Pro Leu Gln Pro Asp Val Thr Gly Ile Arg
835 840 845

Leu Leu Ser Leu Gly Ala Gly Glu Phe Lys Ser Gln Glu His Ala Lys
850 855 860

His Lys Gly Pro Lys Val Glu Arg Asp Gln Ala Ala Lys His Arg Phe
865 870 875 880

Ser Trp Met Lys Leu Leu Ala His Lys Val Gly Arg His Leu Ser Gln
885 890 895

Asp Thr Gly Ser Pro Ser Gly Met Arg Pro Trp Glu Asp Leu Pro Ser
900 905 910

Gln Asp Thr Gly Ser Pro Ser Arg Met Arg Pro Trp Lys Asp Pro Pro
915 920 925

Ser Asp Leu Leu Leu Leu Lys Gln Ser Asn Ser Ser Lys Ile Leu Val
930 935 940

Gly Arg Trp His Leu Ala Ser Glu Lys Gly Ser Tyr Glu Ile Ile Gln
945 950 955 960

Asp Thr Asp Glu Asp Thr Ala Val Asn Asn Trp Leu Ile Ser Pro Gln
965 970 975

Asn Ala Ser Arg Ala Trp Gly Glu Ser Thr Pro Leu Ala Asn Lys Pro
980 985 990

Gly Lys Gln Ser Gly His Pro Lys Phe Pro Arg Val Arg His Lys Ser
995 1000 1005

Leu Gln Val Arg Gln Asp Gly Gly Lys Ser Arg Leu Lys Lys Ser
1010 1015 1020

```
Gln Phe  Leu Ile Lys Thr Arg  Lys Lys Lys Lys Glu  Lys His Thr
    1025                 1030                 1035

His His  Ala Pro Leu Ser Pro  Arg Thr Phe His Pro  Leu Arg Ser
    1040                 1045                 1050

Glu Ala  Tyr Asn Thr Phe Ser  Glu Arg Arg Leu Lys  His Ser Leu
    1055                 1060                 1065

Val Leu  His Lys Ser Asn Glu  Thr Ser Leu Pro Thr  Asp Leu Asn
    1070                 1075                 1080

Gln Thr  Leu Pro Ser Met Asp  Phe Gly Trp Ile Ala  Ser Leu Pro
    1085                 1090                 1095

Asp His  Asn Gln Asn Ser Ser  Asn Asp Thr Gly Gln  Ala Ser Cys
    1100                 1105                 1110

Pro Pro  Gly Leu Tyr Gln Thr  Val Pro Pro Glu Glu  His Tyr Gln
    1115                 1120                 1125

Thr Phe  Pro Ile Gln Asp Pro  Asp Gln Met His Ser  Thr Ser Asp
    1130                 1135                 1140

Pro Ser  His Arg Ser Ser Ser  Pro Glu Leu Ser Glu  Met Leu Glu
    1145                 1150                 1155

Tyr Asp  Arg Ser His Lys Ser  Phe Pro Thr Asp Ile  Ser Gln Met
    1160                 1165                 1170

Ser Pro  Ser Ser Glu His Glu  Val Trp Gln Thr Val  Ile Ser Pro
    1175                 1180                 1185

Asp Leu  Ser Gln Val Thr Leu  Ser Pro Glu Leu Ser  Gln Thr Asn
    1190                 1195                 1200

Leu Ser  Pro Asp Leu Ser His  Thr Thr Leu Ser Pro  Glu Leu Ile
    1205                 1210                 1215

Gln Arg  Asn Leu Ser Pro Ala  Leu Gly Gln Met Pro  Ile Ser Pro
    1220                 1225                 1230

Asp Leu  Ser His Thr Thr Leu  Ser Pro Asp Leu Ser  His Thr Thr
    1235                 1240                 1245

Leu Ser  Leu Asp Leu Ser Gln  Thr Asn Leu Ser Pro  Glu Leu Ser
    1250                 1255                 1260
```

Gln Thr Asn Leu Ser Pro Ala Leu Gly Gln Met Pro Leu Ser Pro
1265 1270 1275

Asp Leu Ser His Thr Thr Leu Ser Leu Asp Phe Ser Gln Thr Asn
1280 1285 1290

Leu Ser Pro Glu Leu Ser His Met Thr Leu Ser Pro Glu Leu Ser
1295 1300 1305

Gln Thr Asn Leu Ser Pro Ala Leu Gly Gln Met Pro Ile Ser Pro
1310 1315 1320

Asp Leu Ser His Thr Thr Leu Ser Leu Asp Phe Ser Gln Thr Asn
1325 1330 1335

Leu Ser Pro Glu Leu Ser Gln Thr Asn Leu Ser Pro Ala Leu Gly
1340 1345 1350

Gln Met Pro Leu Ser Pro Asp Pro Ser His Thr Thr Leu Ser Leu
1355 1360 1365

Asp Leu Ser Gln Thr Asn Leu Ser Pro Glu Leu Ser Gln Thr Asn
1370 1375 1380

Leu Ser Pro Asp Leu Ser Glu Met Pro Leu Phe Ala Asp Leu Ser
1385 1390 1395

Gln Ile Pro Leu Thr Pro Asp Leu Asp Gln Met Thr Leu Ser Pro
1400 1405 1410

Asp Leu Gly Glu Thr Asp Leu Ser Pro Asn Phe Gly Gln Met Ser
1415 1420 1425

Leu Ser Pro Asp Leu Ser Gln Val Thr Leu Ser Pro Asp Ile Ser
1430 1435 1440

Asp Thr Thr Leu Leu Pro Asp Leu Ser Gln Ile Ser Pro Pro Pro
1445 1450 1455

Asp Leu Asp Gln Ile Phe Tyr Pro Ser Glu Ser Ser Gln Ser Leu
1460 1465 1470

Leu Leu Gln Glu Phe Asn Glu Ser Phe Pro Tyr Pro Asp Leu Gly
1475 1480 1485

Gln Met Pro Ser Pro Ser Ser Pro Thr Leu Asn Asp Thr Phe Leu

1490 1495 1500

Ser Lys Glu Phe Asn Pro Leu Val Ile Val Gly Leu Ser Lys Asp
1505 1510 1515

Gly Thr Asp Tyr Ile Glu Ile Ile Pro Lys Glu Glu Val Gln Ser
1520 1525 1530

Ser Glu Asp Asp Tyr Ala Glu Ile Asp Tyr Val Pro Tyr Asp Asp
1535 1540 1545

Pro Tyr Lys Thr Asp Val Arg Thr Asn Ile Asn Ser Ser Arg Asp
1550 1555 1560

Pro Asp Asn Ile Ala Ala Trp Tyr Leu Arg Ser Asn Asn Gly Asn
1565 1570 1575

Arg Arg Asn Tyr Tyr Ile Ala Ala Glu Glu Ile Ser Trp Asp Tyr
1580 1585 1590

Ser Glu Phe Val Gln Arg Glu Thr Asp Ile Glu Asp Ser Asp Asp
1595 1600 1605

Ile Pro Glu Asp Thr Thr Tyr Lys Lys Val Val Phe Arg Lys Tyr
1610 1615 1620

Leu Asp Ser Thr Phe Thr Lys Arg Asp Pro Arg Gly Glu Tyr Glu
1625 1630 1635

Glu His Leu Gly Ile Leu Gly Pro Ile Ile Arg Ala Glu Val Asp
1640 1645 1650

Asp Val Ile Gln Val Arg Phe Lys Asn Leu Ala Ser Arg Pro Tyr
1655 1660 1665

Ser Leu His Ala His Gly Leu Ser Tyr Glu Lys Ser Ser Glu Gly
1670 1675 1680

Lys Thr Tyr Glu Asp Asp Ser Pro Glu Trp Phe Lys Glu Asp Asn
1685 1690 1695

Ala Val Gln Pro Asn Ser Ser Tyr Thr Tyr Val Trp His Ala Thr
1700 1705 1710

Glu Arg Ser Gly Pro Glu Ser Pro Gly Ser Ala Cys Arg Ala Trp
1715 1720 1725

```
Ala Tyr  Tyr Ser Ala Val Asn  Pro Glu Lys Asp Ile  His Ser Gly
    1730                 1735                 1740

Leu Ile  Gly Pro Leu Leu Ile  Cys Gln Lys Gly Ile  Leu His Lys
    1745                 1750                 1755

Asp Ser  Asn Met Pro Met Asp  Met Arg Glu Phe Val  Leu Leu Phe
    1760                 1765                 1770

Met Thr  Phe Asp Glu Lys Lys  Ser Trp Tyr Tyr Glu  Lys Lys Ser
    1775                 1780                 1785

Arg Ser  Ser Trp Arg Leu Thr  Ser Ser Glu Met Lys  Lys Ser His
    1790                 1795                 1800

Glu Phe  His Ala Ile Asn Gly  Met Ile Tyr Ser Leu  Pro Gly Leu
    1805                 1810                 1815

Lys Met  Tyr Glu Gln Glu Trp  Val Arg Leu His Leu  Leu Asn Ile
    1820                 1825                 1830

Gly Gly  Ser Gln Asp Ile His  Val Val His Phe His  Gly Gln Thr
    1835                 1840                 1845

Leu Leu  Glu Asn Gly Asn Lys  Gln His Gln Leu Gly  Val Trp Pro
    1850                 1855                 1860

Leu Leu  Pro Gly Ser Phe Lys  Thr Leu Glu Met Lys  Ala Ser Lys
    1865                 1870                 1875

Pro Gly  Trp Trp Leu Leu Asn  Thr Glu Val Gly Glu  Asn Gln Arg
    1880                 1885                 1890

Ala Gly  Met Gln Thr Pro Phe  Leu Ile Met Asp Arg  Asp Cys Arg
    1895                 1900                 1905

Met Pro  Met Gly Leu Ser Thr  Gly Ile Ile Ser Asp  Ser Gln Ile
    1910                 1915                 1920

Lys Ala  Ser Glu Phe Leu Gly  Tyr Trp Glu Pro Arg  Leu Ala Arg
    1925                 1930                 1935

Leu Asn  Asn Gly Gly Ser Tyr  Asn Ala Trp Ser Val  Glu Lys Leu
    1940                 1945                 1950

Ala Ala  Glu Phe Ala Ser Lys  Pro Trp Ile Gln Val  Asp Met Gln
    1955                 1960                 1965
```

```
Lys Glu  Val Ile Ile Thr Gly  Ile Gln Thr Gln Gly  Ala Lys His
    1970                 1975                 1980

Tyr Leu  Lys Ser Cys Tyr Thr  Thr Glu Phe Tyr Val  Ala Tyr Ser
    1985                 1990                 1995

Ser Asn  Gln Ile Asn Trp Gln  Ile Phe Lys Gly Asn  Ser Thr Arg
    2000                 2005                 2010

Asn Val  Met Tyr Phe Asn Gly  Asn Ser Asp Ala Ser  Thr Ile Lys
    2015                 2020                 2025

Glu Asn  Gln Phe Asp Pro Pro  Ile Val Ala Arg Tyr  Ile Arg Ile
    2030                 2035                 2040

Ser Pro  Thr Arg Ala Tyr Asn  Arg Pro Thr Leu Arg  Leu Glu Leu
    2045                 2050                 2055

Gln Gly  Cys Glu Val Asn Gly  Cys Ser Thr Pro Leu  Gly Met Glu
    2060                 2065                 2070

Asn Gly  Lys Ile Glu Asn Lys  Gln Ile Thr Ala Ser  Ser Phe Lys
    2075                 2080                 2085

Lys Ser  Trp Trp Gly Asp Tyr  Trp Glu Pro Phe Arg  Ala Arg Leu
    2090                 2095                 2100

Asn Ala  Gln Gly Arg Val Asn  Ala Trp Gln Ala Lys  Ala Asn Asn
    2105                 2110                 2115

Asn Lys  Gln Trp Leu Glu Ile  Asp Leu Leu Lys Ile  Lys Lys Ile
    2120                 2125                 2130

Thr Ala  Ile Ile Thr Gln Gly  Cys Lys Ser Leu Ser  Ser Glu Met
    2135                 2140                 2145

Tyr Val  Lys Ser Tyr Thr Ile  His Tyr Ser Glu Gln  Gly Val Glu
    2150                 2155                 2160

Trp Lys  Pro Tyr Arg Leu Lys  Ser Ser Met Val Asp  Lys Ile Phe
    2165                 2170                 2175

Glu Gly  Asn Thr Asn Thr Lys  Gly His Val Lys Asn  Phe Phe Asn
    2180                 2185                 2190

Pro Pro  Ile Ile Ser Arg Phe  Ile Arg Val Ile Pro  Lys Thr Trp
    2195                 2200                 2205
```

Asn Gln Ser Ile Ala Leu Arg Leu Glu Leu Phe Gly Cys Asp Ile
2210 2215 2220

Tyr

<210> 38
<211> 9179
<212> DNA
<213> Homo sapiens

<400> 38

gcaagaactg caggggagga ggacgctgcc acccacagcc tctagagctc attgcagctg 60
ggacagcccg gagtgtggtt agcagctcgg caagcgctgc ccaggtcctg gggtggtggc 120
agccagcggg agcaggaaag gaagcatgtt cccaggctgc ccacgcctct gggtcctggt 180
ggtcttgggc accagctggg taggctgggg gagccaaggg acagaagcgg cacagctaag 240
gcagttctac gtggctgctc agggcatcag ttggagctac cgacctgagc ccacaaactc 300
aagtttgaat ctttctgtaa cttcctttaa gaaaattgtc tacagagagt atgaaccata 360
ttttaagaaa gaaaaaccac aatctaccat ttcaggactt cttgggccta ctttatatgc 420
tgaagtcgga gacatcataa aagttcactt taaaaataag gcagataagc ccttgagcat 480
ccatcctcaa ggaattaggt acagtaaatt atcagaaggt gcttcttacc ttgaccacac 540
attccctgcg gagaagatgg acgacgctgt ggctccaggc cgagaataca cctatgaatg 600
gagtatcagt gaggacagtg gacccaccca tgatgaccct ccatgcctca cacacatcta 660
ttactcccat gaaaatctga tcgaggattt caactcgggg ctgattgggc cctgcttat 720
ctgtaaaaaa gggaccctaa ctgagggtgg gacacagaag acgtttgaca agcaaatcgt 780
gctactattt gctgtgtttg atgaaagcaa gagctggagc cagtcatcat ccctaatgta 840
cacagtcaat ggatatgtga atgggacaat gccagatata acagtttgtg cccatgacca 900
catcagctgg catctgctgg gaatgagctc ggggccagaa ttattctcca ttcatttcaa 960
cggccaggtc ctggagcaga accatcataa ggtctcagcc atcacccttg tcagtgctac 1020
atccactacc gcaaatatga ctgtgggccc agagggaaag tggatcatat cttctctcac 1080
cccaaaacat ttgcaagctg ggatgcaggc ttacattgac attaaaaact gcccaaagaa 1140
aaccaggaat cttaagaaaa taactcgtga gcagaggcgg cacatgaaga ggtgggaata 1200
cttcattgct gcagaggaag tcatttggga ctatgcacct gtaataccag cgaatatgga 1260
caaaaaatac aggtctcagc atttggataa tttctcaaac caaattggaa aacattataa 1320
gaaagttatg tacacacagt acgaagatga gtccttcacc aaacatacag tgaatcccaa 1380
tatgaaagaa gatgggattt tgggtcctat tatcagagcc caggtcagag acacactcaa 1440

aatcgtgttc aaaaatatgg ccagccgccc ctatagcatt taccctcatg gagtgacctt 1500
ctcgccttat gaagatgaag tcaactcttc tttcacctca ggcaggaaca acaccatgat 1560
cagagcagtt caaccagggg aaacctatac ttataagtgg aacatcttag agtttgatga 1620
acccacagaa aatgatgccc agtgcttaac aagaccatac tacagtgacg tggacatcat 1680
gagagacatc gcctctgggc taataggact acttctaatc tgtaagagca gatccctgga 1740
caggcgagga atacagaggg cagcagacat cgaacagcag gctgtgtttg ctgtgtttga 1800
tgagaacaaa agctggtacc ttgaggacaa catcaacaag ttttgtgaaa atcctgatga 1860
ggtgaaacgt gatgacccca agttttatga atcaaacatc atgagcacta tcaatggcta 1920
tgtgcctgag agcataacta ctcttggatt ctgctttgat gacactgtcc agtggcactt 1980
ctgtagtgtg gggacccaga atgaaatttt gaccatccac ttcactgggc actcattcat 2040
ctatggaaag aggcatgagg acaccttgac cctcttcccc atgcgtggag aatctgtgac 2100
ggtcacaatg gataatgttg gaacttggat gttaacttcc atgaattcta gtccaagaag 2160
caaaaagctg aggctgaaat tcagggatgt taaatgtatc ccagatgatg atgaagactc 2220
atatgagatt tttgaacctc cagaatctac agtcatggct acacggaaaa tgcatgatcg 2280
tttagaacct gaagatgaag agagtgatgc tgactatgat taccagaaca gactggctgc 2340
agcattagga atcaggtcat tccgaaactc atcattgaat caggaagaag aagagttcaa 2400
tcttactgcc ctagctctgg agaatggcac tgaattcgtt tcttcaaaca cagatataat 2460
tgttggttca aattattctt ccccaagtaa tattagtaag ttcactgtca ataaccttgc 2520
agaacctcag aaagcccctt ctcaccaaca agccaccaca gctggttccc cactgagaca 2580
cctcattggc aagaactcag ttctcaattc ttccacagca gagcattcca gcccatattc 2640
tgaagaccct atagaggatc ctctacagcc agatgtcaca gggatacgtc tactttcact 2700
tggtgctgga gaattcaaaa gtcaagaaca tgctaagcat aagggaccca aggtagaaag 2760
agatcaagca gcaaagcaca ggttctcctg gatgaaatta ctagcacata aagttgggag 2820
acacctaagc caagacactg gttctccttc cggaatgagg ccctgggagg accttcctag 2880
ccaagacact ggttctcctt ccagaatgag gccctggaag gaccctccta gtgatctgtt 2940
actcttaaaa caaagtaact catctaagat tttggttggg agatggcatt tggcttctga 3000
gaaaggtagc tatgaaataa tccaagatac tgatgaagac acagctgtta acaattggct 3060
gatcagcccc cagaatgcct cacgtgcttg ggagagaaagc acccctcttg ccaacaagcc 3120
tggaaagcag agtggccacc caaagtttcc tagagttaga cataaatctc tacaagtaag 3180
acaggatgga ggaaagagta gactgaagaa aagccagttt ctcattaaga cacgaaaaaa 3240
gaaaaaagag aagcacacac accatgctcc tttatctccg aggacctttc accctctaag 3300

aagtgaagcc tacaacacat tttcagaaag aagacttaag cattcgttgg tgcttcataa 3360
atccaatgaa acatctcttc ccacagacct caatcagaca ttgccctcta tggattttgg 3420
ctggatagcc tcacttcctg accataatca gaattcctca aatgacactg gtcaggcaag 3480
ctgtcctcca ggtctttatc agacagtgcc cccagaggaa cactatcaaa cattccccat 3540
tcaagaccct gatcaaatgc actctacttc agaccccagt cacagatcct cttctccaga 3600
gctcagtgaa atgcttgagt atgaccgaag tcacaagtcc ttccccacag atataagtca 3660
aatgtcccct tcctcagaac atgaagtctg gcagacagtc atctctccag acctcagcca 3720
ggtgaccctc tctccagaac tcagccagac aaacctctct ccagacctca gccacacgac 3780
tctctctcca gaactcattc agagaaacct ttccccagcc ctcggtcaga tgcccatttc 3840
tccagacctc agccatacaa ccctttctcc agacctcagc catacaaccc tttctttaga 3900
cctcagccag acaaacctct ctccagaact cagtcagaca aacctttctc cagccctcgg 3960
tcagatgccc ctttctccag acctcagcca tacaaccctt tctctagact tcagccagac 4020
aaacctctct ccagaactca gccatatgac tctctctcca gaactcagtc agacaaacct 4080
ttccccagcc ctcggtcaga tgcccatttc tccagacctc agccatacaa ccctttctct 4140
agacttcagc cagacaaacc tctctccaga actcagtcaa acaaaccttt ccccagccct 4200
cggtcagatg cccctttctc cagaccccag ccatacaacc ctttctctag acctcagcca 4260
gacaaacctc tctccagaac tcagtcagac aaacctttcc ccagacctca gtgagatgcc 4320
cctctttgca gatctcagtc aaattcccct taccccagac ctcgaccaga tgacactttc 4380
tccagacctt ggtgagacag atctttcccc aaactttggt cagatgtccc tttccccaga 4440
cctcagccag gtgactctct ctccagacat cagtgacacc acccttctcc cggatctcag 4500
ccagatatca cctcctccag accttgatca gatattctac ccttctgaat ctagtcagtc 4560
attgcttctt caagaattta atgagtcttt tccttatcca gaccttggtc agatgccatc 4620
tccttcatct cctactctca atgatacttt tctatcaaag gaatttaatc cactggttat 4680
agtgggcctc agtaaagatg gtacagatta cattgagatc attccaaagg aagaggtcca 4740
gagcagtgaa gatgactatg ctgaaattga ttatgtgccc tatgatgacc cctacaaaac 4800
tgatgttagg acaaacatca actcctccag agatcctgac aacattgcag catggtacct 4860
ccgcagcaac aatggaaaca gaagaaatta ttacattgct gctgaagaaa tatcctggga 4920
ttattcagaa tttgtacaaa gggaaacaga tattgaagac tctgatgata ttccagaaga 4980
taccacatat aagaaagtag tttttcgaaa gtacctcgac agcactttta ccaaacgtga 5040
tcctcgaggg gagtatgaag agcatctcgg aattcttggt cctattatca gagctgaagt 5100
ggatgatgtt atccaagttc gttttaaaaa tttagcatcc agaccgtatt ctctacatgc 5160
ccatggactt tcctatgaaa aatcatcaga gggaaagact tatgaagatg actctcctga 5220

atggtttaag gaagataatg ctgttcagcc aaatagcagt tatacctacg tatggcatgc 5280
cactgagcga tcagggccag aaagtcctgg ctctgcctgt cgggcttggg cctactactc 5340
agctgtgaac ccagaaaaag atattcactc aggcttgata ggtcccctcc taatctgcca 5400
aaaaggaata ctacataagg acagcaacat gcctatggac atgagagaat ttgtcttact 5460
atttatgacc tttgatgaaa agaagagctg gtactatgaa aagaagtccc gaagttcttg 5520
gagactcaca tcctcagaaa tgaaaaaatc ccatgagttt cacgccatta atgggatgat 5580
ctacagcttg cctggcctga aaatgtatga gcaagagtgg gtgaggttac acctgctgaa 5640
cataggcggc tcccaagaca ttcacgtggt tcactttcac ggccagacct tgctggaaaa 5700
tggcaataaa cagcaccagt taggggtctg gccccttctg cctggttcat ttaaaactct 5760
tgaaatgaag gcatcaaaac ctggctggtg gctcctaaac acagaggttg gagaaaacca 5820
gagagcaggg atgcaaacgc catttcttat catggacaga gactgtagga tgccaatggg 5880
actaagcact ggtatcatat ctgattcaca gatcaaggct tcagagtttc tgggttactg 5940
ggagcccaga ttagcaagat taaacaatgg tggatcttat aatgcttgga gtgtagaaaa 6000
acttgcagca gaatttgcct ctaaaccttg gatccaggtg gacatgcaaa aggaagtcat 6060
aatcacaggg atccagaccc aaggtgccaa acactacctg aagtcctgct ataccacaga 6120
gttctatgta gcttacagtt ccaaccagat caactggcag atcttcaaag ggaacagcac 6180
aaggaatgtg atgtatttta atggcaattc agatgcctct acaataaaag agaatcagtt 6240
tgacccacct attgtggcta gatatattag gatctctcca actcgagcct ataacagacc 6300
tacccttcga ttggaactgc aaggttgtga ggtaaatgga tgttccacac ccctgggtat 6360
ggaaaatgga aagatagaaa acaagcaaat cacagcttct tcgtttaaga aatcttggtg 6420
gggagattac tgggaaccct tccgtgcccg tctgaatgcc cagggacgtg tgaatgcctg 6480
gcaagccaag gcaaacaaca ataagcagtg gctagaaatt gatctactca agatcaagaa 6540
gataacggca attataacac agggctgcaa gtctctgtcc tctgaaatgt atgtaaagag 6600
ctataccatc cactacagtg agcagggagt ggaatggaaa ccatacaggc tgaaatcctc 6660
catggtggac aagatttttg aaggaaatac taataccaaa ggacatgtga agaacttttt 6720
caacccccca atcatttcca ggtttatccg tgtcattcct aaaacatgga atcaaagtat 6780
tgcacttcgc ctggaactct ttggctgtga tatttactag aattgaacat tcaaaaaccc 6840
ctggaagaga ctctttaaga cctcaaacca tttagaatgg gcaatgtatt ttacgctgtg 6900
ttaaatgtta acagttttcc actatttctc tttcttttct attagtgaat aaaattttat 6960
acaagaagct tttataatgt aactccttgc taccagtaag taagataatg gctattactt 7020
ctgcattaat ttgaatacag gtaggaaaat atcaagaacc aacaagaaaa gggcttatct 7080

ttcttaatga ttgaaaatgc tatgaagtaa tatttatgta gttaaaatgc ttcattataa 7140
ctcttttaaa tcctttacac actagtaaaa cagatattac tttaaataat aattgataga 7200
cctggataac tttcacaaac acatgatttt ttaatggttt ttcttgagtg aagagaaaaa 7260
caatattatc aaatgaaata agtacttaaa atatcctgtc tttcccatat aacaatgatt 7320
tttctgactt tccatgagta aaaaaacagc caagcatctt tccagtagcc ccattgaaat 7380
tgtgaatccg tcctggtctc cctaaggact gcacacattg atattcaagg ttggtggtca 7440
ttagatatgg aacagaactg aaataaccat ggtagaactg aatgtgtaat gttggcttta 7500
ttctagctgg tactacatgg cacacagttt caaaacataa tttcacctac tggaaagctc 7560
agacctgtaa aacagagcat gggaactgct ggtctaaatg cagttgttcc tgctcaaaga 7620
gacctctggc caaactggca agcagttaaa gttttctttc agggccttcc tctctatggc 7680
ctcaacttcc tcctctctct tcttccagca acttcccctt tcatcattcc tttccctggg 7740
gacttggcat tcagtgatcc tgtagatatt gcacaactgg ggaaccttta gacatcctta 7800
aaatcacatg agatagacag tcatttgggg tgtctgaaat aaaccacccc aaaacttagt 7860
gttaaaagag caaccaaaaa aaatttatgt gagattatgg atttgttact tagcttgatt 7920
taatcatcct gtaacgtgta catatatcaa aatgttatgt ataccataaa tatataaaat 7980
tttatcaacg aaattcataa caatctctca gaccacagag aaatcaaatt agaactgagg 8040
actaagaaac tcactcgaaa ccacacaact acatggaaac tgaacaacct gctcctgaat 8100
gactactggg taaataatga aattaaggca gaaataaata agttccttaa aaccaatgag 8160
aacaaagaga caacatacca gaatctctag gagacagggc tttgcttttg ctgcattcta 8220
ttcgttgtga acacaaatta caggccagtc tcgattcagt gtagaaggga actgcataag 8280
gaccacatac caggaggcat aattcactgg gagcatcttt agaaactacc agagttacct 8340
gttgcccata ccagtggggt aagccctatg aatgtatatg agagtttcaa acatccacaa 8400
aacattggct ttctaatatt cgtattccca ctattccttt cttttcatga ttcatgtcat 8460
tgtcccatca acatttctaa gatttccatt ccgttaagag caaaagagaa tgttggaagg 8520
tggggggaaaa catttctttg ttttctacag ggccagcttc ttggatgtgt gtgatctgtt 8580
cagttgcaaa gggtcacatg ctcagaagga ccgcatgcta aatttaatgc tttgcagtta 8640
ccctcttgaa atcctttatt ttttaagaag gaattcgaca tttccatttt tcaatgagcc 8700
ccacaaatta cgcagctagt cctgggcttc tctactctga aattgggcag gatctctctt 8760
gatctagaat ttactaaggc ataatagggg caagaaaatc ttatgaaata atggggggta 8820
gggaagagat gggaatggag catgagatcc agcttcgtta ttctctactt gagaaaaata 8880
aggccccaaa gattaaacaa cttgcccaag gatattgctt gttagtgtca gaactgaaac 8940
cagaaaccaa atgatcatat ccctagactt ttagtctgct ttctcttcca taaaatgaaa 9000

```
cttataatgt ttctaatcca ttgctcagac aggtagacat gaatattaat tgataatgac     9060
tattaattga tctggaaaat acttgtttgg ggatcaataa tatgtttggg ctattatcta     9120
atgctgtgta gaaatattaa aacccctgtt attttgaaat aaaaaagata cccactttt      9179
```

<210> 39
<211> 694
<212> PRT
<213> Homo sapiens

<400> 39

```
Met Glu Trp Gly Tyr Leu Leu Glu Val Thr Ser Leu Leu Ala Ala Leu
1               5                   10                  15

Ala Leu Leu Gln Arg Ser Ser Gly Ala Ala Ala Ala Ser Ala Lys Glu
            20                  25                  30

Leu Ala Cys Gln Glu Ile Thr Val Pro Leu Cys Lys Gly Ile Gly Tyr
        35                  40                  45

Asn Tyr Thr Tyr Met Pro Asn Gln Phe Asn His Asp Thr Gln Asp Glu
    50                  55                  60

Ala Gly Leu Glu Val His Gln Phe Trp Pro Leu Val Glu Ile Gln Cys
65                  70                  75                  80

Ser Pro Asp Leu Lys Phe Phe Leu Cys Ser Met Tyr Thr Pro Ile Cys
                85                  90                  95

Leu Glu Asp Tyr Lys Lys Pro Leu Pro Pro Cys Arg Ser Val Cys Glu
            100                 105                 110

Arg Ala Lys Ala Gly Cys Ala Pro Leu Met Arg Gln Tyr Gly Phe Ala
        115                 120                 125

Trp Pro Asp Arg Met Arg Cys Asp Arg Leu Pro Glu Gln Gly Asn Pro
    130                 135                 140

Asp Thr Leu Cys Met Asp Tyr Asn Arg Thr Asp Leu Thr Thr Ala Ala
145                 150                 155                 160

Pro Ser Pro Pro Arg Arg Leu Pro Pro Pro Pro Pro Gly Glu Gln Pro
                165                 170                 175

Pro Ser Gly Ser Gly His Gly Arg Pro Pro Gly Ala Arg Pro Pro His
            180                 185                 190

Arg Gly Gly Gly Arg Gly Gly Gly Gly Gly Asp Ala Ala Ala Pro Pro
```

```
        195                 200                 205

Ala Arg Gly Gly Gly Gly Gly Gly Lys Ala Arg Pro Pro Gly Gly Gly
    210                 215                 220

Ala Ala Pro Cys Glu Pro Gly Cys Gln Cys Arg Ala Pro Met Val Ser
225                 230                 235                 240

Val Ser Ser Glu Arg His Pro Leu Tyr Asn Arg Val Lys Thr Gly Gln
                245                 250                 255

Ile Ala Asn Cys Ala Leu Pro Cys His Asn Pro Phe Phe Ser Gln Asp
            260                 265                 270

Glu Arg Ala Phe Thr Val Phe Trp Ile Gly Leu Trp Ser Val Leu Cys
        275                 280                 285

Phe Val Ser Thr Phe Ala Thr Val Ser Thr Phe Leu Ile Asp Met Glu
    290                 295                 300

Arg Phe Lys Tyr Pro Glu Arg Pro Ile Ile Phe Leu Ser Ala Cys Tyr
305                 310                 315                 320

Leu Phe Val Ser Val Gly Tyr Leu Val Arg Leu Val Ala Gly His Glu
                325                 330                 335

Lys Val Ala Cys Ser Gly Gly Ala Pro Gly Ala Gly Gly Ala Gly Gly
            340                 345                 350

Ala Gly Gly Ala Ala Ala Gly Ala Gly Ala Ala Gly Ala Gly Ala Gly
        355                 360                 365

Gly Pro Gly Gly Arg Gly Glu Tyr Glu Glu Leu Gly Ala Val Glu Gln
    370                 375                 380

His Val Arg Tyr Glu Thr Thr Gly Pro Ala Leu Cys Thr Val Val Phe
385                 390                 395                 400

Leu Leu Val Tyr Phe Phe Gly Met Ala Ser Ser Ile Trp Trp Val Ile
                405                 410                 415

Leu Ser Leu Thr Trp Phe Leu Ala Ala Gly Met Lys Trp Gly Asn Glu
            420                 425                 430

Ala Ile Ala Gly Tyr Ser Gln Tyr Phe His Leu Ala Ala Trp Leu Val
        435                 440                 445
```

Pro Ser Val Lys Ser Ile Ala Val Leu Ala Leu Ser Ser Val Asp Gly
450 455 460

Asp Pro Val Ala Gly Ile Cys Tyr Val Gly Asn Gln Ser Leu Asp Asn
465 470 475 480

Leu Arg Gly Phe Val Leu Ala Pro Leu Val Ile Tyr Leu Phe Ile Gly
485 490 495

Thr Met Phe Leu Leu Ala Gly Phe Val Ser Leu Phe Arg Ile Arg Ser
500 505 510

Val Ile Lys Gln Gln Asp Gly Pro Thr Lys Thr His Lys Leu Glu Lys
515 520 525

Leu Met Ile Arg Leu Gly Leu Phe Thr Val Leu Tyr Thr Val Pro Ala
530 535 540

Ala Val Val Val Ala Cys Leu Phe Tyr Glu Gln His Asn Arg Pro Arg
545 550 555 560

Trp Glu Ala Thr His Asn Cys Pro Cys Leu Arg Asp Leu Gln Pro Asp
565 570 575

Gln Ala Arg Arg Pro Asp Tyr Ala Val Phe Met Leu Lys Tyr Phe Met
580 585 590

Cys Leu Val Val Gly Ile Thr Ser Gly Val Trp Val Trp Ser Gly Lys
595 600 605

Thr Leu Glu Ser Trp Arg Ser Leu Cys Thr Arg Cys Cys Trp Ala Ser
610 615 620

Lys Gly Ala Ala Val Gly Gly Gly Ala Gly Ala Thr Ala Ala Gly Gly
625 630 635 640

Gly Gly Gly Pro Gly Gly Gly Gly Gly Gly Gly Pro Gly Gly Gly Gly
645 650 655

Gly Pro Gly Gly Gly Gly Gly Ser Leu Tyr Ser Asp Val Ser Thr Gly
660 665 670

Leu Thr Trp Arg Ser Gly Thr Ala Ser Ser Val Ser Tyr Pro Lys Gln
675 680 685

Met Pro Leu Ser Gln Val
690

<210> 40
<211> 3195
<212> DNA
<213> Homo sapiens

<400> 40

acagcatgga gtggggttac ctgttggaag tgacctcgct gctggccgcc ttggcgctgc 60

tgcagcgctc tagcggcgct gcggccgcct cggccaagga gctggcatgc caagagatca 120

ccgtgccgct gtgtaagggc atcggctaca actacaccta catgcccaat cagttcaacc 180

acgacacgca agacgaggcg ggcctggagg tgcaccagtt ctggccgctg gtggagatcc 240

agtgctcgcc cgatctcaag ttcttcctgt gcagcatgta cacgcccatc tgcctagagg 300

actacaagaa gccgctgccg ccctgccgct cggtgtgcga gcgcgccaag gccggctgcg 360

cgccgctcat gcgccagtac ggcttcgcct ggcccgaccg catgcgctgc gaccggctgc 420

ccgagcaagg caaccctgac acgctgtgca tggactacaa ccgcaccgac ctaaccaccg 480

ccgcgcccag cccgccgcgc cgcctgccgc cgccgccgcc cggcgagcag ccgccttcgg 540

gcagcggcca cggccgcccg ccgggggcca ggcccccgca ccgcggaggc ggcaggggcg 600

gtggcggcgg ggacgcggcg gcgcccccag ctcgcggcgg cggcggtggc gggaaggcgc 660

ggcccccctgg cggcggcgcg gctccctgcg agcccgggtg ccagtgccgc gcgcctatgg 720

tgagcgtgtc cagcgagcgc cacccgctct acaaccgcgt caagacaggc cagatcgcta 780

actgcgcgct gccctgccac aacccctttt tcagccagga cgagcgcgcc ttcaccgtct 840

tctggatcgg cctgtggtcg gtgctctgct tcgtgtccac cttcgccacc gtctccacct 900

tccttatcga catggagcgc ttcaagtacc cggagcggcc cattatcttc ctctcggcct 960

gctacctctt cgtgtcggtg ggctacctag tgcgcctggt ggcgggccac gagaaggtgg 1020

cgtgcagcgg tggcgcgccg ggcgcggggg gcgctggggg cgcgggcggc gcggcggcgg 1080

gcgcgggcgc ggcgggcgcg ggcgcgggcg gcccgggcgg gcgcggcgag tacgaggagc 1140

tgggcgcggt ggagcagcac gtgcgctacg agaccaccgg ccccgcgctg tgcaccgtgg 1200

tcttcttgct ggtctacttc ttcggcatgg ccagctccat ctggtgggtg atcttgtcgc 1260

tcacatggtt cctggcggcc ggtatgaagt ggggcaacga agccatcgcc ggctactcgc 1320

agtacttcca cctggccgcg tggcttgtgc ccagcgtcaa gtccatcgcg gtgctggcgc 1380

tcagctcggt ggacggcgac ccggtggcgg gcatctgcta cgtgggcaac cagagcctgg 1440

acaacctgcg cggcttcgtg ctggcgccgc tggtcatcta cctcttcatc ggcaccatgt 1500

tcctgctggc cggcttcgtg tccctgttcc gcatccgctc ggtcatcaag caacaggacg 1560

gccccaccaa gacgcacaag ctggagaagc tgatgatccg cctgggcctg ttcaccgtgc 1620

tctacaccgt gcccgccgcg gtggtggtcg cctgcctctt ctacgagcag cacaaccgcc 1680

cgcgctggga ggccacgcac aactgcccgt gcctgcggga cctgcagccc gaccaggcac 1740

gcaggcccga ctacgccgtc ttcatgctca agtacttcat gtgcctagtg gtgggcatca 1800

cctcgggcgt gtgggtctgg tccggcaaga cgctggagtc ctggcgctcc ctgtgcaccc 1860

gctgctgctg ggccagcaag ggcgccgcgg tgggcggggg cgcgggcgcc acggccgcgg 1920

ggggtggcgg cgggccgggg ggcggcggcg gcgggggacc cggcggcggc gggggggccgg 1980

gcggcggcgg gggctccctc tacagcgacg tcagcactgg cctgacgtgg cggtcgggca 2040

cggcgagctc cgtgtcttat ccaaagcaga tgccattgtc ccaggtctga gcggaggggga 2100

gggggcgccc aggaggggtg gggaggggggg cgaggagacc caagtgcagc gaagggacac 2160

ttgatgggct gaggttccca ccccttcaca gtgttgattg ctattagcat gataatgaac 2220

tcttaatggt atccattagc tgggacttaa atgactcact tagaacaaag tacctggcat 2280

tgaagcctcc cagacccagc cccttttcct ccattgatgt gcggggagct cctcccgcca 2340

cgcgttaatt tctgttggct gaggagggtg gactctgcgg cgtttccaga acccgagatt 2400

tggagccctc cctggctgca cttggctggg tttgcagtca gatacacaga tttcacctgg 2460

gagaacctct ttttctccct cgactcttcc tacgtaaact cccaccccctg acttaccctg 2520

gaggaggggt gaccgccacc tgatgggatt gcacggtttg ggtattctta atgaccaggc 2580

aaatgcctta agtaaacaaa caagaaatgt cttaattata caccccacgt aaatacgggt 2640

ttcttacatt agaggatgta tttatataat tatttgttaa attgtaaaaa aaaaaagtgt 2700

aaaatatgta tatatccaaa gatatagtgt gtacattttt ttgtaaaaag tttagaggct 2760

taccccctgta agaacagata taagtattct attttgtcaa taaaatgact tttgataaat 2820

gatttaacca ttgccctctc ccccgcctct tctgagctgt caccttttaaa gtgcttgcta 2880

aggacgcatg gggaaaatgg acattttctg gcttgtcatt ctgtacactg accttaggca 2940

tggagaaaat tacttgttaa actctagttc ttaagttgtt agccaagtaa atatcattgt 3000

tgaactgaaa tcaaaattga gtttttgcac cttccccaaa gacggtgttt ttcatgggag 3060

ctcttttctg atccatggat aacaactctc actttagtgg atgtaaatgg aacttctgca 3120

aggcagtaat tcccccttagg ccttgttatt tatcctgcat ggtatcacta aaggtttcaa 3180

aaccctgaaa aaaaa 3195

<210> 41
<211> 220
<212> PRT
<213> Homo sapiens

<400> 41

Met Ser Met Gly Leu Glu Ile Thr Gly Thr Ala Leu Ala Val Leu Gly
1 5 10 15

```
Trp Leu Gly Thr Ile Val Cys Cys Ala Leu Pro Met Trp Arg Val Ser
            20                  25                  30

Ala Phe Ile Gly Ser Asn Ile Ile Thr Ser Gln Asn Ile Trp Glu Gly
        35                  40                  45

Leu Trp Met Asn Cys Val Val Gln Ser Thr Gly Gln Met Gln Cys Lys
    50                  55                  60

Val Tyr Asp Ser Leu Leu Ala Leu Pro Gln Asp Leu Gln Ala Ala Arg
65                  70                  75                  80

Ala Leu Ile Val Val Ala Ile Leu Leu Ala Ala Phe Gly Leu Leu Val
                85                  90                  95

Ala Leu Val Gly Ala Gln Cys Thr Asn Cys Val Gln Asp Asp Thr Ala
            100                 105                 110

Lys Ala Lys Ile Thr Ile Val Ala Gly Val Leu Phe Leu Leu Ala Ala
        115                 120                 125

Leu Leu Thr Leu Val Pro Val Ser Trp Ser Ala Asn Thr Ile Ile Arg
    130                 135                 140

Asp Phe Tyr Asn Pro Val Val Pro Glu Ala Gln Lys Arg Glu Met Gly
145                 150                 155                 160

Ala Gly Leu Tyr Val Gly Trp Ala Ala Ala Ala Leu Gln Leu Leu Gly
                165                 170                 175

Gly Ala Leu Leu Cys Cys Ser Cys Pro Pro Arg Glu Lys Lys Tyr Thr
            180                 185                 190

Ala Thr Lys Val Val Tyr Ser Ala Pro Arg Ser Thr Gly Pro Gly Ala
        195                 200                 205

Ser Leu Gly Thr Gly Tyr Asp Arg Lys Asp Tyr Val
    210                 215                 220
```

<210> 42
<211> 1318
<212> DNA
<213> Homo sapiens

<400> 42

agcccagtcg ccgccgcccg cccacaaagc cacaggcagg tgcaggcgca gccgcggcga 60

gagcgtatgg agccgagccg ttagcgcgcg ccgtcggtga gtcagtccgt ccgtccgtcc 120

gtccgtcggg gcgccgcagc tcccgccagg cccagcggcc ccggcccctc gtctccccgc 180

acccggagcc acccggtgga gcgggccttg ccgcggcagc catgtccatg ggcctggaga 240

tcacgggcac cgcgctggcc gtgctgggct ggctgggcac catcgtgtgc tgcgcgttgc 300

ccatgtggcg cgtgtcggcc ttcatcggca gcaacatcat cacgtcgcag aacatctggg 360

agggcctgtg gatgaactgc gtggtgcaga gcaccggcca gatgcagtgc aaggtgtacg 420

actcgctgct ggcactgcca caggaccttc aggcggcccg cgccctcatc gtggtggcca 480

tcctgctggc cgccttcggg ctgctagtgg cgctggtggg cgcccagtgc accaactgcg 540

tgcaggacga cacggccaag gccaagatca ccatcgtggc aggcgtgctg ttccttctcg 600

ccgccctgct caccctcgtg ccggtgtcct ggtcggccaa caccattatc cgggacttct 660

acaaccccgt ggtgcccgag gcgcagaagc gcgagatggg cgcgggcctg tacgtgggct 720

gggcggccgc ggcgctgcag ctgctggggg gcgcgctgct ctgctgctcg tgtcccccac 780

gcgagaagaa gtacacggcc accaaggtcg tctactccgc gccgcgctcc accggcccgg 840

gagccagcct gggcacaggc tacgaccgca aggactacgt ctaagggaca gacgcaggga 900

gaccccacca ccaccaccac caccaacacc accaccacca ccgcgagctg gagcgcgcac 960

caggccatcc agcgtgcagc cttgcctcgg aggccagccc acccccagaa gccaggaagc 1020

ccccgcgctg gactggggca gcttccccag cagccacggc tttgcgggcc gggcagtcga 1080

cttcggggcc cagggaccaa cctgcatgga ctgtgaaacc tcacccttct ggagcacggg 1140

gcctgggtga ccgccaatac ttgaccaccc cgtcgagccc catcgggccg ctgcccccat 1200

gctcgcgctg ggcagggacc ggcagccctg gaaggggcac ttgatatttt tcaataaaag 1260

cctttcgttt tgcaaaaaaa aaaaaaaaaa aaaaaaaaaa aaaaaaaaaa aaaaaaaa 1318

<210> 43
<211> 264
<212> PRT
<213> Homo sapiens

<400> 43

```
Met Ile Met Ser Ser Tyr Leu Met Asp Ser Asn Tyr Ile Asp Pro Lys
1               5                   10                  15

Phe Pro Pro Cys Glu Glu Tyr Ser Gln Asn Ser Tyr Ile Pro Glu His
            20                  25                  30

Ser Pro Glu Tyr Tyr Gly Arg Thr Arg Glu Ser Gly Phe Gln His His
        35                  40                  45

His Gln Glu Leu Tyr Pro Pro Pro Pro Pro Arg Pro Ser Tyr Pro Glu
    50                  55                  60

Arg Gln Tyr Ser Cys Thr Ser Leu Gln Gly Pro Gly Asn Ser Arg Gly
```

```
65                  70                  75                  80

His Gly Pro Ala Gln Ala Gly His His His Pro Glu Lys Ser Gln Ser
                85                  90                  95

Leu Cys Glu Pro Ala Pro Leu Ser Gly Ala Ser Ala Ser Pro Ser Pro
            100                 105                 110

Ala Pro Pro Ala Cys Ser Gln Pro Ala Pro Asp His Pro Ser Ser Ala
        115                 120                 125

Ala Ser Lys Gln Pro Ile Val Tyr Pro Trp Met Lys Lys Ile His Val
    130                 135                 140

Ser Thr Val Asn Pro Asn Tyr Asn Gly Gly Glu Pro Lys Arg Ser Arg
145                 150                 155                 160

Thr Ala Tyr Thr Arg Gln Gln Val Leu Glu Leu Glu Lys Glu Phe His
                165                 170                 175

Tyr Asn Arg Tyr Leu Thr Arg Arg Arg Arg Ile Glu Ile Ala His Ser
            180                 185                 190

Leu Cys Leu Ser Glu Arg Gln Ile Lys Ile Trp Phe Gln Asn Arg Arg
        195                 200                 205

Met Lys Trp Lys Lys Asp His Arg Leu Pro Asn Thr Lys Val Arg Ser
    210                 215                 220

Ala Pro Pro Ala Gly Ala Ala Pro Ser Thr Leu Ser Ala Ala Thr Pro
225                 230                 235                 240

Gly Thr Ser Glu Asp His Ser Gln Ser Ala Thr Pro Pro Glu Gln Gln
                245                 250                 255

Arg Ala Glu Asp Ile Thr Arg Leu
            260
```

<210> 44
<211> 2328
<212> DNA
<213> Homo sapiens

<400> 44

```
aactttttat tgtggtttgt ccgttccgag cgctccgcag aacagtcctc cctgtaagag      60
cctaaccatt gccagggaaa cctgccctgg gcgctccctt cattagcagt atttttttta     120
aattaatctg attaataatt atttttcccc catttaattt tttttcctcc caggtggagt     180
```

tgccgaagct gggggcagct ggggagggtg gggatgggag gggagagaca gaagttgagg 240
gcatctctct cttccttccc gaccctctgg cccccaaggg gcaggaggaa tgcaggagca 300
ggagttgagc ttgggagctg cagatgcctc cgcccctcct ctctcccagg ctcttcctcc 360
tgccccccttc ttgcaactct ccttaatttt gtttggcttt tggatgatta taattatttt 420
tatttttgaa tttatataaa gtatatgtgt gtgtgtgtgg agctgagaca ggctcggcag 480
cggcacagaa tgagggaaga cgagaaagag agtgggagag agagaggcag agagggagag 540
agggagagtg acagcagcgc tcgcgggggc tcaaccccca gacctccaga aatgacgtca 600
gaatcatttg catcccgctg cctctacctg cctggtccag ctgggaccct gcctcgccgg 660
ccgcatggcc agagggttgg aaattaatga tcatgagctc gtatttgatg gactctaact 720
acatcgatcc gaaatttcct ccatgcgaag aatattcgca aaatagctac atccctgaac 780
acagtccgga atattacggc cggaccaggg aatcgggatt ccagcatcac caccaggagc 840
tgtacccacc accgcctccg cgccctagct accctgagcg ccagtatagc tgcaccagtc 900
tccaggggcc cggcaattcg cgaggccacg ggccggccca ggcgggccac caccaccccg 960
agaaatcaca gtcgctctgc gagccggcgc ctctctcagg cgcctccgcc tccccgtccc 1020
cagccccgcc agcctgcagc cagccagccc ccgaccatcc ctccagcgcc gccagcaagc 1080
aacccatagt ctacccatgg atgaaaaaaa ttcacgttag cacggtgaac cccaattata 1140
acggagggga acccaagcgc tcgaggacag cctatacccg gcagcaagtc ctggaattag 1200
agaaagagtt tcattacaac cgctacctga cccgaaggag aaggatcgag atcgcccact 1260
cgctgtgcct ctctgagagg cagatcaaaa tctggttcca aaaccgtcgc atgaaatgga 1320
agaaggacca ccgactcccc aacaccaaag tcaggtcagc acccccggcc ggcgctgcgc 1380
ccagcaccct ttcggcagct accccgggta cttctgaaga ccactcccag agcgccacgc 1440
cgccggagca gcaacgggca gaggacatta ccaggttata aaacataact cacacccctg 1500
cccccacccc atgcccccac cctcccctca cacacaaatt gactcttatt tatagaattt 1560
aatatatata tatatatata tatatatagg ttcttttctc tcttcctctc accttgtccc 1620
ttgtcagttc caaacagaca aaacagataa acaaacaagc cccctgccct cctctccctc 1680
ccactgttaa ggaccctttt aagcatgtga tgttgtctta gcatggtacc tgctgggtgt 1740
ttttttttaa aaggccattt tgggggggtta tttatttttt aagaaaaaaa gctgcaaaaa 1800
ttatatattg caaggtgtga tggtctggct tgggtgaatt tcaggggaaa tgaggaaaag 1860
aaaaaaggaa agaaatttta aagccaattc tcatccttct cctcctcctc cttccccccc 1920
tctttcctta ggccttttgc attgaaaatg caccagggga ggttagtgag ggggaagtca 1980
ttttaaggag aacaaagcta tgaagttctt ttgtattatt gttggggggg ggtgtgggag 2040
gagaggggc gaagacagca gacaaagcta aatgcatctg gagagcctct cagagctgtt 2100

```
cagtttgagg agccaaaaga aaatcaaaat gaactttcag ttcagagagg cagtctatag     2160

gtagaatctc tccccacccc tatcgtggtt attgtgtttt tggactgaat ttacttgatt     2220

attgtaaaac ttgcaataaa gaattttagt gtcgatgtga aatgccccgt gatcaataat     2280

aaaccagtgg atgtgaatta gttttaaaaa aaaaaaaaaa aaaaaaaa                  2328
```

<210> 45
<211> 264
<212> PRT
<213> Homo sapiens

<400> 45

```
Met Ile Met Ser Ser Tyr Leu Met Asp Ser Asn Tyr Ile Asp Pro Lys
1               5                   10                  15

Phe Pro Pro Cys Glu Glu Tyr Ser Gln Asn Ser Tyr Ile Pro Glu His
            20                  25                  30

Ser Pro Glu Tyr Tyr Gly Arg Thr Arg Glu Ser Gly Phe Gln His His
        35                  40                  45

His Gln Glu Leu Tyr Pro Pro Pro Pro Pro Arg Pro Ser Tyr Pro Glu
    50                  55                  60

Arg Gln Tyr Ser Cys Thr Ser Leu Gln Gly Pro Gly Asn Ser Arg Gly
65                  70                  75                  80

His Gly Pro Ala Gln Ala Gly His His His Pro Glu Lys Ser Gln Ser
                85                  90                  95

Leu Cys Glu Pro Ala Pro Leu Ser Gly Ala Ser Ala Ser Pro Ser Pro
            100                 105                 110

Ala Pro Pro Ala Cys Ser Gln Pro Ala Pro Asp His Pro Ser Ser Ala
        115                 120                 125

Ala Ser Lys Gln Pro Ile Val Tyr Pro Trp Met Lys Lys Ile His Val
    130                 135                 140

Ser Thr Val Asn Pro Asn Tyr Asn Gly Gly Glu Pro Lys Arg Ser Arg
145                 150                 155                 160

Thr Ala Tyr Thr Arg Gln Gln Val Leu Glu Leu Glu Lys Glu Phe His
                165                 170                 175

Tyr Asn Arg Tyr Leu Thr Arg Arg Arg Arg Ile Glu Ile Ala His Ser
            180                 185                 190
```

```
Leu Cys Leu Ser Glu Arg Gln Ile Lys Ile Trp Phe Gln Asn Arg Arg
        195                 200                 205

Met Lys Trp Lys Lys Asp His Arg Leu Pro Asn Thr Lys Val Arg Ser
    210                 215                 220

Ala Pro Pro Ala Gly Ala Ala Pro Ser Thr Leu Ser Ala Ala Thr Pro
225                 230                 235                 240

Gly Thr Ser Glu Asp His Ser Gln Ser Ala Thr Pro Pro Glu Gln Gln
                245                 250                 255

Arg Ala Glu Asp Ile Thr Arg Leu
            260
```

<210> 46
<211> 2459
<212> DNA
<213> Homo sapiens

<400> 46

```
acacacgtgt tacatggata cagctcctag ccagaagcaa gcaggctcta cccacacagg      60
cctccctctt agctagcggg ggctcaaccc ccagacctcc agaaatgacg tcagaatcat     120
ttgcatcccg ctgcctctac ctgcctggtc cagctgggac cctgcctcgc cggccgcatg     180
gccagagggt tgggtgagtg tgtatgggga agaggggctg gactctggta tccttggatg     240
gggggcactc caggctctcc agcctcctcg gctcagcctg ggcccctccc catccaacat     300
ccactccagt cctcattcaa cttcctcttc ctgcgaaaga ggggcgctgc cccgtgacct     360
acacagactg agacacgatc gccatgaatg gagacctctg gaaaagctca ggagccgagg     420
cccacggggc ccagcagagg cctgagggga gaccctgggc gggggctgaa tcactgcctc     480
ccgacagtcc cccaatgccc gggctttgga ggggagccgg gagcttccca tctccttttg     540
caggggaggg ttgtcagtct ggcgggatgt gcactggggg cactccaacc tctgctagct     600
aaccccacat caccacccac ccccgcctcc cagcaccacc accaccacac acacaaaaaa     660
attggataca ttttgaataa agcgattcgg ttccttatcc ggggactggg ttgctccgtg     720
tgattggccg gaggagtcac atggtgaaag taactttaca gggtcgctag ctagtaggag     780
ggctttatgg agcagaaaaa cgacaaagcg agaaaaatta ttttccactc cagaaattaa     840
tgatcatgag ctcgtatttg atggactcta actacatcga tccgaaattt cctccatgcg     900
aagaatattc gcaaaatagc tacatccctg aacacagtcc ggaatattac ggccggacca     960
gggaatcggg attccagcat caccaccagg agctgtaccc accaccgcct ccgcgcccta    1020
gctaccctga gcgccagtat agctgcacca gtctccaggg gcccggcaat tcgcgaggcc    1080
```

acgggccggc ccaggcgggc caccaccacc ccgagaaatc acagtcgctc tgcgagccgg 1140
cgcctctctc aggcgcctcc gcctccccgt ccccagcccc gccagcctgc agccagccag 1200
cccccgacca tccctccagc gccgccagca agcaacccat agtctaccca tggatgaaaa 1260
aaattcacgt tagcacggtg aaccccaatt ataacggagg ggaacccaag cgctcgagga 1320
cagcctatac ccggcagcaa gtcctggaat tagagaaaga gtttcattac aaccgctacc 1380
tgacccgaag gagaaggatc gagatcgccc actcgctgtg cctctctgag aggcagatca 1440
aaatctggtt ccaaaaccgt cgcatgaaat ggaagaagga ccaccgactc cccaacacca 1500
aagtcaggtc agcacccccg gccggcgctg cgcccagcac cctttcggca gctaccccgg 1560
gtacttctga agaccactcc cagagcgcca cgccgccgga gcagcaacgg gcagaggaca 1620
ttaccaggtt ataaaacata actcacaccc ctgcccccac cccatgcccc caccctcccc 1680
tcacacacaa attgactctt atttatagaa tttaatatat atatatatat atatatatat 1740
aggttctttt ctctcttcct ctcaccttgt cccttgtcag ttccaaacag acaaaacaga 1800
taaacaaaca agccccctgc cctcctctcc ctcccactgt taaggaccct tttaagcatg 1860
tgatgttgtc ttagcatggt acctgctggg tgtttttttt taaaaggcca ttttgggggg 1920
ttatttattt tttaagaaaa aaagctgcaa aaattatata ttgcaaggtg tgatggtctg 1980
gcttgggtga atttcagggg aaatgaggaa aagaaaaaag gaaagaaatt ttaaagccaa 2040
ttctcatcct tctcctcctc ctccttcccc ccctctttcc ttaggccttt tgcattgaaa 2100
atgcaccagg ggaggttagt gagggggaag tcattttaag gagaacaaag ctatgaagtt 2160
cttttgtatt attgttgggg gggggtgtgg gaggagaggg ggcgaagaca gcagacaaag 2220
ctaaatgcat ctggagagcc tctcagagct gttcagtttg aggagccaaa agaaaatcaa 2280
aatgaacttt cagttcagag aggcagtcta taggtagaat ctctccccac ccctatcgtg 2340
gttattgtgt ttttggactg aatttacttg attattgtaa aacttgcaat aaagaatttt 2400
agtgtcgatg tgaaatgccc cgtgatcaat aataaaccag tggatgtgaa ttagtttta 2459

<210> 47
<211> 2120
<212> PRT
<213> Homo sapiens

<400> 47

```
Met Arg Ser Phe Lys Arg Val Asn Phe Gly Thr Leu Leu Ser Ser Gln
1               5                   10                  15

Lys Glu Ala Glu Glu Leu Leu Pro Ala Leu Lys Glu Phe Leu Ser Asn
            20                  25                  30

Pro Pro Ala Gly Phe Pro Ser Ser Arg Ser Asp Ala Glu Arg Arg Gln
        35                  40                  45
```

```
Ala Cys Asp Ala Ile Leu Arg Ala Cys Asn Gln Gln Leu Thr Ala Lys
    50                  55                  60

Leu Ala Cys Pro Arg His Leu Gly Ser Leu Leu Glu Leu Ala Glu Leu
65                  70                  75                  80

Ala Cys Asp Gly Tyr Leu Val Ser Thr Pro Gln Arg Pro Pro Leu Tyr
                85                  90                  95

Leu Glu Arg Ile Leu Phe Val Leu Leu Arg Asn Ala Ala Ala Gln Gly
            100                 105                 110

Ser Pro Glu Ala Thr Leu Arg Leu Ala Gln Pro Leu His Ala Cys Leu
        115                 120                 125

Val Gln Cys Ser Arg Glu Ala Ala Pro Gln Asp Tyr Glu Ala Val Ala
    130                 135                 140

Arg Gly Ser Phe Ser Leu Leu Trp Lys Gly Ala Glu Ala Leu Leu Glu
145                 150                 155                 160

Arg Arg Ala Ala Phe Ala Ala Arg Leu Lys Ala Leu Ser Phe Leu Val
                165                 170                 175

Leu Leu Glu Asp Glu Ser Thr Pro Cys Glu Val Pro His Phe Ala Ser
            180                 185                 190

Pro Thr Ala Cys Arg Ala Val Ala Ala His Gln Leu Phe Asp Ala Ser
        195                 200                 205

Gly His Gly Leu Asn Glu Ala Asp Ala Asp Phe Leu Asp Asp Leu Leu
    210                 215                 220

Ser Arg His Val Ile Arg Ala Leu Val Gly Glu Arg Gly Ser Ser Ser
225                 230                 235                 240

Gly Leu Leu Ser Pro Gln Arg Ala Leu Cys Leu Leu Glu Leu Thr Leu
                245                 250                 255

Glu His Cys Arg Arg Phe Cys Trp Ser Arg His His Asp Lys Ala Ile
            260                 265                 270

Ser Ala Val Glu Lys Ala His Ser Tyr Leu Arg Asn Thr Asn Leu Ala
        275                 280                 285

Pro Ser Leu Gln Leu Cys Gln Leu Gly Val Lys Leu Leu Gln Val Gly
```

290 295 300

Glu Glu Gly Pro Gln Ala Val Ala Lys Leu Leu Ile Lys Ala Ser Ala
305 310 315 320

Val Leu Ser Lys Ser Met Glu Ala Pro Ser Pro Pro Leu Arg Ala Leu
325 330 335

Tyr Glu Ser Cys Gln Phe Phe Leu Ser Gly Leu Glu Arg Gly Thr Lys
340 345 350

Arg Arg Tyr Arg Leu Asp Ala Ile Leu Ser Leu Phe Ala Phe Leu Gly
355 360 365

Gly Tyr Cys Ser Leu Leu Gln Gln Leu Arg Asp Asp Gly Val Tyr Gly
370 375 380

Gly Ser Ser Lys Gln Gln Gln Ser Phe Leu Gln Met Tyr Phe Gln Gly
385 390 395 400

Leu His Leu Tyr Thr Val Val Val Tyr Asp Phe Ala Gln Gly Cys Gln
405 410 415

Ile Val Asp Leu Ala Asp Leu Thr Gln Leu Val Asp Ser Cys Lys Ser
420 425 430

Thr Val Val Trp Met Leu Glu Ala Leu Glu Gly Leu Ser Gly Gln Glu
435 440 445

Leu Thr Asp His Met Gly Met Thr Ala Ser Tyr Thr Ser Asn Leu Ala
450 455 460

Tyr Ser Phe Tyr Ser His Lys Leu Tyr Ala Glu Ala Cys Ala Ile Ser
465 470 475 480

Glu Pro Leu Cys Gln His Leu Gly Leu Val Lys Pro Gly Thr Tyr Pro
485 490 495

Glu Val Pro Pro Glu Lys Leu His Arg Cys Phe Arg Leu Gln Val Glu
500 505 510

Ser Leu Lys Lys Leu Gly Lys Gln Ala Gln Gly Cys Lys Met Val Ile
515 520 525

Leu Trp Leu Ala Ala Leu Gln Pro Cys Ser Pro Glu His Met Ala Glu
530 535 540

```
Pro Val Thr Phe Trp Val Arg Val Lys Met Asp Ala Ala Arg Ala Gly
545                 550                 555                 560

Asp Lys Glu Leu Gln Leu Lys Thr Leu Arg Asp Ser Leu Ser Gly Trp
                565                 570                 575

Asp Pro Glu Thr Leu Ala Leu Leu Leu Arg Glu Glu Leu Gln Ala Tyr
            580                 585                 590

Lys Ala Val Arg Ala Asp Thr Gly Gln Glu Arg Phe Asn Ile Ile Cys
        595                 600                 605

Asp Leu Leu Glu Leu Ser Pro Glu Glu Thr Pro Ala Gly Ala Trp Ala
    610                 615                 620

Arg Ala Thr His Leu Val Glu Leu Ala Gln Val Leu Cys Tyr His Asp
625                 630                 635                 640

Phe Thr Gln Gln Thr Asn Cys Ser Ala Leu Asp Ala Ile Arg Glu Ala
                645                 650                 655

Leu Gln Leu Leu Asp Ser Val Arg Pro Glu Ala Gln Ala Arg Asp Gln
            660                 665                 670

Leu Leu Asp Asp Lys Ala Gln Ala Leu Leu Trp Leu Tyr Ile Cys Thr
        675                 680                 685

Leu Glu Ala Lys Met Gln Glu Gly Ile Glu Arg Asp Arg Arg Ala Gln
    690                 695                 700

Ala Pro Gly Asn Leu Glu Glu Phe Glu Val Asn Asp Leu Asn Tyr Glu
705                 710                 715                 720

Asp Lys Leu Gln Glu Asp Arg Phe Leu Tyr Ser Asn Ile Ala Phe Asn
                725                 730                 735

Leu Ala Ala Asp Ala Ala Gln Ser Lys Cys Leu Asp Gln Ala Leu Ala
            740                 745                 750

Leu Trp Lys Glu Leu Leu Thr Lys Gly Gln Ala Pro Ala Val Arg Cys
        755                 760                 765

Leu Gln Gln Thr Ala Ala Ser Leu Gln Ile Leu Ala Ala Leu Tyr Gln
    770                 775                 780

Leu Val Ala Lys Pro Met Gln Ala Leu Glu Val Leu Leu Leu Leu Arg
785                 790                 795                 800
```

```
Ile Val Ser Glu Arg Leu Lys Asp His Ser Lys Ala Ala Gly Ser Ser
                805                 810                 815

Cys His Ile Thr Gln Leu Leu Leu Thr Leu Gly Cys Pro Ser Tyr Ala
            820                 825                 830

Gln Leu His Leu Glu Glu Ala Ala Ser Ser Leu Lys His Leu Asp Gln
        835                 840                 845

Thr Thr Asp Thr Tyr Leu Leu Leu Ser Leu Thr Cys Asp Leu Leu Arg
    850                 855                 860

Ser Gln Leu Tyr Trp Thr His Gln Lys Val Thr Lys Gly Val Ser Leu
865                 870                 875                 880

Leu Leu Ser Val Leu Arg Asp Pro Ala Leu Gln Lys Ser Ser Lys Ala
                885                 890                 895

Trp Tyr Leu Leu Arg Val Gln Val Leu Gln Leu Val Ala Ala Tyr Leu
            900                 905                 910

Ser Leu Pro Ser Asn Asn Leu Ser His Ser Leu Trp Glu Gln Leu Cys
        915                 920                 925

Ala Gln Gly Trp Gln Thr Pro Glu Ile Ala Leu Ile Asp Ser His Lys
    930                 935                 940

Leu Leu Arg Ser Ile Ile Leu Leu Leu Met Gly Ser Asp Ile Leu Ser
945                 950                 955                 960

Thr Gln Lys Ala Ala Val Glu Thr Ser Phe Leu Asp Tyr Gly Glu Asn
                965                 970                 975

Leu Val Gln Lys Trp Gln Val Leu Ser Glu Val Leu Ser Cys Ser Glu
            980                 985                 990

Lys Leu Val Cys His Leu Gly Arg  Leu Gly Ser Val Ser  Glu Ala Lys
        995                 1000                1005

Ala Phe  Cys Leu Glu Ala Leu  Lys Leu Thr Thr Lys  Leu Gln Ile
    1010                1015                1020

Pro Arg  Gln Cys Ala Leu Phe  Leu Val Leu Lys Gly  Glu Leu Glu
    1025                1030                1035

Leu Ala  Arg Asn Asp Ile Asp  Leu Cys Gln Ser Asp  Leu Gln Gln
    1040                1045                1050
```

Val Leu Phe Leu Leu Glu Ser Cys Thr Glu Phe Gly Gly Val Thr
1055 1060 1065

Gln His Leu Asp Ser Val Lys Lys Val His Leu Gln Lys Gly Lys
1070 1075 1080

Gln Gln Ala Gln Val Pro Cys Pro Pro Gln Leu Pro Glu Glu Glu
1085 1090 1095

Leu Phe Leu Arg Gly Pro Ala Leu Glu Leu Val Ala Thr Val Ala
1100 1105 1110

Lys Glu Pro Gly Pro Ile Ala Pro Ser Thr Asn Ser Ser Pro Val
1115 1120 1125

Leu Lys Thr Lys Pro Gln Pro Ile Pro Asn Phe Leu Ser His Ser
1130 1135 1140

Pro Thr Cys Asp Cys Ser Leu Cys Ala Ser Pro Val Leu Thr Ala
1145 1150 1155

Val Cys Leu Arg Trp Val Leu Val Thr Ala Gly Val Arg Leu Ala
1160 1165 1170

Met Gly His Gln Ala Gln Gly Leu Asp Leu Leu Gln Val Val Leu
1175 1180 1185

Lys Gly Cys Pro Glu Ala Ala Glu Arg Leu Thr Gln Ala Leu Gln
1190 1195 1200

Ala Ser Leu Asn His Lys Thr Pro Pro Ser Leu Val Pro Ser Leu
1205 1210 1215

Leu Asp Glu Ile Leu Ala Gln Ala Tyr Thr Leu Leu Ala Leu Glu
1220 1225 1230

Gly Leu Asn Gln Pro Ser Asn Glu Ser Leu Gln Lys Val Leu Gln
1235 1240 1245

Ser Gly Leu Lys Phe Val Ala Ala Arg Ile Pro His Leu Glu Pro
1250 1255 1260

Trp Arg Ala Ser Leu Leu Leu Ile Trp Ala Leu Thr Lys Leu Gly
1265 1270 1275

Gly Leu Ser Cys Cys Thr Thr Gln Leu Phe Ala Ser Ser Trp Gly

1280 1285 1290

Trp Gln Pro Pro Leu Ile Lys Ser Val Pro Gly Ser Glu Pro Ser
1295 1300 1305

Lys Thr Gln Gly Gln Lys Arg Ser Gly Arg Gly Arg Gln Lys Leu
1310 1315 1320

Ala Ser Ala Pro Leu Arg Leu Asn Asn Thr Ser Gln Lys Gly Leu
1325 1330 1335

Glu Gly Arg Gly Leu Pro Cys Thr Pro Lys Pro Pro Asp Arg Ile
1340 1345 1350

Arg Gln Ala Gly Pro His Val Pro Phe Thr Val Phe Glu Glu Val
1355 1360 1365

Cys Pro Thr Glu Ser Lys Pro Glu Val Pro Gln Ala Pro Arg Val
1370 1375 1380

Gln Gln Arg Val Gln Thr Arg Leu Lys Val Asn Phe Ser Asp Asp
1385 1390 1395

Ser Asp Leu Glu Asp Pro Val Ser Ala Glu Ala Trp Leu Ala Glu
1400 1405 1410

Glu Pro Lys Arg Arg Gly Thr Ala Ser Arg Gly Arg Gly Arg Ala
1415 1420 1425

Arg Lys Gly Leu Ser Leu Lys Thr Asp Ala Val Val Ala Pro Gly
1430 1435 1440

Ser Ala Pro Gly Asn Pro Gly Leu Asn Gly Arg Ser Arg Arg Ala
1445 1450 1455

Lys Lys Val Ala Ser Arg His Cys Glu Glu Arg Arg Pro Gln Arg
1460 1465 1470

Ala Ser Asp Gln Ala Arg Pro Gly Pro Glu Ile Met Arg Thr Ile
1475 1480 1485

Pro Glu Glu Glu Leu Thr Asp Asn Trp Arg Lys Met Ser Phe Glu
1490 1495 1500

Ile Leu Arg Gly Ser Asp Gly Glu Asp Ser Ala Ser Gly Gly Lys
1505 1510 1515

```
Thr Pro  Ala Pro Gly Pro Glu  Ala Ala Ser Gly Glu  Trp Glu Leu
    1520                 1525                 1530

Leu Arg  Leu Asp Ser Ser Lys  Lys Lys Leu Pro Ser  Pro Cys Pro
    1535                 1540                 1545

Asp Lys  Glu Ser Asp Lys Asp  Leu Gly Pro Arg Leu  Arg Leu Pro
    1550                 1555                 1560

Ser Ala  Pro Val Ala Thr Gly  Leu Ser Thr Leu Asp  Ser Ile Cys
    1565                 1570                 1575

Asp Ser  Leu Ser Val Ala Phe  Arg Gly Ile Ser His  Cys Pro Pro
    1580                 1585                 1590

Ser Gly  Leu Tyr Ala His Leu  Cys Arg Phe Leu Ala  Leu Cys Leu
    1595                 1600                 1605

Gly His  Arg Asp Pro Tyr Ala  Thr Ala Phe Leu Val  Thr Glu Ser
    1610                 1615                 1620

Val Ser  Ile Thr Cys Arg His  Gln Leu Leu Thr His  Leu His Arg
    1625                 1630                 1635

Gln Leu  Ser Lys Ala Gln Lys  His Arg Gly Ser Leu  Glu Ile Ala
    1640                 1645                 1650

Asp Gln  Leu Gln Gly Leu Ser  Leu Gln Glu Met Pro  Gly Asp Val
    1655                 1660                 1665

Pro Leu  Ala Arg Ile Gln Arg  Leu Phe Ser Phe Arg  Ala Leu Glu
    1670                 1675                 1680

Ser Gly  His Phe Pro Gln Pro  Glu Lys Glu Ser Phe  Gln Glu Arg
    1685                 1690                 1695

Leu Ala  Leu Ile Pro Ser Gly  Val Thr Val Cys Val  Leu Ala Leu
    1700                 1705                 1710

Ala Thr  Leu Gln Pro Gly Thr  Val Gly Asn Thr Leu  Leu Leu Thr
    1715                 1720                 1725

Arg Leu  Glu Lys Asp Ser Pro  Pro Val Ser Val Gln  Ile Pro Thr
    1730                 1735                 1740

Gly Gln  Asn Lys Leu His Leu  Arg Ser Val Leu Asn  Glu Phe Asp
    1745                 1750                 1755
```

Ala Ile Gln Lys Ala Gln Lys Glu Asn Ser Ser Cys Thr Asp Lys
1760 1765 1770

Arg Glu Trp Trp Thr Gly Arg Leu Ala Leu Asp His Arg Met Glu
1775 1780 1785

Val Leu Ile Ala Ser Leu Glu Lys Ser Val Leu Gly Cys Trp Lys
1790 1795 1800

Gly Leu Leu Leu Pro Ser Ser Glu Glu Pro Gly Pro Ala Gln Glu
1805 1810 1815

Ala Ser Arg Leu Gln Glu Leu Leu Gln Asp Cys Gly Trp Lys Tyr
1820 1825 1830

Pro Asp Arg Thr Leu Leu Lys Ile Met Leu Ser Gly Ala Gly Ala
1835 1840 1845

Leu Thr Pro Gln Asp Ile Gln Ala Leu Ala Tyr Gly Leu Cys Pro
1850 1855 1860

Thr Gln Pro Glu Arg Ala Gln Glu Leu Leu Asn Glu Ala Val Gly
1865 1870 1875

Arg Leu Gln Gly Leu Thr Val Pro Ser Asn Ser His Leu Val Leu
1880 1885 1890

Val Leu Asp Lys Asp Leu Gln Lys Leu Pro Trp Glu Ser Met Pro
1895 1900 1905

Ser Leu Gln Ala Leu Pro Val Thr Arg Leu Pro Ser Phe Arg Phe
1910 1915 1920

Leu Leu Ser Tyr Ser Ile Ile Lys Glu Tyr Gly Ala Ser Pro Val
1925 1930 1935

Leu Ser Gln Gly Val Asp Pro Arg Ser Thr Phe Tyr Val Leu Asn
1940 1945 1950

Pro His Asn Asn Leu Ser Ser Thr Glu Glu Gln Phe Arg Ala Asn
1955 1960 1965

Phe Ser Ser Glu Ala Gly Trp Arg Gly Val Val Gly Glu Val Pro
1970 1975 1980

Arg Pro Glu Gln Val Gln Glu Ala Leu Thr Lys His Asp Leu Tyr
1985 1990 1995

Ile Tyr Ala Gly His Gly Ala Gly Ala Arg Phe Leu Asp Gly Gln
2000 2005 2010

Ala Val Leu Arg Leu Ser Cys Arg Ala Val Ala Leu Leu Phe Gly
2015 2020 2025

Cys Ser Ser Ala Ala Leu Ala Val Arg Gly Asn Leu Glu Gly Ala
2030 2035 2040

Gly Ile Val Leu Lys Tyr Ile Met Ala Gly Cys Pro Leu Phe Leu
2045 2050 2055

Gly Asn Leu Trp Asp Val Thr Asp Arg Asp Ile Asp Arg Tyr Thr
2060 2065 2070

Glu Ala Leu Leu Gln Gly Trp Leu Gly Ala Gly Pro Gly Ala Pro
2075 2080 2085

Leu Leu Tyr Tyr Val Asn Gln Ala Arg Gln Ala Pro Arg Leu Lys
2090 2095 2100

Tyr Leu Ile Gly Ala Ala Pro Ile Ala Tyr Gly Leu Pro Val Ser
2105 2110 2115

Leu Arg
2120

<210> 48
<211> 6641
<212> DNA
<213> Homo sapiens

<400> 48

ggttacattt tggatcctcg cggagtactg gtcaggcggt taagtcctgt acctaggaaa 60

gagggcgagc tctggggcgc tctccggtgt catgaggagc ttcaaaagag tcaactttgg 120

gactctgcta agcagccaga aggaggctga agagttgctg cccgccttga aggagttcct 180

gtccaaccct ccagctggtt ttcccagcag ccgatctgat gctgagagga gacaagcttg 240

tgatgccatc ctgagggctt gcaaccagca gctgactgct aagctagctt gccctaggca 300

tctggggagc ctgctggagc tggcagagct ggcctgtgat ggctacttag tgtctacccc 360

acagcgtcct cccctctacc tggaacgaat tctctttgtc ttactgcgga atgctgctgc 420

acaaggaagc ccagaggcca cactccgcct tgctcagccc ctccatgcct gcttggtgca 480

gtgctctcgc gaggctgctc cccaggacta tgaggccgtg gctcggggca gcttttctct 540

gctttggaag gggcagaag ccctgttgga acggcgagct gcatttgcag ctcggctgaa 600

ggccttgagc ttcctagtac tcttggagga tgaaagtacc ccttgtgagg ttcctcactt 660

tgcttctcca acagcctgtc gagcggtagc tgcccatcag ctatttgatg ccagtggcca 720

tggtctaaat gaagcagatg ctgatttcct agatgacctg ctctccaggc acgtgatcag 780

agccttggtg ggtgagagag ggagctcttc tgggcttctt tctccccaga gggccctctg 840

cctcttggag ctcaccttgg aacactgccg tcgcttttgc tggagccgcc accatgacaa 900

agccatcagc gcagtggaga aggctcacag ttacctaagg aacaccaatc tagcccctag 960

ccttcagcta tgtcagctgg gggttaagct gctgcaggtt ggggaggaag gacctcaggc 1020

agtggccaag cttctgatca aggcatcagc tgtcctgagc aagagtatgg aggcaccatc 1080

acccccactt cgggcattgt atgagagctg ccagttcttc ctttcaggcc tggaacgagg 1140

caccaagagg cgctatagac ttgatgccat tctgagcctc tttgcttttc ttggagggta 1200

ctgctctctt ctgcagcagc tgcgggatga tggtgtgtat gggggctcct ccaagcaaca 1260

gcagtctttt cttcagatgt actttcaggg acttcacctc tacactgtgg tggtttatga 1320

ctttgcccaa ggctgtcaga tagttgattt ggctgacctg acccaactag tggacagttg 1380

taaatctacc gttgtctgga tgctggaggc cttagagggc ctgtcgggcc aagagctgac 1440

ggaccacatg ggatgaccg cttcttacac cagtaatttg gcctacagct tctatagtca 1500

caagctctat gccgaggcct gtgccatctc tgagccgctc tgtcagcacc tgggtttggt 1560

gaagccaggc acttatcccg aggtgcctcc tgagaagttg cacaggtgct tccggctaca 1620

agtagagagt ttgaagaaac tgggtaaaca ggcccagggc tgcaagatgg tgattttgtg 1680

gctggcagcc ctgcaaccct gtagccctga acacatggct gagccagtca ctttctgggt 1740

tcgggtcaag atggatgcgg ccagggctgg agacaaggag ctacagctaa agactctgcg 1800

agacagcctc agtggctggg acccggagac cctggccctc ctgctgaggg aggagctgca 1860

ggcctacaag gcggtgcggg ccgacactgg acaggaacgc ttcaacatca tctgtgacct 1920

cctggagctg agccccgagg agacaccagc cggggcctgg gcacgagcca cccacctggt 1980

agaactggct caggtgctct gctaccacga ctttacgcag cagaccaact gctctgctct 2040

ggatgctatc cgggaagccc tgcagcttct ggactctgtg aggcctgagg cccaggccag 2100

agatcagctt ctggacgata aagcacaggc cttgctgtgg ctttacatct gtactctgga 2160

agccaaaatg caggaaggta tcgagcggga tcggagagcc caggcccctg gtaacttgga 2220

ggaatttgaa gtcaatgacc tgaactatga agataaactc caggaagatc gtttcctata 2280

cagtaacatt gccttcaacc tggctgcaga tgctgctcag tccaaatgcc tggaccaagc 2340

cctggccctg tggaaggagc tgcttacaaa ggggcaggcc ccagctgtac ggtgtctcca 2400

gcagacagca gcctcactgc agatcctagc agccctctac cagctggtgg caaagcccat 2460

gcaggctctg gaggtcctcc tgctgctacg gattgtctct gagagactga aggaccactc 2520
gaaggcagct ggctcctcct gccacatcac ccagctcctc ctgaccctcg gctgtcccag 2580
ctatgcccag ttacacctgg aagaggcagc atcgagcctg aagcatctcg atcagactac 2640
tgacacatac ctgctccttt ccctgacctg tgatctgctt cgaagtcaac tctactggac 2700
tcaccagaag gtgaccaagg gtgtctctct gctgctgtct gtgcttcggg atcctgccct 2760
ccagaagtcc tccaaggctt ggtacttgct gcgtgtccag gtcctgcagc tggtggcagc 2820
ttaccttagc ctcccgtcaa acaacctctc acactccctg tgggagcagc tctgtgccca 2880
aggctggcag acacctgaga tagctctcat agactcccat aagctcctcc gaagcatcat 2940
cctcctgctg atgggcagtg acattctctc aactcagaaa gcagctgtgg agacatcgtt 3000
tttggactat ggtgaaaatc tggtacaaaa atggcaggtt ctttcagagg tgctgagctg 3060
ctcagagaag ctggtctgcc acctgggccg cctgggtagt gtgagtgaag ccaaggcctt 3120
ttgcttggag gccctaaaac ttacaacaaa gctgcagata ccacgccagt gtgccctgtt 3180
cctggtgctg aagggcgagc tggagctggc ccgcaatgac attgatctct gtcagtcgga 3240
cctgcagcag gttctgttct tgcttgagtc ttgcacagag tttggtgggg tgactcagca 3300
cctggactct gtgaagaagg tccacctgca gaaggggaag cagcaggccc aggtcccctg 3360
tcctccacag ctcccagagg aggagctctt cctaagaggc cctgctctag agctggtggc 3420
cactgtggcc aaggagcctg gccccatagc accttctaca aactcctccc cagtcttgaa 3480
aaccaagccc cagcccatac ccaacttcct gtcccattca cccacctgtg actgctcgct 3540
ctgcgccagc cctgtcctca cagcagtctg tctgcgctgg gtattggtca cggcaggggt 3600
gaggctggcc atgggccacc aagcccaggg tctggatctg ctgcaggtcg tgctgaaggg 3660
ctgtcctgaa gccgctgagc gcctcaccca agctctccaa gcttccctga atcataaaac 3720
accccccctcc ttggttccaa gcctcttgga tgagatcttg gctcaagcat acacactgtt 3780
ggcactggag ggcctgaacc agccatcaaa cgagagcctg cagaaggttc tacagtcagg 3840
gctgaagttt gtagcagcac ggatacccca cctagagccc tggcgagcca gcctgctctt 3900
gatttgggcc ctcacaaaac taggtggcct cagctgctgt actacccaac tttttgcaag 3960
ctcctggggc tggcagccac cattaataaa aagtgtccct ggctcagagc cctctaagac 4020
tcagggccaa aaacgttctg gacgagggcg ccaaaagtta gcctctgctc ccctgcgcct 4080
caataatacc tctcagaaag gtctggaagg tagaggactg ccctgcacac ctaaaccccc 4140
agaccggatc aggcaagctg gccctcatgt ccccttcacg gtgtttgagg aagtctgccc 4200
tacagagagc aagcctgaag taccccaggc cccccagggta caacagagag tccagacgcg 4260
cctcaaggtg aacttcagtg atgacagtga cttggaagac cctgtctcag ctgaggcctg 4320
gctggcagag gagcctaaga gacggggcac tgcttcccgg ggccgggggc gagcaaggaa 4380

gggcctgagc ctaaagacgg atgccgtggt tgccccaggt agtgcccctg ggaaccctgg 4440
cctgaatggc aggagccgga gggccaagaa ggtggcatca agacattgtg aggagcggcg 4500
tccccagagg gccagtgacc aggccaggcc tggccctgag atcatgagga ccatccctga 4560
ggaagaactg actgacaact ggagaaaaat gagctttgag atcctcaggg gctctgacgg 4620
ggaagactca gcctcaggtg ggaagactcc agctccgggc cctgaggcag cttctggaga 4680
atgggagctg ctgaggctgg attccagcaa gaagaagctg cccagcccat gcccagacaa 4740
ggagagtgac aaggaccttg gtcctcggct ccggctcccc tcagcccccg tagccactgg 4800
tctttctacc ctggactcca tctgtgactc cctgagtgtt gctttccggg gcattagtca 4860
ctgtcctcct agtgggctct atgcccacct ctgccgcttc ctggccttgt gcctgggcca 4920
ccgggatcct tatgccactg ctttccttgt caccgagtct gtctccatca cctgtcgcca 4980
ccagctgctc acccacctcc acagacagct cagcaaggcc cagaagcacc gaggatcact 5040
tgaaatagca gaccagctgc aggggctgag ccttcaggag atgcctggag atgtccccct 5100
ggcccgcatc cagcgcctct tttccttcag ggctttggaa tctggccact tcccccagcc 5160
tgaaaaggag agtttccagg agcgcctggc tctgatcccc agtggggtga ctgtgtgtgt 5220
gttggccctg gccaccctcc agcccggaac cgtgggcaac accctcctgc tgacccggct 5280
ggaaaaggac agtcccccag tcagtgtgca gattcccact ggccagaaca agcttcatct 5340
gcgttcagtc ctgaatgagt ttgatgccat ccagaaggca cagaaagaga acagcagctg 5400
tactgacaag cgagaatggt ggacagggcg gctggcactg gaccacagga tggaggttct 5460
catcgcttcc ctagagaagt ctgtgctggg ctgctggaag gggctgctgc tgccgtccag 5520
tgaggagccc ggccctgccc aggaggcctc ccgcctacag gagctgctac aggactgtgg 5580
ctggaaatat cctgaccgca ctctgctgaa aatcatgctc agtggtgccg gtgccctcac 5640
ccctcaggac attcaggccc tggcctacgg gctgtgccca acccagccag agcgagccca 5700
ggagctcctg aatgaggcag taggacgtct acagggcctg acagtaccaa gcaatagcca 5760
ccttgtcttg gtcctagaca aggacttgca gaagctgccg tgggaaagca tgcccagcct 5820
ccaagcactg cctgtcaccc ggctgccctc cttccgcttc ctactcagct actccatcat 5880
caaagagtat ggggcctcgc cagtgctgag tcaaggggtg gatccacgaa gtaccttcta 5940
tgtcctgaac cctcacaata acctgtcaag cacagaggag caatttcgag ccaatttcag 6000
cagtgaagct ggctggagag gagtggttgg ggaggtgcca agacctgaac aggtgcagga 6060
agccctgaca aagcatgatt tgtatatcta tgcagggcat ggggctggtg cccgcttcct 6120
tgatgggcag gctgtcctgc ggctgagctg tcgggcagtg gccctgctgt ttggctgtag 6180
cagtgcggcc ctggctgtgc gtggaaacct ggagggggct ggcatcgtgc tcaagtacat 6240

```
catggctggt tgccccttgt ttctgggtaa tctctgggat gtgactgacc gcgacattga      6300
ccgctacacg gaagctctgc tgcaaggctg gcttggagca ggcccagggg ccccccttct      6360
ctactatgta aaccaggccc gccaagctcc ccgactcaag tatcttattg gggctgcacc      6420
tatagcctat ggcttgcctg tctctctgcg gtaaccccat ggagctgtct tattgatgct      6480
agaagcctca taactgttct acctccaagg ttagatttaa tccttaggat aactctttta      6540
aagtgatttt ccccagtgtt ttatatgaaa catttccttt tgatttaacc tcagtataat      6600
aaagatacat catttaaacc ctgaaaaaaa aaaaaaaaaa a                          6641
```

<210> 49
<211> 707
<212> PRT
<213> Homo sapiens

<400> 49

```
Met Ser Leu Trp Gln Pro Leu Val Leu Val Leu Leu Val Leu Gly Cys
1               5                   10                  15

Cys Phe Ala Ala Pro Arg Gln Arg Gln Ser Thr Leu Val Leu Phe Pro
            20                  25                  30

Gly Asp Leu Arg Thr Asn Leu Thr Asp Arg Gln Leu Ala Glu Glu Tyr
        35                  40                  45

Leu Tyr Arg Tyr Gly Tyr Thr Arg Val Ala Glu Met Arg Gly Glu Ser
    50                  55                  60

Lys Ser Leu Gly Pro Ala Leu Leu Leu Leu Gln Lys Gln Leu Ser Leu
65                  70                  75                  80

Pro Glu Thr Gly Glu Leu Asp Ser Ala Thr Leu Lys Ala Met Arg Thr
                85                  90                  95

Pro Arg Cys Gly Val Pro Asp Leu Gly Arg Phe Gln Thr Phe Glu Gly
            100                 105                 110

Asp Leu Lys Trp His His His Asn Ile Thr Tyr Trp Ile Gln Asn Tyr
        115                 120                 125

Ser Glu Asp Leu Pro Arg Ala Val Ile Asp Asp Ala Phe Ala Arg Ala
    130                 135                 140

Phe Ala Leu Trp Ser Ala Val Thr Pro Leu Thr Phe Thr Arg Val Tyr
145                 150                 155                 160

Ser Arg Asp Ala Asp Ile Val Ile Gln Phe Gly Val Ala Glu His Gly
                165                 170                 175
```

Asp Gly Tyr Pro Phe Asp Gly Lys Asp Gly Leu Leu Ala His Ala Phe
180 185 190

Pro Pro Gly Pro Gly Ile Gln Gly Asp Ala His Phe Asp Asp Asp Glu
195 200 205

Leu Trp Ser Leu Gly Lys Gly Val Val Val Pro Thr Arg Phe Gly Asn
210 215 220

Ala Asp Gly Ala Ala Cys His Phe Pro Phe Ile Phe Glu Gly Arg Ser
225 230 235 240

Tyr Ser Ala Cys Thr Thr Asp Gly Arg Ser Asp Gly Leu Pro Trp Cys
245 250 255

Ser Thr Thr Ala Asn Tyr Asp Thr Asp Asp Arg Phe Gly Phe Cys Pro
260 265 270

Ser Glu Arg Leu Tyr Thr Gln Asp Gly Asn Ala Asp Gly Lys Pro Cys
275 280 285

Gln Phe Pro Phe Ile Phe Gln Gly Gln Ser Tyr Ser Ala Cys Thr Thr
290 295 300

Asp Gly Arg Ser Asp Gly Tyr Arg Trp Cys Ala Thr Thr Ala Asn Tyr
305 310 315 320

Asp Arg Asp Lys Leu Phe Gly Phe Cys Pro Thr Arg Ala Asp Ser Thr
325 330 335

Val Met Gly Gly Asn Ser Ala Gly Glu Leu Cys Val Phe Pro Phe Thr
340 345 350

Phe Leu Gly Lys Glu Tyr Ser Thr Cys Thr Ser Glu Gly Arg Gly Asp
355 360 365

Gly Arg Leu Trp Cys Ala Thr Thr Ser Asn Phe Asp Ser Asp Lys Lys
370 375 380

Trp Gly Phe Cys Pro Asp Gln Gly Tyr Ser Leu Phe Leu Val Ala Ala
385 390 395 400

His Glu Phe Gly His Ala Leu Gly Leu Asp His Ser Ser Val Pro Glu
405 410 415

Ala Leu Met Tyr Pro Met Tyr Arg Phe Thr Glu Gly Pro Pro Leu His

420 425 430

Lys Asp Asp Val Asn Gly Ile Arg His Leu Tyr Gly Pro Arg Pro Glu
435 440 445

Pro Glu Pro Arg Pro Pro Thr Thr Thr Thr Pro Gln Pro Thr Ala Pro
450 455 460

Pro Thr Val Cys Pro Thr Gly Pro Pro Thr Val His Pro Ser Glu Arg
465 470 475 480

Pro Thr Ala Gly Pro Thr Gly Pro Pro Ser Ala Gly Pro Thr Gly Pro
485 490 495

Pro Thr Ala Gly Pro Ser Thr Ala Thr Thr Val Pro Leu Ser Pro Val
500 505 510

Asp Asp Ala Cys Asn Val Asn Ile Phe Asp Ala Ile Ala Glu Ile Gly
515 520 525

Asn Gln Leu Tyr Leu Phe Lys Asp Gly Lys Tyr Trp Arg Phe Ser Glu
530 535 540

Gly Arg Gly Ser Arg Pro Gln Gly Pro Phe Leu Ile Ala Asp Lys Trp
545 550 555 560

Pro Ala Leu Pro Arg Lys Leu Asp Ser Val Phe Glu Glu Arg Leu Ser
565 570 575

Lys Lys Leu Phe Phe Phe Ser Gly Arg Gln Val Trp Val Tyr Thr Gly
580 585 590

Ala Ser Val Leu Gly Pro Arg Arg Leu Asp Lys Leu Gly Leu Gly Ala
595 600 605

Asp Val Ala Gln Val Thr Gly Ala Leu Arg Ser Gly Arg Gly Lys Met
610 615 620

Leu Leu Phe Ser Gly Arg Arg Leu Trp Arg Phe Asp Val Lys Ala Gln
625 630 635 640

Met Val Asp Pro Arg Ser Ala Ser Glu Val Asp Arg Met Phe Pro Gly
645 650 655

Val Pro Leu Asp Thr His Asp Val Phe Gln Tyr Arg Glu Lys Ala Tyr
660 665 670

```
Phe Cys Gln Asp Arg Phe Tyr Trp Arg Val Ser Ser Arg Ser Glu Leu
        675                 680                 685

Asn Gln Val Asp Gln Val Gly Tyr Val Thr Tyr Asp Ile Leu Gln Cys
    690                 695                 700

Pro Glu Asp
705
```

<210> 50
<211> 2387
<212> DNA
<213> Homo sapiens

<400> 50

```
agacacctct gccctcacca tgagcctctg gcagcccctg gtcctggtgc tcctggtgct      60
gggctgctgc tttgctgccc ccagacagcg ccagtccacc cttgtgctct tccctggaga     120
cctgagaacc aatctcaccg acaggcagct ggcagaggaa tacctgtacc gctatggtta     180
cactcgggtg gcagagatgc gtggagagtc gaaatctctg gggcctgcgc tgctgcttct     240
ccagaagcaa ctgtccctgc ccgagaccgg tgagctggat agcgccacgc tgaaggccat     300
gcgaacccca cggtgcgggg tcccagacct gggcagattc caaacctttg agggcgacct     360
caagtggcac caccacaaca tcacctattg gatccaaaac tactcggaag acttgccgcg     420
ggcggtgatt gacgacgcct ttgcccgcgc cttcgcactg tggagcgcgg tgacgccgct     480
caccttcact cgcgtgtaca gccgggacgc agacatcgtc atccagtttg gtgtcgcgga     540
gcacggagac gggtatccct tcgacgggaa ggacgggctc ctggcacacg cctttcctcc     600
tggccccggc attcagggag acgcccattt cgacgatgac gagttgtggt ccctgggcaa     660
gggcgtcgtg gttccaactc ggtttggaaa cgcagatggc gcggcctgcc acttcccctt     720
catcttcgag ggccgctcct actctgcctg caccaccgac ggtcgctccg acggcttgcc     780
ctggtgcagt accacggcca actacgacac cgacgaccgg tttggcttct gccccagcga     840
gagactctac acccaggacg gcaatgctga tgggaaaccc tgccagtttc cattcatctt     900
ccaaggccaa tcctactccg cctgcaccac ggacggtcgc tccgacggct accgctggtg     960
cgccaccacc gccaactacg accgggacaa gctcttcggc ttctgcccga cccgagctga    1020
ctcgacggtg atggggggca actcggcggg ggagctgtgc gtcttcccct tcactttcct    1080
gggtaaggag tactcgacct gtaccagcga gggccgcgga gatgggcgcc tctggtgcgc    1140
taccacctcg aactttgaca gcgacaagaa gtggggcttc tgcccggacc aaggatacag    1200
tttgttcctc gtggcggcgc atgagttcgg ccacgcgctg ggcttagatc attcctcagt    1260
gccggaggcg ctcatgtacc ctatgtaccg cttcactgag gggccccccct tgcataagga    1320
cgacgtgaat ggcatccggc acctctatgg tcctcgccct gaacctgagc cacggcctcc    1380
```

aaccaccacc acaccgcagc ccacggctcc cccgacggtc tgccccaccg gaccccccac 1440

tgtccacccc tcagagcgcc ccacagctgg ccccacaggt ccccccctcag ctggccccac 1500

aggtcccccc actgctggcc cttctacggc cactactgtg cctttgagtc cggtggacga 1560

tgcctgcaac gtgaacatct tcgacgccat cgcggagatt gggaaccagc tgtatttgtt 1620

caaggatggg aagtactggc gattctctga gggcaggggg agccggccgc agggcccctt 1680

ccttatcgcc gacaagtggc ccgcgctgcc ccgcaagctg gactcggtct ttgaggagcg 1740

gctctccaag aagcttttct tcttctctgg gcgccaggtg tgggtgtaca caggcgcgtc 1800

ggtgctgggc ccgaggcgtc tggacaagct gggcctggga gccgacgtgg cccaggtgac 1860

cggggccctc cggagtggca gggggaagat gctgctgttc agcgggcggc gcctctggag 1920

gttcgacgtg aaggcgcaga tggtggatcc ccggagcgcc agcgaggtgg accggatgtt 1980

ccccggggtg cctttggaca cgcacgacgt cttccagtac cgagagaaag cctatttctg 2040

ccaggaccgc ttctactggc gcgtgagttc ccggagtgag ttgaaccagg tggaccaagt 2100

gggctacgtg acctatgaca tcctgcagtg ccctgaggac tagggctccc gtcctgcttt 2160

ggcagtgcca tgtaaatccc cactgggacc aaccctgggg aaggagccag tttgccggat 2220

acaaactggt attctgttct ggaggaaagg gaggagtgga ggtgggctgg gccctctctt 2280

ctcacctttg ttttttgttg gagtgtttct aataaacttg gattctctaa cctttaaaaa 2340

aaaaaaaaaa aaaaaaaaaa aaaaaaaaaa aaaaaaaaaa aaaaaaa 2387

<210> 51
<211> 5058
<212> PRT
<213> Homo sapiens

<220>
<221> MOD_RES
<222> (283)..(283)
<223> Any amino acid

<220>
<221> MOD_RES
<222> (2674)..(2674)
<223> Any amino acid

<400> 51

```
Met Gly His Ala Gly Cys Gln Phe Lys Ala Leu Leu Trp Lys Asn Trp
1               5                   10                  15

Leu Cys Arg Leu Arg Asn Pro Val Leu Phe Leu Ala Glu Phe Phe Trp
            20                  25                  30

Pro Cys Ile Leu Phe Val Ile Leu Thr Val Leu Arg Phe Gln Glu Pro
        35                  40                  45
```

```
Pro Arg Tyr Arg Asp Ile Cys Tyr Leu Gln Pro Arg Asp Leu Pro Ser
    50                  55                  60

Cys Gly Val Ile Pro Phe Val Gln Ser Leu Leu Cys Asn Thr Gly Ser
65                  70                  75                  80

Arg Cys Arg Asn Phe Ser Tyr Glu Gly Ser Met Glu His His Phe Arg
                85                  90                  95

Leu Ser Arg Phe Gln Thr Ala Ala Asp Pro Lys Lys Val Asn Asn Leu
            100                 105                 110

Ala Phe Leu Lys Glu Ile Gln Asp Leu Ala Glu Glu Ile His Gly Met
        115                 120                 125

Met Asp Lys Ala Lys Asn Leu Lys Arg Leu Trp Val Glu Arg Ser Asn
    130                 135                 140

Thr Pro Asp Ser Ser Tyr Gly Ser Ser Phe Phe Thr Met Asp Leu Asn
145                 150                 155                 160

Lys Thr Glu Glu Val Ile Leu Lys Leu Glu Ser Leu His Gln Gln Pro
                165                 170                 175

His Ile Trp Asp Phe Leu Leu Leu Leu Pro Arg Leu His Thr Ser His
            180                 185                 190

Asp His Val Glu Asp Gly Met Asp Val Ala Val Asn Leu Leu Gln Thr
        195                 200                 205

Ile Leu Asn Ser Leu Ile Ser Leu Glu Asp Leu Asp Trp Leu Pro Leu
    210                 215                 220

Asn Gln Thr Phe Ser Gln Val Ser Glu Leu Val Leu Asn Val Thr Ile
225                 230                 235                 240

Ser Thr Leu Thr Phe Leu Gln Gln His Gly Val Ala Val Thr Glu Pro
                245                 250                 255

Val Tyr His Leu Ser Met Gln Asn Ile Val Trp Asp Pro Gln Lys Val
            260                 265                 270

Gln Tyr Asp Leu Lys Ser Gln Phe Gly Phe Xaa Asp Leu His Thr Glu
        275                 280                 285

Gln Ile Leu Asn Ser Ser Ala Glu Leu Lys Glu Ile Pro Thr Asp Thr
```

290 295 300

Ser Leu Glu Lys Met Val Cys Ser Val Leu Ser Ser Thr Ser Glu Asp
305 310 315 320

Glu Ala Glu Lys Trp Gly His Val Gly Gly Cys His Pro Lys Trp Ser
325 330 335

Glu Ala Lys Asn Tyr Leu Val His Ala Val Ser Trp Leu Arg Val Tyr
340 345 350

Gln Gln Val Phe Val Gln Trp Gln Gln Gly Ser Leu Leu Gln Lys Thr
355 360 365

Leu Thr Gly Met Gly His Ser Leu Glu Ala Leu Arg Asn Gln Phe Glu
370 375 380

Glu Glu Ser Lys Pro Trp Lys Val Val Glu Ala Leu His Thr Ala Leu
385 390 395 400

Leu Leu Leu Asn Asp Ser Leu Ser Ala Asp Gly Pro Lys Asp Asn His
405 410 415

Thr Phe Pro Lys Ile Leu Gln His Leu Trp Lys Leu Gln Ser Leu Leu
420 425 430

Gln Asn Leu Pro Gln Trp Pro Ala Leu Lys Arg Phe Leu Gln Leu Asp
435 440 445

Gly Ala Leu Arg Asn Ala Ile Ala Gln Asn Leu His Phe Val Gln Glu
450 455 460

Val Leu Ile Cys Leu Glu Thr Ser Ala Asn Asp Phe Lys Trp Phe Glu
465 470 475 480

Leu Asn Gln Leu Lys Leu Glu Lys Asp Val Phe Phe Trp Glu Leu Lys
485 490 495

Gln Met Leu Ala Lys Asn Ala Val Cys Pro Asn Gly Arg Phe Ser Glu
500 505 510

Lys Glu Val Phe Leu Pro Pro Gly Asn Ser Ser Ile Trp Gly Gly Leu
515 520 525

Gln Gly Leu Leu Cys Tyr Cys Asn Ser Ser Glu Thr Ser Val Leu Asn
530 535 540

```
Lys Leu Leu Gly Ser Val Glu Asp Ala Asp Arg Ile Leu Gln Glu Val
545                 550                 555                 560

Ile Thr Trp His Lys Asn Met Ser Val Leu Ile Pro Glu Glu Tyr Leu
                565                 570                 575

Asp Trp Gln Glu Leu Glu Met Gln Leu Ser Glu Ala Ser Leu Ser Cys
            580                 585                 590

Thr Arg Leu Phe Leu Leu Leu Gly Ala Asp Pro Ser Pro Glu Asn Asp
        595                 600                 605

Val Phe Ser Ser Asp Cys Lys His Gln Leu Val Ser Thr Val Ile Phe
    610                 615                 620

His Thr Leu Glu Lys Thr Gln Phe Phe Leu Glu Gln Ala Tyr Tyr Trp
625                 630                 635                 640

Lys Ala Phe Lys Lys Phe Ile Arg Lys Thr Cys Glu Val Ala Gln Tyr
                645                 650                 655

Val Asn Met Gln Glu Ser Phe Gln Asn Arg Leu Leu Ala Phe Pro Glu
            660                 665                 670

Glu Ser Pro Cys Phe Glu Glu Asn Met Asp Trp Lys Met Ile Ser Asp
        675                 680                 685

Asn Tyr Phe Gln Phe Leu Asn Asn Leu Leu Lys Ser Pro Thr Ala Ser
    690                 695                 700

Ile Ser Arg Ala Leu Asn Phe Thr Lys His Leu Leu Met Met Glu Lys
705                 710                 715                 720

Lys Leu His Thr Leu Glu Asp Glu Gln Met Asn Phe Leu Leu Ser Phe
                725                 730                 735

Val Glu Phe Phe Glu Lys Leu Leu Leu Pro Asn Leu Phe Asp Ser Ser
            740                 745                 750

Ile Val Pro Ser Phe His Ser Leu Pro Ser Leu Thr Glu Asp Ile Leu
        755                 760                 765

Asn Ile Ser Ser Leu Trp Thr Asn His Leu Lys Ser Leu Lys Arg Asp
    770                 775                 780

Pro Ser Ala Thr Asp Ala Gln Lys Leu Leu Glu Phe Gly Asn Glu Val
785                 790                 795                 800
```

```
Ile Trp Lys Met Gln Thr Leu Gly Ser His Trp Ile Arg Lys Glu Pro
                805                 810                 815

Lys Asn Leu Leu Arg Phe Ile Glu Leu Ile Leu Phe Glu Ile Asn Pro
            820                 825                 830

Lys Leu Leu Glu Leu Trp Ala Tyr Gly Ile Ser Lys Gly Lys Arg Ala
        835                 840                 845

Lys Leu Glu Asn Phe Phe Thr Leu Leu Asn Phe Ser Val Pro Glu Asn
    850                 855                 860

Glu Ile Leu Ser Thr Ser Phe Asn Phe Ser Gln Leu Phe His Ser Asp
865                 870                 875                 880

Trp Pro Lys Ser Pro Ala Met Asn Ile Asp Phe Val Arg Leu Ser Glu
                885                 890                 895

Ala Ile Ile Thr Ser Leu His Glu Phe Gly Phe Leu Glu Gln Glu Gln
            900                 905                 910

Ile Ser Glu Ala Leu Asn Thr Val Tyr Ala Ile Arg Asn Ala Ser Asp
        915                 920                 925

Leu Phe Ser Ala Leu Ser Glu Pro Gln Lys Gln Glu Val Asp Lys Ile
    930                 935                 940

Leu Thr His Ile His Leu Asn Val Phe Gln Asp Lys Asp Ser Ala Leu
945                 950                 955                 960

Leu Leu Gln Ile Tyr Ser Ser Phe Tyr Arg Tyr Ile Tyr Glu Leu Leu
                965                 970                 975

Asn Ile Gln Ser Arg Gly Ser Ser Leu Thr Phe Leu Thr Gln Ile Ser
            980                 985                 990

Lys His Ile Leu Asp Ile Ile Lys  Gln Phe Asn Phe Gln  Asn Ile Ser
        995                 1000                1005

Lys Ala  Phe Ala Phe Leu Phe  Lys Thr Ala Glu Val  Leu Gly Gly
    1010                1015                1020

Ile Ser  Asn Val Ser Tyr Cys  Gln Gln Leu Leu Ser  Ile Phe Asn
    1025                1030                1035

Phe Leu  Glu Leu Gln Ala Gln  Ser Phe Met Ser Thr  Glu Gly Gln
    1040                1045                1050
```

Glu Leu Glu Val Ile His Thr Thr Leu Thr Gly Leu Lys Gln Leu
1055 1060 1065

Leu Ile Ile Asp Glu Asp Phe Arg Ile Ser Leu Phe Gln Tyr Met
1070 1075 1080

Ser Gln Phe Phe Asn Ser Ser Val Glu Asp Leu Leu Asp Asn Lys
1085 1090 1095

Cys Leu Ile Ser Asp Asn Lys His Ile Ser Ser Val Asn Tyr Ser
1100 1105 1110

Thr Ser Glu Glu Ser Ser Phe Val Phe Pro Leu Ala Gln Ile Phe
1115 1120 1125

Ser Asn Leu Ser Ala Asn Val Ser Val Phe Asn Lys Phe Met Ser
1130 1135 1140

Ile His Cys Thr Val Ser Trp Leu Gln Met Trp Thr Glu Ile Trp
1145 1150 1155

Glu Thr Ile Ser Gln Leu Phe Lys Phe Asp Met Asn Val Phe Thr
1160 1165 1170

Ser Leu His His Gly Phe Thr Gln Leu Leu Asp Glu Leu Glu Asp
1175 1180 1185

Asp Val Lys Val Ser Lys Ser Cys Gln Gly Ile Leu Pro Thr His
1190 1195 1200

Asn Val Ala Arg Leu Ile Leu Asn Leu Phe Lys Asn Val Thr Gln
1205 1210 1215

Ala Asn Asp Phe His Asn Trp Glu Asp Phe Leu Asp Leu Arg Asp
1220 1225 1230

Phe Leu Val Ala Leu Gly Asn Ala Leu Val Ser Val Lys Lys Leu
1235 1240 1245

Asn Leu Glu Gln Val Glu Lys Ser Leu Phe Thr Met Glu Ala Ala
1250 1255 1260

Leu His Gln Leu Lys Thr Phe Pro Phe Asn Glu Ser Thr Ser Arg
1265 1270 1275

Glu Phe Leu Asn Ser Leu Leu Glu Val Phe Ile Glu Phe Ser Ser

1280 1285 1290

Thr Ser Glu Tyr Ile Val Arg Asn Leu Asp Ser Ile Asn Asp Phe
1295 1300 1305

Leu Ser Asn Asn Leu Thr Asn Tyr Gly Glu Lys Phe Glu Asn Ile
1310 1315 1320

Ile Thr Glu Leu Arg Glu Ala Ile Val Phe Leu Arg Asn Val Ser
1325 1330 1335

His Asp Arg Asp Leu Phe Ser Cys Ala Asp Ile Phe Gln Asn Val
1340 1345 1350

Thr Glu Cys Ile Leu Glu Asp Gly Phe Leu Tyr Val Asn Thr Ser
1355 1360 1365

Gln Arg Met Leu Arg Ile Leu Asp Thr Leu Asn Ser Thr Phe Ser
1370 1375 1380

Ser Glu Asn Thr Ile Ser Ser Leu Lys Gly Cys Ile Val Trp Leu
1385 1390 1395

Asp Val Ile Asn His Leu Tyr Leu Leu Ser Asn Ser Ser Phe Ser
1400 1405 1410

Gln Gly Arg Leu Gln Asn Ile Leu Gly Asn Phe Arg Asp Ile Glu
1415 1420 1425

Asn Lys Met Asn Ser Ile Leu Lys Ile Val Thr Trp Val Leu Asn
1430 1435 1440

Ile Lys Lys Pro Leu Cys Ser Ser Asn Gly Ser His Ile Asn Cys
1445 1450 1455

Val Asn Ile Tyr Leu Lys Asp Val Thr Asp Phe Leu Asn Ile Val
1460 1465 1470

Leu Thr Thr Val Phe Glu Lys Glu Lys Lys Pro Lys Phe Glu Ile
1475 1480 1485

Leu Leu Ala Leu Leu Asn Asp Ser Thr Lys Gln Val Arg Met Ser
1490 1495 1500

Ile Asn Asn Leu Thr Thr Asp Phe Asp Phe Ala Ser Gln Ser Asn
1505 1510 1515

```
Trp Arg  Tyr Phe Thr Glu Leu  Ile Leu Arg Pro Ile  Glu Met Ser
    1520                 1525                 1530

Asp Glu  Ile Pro Asn Gln Phe  Gln Asn Ile Trp Leu  His Leu Ile
    1535                 1540                 1545

Thr Leu  Gly Lys Glu Phe Gln  Lys Leu Val Lys Gly  Ile Tyr Phe
    1550                 1555                 1560

Asn Ile  Leu Glu Asn Asn Ser  Ser Ser Lys Thr Glu  Asn Leu Leu
    1565                 1570                 1575

Asn Ile  Phe Ala Thr Ser Pro  Lys Glu Lys Asp Val  Asn Ser Val
    1580                 1585                 1590

Gly Asn  Ser Ile Tyr His Leu  Ala Ser Tyr Leu Ala  Phe Ser Leu
    1595                 1600                 1605

Ser His  Asp Leu Gln Asn Ser  Pro Lys Ile Ile Ile  Ser Pro Glu
    1610                 1615                 1620

Ile Met  Lys Ala Thr Gly Leu  Gly Ile Gln Leu Ile  Arg Asp Val
    1625                 1630                 1635

Phe Asn  Ser Leu Met Pro Val  Val His His Thr Ser  Pro Gln Asn
    1640                 1645                 1650

Ala Gly  Tyr Met Gln Ala Leu  Lys Lys Val Thr Ser  Val Met Arg
    1655                 1660                 1665

Thr Leu  Lys Lys Ala Asp Ile  Asp Leu Leu Val Asp  Gln Leu Glu
    1670                 1675                 1680

Gln Val  Ser Val Asn Leu Met  Asp Phe Phe Lys Asn  Ile Ser Ser
    1685                 1690                 1695

Val Gly  Thr Gly Asn Leu Val  Val Asn Leu Leu Val  Gly Leu Met
    1700                 1705                 1710

Glu Lys  Phe Ala Asp Ser Ser  His Ser Trp Asn Val  Asn His Leu
    1715                 1720                 1725

Leu Gln  Leu Ser Arg Leu Phe  Pro Lys Asp Val Val  Asp Ala Val
    1730                 1735                 1740

Ile Asp  Val Tyr Tyr Val Leu  Pro His Ala Val Arg  Leu Leu Gln
    1745                 1750                 1755
```

Gly Val Pro Gly Lys Asn Ile Thr Glu Gly Leu Lys Asp Val Tyr
1760 1765 1770

Ser Phe Thr Leu Leu His Gly Ile Thr Ile Ser Asn Ile Thr Lys
1775 1780 1785

Glu Asp Phe Ala Ile Val Ile Lys Ile Leu Leu Asp Thr Ile Glu
1790 1795 1800

Leu Val Ser Asp Lys Pro Asp Ile Ile Ser Glu Ala Leu Ala Cys
1805 1810 1815

Phe Pro Val Val Trp Cys Trp Asn His Thr Asn Ser Gly Phe Arg
1820 1825 1830

Gln Asn Ser Lys Ile Asp Pro Cys Asn Val His Gly Leu Met Ser
1835 1840 1845

Ser Ser Phe Tyr Gly Lys Val Ala Ser Ile Leu Asp His Phe His
1850 1855 1860

Leu Ser Pro Gln Gly Glu Asp Ser Pro Cys Ser Asn Glu Ser Ser
1865 1870 1875

Arg Met Glu Ile Thr Arg Lys Val Val Cys Ile Ile His Glu Leu
1880 1885 1890

Val Asp Trp Asn Ser Ile Leu Leu Glu Leu Ser Glu Val Phe His
1895 1900 1905

Val Asn Ile Ser Leu Val Lys Thr Val Gln Lys Phe Trp His Lys
1910 1915 1920

Ile Leu Pro Phe Val Pro Pro Ser Ile Asn Gln Thr Arg Asp Ser
1925 1930 1935

Ile Ser Glu Leu Cys Pro Ser Gly Ser Ile Lys Gln Val Ala Leu
1940 1945 1950

Gln Ile Ile Glu Lys Leu Lys Asn Val Asn Phe Thr Lys Val Thr
1955 1960 1965

Ser Gly Glu Asn Ile Leu Asp Lys Leu Ser Ser Leu Asn Lys Ile
1970 1975 1980

Leu Asn Ile Asn Glu Asp Thr Glu Thr Ser Val Gln Asn Ile Ile
1985 1990 1995

Ser Ser Asn Leu Glu Arg Thr Val Gln Leu Ile Ser Glu Asp Trp
2000 2005 2010

Ser Leu Glu Lys Ser Thr His Asn Leu Leu Ser Leu Phe Met Met
2015 2020 2025

Leu Gln Asn Ala Asn Val Thr Gly Ser Ser Leu Glu Ala Leu Ser
2030 2035 2040

Ser Phe Ile Glu Lys Ser Glu Thr Pro Tyr Asn Phe Glu Glu Leu
2045 2050 2055

Trp Pro Lys Phe Gln Gln Ile Met Lys Asp Leu Thr Gln Asp Phe
2060 2065 2070

Arg Ile Arg His Leu Leu Ser Glu Met Asn Lys Gly Ile Lys Ser
2075 2080 2085

Ile Asn Ser Met Ala Leu Gln Lys Ile Thr Leu Gln Phe Ala His
2090 2095 2100

Phe Leu Glu Ile Leu Asp Ser Pro Ser Leu Lys Thr Leu Glu Ile
2105 2110 2115

Ile Glu Asp Phe Leu Leu Val Thr Lys Asn Trp Leu Gln Glu Tyr
2120 2125 2130

Ala Asn Glu Asp Tyr Ser Arg Met Ile Glu Thr Leu Phe Ile Pro
2135 2140 2145

Val Thr Asn Glu Ser Ser Thr Glu Asp Ile Ala Leu Leu Ala Lys
2150 2155 2160

Ala Ile Ala Thr Phe Trp Gly Ser Leu Lys Asn Ile Ser Arg Ala
2165 2170 2175

Gly Asn Phe Asp Val Ala Phe Leu Thr His Leu Leu Asn Gln Glu
2180 2185 2190

Gln Leu Thr Asn Phe Ser Val Val Gln Leu Leu Phe Glu Asn Ile
2195 2200 2205

Leu Ile Asn Leu Ile Asn Asn Leu Ala Gly Asn Ser Gln Glu Ala
2210 2215 2220

Ala Trp Asn Leu Asn Asp Thr Asp Leu Gln Ile Met Asn Phe Ile

2225 2230 2235

Asn Leu Ile Leu Asn His Met Gln Ser Glu Thr Ser Arg Lys Thr
2240 2245 2250

Val Leu Ser Leu Arg Ser Ile Val Asp Phe Thr Glu Gln Phe Leu
2255 2260 2265

Lys Thr Phe Phe Ser Leu Phe Leu Lys Glu Asp Ser Glu Asn Lys
2270 2275 2280

Ile Ser Leu Leu Leu Lys Tyr Phe His Lys Asp Val Ile Ala Glu
2285 2290 2295

Met Ser Phe Val Pro Lys Asp Lys Ile Leu Glu Ile Leu Lys Leu
2300 2305 2310

Asp Gln Phe Leu Thr Leu Met Ile Gln Asp Arg Leu Met Asn Ile
2315 2320 2325

Phe Ser Ser Leu Lys Glu Thr Ile Tyr His Leu Met Lys Ser Ser
2330 2335 2340

Phe Ile Leu Asp Asn Gly Glu Phe Tyr Phe Asp Thr His Gln Gly
2345 2350 2355

Leu Lys Phe Met Gln Asp Leu Phe Asn Ala Leu Leu Arg Glu Thr
2360 2365 2370

Ser Met Lys Asn Lys Thr Glu Asn Asn Ile Asp Phe Phe Thr Val
2375 2380 2385

Val Ser Gln Leu Phe Phe His Val Asn Lys Ser Glu Asp Leu Phe
2390 2395 2400

Lys Leu Asn Gln Asp Leu Gly Ser Ala Leu His Leu Val Arg Glu
2405 2410 2415

Cys Ser Thr Glu Met Ala Arg Leu Leu Asp Thr Ile Leu His Ser
2420 2425 2430

Pro Asn Lys Asp Phe Tyr Ala Leu Tyr Pro Thr Leu Gln Glu Val
2435 2440 2445

Ile Leu Ala Asn Leu Thr Asp Leu Leu Phe Phe Ile Asn Asn Ser
2450 2455 2460

Phe Pro Leu Arg Asn Arg Ala Thr Leu Glu Ile Thr Lys Arg Leu
2465 2470 2475

Val Gly Ala Ile Ser Arg Ala Ser Glu Glu Ser His Val Leu Lys
2480 2485 2490

Pro Leu Leu Glu Met Ser Gly Thr Leu Val Met Leu Leu Asn Asp
2495 2500 2505

Ser Ala Asp Leu Arg Asp Leu Ala Thr Ser Met Asp Ser Ile Val
2510 2515 2520

Lys Leu Leu Lys Leu Val Lys Lys Val Ser Gly Lys Met Ser Thr
2525 2530 2535

Val Phe Lys Thr His Phe Ile Ser Asn Thr Lys Asp Ser Val Lys
2540 2545 2550

Phe Phe Asp Thr Leu Tyr Ser Ile Met Gln Gln Ser Val Gln Asn
2555 2560 2565

Leu Val Lys Glu Ile Ala Thr Leu Lys Lys Ile Asp His Phe Thr
2570 2575 2580

Phe Glu Lys Ile Asn Asp Leu Leu Val Pro Phe Leu Asp Leu Ala
2585 2590 2595

Phe Glu Met Ile Gly Val Glu Pro Tyr Ile Ser Ser Asn Ser Asp
2600 2605 2610

Ile Phe Ser Met Ser Pro Ser Ile Leu Ser Tyr Met Asn Gln Ser
2615 2620 2625

Lys Asp Phe Ser Asp Ile Leu Glu Glu Ile Ala Glu Phe Leu Thr
2630 2635 2640

Ser Val Lys Met Asn Leu Glu Asp Met Arg Ser Leu Ala Val Ala
2645 2650 2655

Phe Asn Asn Glu Thr Gln Thr Phe Ser Met Asp Ser Val Asn Leu
2660 2665 2670

Xaa Glu Glu Ile Leu Gly Cys Leu Val Pro Ile Asn Asn Ile Thr
2675 2680 2685

Asn Gln Met Asp Phe Leu Tyr Pro Asn Pro Ile Ser Thr His Ser
2690 2695 2700

Gly Pro Gln Asp Ile Lys Trp Glu Ile Ile His Glu Val Ile Leu
2705 2710 2715

Phe Leu Asp Lys Ile Leu Ser Gln Asn Ser Thr Glu Ile Gly Ser
2720 2725 2730

Phe Leu Lys Met Val Ile Cys Leu Thr Leu Glu Ala Leu Trp Lys
2735 2740 2745

Asn Leu Lys Lys Asp Asn Trp Asn Val Ser Asn Val Leu Met Thr
2750 2755 2760

Phe Thr Gln His Pro Asn Asn Leu Leu Lys Thr Ile Glu Thr Val
2765 2770 2775

Leu Glu Ala Ser Ser Gly Ile Lys Ser Asp Tyr Glu Gly Asp Leu
2780 2785 2790

Asn Lys Ser Leu Tyr Phe Asp Thr Pro Leu Ser Gln Asn Ile Thr
2795 2800 2805

His His Gln Leu Glu Lys Ala Ile His Asn Val Leu Ser Arg Ile
2810 2815 2820

Ala Leu Trp Arg Lys Gly Leu Arg Phe Asn Asn Ser Glu Trp Ile
2825 2830 2835

Thr Ser Thr Arg Thr Leu Phe Gln Pro Leu Phe Glu Ile Phe Ile
2840 2845 2850

Lys Ala Thr Thr Gly Lys Asn Val Thr Ser Glu Lys Glu Glu Arg
2855 2860 2865

Thr Glu Lys Glu Met Ile Asp Phe Pro Tyr Ser Phe Lys Pro Phe
2870 2875 2880

Phe Cys Leu Glu Lys Tyr Leu Gly Gly Leu Phe Val Leu Thr Lys
2885 2890 2895

Tyr Trp Gln Gln Ile Pro Leu Thr Asp Gln Ser Val Val Glu Ile
2900 2905 2910

Cys Glu Val Phe Gln Gln Thr Val Lys Pro Ser Glu Ala Met Glu
2915 2920 2925

Met Leu Gln Lys Val Lys Met Met Val Val Arg Val Leu Thr Ile
2930 2935 2940

Val Ala Glu Asn Pro Ser Trp Thr Lys Asp Ile Leu Cys Ala Thr
2945 2950 2955

Leu Ser Cys Lys Gln Asn Gly Ile Arg His Leu Ile Leu Ser Ala
2960 2965 2970

Ile Gln Gly Val Thr Leu Ala Gln Asp His Phe Gln Glu Ile Glu
2975 2980 2985

Lys Ile Trp Ser Ser Pro Asn Gln Leu Asn Cys Glu Ser Leu Ser
2990 2995 3000

Lys Asn Leu Ser Ser Thr Leu Glu Ser Phe Lys Ser Ser Leu Glu
3005 3010 3015

Asn Ala Thr Gly Gln Asp Cys Thr Ser Gln Pro Arg Leu Glu Thr
3020 3025 3030

Val Gln Gln His Leu Tyr Met Leu Ala Lys Ser Leu Glu Glu Thr
3035 3040 3045

Trp Ser Ser Gly Asn Pro Ile Met Thr Phe Leu Ser Asn Phe Thr
3050 3055 3060

Val Thr Glu Asp Val Lys Ile Lys Asp Leu Met Lys Asn Ile Thr
3065 3070 3075

Lys Leu Thr Glu Glu Leu Arg Ser Ser Ile Gln Ile Ser Asn Glu
3080 3085 3090

Thr Ile His Ser Ile Leu Glu Ala Asn Ile Ser His Ser Lys Val
3095 3100 3105

Leu Phe Ser Ala Leu Thr Val Ala Leu Ser Gly Lys Cys Asp Gln
3110 3115 3120

Glu Ile Leu His Leu Leu Leu Thr Phe Pro Lys Gly Glu Lys Ser
3125 3130 3135

Trp Ile Ala Ala Glu Glu Leu Cys Ser Leu Pro Gly Ser Lys Val
3140 3145 3150

Tyr Ser Leu Ile Val Leu Leu Ser Arg Asn Leu Asp Val Arg Ala
3155 3160 3165

Phe Ile Tyr Lys Thr Leu Met Pro Ser Glu Ala Asn Gly Leu Leu

3170 3175 3180

Asn Ser Leu Leu Asp Ile Val Ser Ser Leu Ser Ala Leu Leu Ala
3185 3190 3195

Lys Ala Gln His Val Phe Glu Tyr Leu Pro Glu Phe Leu His Thr
3200 3205 3210

Phe Lys Ile Thr Ala Leu Leu Glu Thr Leu Asp Phe Gln Gln Val
3215 3220 3225

Ser Gln Asn Val Gln Ala Arg Ser Ser Ala Phe Gly Ser Phe Gln
3230 3235 3240

Phe Val Met Lys Met Val Cys Lys Asp Gln Ala Ser Phe Leu Ser
3245 3250 3255

Asp Ser Asn Met Phe Ile Asn Leu Pro Arg Val Lys Glu Leu Leu
3260 3265 3270

Glu Asp Asp Lys Glu Lys Phe Asn Ile Pro Glu Asp Ser Thr Pro
3275 3280 3285

Phe Cys Leu Lys Leu Tyr Gln Glu Ile Leu Gln Leu Pro Asn Gly
3290 3295 3300

Ala Leu Val Trp Thr Phe Leu Lys Pro Ile Leu His Gly Lys Ile
3305 3310 3315

Leu Tyr Thr Pro Asn Thr Pro Glu Ile Asn Lys Val Ile Gln Lys
3320 3325 3330

Ala Asn Tyr Thr Phe Tyr Ile Val Asp Lys Leu Lys Thr Leu Ser
3335 3340 3345

Glu Thr Leu Leu Glu Met Ser Ser Leu Phe Gln Arg Ser Gly Ser
3350 3355 3360

Gly Gln Met Phe Asn Gln Leu Gln Glu Ala Leu Arg Asn Lys Phe
3365 3370 3375

Val Arg Asn Phe Val Glu Asn Gln Leu His Ile Asp Val Asp Lys
3380 3385 3390

Leu Thr Glu Lys Leu Gln Thr Tyr Gly Gly Leu Leu Asp Glu Met
3395 3400 3405

Phe Asn His Ala Gly Ala Gly Arg Phe Arg Phe Leu Gly Ser Ile
3410 3415 3420

Leu Val Asn Leu Ser Ser Cys Val Ala Leu Asn Arg Phe Gln Ala
3425 3430 3435

Leu Gln Ser Val Asp Ile Leu Glu Thr Lys Ala His Glu Leu Leu
3440 3445 3450

Gln Gln Asn Ser Phe Leu Ala Ser Ile Ile Phe Ser Asn Ser Leu
3455 3460 3465

Phe Asp Lys Asn Phe Arg Ser Glu Ser Val Lys Leu Pro Pro His
3470 3475 3480

Val Ser Tyr Thr Ile Arg Thr Asn Val Leu Tyr Ser Val Arg Thr
3485 3490 3495

Asp Val Val Lys Asn Pro Ser Trp Lys Phe His Pro Gln Asn Leu
3500 3505 3510

Pro Ala Asp Gly Phe Lys Tyr Asn Tyr Val Phe Ala Pro Leu Gln
3515 3520 3525

Asp Met Ile Glu Arg Ala Ile Ile Leu Val Gln Thr Gly Gln Glu
3530 3535 3540

Ala Leu Glu Pro Ala Ala Gln Thr Gln Ala Ala Pro Tyr Pro Cys
3545 3550 3555

His Thr Ser Asp Leu Phe Leu Asn Asn Val Gly Phe Phe Phe Pro
3560 3565 3570

Leu Ile Met Met Leu Thr Trp Met Val Ser Val Ala Ser Met Val
3575 3580 3585

Arg Lys Leu Val Tyr Glu Gln Glu Ile Gln Ile Glu Glu Tyr Met
3590 3595 3600

Arg Met Met Gly Val His Pro Val Ile His Phe Leu Ala Trp Phe
3605 3610 3615

Leu Glu Asn Met Ala Val Leu Thr Ile Ser Ser Ala Thr Leu Ala
3620 3625 3630

Ile Val Leu Lys Thr Ser Gly Ile Phe Ala His Ser Asn Thr Phe
3635 3640 3645

Ile Val Phe Leu Phe Leu Leu Asp Phe Gly Met Ser Val Val Met
3650 3655 3660

Leu Ser Tyr Leu Leu Ser Ala Phe Phe Ser Gln Ala Asn Thr Ala
3665 3670 3675

Ala Leu Cys Thr Ser Leu Val Tyr Met Ile Ser Phe Leu Pro Tyr
3680 3685 3690

Ile Val Leu Leu Val Leu His Asn Gln Leu Ser Phe Val Asn Gln
3695 3700 3705

Thr Phe Leu Cys Leu Leu Ser Thr Thr Ala Phe Gly Gln Gly Val
3710 3715 3720

Phe Phe Ile Thr Phe Leu Glu Gly Gln Glu Thr Gly Ile Gln Trp
3725 3730 3735

Asn Asn Met Tyr Gln Ala Leu Glu Gln Gly Gly Met Thr Phe Gly
3740 3745 3750

Trp Val Cys Trp Met Ile Leu Phe Asp Ser Ser Leu Tyr Phe Leu
3755 3760 3765

Cys Gly Trp Tyr Leu Ser Asn Leu Ile Pro Gly Thr Phe Gly Leu
3770 3775 3780

Arg Lys Pro Trp Tyr Phe Pro Phe Thr Ala Ser Tyr Trp Lys Ser
3785 3790 3795

Val Gly Phe Leu Val Glu Lys Arg Gln Tyr Phe Leu Ser Ser Ser
3800 3805 3810

Leu Phe Phe Phe Asn Glu Asn Phe Asp Asn Lys Gly Ser Ser Leu
3815 3820 3825

Gln Asn Arg Glu Gly Glu Leu Glu Gly Ser Ala Pro Gly Val Thr
3830 3835 3840

Leu Val Ser Val Thr Lys Glu Tyr Glu Gly His Lys Ala Val Val
3845 3850 3855

Gln Asp Leu Ser Leu Thr Phe Tyr Arg Asp Gln Ile Thr Ala Leu
3860 3865 3870

Leu Gly Thr Asn Gly Ala Gly Lys Thr Thr Ile Ile Ser Met Leu
3875 3880 3885

Thr Gly Leu His Pro Pro Thr Ser Gly Thr Ile Ile Ile Asn Gly
3890 3895 3900

Lys Asn Leu Gln Thr Asp Leu Ser Arg Val Arg Met Glu Leu Gly
3905 3910 3915

Val Cys Pro Gln Gln Asp Ile Leu Leu Asp Asn Leu Thr Val Arg
3920 3925 3930

Glu His Leu Leu Leu Phe Ala Ser Ile Lys Ala Pro Gln Trp Thr
3935 3940 3945

Lys Lys Glu Leu His Gln Gln Val Asn Gln Thr Leu Gln Asp Val
3950 3955 3960

Asp Leu Thr Gln His Gln His Lys Gln Thr Arg Ala Leu Ser Gly
3965 3970 3975

Gly Leu Lys Arg Lys Leu Ser Leu Gly Ile Ala Phe Met Gly Met
3980 3985 3990

Ser Arg Thr Val Val Leu Asp Glu Pro Thr Ser Gly Val Asp Pro
3995 4000 4005

Cys Ser Arg His Ser Leu Trp Asp Ile Leu Leu Lys Tyr Arg Glu
4010 4015 4020

Gly Arg Thr Ile Ile Phe Thr Thr His His Leu Asp Glu Ala Glu
4025 4030 4035

Ala Leu Ser Asp Arg Val Ala Val Leu Gln His Gly Arg Leu Arg
4040 4045 4050

Cys Cys Gly Pro Pro Phe Cys Leu Lys Glu Ala Tyr Gly Gln Gly
4055 4060 4065

Leu Arg Leu Thr Leu Thr Arg Gln Pro Ser Val Leu Glu Ala His
4070 4075 4080

Asp Leu Lys Asp Met Ala Cys Val Thr Ser Leu Ile Lys Ile Tyr
4085 4090 4095

Ile Pro Gln Ala Phe Leu Lys Asp Ser Ser Gly Ser Glu Leu Thr
4100 4105 4110

Tyr Thr Ile Pro Lys Asp Thr Asp Lys Ala Cys Leu Lys Gly Leu

4115 4120 4125

Phe Gln Ala Leu Asp Glu Asn Leu His Gln Leu His Leu Thr Gly
4130 4135 4140

Tyr Gly Ile Ser Asp Thr Thr Leu Glu Glu Val Phe Leu Met Leu
4145 4150 4155

Leu Gln Asp Ser Asn Lys Lys Ser His Ile Ala Leu Gly Thr Glu
4160 4165 4170

Ser Glu Leu Gln Asn His Arg Pro Thr Gly His Leu Ser Gly Tyr
4175 4180 4185

Cys Gly Ser Leu Ala Arg Pro Ala Thr Val Gln Gly Val Gln Leu
4190 4195 4200

Leu Arg Ala Gln Val Ala Ala Ile Leu Ala Arg Arg Leu Arg Arg
4205 4210 4215

Thr Leu Arg Ala Gly Lys Ser Thr Leu Ala Asp Leu Leu Leu Pro
4220 4225 4230

Val Leu Phe Val Ala Leu Ala Met Gly Leu Phe Met Val Arg Pro
4235 4240 4245

Leu Ala Thr Glu Tyr Pro Pro Leu Arg Leu Thr Pro Gly His Tyr
4250 4255 4260

Gln Arg Ala Glu Thr Tyr Phe Phe Ser Ser Gly Gly Asp Asn Leu
4265 4270 4275

Asp Leu Thr Arg Val Leu Leu Arg Lys Phe Arg Asp Gln Asp Leu
4280 4285 4290

Pro Cys Ala Asp Leu Asn Pro Arg Gln Lys Asn Ser Ser Cys Trp
4295 4300 4305

Arg Thr Asp Pro Phe Ser His Pro Glu Phe Gln Asp Ser Cys Gly
4310 4315 4320

Cys Leu Lys Cys Pro Asn Arg Ser Ala Ser Ala Pro Tyr Leu Thr
4325 4330 4335

Asn His Leu Gly His Thr Leu Leu Asn Leu Ser Gly Phe Asn Met
4340 4345 4350

Glu Glu Tyr Leu Leu Ala Pro Ser Glu Lys Pro Arg Leu Gly Gly
4355 4360 4365

Trp Ser Phe Gly Leu Lys Ile Pro Ser Glu Ala Gly Gly Ala Asn
4370 4375 4380

Gly Asn Ile Ser Lys Pro Pro Thr Leu Ala Lys Val Trp Tyr Asn
4385 4390 4395

Gln Lys Gly Phe His Ser Leu Pro Ser Tyr Leu Asn His Leu Asn
4400 4405 4410

Asn Leu Ile Leu Trp Gln His Leu Pro Pro Thr Val Asp Trp Arg
4415 4420 4425

Gln Tyr Gly Ile Thr Leu Tyr Ser His Pro Tyr Gly Gly Ala Leu
4430 4435 4440

Leu Asn Glu Asp Lys Ile Leu Glu Ser Ile Arg Gln Cys Gly Val
4445 4450 4455

Ala Leu Cys Ile Val Leu Gly Phe Ser Ile Leu Ser Ala Ser Ile
4460 4465 4470

Gly Ser Ser Val Val Arg Asp Arg Val Ile Gly Ala Lys Arg Leu
4475 4480 4485

Gln His Ile Ser Gly Leu Gly Tyr Arg Met Tyr Trp Phe Thr Asn
4490 4495 4500

Phe Leu Tyr Asp Met Leu Phe Tyr Leu Val Ser Val Cys Leu Cys
4505 4510 4515

Val Ala Val Ile Val Ala Phe Gln Leu Thr Ala Phe Thr Phe Arg
4520 4525 4530

Lys Asn Leu Ala Ala Thr Ala Leu Leu Leu Ser Leu Phe Gly Tyr
4535 4540 4545

Ala Thr Leu Pro Trp Met Tyr Leu Met Ser Arg Ile Phe Ser Ser
4550 4555 4560

Ser Asp Val Ala Phe Ile Ser Tyr Val Ser Leu Asn Phe Ile Phe
4565 4570 4575

Gly Leu Cys Thr Met Pro Ile Thr Ile Met Pro Arg Leu Leu Ala
4580 4585 4590

Ile Ile Ser Lys Ala Lys Asn Leu Gln Asn Ile Tyr Asp Val Leu
4595 4600 4605

Lys Trp Val Phe Thr Ile Phe Pro Gln Phe Cys Leu Gly Gln Gly
4610 4615 4620

Leu Val Glu Leu Cys Tyr Asn Gln Ile Lys Tyr Asp Leu Thr His
4625 4630 4635

Asn Phe Gly Ile Asp Ser Tyr Val Ser Pro Phe Glu Met Asn Phe
4640 4645 4650

Leu Gly Trp Ile Phe Val Gln Leu Ala Ser Gln Gly Thr Val Leu
4655 4660 4665

Leu Leu Leu Arg Val Leu Leu His Trp Asp Leu Leu Arg Trp Pro
4670 4675 4680

Arg Gly His Ser Thr Leu Gln Gly Thr Val Lys Ser Ser Lys Asp
4685 4690 4695

Thr Asp Val Glu Lys Glu Glu Lys Arg Val Phe Glu Gly Arg Thr
4700 4705 4710

Asn Gly Asp Ile Leu Val Leu Tyr Asn Leu Ser Lys His Tyr Arg
4715 4720 4725

Arg Phe Phe Gln Asn Ile Ile Ala Val Gln Asp Ile Ser Leu Gly
4730 4735 4740

Ile Pro Lys Gly Glu Cys Phe Gly Leu Leu Gly Val Asn Gly Ala
4745 4750 4755

Gly Lys Ser Thr Thr Phe Lys Met Leu Asn Gly Glu Val Ser Leu
4760 4765 4770

Thr Ser Gly His Ala Ile Ile Arg Thr Pro Met Gly Asp Ala Val
4775 4780 4785

Asp Leu Ser Ser Ala Gly Thr Ala Gly Val Leu Ile Gly Tyr Cys
4790 4795 4800

Pro Gln Gln Asp Ala Leu Asp Glu Leu Leu Thr Gly Trp Glu His
4805 4810 4815

Leu Tyr Tyr Tyr Cys Ser Leu Arg Gly Ile Pro Arg Gln Cys Ile
4820 4825 4830

Pro Glu Val Ala Gly Asp Leu Ile Arg Arg Leu His Leu Glu Ala
4835 4840 4845

His Ala Asp Lys Pro Val Ala Thr Tyr Ser Gly Gly Thr Lys Arg
4850 4855 4860

Lys Leu Ser Thr Ala Leu Ala Leu Val Gly Lys Pro Asp Ile Leu
4865 4870 4875

Leu Leu Asp Glu Pro Ser Ser Gly Met Asp Pro Cys Ser Lys Arg
4880 4885 4890

Tyr Leu Trp Gln Thr Ile Met Lys Glu Val Arg Glu Gly Cys Ala
4895 4900 4905

Ala Val Leu Thr Ser His Ser Met Glu Glu Cys Glu Ala Leu Cys
4910 4915 4920

Thr Arg Leu Ala Ile Met Val Asn Gly Ser Phe Lys Cys Leu Gly
4925 4930 4935

Ser Pro Gln His Ile Lys Asn Arg Phe Gly Asp Gly Tyr Thr Val
4940 4945 4950

Lys Val Trp Leu Cys Lys Glu Ala Asn Gln His Cys Thr Val Ser
4955 4960 4965

Asp His Leu Lys Leu Tyr Phe Pro Gly Ile Gln Phe Lys Gly Gln
4970 4975 4980

His Leu Asn Leu Leu Glu Tyr His Val Pro Lys Arg Trp Gly Cys
4985 4990 4995

Leu Ala Asp Leu Phe Lys Val Ile Glu Asn Asn Lys Thr Phe Leu
5000 5005 5010

Asn Ile Lys His Tyr Ser Ile Asn Gln Thr Thr Leu Glu Gln Val
5015 5020 5025

Phe Ile Asn Phe Ala Ser Glu Gln Gln Gln Thr Leu Gln Ser Thr
5030 5035 5040

Leu Asp Pro Ser Thr Asp Ser His His Thr His His Leu Pro Ile
5045 5050 5055

<210> 52
<211> 17209

<212> DNA
<213> Homo sapiens

<400> 52

ggactgagag cagggagcag caggcatggg gcatgccggg tgccagttca aagccctgct 60
gtggaagaat tggctctgca gactcaggaa cccggtcctt ttccttgctg aattcttctg 120
gccttgtatc ctgtttgtaa ttctgacagt tcttcgtttt caagaacctc ccagatacag 180
agacatttgt tatttgcagc cccgagatct acccagctgt ggtgttatcc cctttgttca 240
aagccttctt tgtaacactg gatcaaggtg taggaacttc agctatgaag ggtcaatgga 300
gcatcatttt cgtttgtcta ggttccaaac tgcagctgac cccaagaaag tcaacaacct 360
ggccttttta aaagagatac aagacctggc agaggaaatt catggaatga tggacaaggc 420
aaaaaactta aaaagacttt gggtagaacg atccaacact ccagattctt cttatggttc 480
cagttttttt acaatggatc tcaataagac cgaggaggta atattgaaac tggaaagcct 540
ccatcagcag cctcatatct gggattttct acttttactg ccgagactac acacaagcca 600
tgatcatgtg gaagatggca tggatgttgc agtgaacctt ctccagacca ttttgaattc 660
cttaatatcc ctagaagatt tagattggct tccactcaac caaacttttt cccaggtttc 720
tgaacttgta ctgaatgtga ccatttcgac actgacattt ctgcagcaac atggagtagc 780
agtcaccgag ccagtttacc acctgtccat gcagaatata gtgtgggatc cacagaaagt 840
ccagtatgat ctcaaatccc agtttggctt tgrtgatctt cacacggaac agatcctgaa 900
ctcttcagct gaactgaagg agattcccac agacacttcc ttggagaaga tggtgtgttc 960
agtcttgtct agcacatcag aggatgaagc tgagaaatgg ggccacgttg gaggctgcca 1020
ccctaagtgg tcagaagcca aaaactatct tgtccatgca gtcagctggc tgcgagtcta 1080
ccaacaggtg tttgttcagt ggcaacaggg tagcctgctt cagaagacac tcacaggcat 1140
gggccatagt ctggaggctc tcaggaatca gtttgaagaa gagagcaagc cctggaaggt 1200
ggtggaagct ctgcacactg cactgctcct gctgaatgac agcttgtcag cagatggccc 1260
aaaagataat catacatttc caaagatatt acagcatctg tggaaattgc aaagcttgct 1320
gcaaaacctg ccccagtggc cggcactgaa gagatttctt cagcttgatg gagctctcag 1380
aaatgcgata gctcagaatt tacattttgt ccaagaagtc ctcatttgcc tggagacatc 1440
agctaatgat tttaaatggt ttgaacttaa ccaattgaaa ctggaaaagg atgtgttctt 1500
ttgggagctg aaacagatgt tggcgaagaa tgctgtctgc ccgaatggtc gtttctctga 1560
gaaggaggtc tttttgccgc ctggaaactc cagcatatgg ggtggtctcc agggactgtt 1620
gtgctattgt aactcctctg agacgagtgt tttaaacaag ctacttggtt cagtagagga 1680
tgctgatcgt attttgcaag aggtcattac ttggcacaaa aatatgtcag ttttaatacc 1740

tgaagaatat ttggactggc aggaacttga gatgcagctg tcagaagcaa gcctttcctg 1800

tactcggctc ttcctgctgc tgggagctga tccctctcct gagaatgatg tcttttctag 1860

tgactgtaag caccagcttg tctccacagt gatatttcat acacttgaaa aaacacaatt 1920

tttcctggaa caagcatatt attggaaagc cttcaaaaag tttatcagga agacttgcga 1980

agtggcccaa tatgtaaata tgcaagagag tttccagaac agactattgg cttttcctga 2040

ggaatctcct tgttttgaag aaaacatgga ttggaaaatg atcagtgata attattttca 2100

atttttgaat aacttactca agtctccaac agcttccata tccagggctt taaatttcac 2160

aaagcacctt ctaatgatgg aaaagaagtt gcacaccctt gaggatgaac aaatgaactt 2220

tcttttatca tttgtggaat tttttgagaa attattgttg cctaatcttt ttgactcctc 2280

cattgttccc agtttccaca gcctcccatc tctcacagag gatattctga atataagttc 2340

tctgtggaca aatcatttaa aaagtttaaa gagagaccca tctgccactg atgctcagaa 2400

actcttggaa tttggcaacg aagtgatttg gaaaatgcag actctcggaa gtcactggat 2460

aaggaaggaa ccaaaaaatc ttttgagatt catagaatta atactttttg aaattaatcc 2520

caaattacta gaattatggg cctatggcat ttcaaaagga aaaagagcta aattggaaaa 2580

cttctttaca cttttaaatt tttctgttcc agaaaatgag attctgagta caagttttaa 2640

cttttcccag ttgttccatt cagattggcc taaatcacca gctatgaaca tagattttgt 2700

acgtttaagt gaggctataa taactagtct ccatgaattt ggatttttgg agcaggaaca 2760

gatctcagaa gctctgaaca cagtctacgc tatcaggaat gcatctgatc ttttctcagc 2820

cctttctgaa ccacaaaaac aagaagttga taaaattttg actcacatac acctaaatgt 2880

cttccaggac aaggattcag ctttacttct gcaaatttat tcttcatttt accgatatat 2940

ttatgaatta ttgaatattc agagtagagg ctcttcgttg actttcctta cacaaatctc 3000

aaaacacatt ttggatatca taaaacaatt taatttccaa aacatcagta aagcatttgc 3060

atttttattt aagacagcag aggttcttgg gggaatttct aatgtatctt actgtcagca 3120

attgctttca atttttaact ttttggagct tcaggcccaa tccttcatgt ctacagaggg 3180

ccaagaactg gaagtgatcc acactacttt gacaggcctc aaacagctgc tcataattga 3240

tgaagatttt cgtatttctt tatttcaata tatgagccaa ttcttcaaca gttcagtaga 3300

agacctattg gataataaat gcttgatttc ggacaataaa cacatttctt ccgtaaatta 3360

ttcaacaagt gaggagtctt catttgtttt tccattggca caaatttttt caaacctctc 3420

agcaaatgtc agtgtgttca acaagtttat gtccattcac tgtaccgttt catggcttca 3480

aatgtggact gaaatctggg aaaccatatc tcaattattt aagtttgaca tgaatgtttt 3540

cacatctctt catcatggtt tcactcagct tttggatgaa ttggaagatg atgtgaaagt 3600

ctctaaaagc tgccagggta tacttcccac ccataatgtt gctagactca tattaaattt 3660

gtttaaaaat gtaactcaag ccaatgactt ccataattgg gaggacttcc tggatctcag 3720
ggattttttg gtagctttag gtaatgcatt agtttcagta aaaaaactta acttggagca 3780
agtggagaaa tccctttca ccatggaagc tgccctgcat cagttgaaga catttccatt 3840
caacgaaagt acaagcagag agtttttaaa ttctctgctt gaagttttca ttgagtttag 3900
cagtacctca gaatatatag tcagaaatct agattcaata aatgactttc tttcaaataa 3960
tctcacaaat tatggagaaa aatttgaaaa tatcatcact gagctaagag aagcaatagt 4020
atttcttaga aatgtatcac atgatcgaga tttgttttcc tgtgctgata ttttccaaaa 4080
tgttactgag tgtattttag aagatggctt tttatatgta aatacctcac agaggatgtt 4140
acgtattcta gacacgttaa attccacatt ttcctctgag aacacaatta gcagtctgaa 4200
aggatgcatt gtatggttag atgtcataaa ccatttgtat ttgttgtcta actccagttt 4260
ttcacaaggt cgtcttcaaa atattttggg gaatttcaga gatatagaaa acaaaatgaa 4320
ctctatatta aaaattgtaa cttgggtgtt aaatataaaa aaacctcttt gttcatcaaa 4380
tggctcacat ataaattgtg tcaatattta cttgaaagat gtaactgact ttctaaatat 4440
tgtacttact acagtctttg aaaaagagaa gaaacctaaa tttgagattt tattagctct 4500
tttaaatgat tccacaaagc aagtaaggat gagtatcaac aacttaacaa cagactttga 4560
ttttgcatct cagtccaatt ggagatattt tactgaatta attctaagac caatagaaat 4620
gtcagatgaa attcctaatc agtttcaaaa tatttggctt catttaataa cactggggaa 4680
ggaatttcag aagcttgtaa aaggtattta ttttaacatc ctggaaaata attcctcttc 4740
taaaactgaa aacttgttaa acatatttgc caccagtcca aaagaaaagg atgtaaacag 4800
tgtaggcaat tccatttatc acttagctag ttaccttgcc ttcagcttat ctcatgacct 4860
ccaaaattca ccaaaaataa taatttcacc tgaaataatg aaagctacag gtcttggtat 4920
tcaactgata agggatgtgt tcaactcctt aatgcctgta gttcatcaca ctagtccaca 4980
aaatgcaggt tatatgcaag ctttgaagaa ggtaacttct gtcatgcgta cccttaagaa 5040
agcagacata gaccttttag tggatcagct tgaacaagtt agtgtaaacc taatggattt 5100
ctttaagaat atcagtagtg tgggaactgg caatttagtg gtcaatttgc ttgttggctt 5160
gatggaaaaa tttgcagaca gctcacattc ttggaatgtt aatcatctgc tgcagctctc 5220
acgcctgttt cctaaagatg ttgtggatgc tgtgatagat gtgtactatg tgcttcctca 5280
tgctgtaagg ctcctgcagg gagtacctgg taaaaacatc actgaaggcc tcaaggatgt 5340
ctacagcttc acactccttc atggcataac catttcaaat atcaccaagg aagacttcgc 5400
aattgtgata aaaattcttt tggatacaat tgaattagta tcagataagc cagatattat 5460
ttcagaggct ttagcttgtt ttcctgtggt ttggtgctgg aatcacacaa attctggatt 5520

tcggcagaat tcaaagatag acccctgcaa tgtccatggg ctcatgtctt cttcctttta 5580
tggcaaagtg gccagtatac ttgatcattt ccacctgtct ccccaaggtg aagattcacc 5640
atgttcaaat gaaagctccc gaatggaaat aactaggaaa gtggtctgca taattcatga 5700
attagtggac tggaattcta ttcttctgga gctctctgaa gtcttccatg ttaacatttc 5760
tcttgtgaaa actgtgcaga aattttggca taagatatta ccgtttgtcc caccttcaat 5820
aaatcaaact agggatagca tctctgaact ctgtcctagt ggttccataa agcaagttgc 5880
tttgcaaatc atagaaaaac ttaaaaatgt caactttaca aaagttacat caggtgaaaa 5940
tattcttgac aaactaagta gtttaaacaa gatccttaac attaatgaag acacagagac 6000
atctgttcaa aatattattt cctcaaattt ggaaaggaca gtacaattga tttctgaaga 6060
ctggagccta gaaaaaagta cgcataatct actctcttta ttcatgatgc tccagaatgc 6120
aaatgtcaca ggtagcagtt tagaagcatt atcaagtttt attgaaaaaa gtgaaacacc 6180
ttacaacttt gaagaactat ggcccaagtt tcaacaaatc atgaaagacc taacccaaga 6240
ttttagaatc agacacctgc tttctgaaat gaacaaagga atcaaaagta taaattcaat 6300
ggctcttcaa aagataactt tgcagtttgc ccatttcctg gaaatcctgg attcaccgtc 6360
attgaagaca ttagaaatta ttgaagattt tctattggtc acaaaaaact ggcttcagga 6420
atatgcaaat gaggattact ccagaatgat agaaacatta ttcattcctg tgaccaatga 6480
gagttcaact gaagatatag ctttgttagc caaagctatt gctacttttt ggggctcttt 6540
aaaaaatata tctagagcag gcaattttga tgttgccttt cttacccatc tgctaaatca 6600
agaacagctg actaatttct cagttgttca gctgcttttt gaaaacatcc taattaattt 6660
gatcaataac ttagctggga attctcagga agcagcttgg aacttaaatg atactgacct 6720
tcaaataatg aatttcatta accttatctt gaaccatatg cagtcagaaa ctagtaggaa 6780
aacagttctc tctctgagaa gcatagtaga tttcacagaa cagtttttga aaacattctt 6840
ctcccttttt ctaaaggaag attctgagaa caaaatatct cttctgctga aatatttcca 6900
caaagatgtt attgcagaga tgagttttgt cccaaaagat aaaattctag aaattctgaa 6960
actggatcaa tttcttaccc tgatgataca agacagattg atgaacattt tttcaagttt 7020
aaaggagact atatatcacc taatgaaaag ttcatttata ttagacaatg gagaatttta 7080
ttttgatact catcaaggac tgaagttcat gcaagattta tttaatgccc ttctcaggga 7140
aacttcaatg aaaaataaga ctgaaaataa tatagacttt ttcacagtgg tgagtcagtt 7200
gtttttccat gtgaataagt ctgaggacct cttcaaactc aatcaagatc ttgggtcagc 7260
tcttcacctt gtaagagaat gttcaacaga gatggcaaga cttctggata caattttaca 7320
ctctcctaat aaggacttct atgctttgta tcctaccctc caagaagtta tacttgctaa 7380
tctaacggat ttgcttttct ttataaataa ttcattccct ctaagaaaca gagcaacatt 7440

agaaattact aagagattag ttggtgctat ttcaagagca agtgaagaaa gtcacgtcct 7500
gaaacccctc ttagaaatgt ctgggactct ggtcatgctg ttgaatgaca gtgctgacct 7560
gagagatctt gccacatcaa tggactccat tgtgaaactt cttaagctgg tcaagaaagt 7620
ttcggggaag atgtccacag tttttaaaac tcattttatc tccaatacca aggacagtgt 7680
gaaattcttt gacactctgt attccatcat gcaacaaagt gttcaaaatc ttgtgaaaga 7740
aatagctact ttaaaaaaaa tagatcattt cacatttgaa aagataaatg atttgttggt 7800
gccatttctt gacttggcct ttgaaatgat tggggtagaa ccttatatat catcaaactc 7860
tgatattttc agtatgtcac ctagcatact ctcatatatg aaccaatcta aggacttttc 7920
tgatattttg gaagaaattg ctgaattttt aacatctgtg aaaatgaact tggaagatat 7980
gaggagtctt gcggtagcat ttaacaatga gactcaaaca ttttctatgg attctgtcaa 8040
cttaygggaa gaaattctgg gttgcttagt tcctataaat aacatcacca accaaatgga 8100
cttcttatac cctaatccaa tttccactca tagtggccct caagatataa aatgggaaat 8160
aattcatgaa gtgatccttt ttttggataa aatattatca caaaacagca cagaaatagg 8220
atctttcttg aaaatggtga tctgtctcac cttagaagct ctttggaaaa acttaaagaa 8280
agataattgg aatgtttcta atgtgttgat gacgtttact cagcatccaa ataacctttt 8340
gaaaaccata gaaacagttt tagaggcctc cagtggaatt aaaagtgact atgaaggtga 8400
tttgaataaa agtttatatt ttgacacacc tttgagtcag aatataactc atcatcaact 8460
tgaaaaagca atccataatg ttttaagtag aatagctctc tggaggaaag gacttcgttt 8520
taacaactct gaatggataa cttccacaag aactttgttt cagccacttt ttgagatttt 8580
cattaaagca accaccggaa agaatgtcac atcagaaaaa gaagagagaa ccgagaaaga 8640
gatgattgac tttccttata gtttcaaacc atttttctgt ttggagaaat acctgggagg 8700
attatttgta ttgactaaat actggcaaca aatcccacta acagatcaaa gtgttgttga 8760
gatttgtgaa gttttccagc agactgtgaa gccctcagaa gccatggaga tgctgcagaa 8820
agtgaagatg atggtcgtac gtgtgctcac catcgttgca gaaaaccctt cctggaccaa 8880
ggacattttg tgtgctactc tgagttgcaa gcaaaatggg ataaggcatc tcattttatc 8940
tgctatacaa ggggtcactt tggcgcagga ccacttccag gaaattgaaa agatatggtc 9000
ctcgccgaat cagctaaatt gtgaaagtct tagcaagaat ctttctagca ccttggagag 9060
cttcaagagc agcttggaaa atgccactgg ccaggactgc acaagccagc cgaggctgga 9120
gacggtgcag cagcacttgt acatgttggc caaaagcctc gaggaaactt ggtcatcagg 9180
gaatcccatc atgacttttc tcagcaattt cacagtaact gaggatgtaa aaataaaaga 9240
tttgatgaag aatatcacca agttgactga ggagcttcgc tcttccatcc aaatctcgaa 9300

tgagactatc catagcattc tagaagcaaa tatttcccac tccaaggttc tcttcagtgc 9360
cctcaccgta gctctgtctg gaaagtgtga tcaggaaatc cttcatctcc tgctgacatt 9420
tcccaaaggg gaaaaatctt ggatcgcagc ggaggaactc tgtagcctgc cagggtcaaa 9480
agtgtattct ctgattgtgt tgctgagtcg aaacttggat gtgcgagctt tcatttacaa 9540
gactctgatg ccttctgaag caaatggctt gctcaactcc ttgctggata tagtttccag 9600
cctcagcgcc ttgcttgcca aagcccagca cgtctttgag tatcttcctg agtttcttca 9660
cacatttaaa atcactgcct tgctagaaac cctggacttt caacaggttt cacaaaatgt 9720
ccaggccaga agttcagctt ttggttcttt ccagtttgtg atgaagatgg tttgcaagga 9780
ccaagcatca ttccttagcg attctaatat gtttattaat ttgcccagag ttaaggaact 9840
cttggaagat gacaaagaaa aattcaacat tcctgaagat tcaacaccgt tttgcttgaa 9900
gctttatcag gaaattctac aattgccaaa tggtgctttg gtgtggacct tcctaaaacc 9960
catattgcat ggaaaaatac tatacacacc aaacactcca gaaattaaca aggtcattca 10020
aaaggctaat tacacctttt atattgtgga caaactaaaa actttatcag aaacactgct 10080
ggaaatgtcc agccttttcc agagaagtgg aagtggccag atgttcaacc agctgcagga 10140
ggccctgaga aacaaatttg taagaaactt tgtagaaaac cagttgcaca ttgatgtaga 10200
caaacttact gaaaaactcc agacatacgg agggctgctg gatgagatgt ttaaccatgc 10260
aggcgctgga cgcttccgtt tcttgggcag catcttggtc aatctctctt cctgcgtggc 10320
actgaaccgt ttccaggctc tgcagtctgt cgacatcctg gagactaaag cacatgaact 10380
cttgcagcag aacagcttct tggccagtat cattttcagc aattccttat tcgacaagaa 10440
cttcagatca gagtctgtca aactgccacc ccatgtctca tacacaatcc ggaccaatgt 10500
gttatacagc gtgcgaacag atgtggtaaa aaacccttct tggaagttcc accctcagaa 10560
tctaccagct gatgggttca aatataacta cgtctttgcc ccactgcaag acatgatcga 10620
aagagccatc attttggtgc agactgggca ggaagccctg gaaccagcag cacagactca 10680
ggcggcccct tacccctgcc ataccagcga cctattcctg aacaacgttg gtttcttttt 10740
tccactgata atgatgctga cgtggatggt gtctgtggcc agcatggtca gaaagttggt 10800
gtatgagcag gagatacaga tagaagagta tatgcggatg atgggagtgc atccagtgat 10860
ccatttcctg gcctggttcc tggagaacat ggctgtgttg accataagca gtgctactct 10920
ggccatcgtt ctgaaaacaa gtggcatctt tgcacacagc aataccttta ttgttttcct 10980
ctttctcttg gattttggga tgtcagtcgt catgctgagc tacctcttga gtgcattttt 11040
cagccaagct aatacagcgg ccctttgtac cagcctggtg tacatgatca gctttctgcc 11100
ctacatagtt ctattggttc tacataacca attaagtttt gttaatcaga catttctgtg 11160
ccttctttcg acaaccgcct ttggacaagg ggtatttttt attacattcc tggaaggaca 11220

agagacaggg attcaatgga ataatatgta ccaggctctg gaacaagggg gcatgacatt 11280
tggctgggtt tgctggatga ttctttttga ttcaagcctt tatttttttgt gtggatggta 11340
cttgagcaac ttgattcctg gaacatttgg tttacggaaa ccatggtatt tcccctttac 11400
tgcctcatat tggaagagtg tgggtttctt ggtggagaaa aggcaatact ttctaagttc 11460
tagtctgttc ttcttcaatg agaactttga caataaaggg tcatcactgc aaaacaggga 11520
aggagagctt gaaggaagtg ccccgggagt caccctggtg tctgtgacca aggaatatga 11580
gggccacaag gctgtggtcc aagacctcag cctgaccttc tacagagacc aaatcaccgc 11640
cctgctgggg acaaacggtg ccgggaaaac cactatcata tccatgttga cggggctcca 11700
ccctcccact tctggaacca tcatcatcaa tggcaagaac ctacagacag acctgtcgag 11760
ggtcagaatg gagcttggtg tgtgtccgca gcaggacatc ctgttggaca acctcaccgt 11820
ccgggaacat ttgctgctct ttgcttccat aaaggcgcct cagtggacca agaaggagct 11880
gcatcagcaa gtcaatcaaa ctcttcagga tgtggactta actcagcatc agcacaaaca 11940
gacccgagct ctgtctggag gcctgaagag gaagctctcc cttggcattg ctttcatggg 12000
catgtcgagg accgtggttc tggatgagcc caccagtggg gtggaccctt gctcccggca 12060
tagcctgtgg gacattctgc tcaagtaccg agaaggtcgt acgatcatct tcacaaccca 12120
ccacctggat gaagccgaag cgctgagtga ccgcgtggcc gtcctccagc atgggaggct 12180
caggtgctgc ggtcctccct tctgcctgaa ggaggcatat ggccaggggc tccgcctgac 12240
actcacgagg cagccttctg ttctggaggc ccatgatctg aaagacatgg cttgtgttac 12300
atccctgata aagatctata ttccacaagc atttctcaaa gacagcagtg gaagtgagct 12360
gacctacacc attccaaagg acacagacaa ggcctgcttg aaagggctct tccaggccct 12420
ggatgagaac ctgcatcagc tgcacctgac gggctatggg atctcagaca ccaccttaga 12480
agaggtgttt ttgatgcttt tgcaagattc caacaagaaa tctcacattg ccctggggac 12540
tgagtcagag ctgcagaacc acaggcctac aggacatctg tctggctact gtggctccct 12600
agcacggccc gcaactgtgc agggcgtcca gctgctccgc gcacaagtgg ccgcgatcct 12660
ggcccggagg ctccgccgca cgctgcgcgc cgggaagagc accctcgccg acctgctgct 12720
gccagtcctc ttcgtggcct tggccatggg cttgttcatg gtgagacccc tggccaccga 12780
gtaccctccc ctcagactca cacctggaca ttaccagcgg gccgagacct actttttcag 12840
cagtgggggc gacaacttgg acctcacccg tgtgcttctg cggaagttta gagatcaaga 12900
tttgccctgt gcagatttaa acccacgcca gaagaattct tcatgctggc gcacagatcc 12960
cttttctcac ccagaattcc aggattcatg tggctgcctg aagtgtccaa atagaagtgc 13020
tagtgctccc tacctgacca accacctggg ccacacactg ttgaatctct caggcttcaa 13080

tatggaggag tacttgctgg caccatctga aaaaccaagg cttggaggtt ggtcttttgg 13140
attaaaaatc cccagtgaag ctggaggtgc aaatggaaac atatcaaaac ccccaactct 13200
ggcaaaggtg tggtataatc agaagggttt tcattcccta ccttcctact taaatcatct 13260
aaacaacctt attttgtggc agcacctacc ccctactgtg gactggagac aatacggaat 13320
aacactctac agccacccat atggaggggc cttgctgaac gaggacaaga tcctggagag 13380
catccgtcag tgtggagtgg ccctctgcat cgtgctggga ttctccatcc tgtctgcatc 13440
catcggcagc tctgtggtga gggacagggt gattggagcc aaaaggttgc agcacataag 13500
tggccttggc tacaggatgt actggttcac aaacttccta tatgacatgc tcttttactt 13560
ggtttccgtc tgcctgtgtg ttgccgttat tgtcgccttc cagttaacag cttttacttt 13620
ccgcaagaac ttggcagcca cggccctcct gctgtcactt ttcggatatg caactcttcc 13680
atggatgtac ctgatgtcca gaatcttttc cagttcggac gtggctttca tttcctatgt 13740
ctcactaaac ttcatctttg gcctttgtac catgcccata accattatgc cccggttgct 13800
agccatcatc tccaaagcta agaatttaca gaatatctat gatgtcctca agtgggtctt 13860
tactattttt cctcaattct gtcttggtca aggactggta gaactctgct ataatcagat 13920
caaatatgac ctgacccaca acttcggcat tgattcctat gtgagtccct ttgagatgaa 13980
ctttctgggc tggatcttcg tgcaactggc ctcgcagggc acagtacttc tcctcttgag 14040
ggttctgcta cactgggacc ttctgcgatg gccaaggggt cattctactc tccaaggcac 14100
agtcaaatct tctaaggata cagatgttga aaaagaggaa aagagagtgt ttgaaggaag 14160
gaccaatgga gacattcttg tgttatacaa ccttagtaaa cattatcgac gctttttcca 14220
gaatattatt gctgtgcaag atattagttt gggcatacca aaaggagagt gctttggact 14280
tctaggggtg aatggagctg ggaagagcac gactttcaaa atgctgaatg gtgaagtttc 14340
tctaacttca ggacatgcta tcatcaggac tcccatggga gacgccgtgg acctgtcttc 14400
tgctggcacg gcaggcgtgc tcattggcta ctgtccccag caggatgccc tggacgagct 14460
tctgactggt tgggaacatc tctattatta ctgtagctta cgcgggattc caaggcagtg 14520
catccctgag gttgctggag acctcatcag gcgcttacac ctcgaagccc acgcggacaa 14580
acctgtggcc acctacagtg gggggaacca gcggaaactc tctacagccc tggccctggt 14640
ggggaaacct gacattcttt tattggatga gcccagctct gggatggatc cctgctctaa 14700
gcggtacctg tggcaaacaa taatgaagga ggttcgggaa ggctgtgctg cggtgctgac 14760
ctcccacagc atggaggagt gtgaggctct ttgcacaaga ctggccataa tggttaacgg 14820
cagcttcaaa tgtcttggtt ctcctcagca catcaaaaat aggtttggtg atggttatac 14880
agtcaaagtt tggctctgta aggaagcaaa tcaacattgc actgtttctg accacttgaa 14940
gctttatttt ccaggaattc agttcaaggg acagcacctg aatttattag aatatcatgt 15000

```
gccaaaaaga tggggatgcc tagctgactt gttcaaagtt atagagaaca ataaaacctt    15060
cttgaatatt aagcattatt ccattaacca aaccactttg gagcaggtat ttattaattt    15120
tgcttctgag cagcagcaaa ctctacaatc tactcttgat ccatccactg acagtcacca    15180
cacacatcac ttgcccatct gagcactaaa gaagtttcca taaggaataa aaccttgtct    15240
tccattacaa ttaacagtca aggataaaac aagcacgcgc acaatcaagg agctggaaca    15300
cactctccag gccgtcaaat tattctcttg ttcattttct attttgaatc tccttgttag    15360
ttaataacca ccaaatggaa aggtcattct ttctgcagac ttttggggag ctcctccaaa    15420
acatttgttc tctttaccat gccagatgga caccagcttc tttgtgacaa aggcatgaat    15480
gatttgacag tgtccaaact gagacattct ggagctggaa agcctgtcac actagagtgt    15540
gtgtgacatg tccactctaa acatgtcact tttctgttaa gaaaactgag ccccctcccc    15600
acaggttaaa aaactttagt aacttgtttg tatagaaaat agtaacaagg actattttct    15660
attgttgtca tctatttact agatacatgt ttttaatgat tttaatgtaa gcttttatta    15720
atactgatga cattatatgg tatgatatga aaaaatcacc aatttttttac atataaaaga    15780
taccttttta aaaaaatagg ttttaagagc tcttttagta tacactttag caaaattaat    15840
taaattgaac tagttactct gtatcaatta cagtagttct accagaatct cccaggttat    15900
aatttatgag ggtagagaaa taaaatgtag atgcattttc tttttcttca tttggatgaa    15960
taattactgt tttttgttat tctaagtcag tgtttttcaa agcgtagtgg tccccatatt    16020
agctgcattg ccatcttctg ggagcttgca agaaatgtac attctcagga tccactccag    16080
acctattgaa tctcaaattc tggggcttaa acaagcacat tccaagttaa gaaccaatga    16140
cctaagggaa tgtctggtta cctcctagtt atacaagcaa aatctgcata gtatgtagtc    16200
tttttttattt atttattttt tttttttgag gcggagtctc gctctgtcac ccaggctgga    16260
gtgcagtggc gcgatgtcgg ctcactgcaa gctccgcctc ctgggttcac gccattctcc    16320
tgcctcagcc tccccagcag ctgggactac aggcacacat cgccacaccc ggctaatttt    16380
tagtattttt agtagagacg gggtttcacc gtgttagcca ggatggtctc tatctcctga    16440
ccttgtgatc cgccccccctc cacctcccaa agtgctggga ttacaggcgt gagccaccgt    16500
gtccggccgt agtttatttt aaaatatatt tttaaaagct ttgtaaaaat tatgtcattc    16560
tcagaattgt tgtcttcaaa gcattgtcag atgtagagtg ctcagatgtg gctcttaaag    16620
actatataca tctgaatttt tcatcctata gttagtaaga tgcataaaat caatccacta    16680
ctgaaatagt ttccagtcag acatttctga gttcagacat ttctcaacat tcttttaaca    16740
acatttttct gaatcctcaa tagaaaatca cattaatctt attttaaaat ttggcctttt    16800
tcaacactaa cgttgagtac cggtagcttt gtgatcaaag gcatatactt ccttatgaga    16860
```

```
tttctttact aaagcaagat ttcattaaat ctctatttcc taaatatcat tctatacaaa    16920
agatattttt taaacggtaa ggattaagac aatcactgat agctttgttg tgagcaattt    16980
tgattcccat gtatcacatg aattacactt ccttaaataa attacagttt atggctgtat    17040
gatttattct ctaattctaa catagtctag ttgtcaaaag gaaatatgta atctttttat    17100
gattgttgaa tcaataaata ccaatttgtg aaacatgaat gtgtttaaac tgcagtgaat    17160
aaatgagatg tgctttaatt taacccaaaa aaaaaaaaaa aaaaaaaaa                 17209
```

<210> 53
<211> 931
<212> PRT
<213> Homo sapiens

<400> 53

```
Met Lys Ile Gln Lys Lys Leu Thr Gly Cys Ser Arg Leu Met Leu Leu
1               5                   10                  15

Cys Leu Ser Leu Glu Leu Leu Leu Glu Ala Gly Ala Gly Asn Ile His
            20                  25                  30

Tyr Ser Val Pro Glu Glu Thr Asp Lys Gly Ser Phe Val Gly Asn Ile
        35                  40                  45

Ala Lys Asp Leu Gly Leu Gln Pro Gln Glu Leu Ala Asp Gly Gly Val
    50                  55                  60

Arg Ile Val Ser Arg Gly Arg Met Pro Leu Phe Ala Leu Asn Pro Arg
65                  70                  75                  80

Ser Gly Ser Leu Ile Thr Ala Arg Arg Ile Asp Arg Glu Glu Leu Cys
                85                  90                  95

Ala Gln Ser Met Pro Cys Leu Val Ser Phe Asn Ile Leu Val Glu Asp
            100                 105                 110

Lys Met Lys Leu Phe Pro Val Glu Val Glu Ile Ile Asp Ile Asn Asp
        115                 120                 125

Asn Thr Pro Gln Phe Gln Leu Glu Glu Leu Glu Phe Lys Met Asn Glu
    130                 135                 140

Ile Thr Thr Pro Gly Thr Arg Val Ser Leu Pro Phe Gly Gln Asp Leu
145                 150                 155                 160

Asp Val Gly Met Asn Ser Leu Gln Ser Tyr Gln Leu Ser Ser Asn Pro
                165                 170                 175
```

```
His Phe Ser Leu Asp Val Gln Gln Gly Ala Asp Gly Pro Gln His Pro
            180                 185                 190

Glu Met Val Leu Gln Ser Pro Leu Asp Arg Glu Glu Glu Ala Val His
        195                 200                 205

His Leu Ile Leu Thr Ala Ser Asp Gly Gly Glu Pro Val Arg Ser Gly
    210                 215                 220

Thr Leu Arg Ile Tyr Ile Gln Val Val Asp Ala Asn Asp Asn Pro Pro
225                 230                 235                 240

Ala Phe Thr Gln Ala Gln Tyr His Ile Asn Val Pro Glu Asn Val Pro
                245                 250                 255

Leu Gly Thr Gln Leu Leu Met Val Asn Ala Thr Asp Pro Asp Glu Gly
            260                 265                 270

Ala Asn Gly Glu Val Thr Tyr Ser Phe His Asn Val Asp His Arg Val
        275                 280                 285

Ala Gln Ile Phe Arg Leu Asp Ser Tyr Thr Gly Glu Ile Ser Asn Lys
    290                 295                 300

Glu Pro Leu Asp Phe Glu Glu Tyr Lys Met Tyr Ser Met Glu Val Gln
305                 310                 315                 320

Ala Gln Asp Gly Ala Gly Leu Met Ala Lys Val Lys Val Leu Ile Lys
                325                 330                 335

Val Leu Asp Val Asn Asp Asn Ala Pro Glu Val Thr Ile Thr Ser Val
            340                 345                 350

Thr Thr Ala Val Pro Glu Asn Phe Pro Pro Gly Thr Ile Ile Ala Leu
        355                 360                 365

Ile Ser Val His Asp Gln Asp Ser Gly Asp Asn Gly Tyr Thr Thr Cys
    370                 375                 380

Phe Ile Pro Gly Asn Leu Pro Phe Lys Leu Glu Lys Leu Val Asp Asn
385                 390                 395                 400

Tyr Tyr Arg Leu Val Thr Glu Arg Thr Leu Asp Arg Glu Leu Ile Ser
                405                 410                 415

Gly Tyr Asn Ile Thr Ile Thr Ala Ile Asp Gln Gly Thr Pro Ala Leu
            420                 425                 430
```

Ser Thr Glu Thr His Ile Ser Leu Leu Val Thr Asp Ile Asn Asp Asn
435 440 445

Ser Pro Val Phe His Gln Asp Ser Tyr Ser Ala Tyr Ile Pro Glu Asn
450 455 460

Asn Pro Arg Gly Ala Ser Ile Phe Ser Val Arg Ala His Asp Leu Asp
465 470 475 480

Ser Asn Glu Asn Ala Gln Ile Thr Tyr Ser Leu Ile Glu Asp Thr Ile
485 490 495

Gln Gly Ala Pro Leu Ser Ala Tyr Leu Ser Ile Asn Ser Asp Thr Gly
500 505 510

Val Leu Tyr Ala Leu Arg Ser Phe Asp Tyr Glu Gln Phe Arg Asp Met
515 520 525

Gln Leu Lys Val Met Ala Arg Asp Ser Gly Asp Pro Pro Leu Ser Ser
530 535 540

Asn Val Ser Leu Ser Leu Phe Leu Leu Asp Gln Asn Asp Asn Ala Pro
545 550 555 560

Glu Ile Leu Tyr Pro Ala Leu Pro Thr Asp Gly Ser Thr Gly Val Glu
565 570 575

Leu Ala Pro Leu Ser Ala Glu Pro Gly Tyr Leu Val Thr Lys Val Val
580 585 590

Ala Val Asp Arg Asp Ser Gly Gln Asn Ala Trp Leu Ser Tyr Arg Leu
595 600 605

Leu Lys Ala Ser Glu Pro Gly Leu Phe Ser Val Gly Leu His Thr Gly
610 615 620

Glu Val Arg Thr Ala Arg Ala Leu Leu Asp Arg Asp Ala Leu Lys Gln
625 630 635 640

Ser Leu Val Val Ala Val Gln Asp His Gly Gln Pro Pro Leu Ser Ala
645 650 655

Thr Val Thr Leu Thr Val Ala Val Ala Asp Arg Ile Ser Asp Ile Leu
660 665 670

Ala Asp Leu Gly Ser Leu Glu Pro Ser Ala Lys Pro Asn Asp Ser Asp
675 680 685

Leu Thr Leu Tyr Leu Val Val Ala Ala Ala Ala Val Ser Cys Val Phe
    690                 695                 700

Leu Ala Phe Val Ile Val Leu Leu Ala His Arg Leu Arg Arg Trp His
705                 710                 715                 720

Lys Ser Arg Leu Leu Gln Ala Ser Gly Gly Gly Leu Ala Ser Met Pro
                725                 730                 735

Gly Ser His Phe Val Gly Val Asp Gly Val Arg Ala Phe Leu Gln Thr
            740                 745                 750

Tyr Ser His Glu Val Ser Leu Thr Ala Asp Ser Arg Lys Ser His Leu
        755                 760                 765

Ile Phe Pro Gln Pro Asn Tyr Ala Asp Thr Leu Ile Ser Gln Glu Ser
    770                 775                 780

Cys Glu Lys Lys Gly Phe Leu Ser Ala Pro Gln Ser Leu Leu Glu Asp
785                 790                 795                 800

Lys Lys Glu Pro Phe Ser Gln Gln Ala Pro Pro Asn Thr Asp Trp Arg
                805                 810                 815

Phe Ser Gln Ala Gln Arg Pro Gly Thr Ser Gly Ser Gln Asn Gly Asp
            820                 825                 830

Asp Thr Gly Thr Trp Pro Asn Asn Gln Phe Asp Thr Glu Met Leu Gln
        835                 840                 845

Ala Met Ile Leu Ala Ser Ala Ser Glu Ala Ala Asp Gly Ser Ser Thr
    850                 855                 860

Leu Gly Gly Gly Ala Gly Thr Met Gly Leu Ser Ala Arg Tyr Gly Pro
865                 870                 875                 880

Gln Phe Thr Leu Gln His Val Pro Asp Tyr Arg Gln Asn Val Tyr Ile
                885                 890                 895

Pro Gly Ser Asn Ala Thr Leu Thr Asn Ala Ala Gly Lys Arg Asp Gly
            900                 905                 910

Lys Ala Pro Ala Gly Gly Asn Gly Asn Lys Lys Lys Ser Gly Lys Lys
        915                 920                 925

Glu Lys Lys

930

<210> 54
<211> 4602
<212> DNA
<213> Homo sapiens

<400> 54

```
atgaagattc agaaaaagct gactggctgc agcaggctga tgcttctgtg tctttctctg      60
gagctgctgt tggaagctgg ggctgggaat attcactact cagtgccgga agagacagac     120
aaaggttcct tcgtaggcaa catcgccaag gacctagggc tgcaacccca ggagctggca     180
gatggcggag tccgcatcgt ctccagaggt aggatgccgc ttttcgctct gaatcctaga     240
agtggcagct tgatcaccgc gcgcaggata gaccgggagg agctctgcgc tcagagcatg     300
ccgtgtctcg tgagttttaa tatccttgtt gaggataaaa tgaagctttt tcctgttgaa     360
gtagaaataa ttgatattaa tgacaacact ccccaattcc agttagagga actggagttt     420
aaaatgaatg aaataacgac tccaggtacc agagtctcat tgccttttgg gcaagacctt     480
gatgtgggta tgaactcact ccagagctac caactcagct ctaaccctca tttctccctg     540
gatgtgcaac agggagccga tgggcctcaa catccagaga tggtgctgca gagtccctta     600
gacagagaag aagaagctgt ccaccacctc atcctcacag cttctgatgg gggtgaacca     660
gtccgttcag ggaccctcag aatttacatt caggtggtgg atgcaaatga caatcctcca     720
gcatttactc aggcacaata ccatataaat gtccccgaaa acgtgccgct gggtactcag     780
ctgctcatgg taaatgccac tgaccctgat gagggagcca atggggaagt aacgtactcc     840
tttcacaatg tagaccacag agtggcccaa atatttcgtt tagattctta cacaggagaa     900
atatcaaata aagaaccact agatttcgaa gaatacaaaa tgtattcaat ggaagttcaa     960
gcccaggatg gtgcggggct catggctaaa gttaaggtac tgatcaaagt tttggatgta    1020
aatgataatg ccccagaagt gaccatcacc tctgtcacca ctgcagttcc agaaaacttt    1080
cctcctggga ccataattgc tcttatcagt gtgcatgacc aggactcagg agacaatggc    1140
tacaccacat gtttcattcc tggaaattta ccctttaaat tggaaaagtt agttgataat    1200
tattaccgtt tagtgactga aagaacactg gacagagaac ttatctctgg gtacaacatc    1260
acaataacag caatagacca aggaactcca gctctatcta ctgaaactca catttcacta    1320
ctagtgacag atatcaatga caactcccca gtcttccatc aggactccta ctctgcctac    1380
attcccgaaa acaaccccag aggagcctcc atcttctctg tgagggccca cgacttggac    1440
agcaatgaga atgcacaaat cacttactcc ctaatagagg acactatcca gggggcaccc    1500
ctatctgcct acctctccat caactccgac actggggtcc tgtatgcgct gcgatccttc    1560
gactatgagc agttccggga catgcaactg aaagtgatgg cgcgggacag tggggatccg    1620
```

cccctcagca gcaacgtgtc tctcagccta ttcctgctgg accagaacga caacgcgccc 1680
gagatcctgt accccgccct ccccacagat ggttctaccg gcgtggagct ggcgcccctc 1740
tccgcagagc ccggctacct ggtgaccaag gtggtggcgg tggacagaga ctcgggccag 1800
aacgcctggc tgtcctaccg cctgctcaag gccagcgagc cgggactctt ctcggtgggt 1860
ctgcacacgg gcgaggtgcg cacggcgcga gccctgctgg acagagacgc gctcaagcag 1920
agtctcgtgg tggccgtcca ggaccacggc cagcccccgc tctccgccac tgtcacgctc 1980
accgtggccg tggccgacag gatctccgac atcctggccg acctgggcag cctcgagccc 2040
tccgccaaac ccaacgattc ggacctcact ctgtacctgg tggtggcggc ggccgcggtc 2100
tcctgcgtct tcctggcctt cgtcatcgtg ctgctggcgc acaggctgcg gcgctggcac 2160
aagtcacgtc tgctacaggc ttcgggaggc ggcttagcga gcatgcccgg ttcgcacttt 2220
gtgggcgtgg acggggttcg ggctttcctg cagacctatt cccacgaggt ctccctcact 2280
gcggactcgc ggaagagcca cctgattttc cccagcccca actatgcgga cacactcatc 2340
agccaggaga gctgtgagaa aaagggtttt ctatcagcac cccagtcttt acttgaagac 2400
aaaaaggaac cattttctca gcaagccccg cccaacacgg actggcgttt ctctcaggcc 2460
cagagacccg gcaccagcgg ctcccaaaat ggcgatgaca ccggcacctg gcccaacaac 2520
cagtttgaca cagagatgct gcaagccatg atcttggcgt ccgccagtga agctgctgat 2580
gggagctcca ccctgggagg gggtgccggc accatgggat tgagcgcccg ctacggaccc 2640
cagttcaccc tgcagcacgt gcccgactac cgccagaatg tctacatccc aggcagcaat 2700
gccacactga ccaacgcagc tggcaagcgg gatggcaagg ccccagcagg tggcaatggc 2760
aacaagaaga agtcgggcaa gaaggagaag aagtaacatg gaggccaggc caagagccac 2820
agggcggcct ctccccaacc agcccagctt ctccttacct gcacccaggc ctcagagttt 2880
cagggctaac cccagaata ctggtagggg ccaaggccat gctccccttg ggaaacagaa 2940
acaagtgccc agtcagcacc taccccttcc cccccagggg gttgaatatg caaaagcagt 3000
tccgctggga accccatcc aatcaactgc tgtacccatg gggtagtgg ggttactgta 3060
gacaccaaga accatttgcc acaccccgtt tagttacagc tgaactcctc catcttccaa 3120
atcaatcagg cccatccatc ccatgcctcc ctcctcccca ccccactcca acagttcctc 3180
tttcccgagt aaggtggttg ggtgttgaa gtaccaagta acctacaagc ctcctagttc 3240
tgaaaagttg gaagggcatc atgacctctt ggcctctcct ttgattctca atcttccccc 3300
aaagcatggt ttggtgccag ccccttcacc tccttccaga gcccaagatc aatgctcaag 3360
ttttggagga catgatcacc atccccatgg tactgatgct tgctggattt agggagggca 3420
ttttgctacc aagcctcttc ccaacgccct ggggaccagt cttctgtttt gtttttcatt 3480
gtttgacgtt tccactgcat gccttgactt cccccacctc ctcctcaaac aagagactcc 3540

actgcatgtt ccaagacagt atggggtggt aagataagga agggaagtgt gtggatgtgg 3600

atggtggggg catggacaaa gcttgacaca tcaagttatc aaggccttgg aggaggctct 3660

gtatgtcctc aggggactga caacatcctc cagattccag ccataaacca ataactaggc 3720

tggacccttc ccactacata atagggctca gcccaggcag ccagctttgg gctgagctaa 3780

caggaccaat ggattaaact ggcatttcag tccaaggaag ctcgaagcag gtttaggacc 3840

aggtcccctt gagaggtcag aggggcctct gtgggtgctg ggtactccag aggtgccact 3900

ggtggaaggg tcagcggagc cccagcagga agggtgggcc agccaggcca ttcttagtcc 3960

ctgggttggg gaggcaggga gctagggcag ggaccaaatg aacagaaagt ctcagcccag 4020

gatggggctt cttcaacagg gcccctgccc tcctgaagcc tcagtccttc accttgccag 4080

gtgccgtttc tcttccgtga aggccactgc ccaggtcccc agtgcgcccc ctagtggcca 4140

tagcctggtt aaagttcccc agtgcctcct tgtgcataga ccttcttctc ccacccctt 4200

ctgcccctgg gtccccggcc atccagcggg gctgccagag aaccccagac ctgcccttac 4260

agtagtgtag cgccccctcc ctctttcggc tggtgtagaa tagccagtag tgtagtgcgg 4320

tgtgctttta cgtgatggcg ggtgggcagc gggcggcggg ctccgcgcag ccgtctgtcc 4380

ttgatctgcc cgcggcggcc cgtgttgtgt tttgtgctgt gtccacgcgc taaggcgacc 4440

ccctcccccg tactgacttc tcctataagc gcttctcttc gcatagtcac gtagctccca 4500

ccccaccctc ttcctgtgtc tcacgcaagt tttatactct aatatttata tggctttttt 4560

tcttcgacaa aaaaataata aaacgtttct tctgaaaagc tg 4602

<210> 55
<211> 3114
<212> DNA
<213> Homo sapiens

<400> 55

aatcccagct cgcagccctt gctccgcgtg tactcacggg aggactcgca gacgttactg 60

ccctcttgcg tgccccggcc acccccgggc ggcttgtagc cggtgcgcgg ggtggctggg 120

gctacgtgca gagctgtcgc ggagccggaa cagcagcggt gaagcccctc ggctcggccg 180

agaccgccgt gcccactgct cgcctcggtt gccgccgctt tagccgcagc cgctgctgcc 240

gccgccgggg gagaggcagc ctattgtctt tctccgcggc gaaggtgagg agctgtctcg 300

gctcggcccg cgggggagcc ccgggagccg cacggagatg gaggaggaca tctggacagt 360

gagcaggagg cgcttcggcc catgccgaac atcccagggg acctggagag ccgggaggcc 420

atggtggcat ttttcaactc cgctggagcc aatgcccagg aggaacaaag ggtgtgctgc 480

cagcccctgg ctcacccagt ggcctcgtcc cagaaaaagc cagaggtagc ggccccagcc 540

ccagagagtg ggggtgagtc tgtgtttggg gagacccacc gggccctgca gggggccatg 600

```
gagaagctgc agcgacttta tggaaggaga aggtggacct gaaggagcgg gtagagaaac      660
tagagcttca attcatccac ctctcaggac agacagacac catagggaga aagtacatca      720
gccaggggc agtgtcagag acgcagcact gggagaggag gacatcgtca ggctggccca       780
ggaccaggag gagatgaagg tgaacctgca ggagctgcgg ggcaggtgtt gcagcttgtg      840
ggagaccaca aggaggggca tggcaaattc tgaccattgc ccagaaccct gctgatgagc      900
ccactctagg agccccaata gcccaggagc ttgggtgtgc tgacgagcag ggtgatcacc      960
actggattgc tgacagatag aggacgtggg accgtgacta tcacccctaa tctgcagtgg     1020
atttggctct cggcactccc aggctgggag ctggatacct gccctggcag catgactcag     1080
actgcatgac agtcacagac tcgcctctgc tcctgtggtc cagtggccgg acaccccctg     1140
ggatggctca aaggagtcag gacttggaag tggggacatc agggtagctg aaggaaatcc     1200
acacacccag agcatctcgg agttcagact ctcagacctg aagtaggcgc cccgggact      1260
gggctaggag ttggacggaa tggaggatgg aggacagcga gaagaaagga agagaaatgc     1320
aaagtgtggg cagccgccaa gagtgaaaat agagggaagt gtcatgcaag tgctggacag     1380
aaggcggcag gtgggacgag ccccacagcc ccctcctcaa aaacgaccac ctccaggact     1440
cagtgatccc tgggggcag gctctgccag ccctcggcca cacgtggctc cggcacccat      1500
ggtcccagtg ccttggatgg agacggccag ttctggcggc cagatgtggt gctctggaat     1560
ccagtcccat ttccttcctg gccacgcctg tccagcggcc tcttcagccg cattcagccc     1620
ctacttacct ggggaccccg gctggggcac gagagcacca gggggtagg gcccaaaggg      1680
atcaggggaa gcctctggcc tggagggtat ggggcacgct tccccaaggg cggacccggc     1740
aggaggaagc ccaggagctg ggtcctgccg cccaggagct gggccctgcc acccaggccg     1800
ggctagggac atggcagggc ctgggcatcc tgacgctgga cttgggcgac ctgggaggca     1860
cagggagggg agagatgggc gaccccgccc cagcgcagtg ccggccacac cccaaggcag     1920
ttgccagagc ttaagccccg cccccagcag cgagaacatc ccagctccac accccccccc     1980
cccccgcagc cagtgctcct tgtcaagctc cccccgtcac tccaggtggg agccaccccg     2040
gtgaggggggt gtgccacttg cccccagggc actcctctgg gcatcccggg tgggggattt     2100
tggggccgtg gggggcagtc tttggtacct gtgttcgtca gggatgctct gacaaccagg     2160
tgtcgtccac gggcgggggc atgggcatgg tgacagtggt cctgttgatg tcaccgatga     2220
tgctgagcgc ctccttcagc gcgtggtgca tgtgcagcat ctcgtcgtgc tgctgtgcct     2280
gctctgccaa ctcctccatc agtgtgttct ggttcccaca tgagtacata ttggccagca     2340
gctccgagat gatgaactcc ggggtctgag agtgggcaaa cagggaagaa ggttgggacc     2400
tggtgcctgt gccgccctgg ctgccttgct gggcccttct gggactgtgc gctggacttg     2460
```

gagccccttg gagtatggct tttcacacgg gcttctatac cgcttcgact ggaagatcca 2520

cctccccact gccttttctc actcagatgg ggacaccgag gtccagagga aaagacacct 2580

gtcaaatgtc acagatctgg gaggggactt aagacttatc atgccaagag gacacctgtc 2640

tactcagttt ttttttggtg gggcgggggg cggtgatagg gtctcgctct gtcaccaggc 2700

tggagtacag tgatgactgc tcactgcagc ctccacctcc tgggctcaaa gtgatcctcc 2760

aacgtcagcc tctcgaggag ctaggactac aggcacatgc caccaccaag cccagctatt 2820

tttaaaattt ttgtgtggag acaaggtctc actatgtggc ccaggctggt ctcgaactcc 2880

tgggctcaag tgatcctcct gcctcggcct ccaggagtgg gagttggagt tgatgcctgg 2940

atacaggagc tctgtgggtg ggagtgagac aaaacacagg gtcctgagct ctggggacca 3000

agcaatgtcc tctggtgaaa aaaatcctgg acttgctggc agaagatttg cctcttactc 3060

gccatgtgct ctgaatacat ttacctgccc tctgggaaaa aaaaaaaaaa aaaa 3114

<210> 56
<211> 3357
<212> DNA
<213> Homo sapiens

<400> 56

```
aatcccagct cgcagccctt gctccgcgtg tactcacggg aggactcgca gacgttactg      60
ccctcttgcg tgccccggcc acccccgggc ggcttgtagc cggtgcgcgg ggtggctggg     120
gctacgtgca gagctgtcgc ggagccggaa cagcagcggt gaagcccctc ggctcggccg     180
agaccgccgt gcccactgct cgcctcggtt gccgccgctt tagccgcagc cgctgctgcc     240
gccgccgggg gagaggcagc ctattgtctt tctccgcggc gaaggtgagg agctgtctcg     300
gctcggcccg cgggggagcc ccgggagccg cacggtggca tttttcaact ccgctggagc     360
caatgcccag gaggaacaaa gggtgtgctg ccagcccctg gctcacccag tggcctcgtc     420
ccagaaaaag ccagaggtag cggccccagc cccagagagt gggggtgagt ctgtgtttgg     480
ggagacccac cgggccctgc agggggccat ggagaagctg cagcgacttt atggaaggag     540
aaggtggacc tgaaggagcg ggtagagaaa ctagagcttc aattcatcca cctctcagga     600
cagacagaca ccatagggag aaagtacatc agccaggggg cagtgtcaga gacgcagcac     660
tgggagagga ggacatcgtc aggctggccc aggaccagga ggagatgaag gtgaacctgc     720
aggagctgcg gggcaggtgt tgcagcttgt gggagaccac aaggaggggc atggcaaatt     780
ctgaccattg cccagaaccc tgctgatgag cccactctag gagccccaat agcccaggag     840
cttgggtgtg ctgacgagca gggtgatcac cactggattg ctgacagata gaggacgtgg     900
gaccgtgact atcaccccta atctgcagtg gatttggctc tcggcactcc caggctggga     960
gctggatacc tgccctggca gcatgactca gactgcatga cagagaacgt ggccagtgga    1020
```

gacggcacac tggaaatcag agtgaatgtt cttgaaagag ggtcacgggt caacaaggcc 1080
cagccaaagg atgcagtaga accattttcc ttagaaatct ttgggagtga agtaggcttc 1140
agccactccc atccctgccc tcgcggctac cactacccca ttagtttaga cagggtcggg 1200
cggggagggg tgtggagaag aaatgagctt gcctgtggcc cccaggctcc ctctgtccta 1260
gctcaggtct gggtgccatt ctttacactc gtgtgctcgc tcacgcacac atcacacacc 1320
ttgctggtca cacagtcaca gactcgcctc tgctcctgtg gtccagtggc cggacacccc 1380
ctgggatggc tcaaaggagt caggacttgg aagtggggac atcagggtag ctgaaggaaa 1440
tccacacacc cagagcatct cggagttcag actctcagac ctgaagtagg cgcccccggg 1500
actgggctag gagttggacg gaatggagga tggaggacag cgagaagaaa ggaagagaaa 1560
tgcaaagtgt gggcagccgc caagagtgaa aatagaggga agtgtcatgc aagtgctgga 1620
cagaaggcgg caggtgggac gagccccaca gccccctcct caaaaacgac cacctccagg 1680
actcagtgat ccctgggggg caggctctgc cagccctcgg ccacacgtgg ctccggcacc 1740
catggtccca gtgccttgga tggagacggc cagttctggc ggccagatgt ggtgctctgg 1800
aatccagtcc catttccttc ctggccacgc ctgtccagcg gcctcttcag ccgcattcag 1860
cccctactta cctggggacc ccggctgggg cacgagagca ccagggggt agggcccaaa 1920
gggatcaggg gaagcctctg gcctggaggg tatggggcac gcttccccaa gggcggaccc 1980
ggcaggagga agcccaggag ctgggtcctg ccgcccagga gctgggccct gccacccagg 2040
ccgggctagg gacatggcag ggcctgggca tcctgacgct ggacttgggc gacctgggag 2100
gcacagggag gggagagatg ggcgaccccg ccccagcgca gtgccggcca caccccaagg 2160
cagttgccag agcttaagcc ccgcccccag cagcgagaac atcccagctc cacacccccc 2220
ccccccccgc agccagtgct ccttgtcaag ctcccccgt cactccaggt gggagccacc 2280
ccggtgaggg ggtgtgccac ttgcccccag ggcactcctc tgggcatccc gggtggggga 2340
ttttggggcc gtgggggca gtctttggta cctgtgttcg tcagggatgc tctgacaacc 2400
aggtgtcgtc cacgggcggg ggcatgggca tggtgacagt ggtcctgttg atgtcaccga 2460
tgatgctgag cgcctccttc agcgcgtggt gcatgtgcag catctcgtcg tgctgctgtg 2520
cctgctctgc caactcctcc atcagtgtgt tctggttccc acatgagtac atattggcca 2580
gcagctccga gatgatgaac tccggggtct gagagtgggc aaacagggaa gaaggttggg 2640
acctggtgcc tgtgccgccc tggctgcctt gctgggccct tctgggactg tgcgctggac 2700
ttggagcccc ttggagtatg gcttttcaca cgggcttcta taccgcttcg actggaagat 2760
ccacctcccc actgcctttt ctcactcaga tggggacacc gaggtccaga ggaaaagaca 2820
cctgtcaaat gtcacagatc tgggagggga cttaagactt atcatgccaa gaggacacct 2880
gtctactcag ttttttttg gtggggcggg gggcggtgat agggtctcgc tctgtcacca 2940

```
ggctggagta cagtgatgac tgctcactgc agcctccacc tcctgggctc aaagtgatcc      3000
tccaacgtca gcctctcgag gagctaggac tacaggcaca tgccaccacc aagcccagct      3060
atttttaaaa tttttgtgtg gagacaaggt ctcactatgt ggcccaggct ggtctcgaac      3120
tcctgggctc aagtgatcct cctgcctcgg cctccaggag tgggagttgg agttgatgcc      3180
tggatacagg agctctgtgg gtgggagtga gacaaaacac agggtcctga gctctgggga      3240
ccaagcaatg tcctctggtg aaaaaaatcc tggacttgct ggcagaagat ttgcctctta      3300
ctcgccatgt gctctgaata catttacctg ccctctggga aaaaaaaaaa aaaaaaa         3357
```

<210> 57
<211> 2975
<212> DNA
<213> Homo sapiens

<400> 57

```
aatcccagct cgcagccctt gctccgcgtg tactcacggg aggactcgca gacgttactg      60
ccctcttgcg tgccccggcc acccccgggc ggcttgtagc cggtgcgcgg ggtggctggg     120
gctacgtgca gagctgtcgc ggagccggaa cagcagcggt gaagcccctc ggctcggccg     180
agaccgccgt gcccactgct cgcctcggtt gccgccgctt tagccgcagc cgctgctgcc     240
gccgccgggg gagaggcagc ctattgtctt tctccgcggc gaaggtggca tttttcaact     300
ccgctggagc caatgcccag gaggaacaaa gggtgtgctg ccagcccctg gctcacccag     360
tggcctcgtc ccagaaaaag ccagaggtag cggccccagc cccagagagt gggggtgagt     420
ctgtgtttgg ggagacccac cgggccctgc aggggggcat ggagaagctg cagcgacttt     480
atggaaggag aaggtggacc tgaaggagcg ggtagagaaa ctagagcttc aattcatcca     540
cctctcagga cagacagaca ccatagggag aaagtacatc agccaggggg cagtgtcaga     600
gacgcagcac tgggagagga ggacatcgtc aggctggccc aggaccagga ggagatgaag     660
gtgaacctgc aggagctgcg gggcaggtgt tgcagcttgt gggagaccac aaggaggggc     720
atggcaaatt ctgaccattg cccagaaccc tgctgatgag cccactctag gagccccaat     780
agcccaggag cttgggtgtg ctgacgagca gggtgatcac cactggattg ctgacagata     840
gaggacgtgg gaccgtgact atcaccccta atctgcagtg gatttggctc tcggcactcc     900
caggctggga gctggatacc tgccctggca gcatgactca gactgcatga cagtcacaga     960
ctcgcctctg ctcctgtggt ccagtggccg gacacccccct gggatggctc aaaggagtca    1020
ggacttggaa gtggggacat cagggtagct gaaggaaatc cacacaccca gagcatctcg    1080
gagttcagac tctcagacct gaagtaggcg cccccgggac tgggctagga gttggacgga    1140
atggaggatg gaggacagcg agaagaaagg aagagaaatg caaagtgtgg gcagccgcca    1200
agagtgaaaa tagagggaag tgtcatgcaa gtgctggaca gaaggcggca ggtgggacga    1260
```

gccccacagc cccctcctca aaaacgacca cctccaggac tcagtgatcc ctgggggca 1320
ggctctgcca gccctcggcc acacgtggct ccggcaccca tggtcccagt gccttggatg 1380
gagacggcca gttctggcgg ccagatgtgg tgctctggaa tccagtccca tttccttcct 1440
ggccacgcct gtccagcggc ctcttcagcc gcattcagcc cctacttacc tggggacccc 1500
ggctggggca cgagagcacc agggggtag ggcccaaagg gatcagggga agcctctggc 1560
ctggagggta tggggcacgc ttccccaagg gcggacccgg caggaggaag cccaggagct 1620
gggtcctgcc gcccaggagc tgggccctgc cacccaggcc gggctaggga catggcaggg 1680
cctgggcatc ctgacgctgg acttgggcga cctgggaggc acagggaggg gagagatggg 1740
cgaccccgcc ccagcgcagt gccggccaca ccccaaggca gttgccagag cttaagcccc 1800
gcccccagca gcgagaacat cccagctcca cacccccccc ccccccgcag ccagtgctcc 1860
ttgtcaagct ccccccgtca ctccaggtgg gagccacccc ggtgaggggg tgtgccactt 1920
gcccccaggg cactcctctg ggcatcccgg gtgggggatt ttggggccgt gggggcagt 1980
ctttggtacc tgtgttcgtc agggatgctc tgacaaccag gtgtcgtcca cgggcggggg 2040
catgggcatg gtgacagtgg tcctgttgat gtcaccgatg atgctgagcg cctccttcag 2100
cgcgtggtgc atgtgcagca tctcgtcgtg ctgctgtgcc tgctctgcca actcctccat 2160
cagtgtgttc tggttcccac atgagtacat attggccagc agctccgaga tgatgaactc 2220
cggggtctga gagtgggcaa acagggaaga aggttgggac ctggtgcctg tgccgccctg 2280
gctgccttgc tgggcccttc tgggactgtg cgctggactt ggagcccctt ggagtatggc 2340
ttttcacacg ggcttctata ccgcttcgac tggaagatcc acctccccac tgccttttct 2400
cactcagatg gggacaccga ggtccagagg aaaagacacc tgtcaaatgt cacagatctg 2460
ggaggggact taagacttat catgccaaga ggacacctgt ctactcagtt ttttttggt 2520
ggggcggggg gcggtgatag ggtctcgctc tgtcaccagg ctggagtaca gtgatgactg 2580
ctcactgcag cctccacctc ctgggctcaa agtgatcctc caacgtcagc ctctcgagga 2640
gctaggacta caggcacatg ccaccaccaa gcccagctat ttttaaaatt tttgtgtgga 2700
gacaaggtct cactatgtgg cccaggctgg tctcgaactc ctgggctcaa gtgatcctcc 2760
tgcctcggcc tccaggagtg ggagttggag ttgatgcctg gatacaggag ctctgtgggt 2820
gggagtgaga caaaacacag ggtcctgagc tctggggacc aagcaatgtc ctctggtgaa 2880
aaaaatcctg gacttgctgg cagaagattt gcctcttact cgccatgtgc tctgaataca 2940
tttacctgcc ctctgggaaa aaaaaaaaa aaaaa 2975